# FASCICULE DE BREVET EUROPEEN

(11) **EP 2 170 889 B1**
(45) Date de publication et mention de la délivrance du brevet: **17.08.2016**
(21) Numéro de dépôt: 08826677.0
(22) Date de dépôt: 26.06.2008
(51) Int. Cl.: C07D 487/04, C07D 519/00, A61K 31/5025, A61K 31/551, A61K 31/5386

(54) **DERIVES DE 6-CYCLOAMINO-3-(PYRIDIN-4-YL)IMIDAZO[1,2-B]PYRIDAZINE, LEUR PREPARATION ET LEUR APPLICATION EN THERAPEUTIQUE**
6-CYCLOAMINO-3-(PYRIDIN-4-YL)-IMIDAZO[1,2-B]PYRIDAZINDERIVATE, IHRE HERSTELLUNG UND IHRE THERAPEUTISCHE VERWENDUNG
6-CYCLOAMINO-3-(PYRIDIN-4-YL)IMIDAZO[1,2-B]PYRIDAZINE DERIVATIVES, PREPARATION THEREOF AND THERAPEUTIC USE THEREOF

(30) Priorité: 28.06.2007 FR 0704661; 28.06.2007 US 946785 P
(43) Date de publication de la demande: 07.04.2010
(73) Titulaire: SANOFI, 75008 Paris (FR)
(72) Inventeur: ALMARIO GARCIA, Antonio, F-75013 Paris (FR); BARRAGUE, Matthieu, Bridgewater, NJ 08807 (US); BURNIER, Philippe, F-75013 Paris (FR); ENGUEHARD, Cécile, F-37200 Tours (FR); GAO, Zhongli, Bridgewater, New Jersey 08807 (US); GEORGE, Pascal, F-75013 Paris (FR); GUEIFFIER, Alain, F-37200 Tours (FR); LI, Adrien, Tak, F-75013 Paris (FR); PUECH, Frédéric, F-75013 Paris (FR); VAZ, Roy, Bridgewater, New Jersey 08807 (US); ZHAO, Qiuxia, Bridgewater, New Jersey 08807 (US)
(74) Mandataire: Nony
(86) Numéro de dépôt international: PCT/FR2008/000902
(87) Numéro de publication internationale: WO 2009/016286

(56) Documents cités:
- EP-A- 1 790 650
- WO-A-02/066481
- WO-A-2004/016614
- WO-A-2006/070943
- WO-A-2007/013673
- MAVEL, SYLVIE ET AL: "Synthesis of imidazo[2,1-a]phthalazines, potential inhibitors of p38 MAP kinase. Prediction of binding affinities of protein ligands" ARCHIV DER PHARMAZIE, vol. 335, no. 1, 2002, pages 7-14, XP002474567

## Description

La présente invention se rapporte à des dérivés de 6-cycloamino-3-(pyridin-4-yl)imidazo[1,2-*b*]pyridazine, à leur préparation et à leur application en thérapeutique, dans le traitement ou la prévention de maladies impliquant la caséine kinase 1 epsilon et/ou la caséine kinase 1 delta.

La présente invention a pour objet les composés répondant à la formule générale (I)

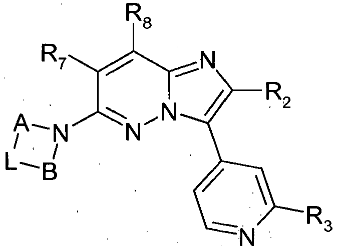

Dans laquelle
- R₂ représente un groupe phényle éventuellement substitué par un ou plusieurs substituants choisis parmi les atomes d'halogène et les groupes C₁₋₆ alkyle, C₁₋₆ alkyloxy, C₁₋₆fluoroalkyle;
- R₃ représente un atome d'hydrogène ou un groupe C₁₋₃ alkyle, -NR₄R₅, hydroxyle ou C₁₋₄ alkyloxy ;
- A représente un groupe C₁₋₇-alkylène éventuellement substitué par un ou deux groupes Rₐ ;
- B représente un groupe C₁₋₇-alkylène éventuellement substitué par un groupe R_{b};
- L représente soit un atome d'azote éventuellement substitué par un groupe R_{c} ou R_{d}, soit un atome de carbone substitué par un groupe Rₑ₁ et un groupé R_{d} ou deux groupes Rₑ₂ ; les atomes de carbone de A et de B étant éventuellement substitués par un ou plusieurs groupes R_{f} identiques ou différents l'un de l'autre ;
   Lorsqu'ils sont présents, Rₐ, R_{b} et R_{c} sont définis en combinaison comme suit :
   soit deux groupes Rₐ forment ensemble un groupe C₁₋₆-alkylène;
   soit Rₐ et R_{b} forment ensemble une liaison ou un groupe C₁₋₆-alkylène;
   soit Rₐ et R_{c} forment ensemble une liaison ou un groupe C₁₋₆-alkylène ;
   soit R_{b} et R_{c} forment ensemble une liaison ou un groupe C₁₋₆-alkylène ;
   R_{d} représente un groupe choisi parmi l'atome d'hydrogène et les groupes C₁₋₆-alkyle; C₃₋₇-cycloalkyle, C₃₋₇-cycloalkyl-C₁₋₆-alkyle, C₁₋₆-alkylthio-C₁₋₆-alkyle, C₁₋₆-alkyloxy-C₁₋₆-alkyle, C₁₋₆-fluoroalkyle, benzyle, C₁₋₆-acyle, hydroxy-C₁₋₆-alkyle ;
   Rₑ₁ représente un groupe -NR₄R₅ ou une monoamine cyclique comportant éventuellement un atome d'oxygène, la monoamine cyclique étant éventuellement substituée par un ou plusieurs substituants choisis parmi l'atome de fluor et les groupes C₁₋₆-alkyle, C₁₋₆-alkyloxy, hydroxyle ;
   Les deux Rₑ₂ forment, avec l'atome de carbone qui les porte, un groupe pyrrolidine, pipéridine, morpholine éventuellement substitué par un ou plusieurs groupes R_{f} identiques ou différents l'un de l'autre ;
   R_{f} représente un groupe C₁₋₆-alkyle, C₃₋₇-cycloalkyle, C₃₋₇cycloalkyle-C₁₋₆-alkyle, C₁₋₆-alkyloxy-C₁₋₆-alkyle, hydroxy-C₁₋₆-alkyle, C₁₋₆-fluoroalkyle ou benzyle ;
   R₄ et R₅ représentent, indépendamment l'un de l'autre un atome d'hydrogène ou un groupe C₁₋₄ alkyle, C₃₋₇-cycloalkyle, C₃₋₇-cycloalkyle-C₁₋₆-alkyle ;
- R₇ et R₈ représentent, indépendamment l'un de l'autre, un atome d'hydrogène ou un groupe un groupe C₁₋₆-alkyle ;
   à l'état de base ou de sel d'addition à un acide.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases où de sels d'addition à des acides. De tels sels d'addition font partie de l'invention. Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvates, à savoir sous forme d'associations ou de combinaisons avec une ou plusieurs molécules d'eau ou avec un solvant. De tels hydrates et solvates font également partie de l'invention.

Dans le cadre de l'invention, on entend par :
- C_{t-z} où t et z peuvent prendre les valeurs de 1 à 7, une chaîne carbonée pouvant avoir de t à z atomes de carbone, par exemple C₁₋₇ une chaîne carbonée qui peut avoir de 1 à 7 atomes de carbone ;
- alkyle, un groupe aliphatique saturé, linéaire ou ramifié; par exemple un groupe C₁₋₆-alkyle représente une chaîne carbonée de 1 à 6 atomes de carbone, linéaire ou ramifiée, par exemple un méthyle, éthyle, propyle, isopropyle, butyle, isobutyle, terbutyle, pentyle, hexyle ;
- alkylène, un groupe alkyle divalent saturé, linéaire ou ramifié, par exemple un groupe C₁₋₆-alkylène représente une chaîne carbonée divalente de 1 à 6 atomes de carbone, linéaire ou ramifiée, par exemple un méthylène, éthylène, 1-méthyléthylène, propylène ;
- cycloalkyle, un groupe alkyle cyclique, par exemple un groupe C₃₋₇-cycloalkyle représente un groupe carboné cyclique de 3 à 7 atomes de carbone, par exemple un cyclopropyle, cyclobutyle, cyclopentyle, cyclohexyle, cycloheptyle ;
- acyle, un groupe alkyle-C(O)- ;
- hydroxyle, un groupe -OH ;
- monoamine cyclique, une chaîne carbonée cyclique saturée comportant 1 atome d'azote ;
- hydroxyalkyle, un groupe alkyle dont un atome d'hydrogène a été substitué par un groupe hydroxyle ;
- alkyloxy, un groupe -O-alkyle ;
- alkylthio, un groupe -S-alkyle ;
- fluoroalkyle, un groupe alkyle dont un ou plusieurs atomes d'hydrogène ont été substitués par un atome de fluor ;
- fluoroalkyloxy, un groupe alkyloxy dont un ou plusieurs atomes d'hydrogène ont été substitués par un atome de fluor ;
- un atome d'halogène, un atome de fluor, de chlore, de brome ou d'iodé ;
- aryle, un groupe aromatique, mono- ou bicyclique comprenant entre 6 et 10 atomes de carbones. A titre d'exemple de groupe aryle, on peut citer les groupes phényle ou naphtyle.

A titre d'exemples d'amines ou diamines cycliques formées par N, A, L et B, on peut notamment citer l'aziridine, l'azétidine, la pyrrolidine, la pipéridine, l'azépine, la morpholine, la thiomorpholine, l'homopipéridine, la décahydro-quinoline, la décahydro-isoquinoline, l'azabicyclo-heptane, l'azabicyclo-octane, l'azabicyclo-nonane, l'aza-oxo-bicyclo-heptane, l'aza-thia-bicyclo-heptane, l'aza-oxo-bicyclo-octane, l'aza-thia-bicyclo-octane ; la pipérazine, l'homopipérazine, la diaza-cyclo-octane, le diaza-cyclo-nonane, le diaza-cyclo-décane, le diaza-cyclo-undécane, l'octahydro-pyrrolo-pyrazine, l'octahydro-pyrrolo-diazepine, l'octahydro-pyrrolo-pyrrole, l'octahydro-pyrrolo-pyridine, le décahydro-naphthyridine, le diaza-bicyclo-heptane, le diaza-bicyclo-octane, le diaza-bicyclo-nonane, diaza-spiro-heptane, diaza-spiro-octane, le diaza-spiro-nonane, le diaza-spiro-décane, le diaza-spiro-undécane, le oxa-diaza-spiro-undécane:

Parmi les composés de formule générale (I) objets de l'invention, un groupe de composés est constitué par les composés pour lesquels R₃ représente un groupement choisi parmi un atome d'hydrogène, un groupe C₁₋₃ alkyle, C₁₋₄ alkyloxy, -NR₄R₅ ; R₄ et R₅ représentent un atome d'hydrogène ou un groupe C₁₋₄-alkyle ;
R₂, A, L, B, R₇ et R₈ étant tels que définis ci-dessus.

Parmi les composés de formule générale (I) objets de l'invention, un autre groupe de composés est constitué par les composés pour lesquels R₃ représente un groupement choisi parmi un atome d'hydrogène, un groupe méthyle et méthoxy;
R₂, A, L, B, R₇ et R₈ étant tels que définis ci-dessus.

Parmi les composés de formule générale (I) objets de l'invention, un autre groupe de composés est constitué par les composés pour lesquels R₃ représente un groupement choisi parmi -NH₂, méthyl-amino et diméthyl-amino ;
R₂, A, L, B, R₇ et R₈ étant tels que définis ci-dessus.

Parmi les composés de formule générale (I) objets de l'invention, un autre groupe de composés peut être décrit, constitué par les composés pour lesquels R₃ représente un groupement choisi parmi -NH₂, diméthyl-amino ;
R₂, A, L, B, R₇ et R₈ étant tels que définis ci-dessus.

Parmi les composés de formule générale (I) objets de l'invention, un autre groupe de composés est constitué par les composés pour lesquels R₇ et R₈ représentent, indépendamment l'un de l'autre un atome d'hydrogène ou un groupe méthyle ;
R₂, R₃, A, L, B, étant tels que définis ci-dessus.

Parmi les composés de formule générale (I) objets de l'invention, un autre groupe de composés est constitué par les composés pour lesquels :
- A représente un groupe C₁₋₇-alkylène ;
- B représente un groupe C₁₋₇-alkylène ;
- L représente un atome de carbone substitué par un groupe Rₑ₁ et un groupe R_{d} ;
- R_{d} représente un atome d'hydrogène ;
- Rₑ₁ représente un groupe NR₄R₅, où R₄ et R₅ représentent indépendamment l'un de l'autre un groupe C₁₋₄-alkyle ; ou bien Rₑ₁ représente une monoamine cyclique comportant éventuellement un atome d'oxygène, la monoamine étant éventuellement substituée par un ou plusieurs groupes choisis parmi l'atome de fluor et les groupes hydroxyle, C₁₋₆-alkyle ;
- R₂, R₃, R₇ et R₈ étant tels que définis ci-dessus.

Parmi les composés de formule générale (I) objets de l'invention, un autre groupe de composés peut être décrit, constitué par les composés pour lesquels :
- A représente un groupe -C₂H₄- ;
- B représente un groupe -C₂H₄- ou -CH₂- ;
- L représente un atome de carbone substitué par un groupe Rₑ₁ et un groupe R_{d} ;
- R_{d} représente un atome d'hydrogène
- Rₑ₁ représente un groupement choisi parmi les groupes diméthylamino, pyrrolidinyle, morpholinyle, diméthyl-morpholinyle, fluoro-pyrrolidinyle, hydroxy-pyrrolidinyle ;
- R₂, R₃, R₇ et R₈ étant tels que définis ci-dessus.

Parmi les composés de formule générale (I) objets de l'invention, un autre groupe de composés peut être décrit, constitué par les composés pour lesquels :
- l'amine cyclique formée par -N-A-L-B- représente une (+/-)-3-diméthylamino-pyrrolidin-1-yle, 4-(pyrrolidin-1-yl)-pipéridin-1-yle, 4-(morpholin-4-yl)-pipéridin-1-yle, 4-(2,6-diméthyl-morpholin-4-yl)-pipéridin-1-yle, 4-diméthylaminopipéridin-1-yle, 4-((*R*)-3-fluoro-pyrrolidin-1-yl)-pipéridin-1-yle, 4-(3-hydroxy-pyrrolidin-1-yl)-pipéridin-1-yle ;
   R₂, R₃, R₇ et R₈ étant tels que définis ci-dessus.

Parmi les composés de formule générale (I) objets de l'invention, un autre groupe de composés peut être décrit, constitué par les composés pour lesquels :
- A représente un groupe C₁₋₇-alkylène ;
- B représente un groupe C₁₋₇-alkylène ;
- L représente un atome de carbone substitué par un groupe Rₑ₁ et un groupe R_{d} ;
- R_{d} représente un atome d'hydrogène ;
- Rₑ₁ représente un groupe NR₄R₅, où R₄ et R₅ représentent indépendamment l'un de l'autre un groupe C₁₋₄-alkyle ; ou bien Rₑ₁ représente une monoamine cyclique comportant éventuellement un atome d'oxygène, la monoamine étant éventuellement substituée par un ou plusieurs groupes choisis parmi les groupes hydroxyle, C₁₋₆-alkyle ;
- R₂, R₃, R₇ et R₈ étant tels que définis ci-dessus.

Parmi les composés de formule générale (I) objets de l'invention, un autre groupe de composés peut être décrit, constitué par les composés pour lesquels :
- A représente un groupe -C₂H₄- ;
- B représente un groupe -C₂H₄- ou -CH₂- ;
- L représente un atome de carbone substitué par un groupe Rₑ₁ et un groupe R_{d} ;
- R_{d} représente un atome d'hydrogène ;
- Rₑ₁ représente un groupement choisi parmi les groupes diméthylamino, pyrrolidinyle, morpholinyle, diméthyl-morpholinyle, hydroxy-pyrrolidinyle ;
- R₂, R₃, R₇ et R₈ étant tels que définis ci-dessus.

Parmi les composés de formule générale (I) objets de l'invention, un autre groupe de composés peut être décrit, constitué par les composés pour lesquels :
- l'amine cyclique formée par -N-A-L-B- représente une (+/-)-3-diméthylamino-pyrrolidin-1-yle, 4-(pyrrolidin-1-yl)-pipéridin-1-yle, 4-(morpholin-4-yl)-pipéridin-1-yle, 4-(2,6-diméthyl-morpholin-4-yl)-pipéridin-1-yle, 4-diméthylaminopipéridin-1-yle, 4-(3-hydroxy-pyrrolidin-1-yl)-pipéridin-1-yle ;
   R₂, R₃, R₇ et R₈ étant tels que définis ci-dessus.

Parmi les composés de formule générale (I) objets de l'invention, un autre groupe de composés est constitué par les composés pour lesquels :
- A représente un groupe C₁₋₇-alkylène éventuellement substitué par un ou deux groupes Rₐ ;
- B représente un groupe C₁₋₇-alkylène éventuellement substitué par un groupe R_{b} ;
- L représente un atome d'azote éventuellement substitué par un groupe R_{c} ou R_{d} ;
les atomes de carbone de A et de B étant éventuellement substitués par un ou plusieurs groupes R_{f} identiques ou différents l'un de l'autre ;
Lorsqu'ils sont présents, Rₐ, R_{b} et R_{c} sont définis en combinaison comme suit :- soit deux groupes Rₐ forment ensemble un groupe C₁₋₆-alkylène ;
- soit Rₐ et R_{b} forment ensemble une liaison ou un groupe C₁₋₆-alkylène;
- soit Rₐ et R_{c} forment ensemble une liaison ou un groupe C₁₋₆-alkylène ;
- soit R_{b} et R_{c} forment ensemble une liaison ou un groupe C₁₋₆-alkylène ;
- R_{d} représente un substituant choisi parmi un groupe C₁₋₆-alkyle, C₃₋₇-cycloalkyle, C₃₋₇-cycloalkyle-C₁₋₆-alkyle, hydroxy-C₁₋₆-alkyle, C₁₋₆-alkyloxy-C₁₋₆-alkyle, C₁₋₆-fluoroalkyle, benzyle, C₁₋₆-acyle ;
- R_{f} représente un groupe C₁₋₆-alkyle ; hydroxy-C₁₋₆-alkyle, fluoro-C₁₋₆-alkyle
R₂, R₃, R₇ et R₈ étant tels que définis ci-dessus.

Parmi les composés de formule générale (I) objets de l'invention, un autre groupe de composés peut être décrit, constitué par les composés pour lesquels :
- l'amine cyclique formée par -N-A-L-B- représente une pipérazinyle, une diazépanyle, hexahydro-pyrrolo-pyrazinyle, diaza-bicyclo-heptyle, octahydro-pyrrolo-diazépinyle, diaza-bicyclo-nonyle, hexahydro-pyrrolo-pyrrolyle, octahydro-pyrrolo-pyridinyle, décahydro-naphtyridinyle, diaza-spiro-décyle, éventuellement substituée par un ou plusieurs groupes choisis parmi les groupes, méthyle, éthyle, isopropyle, butyle, cyclopropyle, cyclohexyle, hydroxyméthyle, hydroxyéthyle, (hydroxyméthyl)propyle, (hydroxyméthyl)butyle, méthoxyéthyle, fluorométhyle, fluoroéthyle, trifluoroéthyle, acétyle, isobutyryle, benzyle ;
   R₂, R₃, R₇ et R₈ étant tels que définis ci-dessus.

Parmi les composés de formule générale (I) objets de l'invention, un autre groupe de composés peut être décrit, constitué par les composés pour lesquels :
- l'amine cyclique formée par -N-A-L-B- représente une pipérazin-1-yle, 3-méthylpipérazin-1-yle, 4-méthylpipérazin-1-yle, 3,3-diméthylpipérazin1-yle, 3,4-diméthylpipérazin-1-yle, *cis*-3,5-diméthylpipérazin-1-yle, 4-(2-hydroxyéthyl)pipérazin-1-yle, 4-(2-méthoxyéthyl)pipérazin-1-yle, 4-(2-fluoroéthyl)pipérazin-1-yle, 4-(2,2,2-trifluoroéthyl)pipérazin-1-yle, 4-cyclopropylpipérazin-1-yle, 4-isopropylpipérazin-1-yle, 4-(2-hydroxy-2-méthylpropyl)pipérazin-1-yle, 4-*n-*butylpipérazin-1-yle, 4-(3-hydroxy-3-méthyl-butyl)pipérazin-1-yle, cyclohexylpipérazin-1-yle, 4-acétyl-pipérazin-1-yle, 4-isobutyryl-pipérazin-1-yle, (+/-)-hexahydropyrrolo[1,2-*a*]pyrazin-2-yle, (*S*)-hexahydropyrrolo[1,2-*a*]pyrazin-2-yle, (1*S*,4*S*)-2,5-diaza-bicyclo[2.2.1]hept-2-y|e, 1(*S*,4*S*)-5-benzyl-2,5-diazabicyclo[2.2.1]hept-2-yle, (1*S*,4*S*)-5-(2-hydroxyéthyl)-2,5-diazabicyclo[2.2.1]hept-2-yle, 5-isopropyl-2,5-diaza[2.2.1]hept-2-yle, 4-méthyl-[1,4]diazépan-1-yle, (+/-)-octahydropyrrolo[1,2-*d*][1,4]diazépin-3-yle, 1,4-diaza-bicyclo[3.3.2]non-4-yle, (+/-)-3,6-diaza-bicyclo[3.2.0]hept-3-yle, hexahydropyrrolo[3,4-*b*]pyrrol-5(1*H*)-yle, hexahydropyrrolo[3,4-*c*]pyrrol-2(1*H*)-yle, 5-benzyl-hexahydropyrrolo[3,4-*c*]pyrrol-2(1*H*)-yle, 5-cyclopropyl-hexahydropyrrolo[3,4-*c*]pyrrol-2(1*H*)-yle, 5-isopropyl-hexahydropyrrolo[3,4-*c*]pyrrol-2(1*H*)-yle, octahydro-6*H*-pyrrolo[3,4-*b*]pyridin-6-yle, (+/-)-(*cis*)-décahydro[2,6]-naphthyridin-2-yle, 3-éthyl-pipérazin-1-yle, 3,3-diéthyl-pipérazin-1-yle, 3-fluorométhyl-pipérazin-1-yle, 4-(3-hydroxyméthyl)pipérazin-1-yle, 3-(1-hydroxy-1-méthyl-éthyl)-pipérazin-1-yle, 3-isopropyl-pipérazin-1-yle, (1*R*,5*R*)-3,6-diaza-bicyclo[3.2.0]heot 2-yle, 5-méthyl-2,5-diaza-bicyclo[2.2.1]hept-2-yl,1-((1*S*,4*S*)-(2,5-diaza-bicyclo[2.2.1]hept-2-yl)-2-méthyl-propan-2-ol, 3,6-diaza-bicyclo[3.1.1]hept-3-yle, 5-méthyl-hexahydro-pyrrolo[3,4-*c*]pyrrol-2(1*H*)-yle, (+/-)-hexahydro-pyrrolo[3,4-*b*]pyrrol-5(1*H*)-ylé, (+)-hexahydro-pyrrolo[3,4-*b*]pyrrol-5(1*H*)-yle, (-)-hexahydro-pyrrolo[3,4-*b*]pyrrol-5(1*H*)-yle, (4a*S*,7a*S*)-octahydro-pyrrolo[3,4-*b*]pyridin-6-yle, 6,9-diaza-spiro[4.5]déc-9-yle ;
   R₂, R₃, R₇ et R₈ étant tels que définis ci-dessus.

Parmi les composés de formule générale (I) objets de l'invention, un autre groupe de composés peut être décrit, constitué par les composés pour lesquels :
- A représente un groupe C₁₋₇alkylène éventuellement substitué par un ou deux groupes Rₐ ;
- B représente un groupe C₁₋₇-alkylène éventuellement substitué par un groupe R_{b} ;
- L représente un atome d'azote éventuellement substitué par un groupe R_{c} ou R_{d} ; les atomes de carbone de A et de B étant éventuellement substitués par un ou plusieurs groupes R_{f} identiques ou différents l'un de l'autre ;
   Lorsqu'ils sont présents, Rₐ, R_{b} et R_{c} sont définis en combinaison comme suit :
   - soit Rₐ et R_{b} forment ensemble une liaison ou un groupe C₁₋₆-alkylène;
   - soit Rₐ et R_{c} forment ensemble une liaison ou un groupe C₁₋₆-alkylène ;
   - soit R_{b} et R_{c} forment ensemble une liaison ou un groupe C₁₋₆-alkylène ;
- R_{d} représente un substituant choisi parmi un groupe C₁₋₆-alkyle, C₃₋₇-cycloalkyle, C₃₋₇-cycloalkyle-C₁₋₆-alkyle, hydroxy-C₁₋₆-alkyle, C₁₋₆-alkyloxy-C₁₋₆-alkyle, C₁₋₆-fluoroalkyle, benzyle, C₁₋₆-acyle
- R_{f} représente un groupe C₁₋₆-alkyle ;
   R₂, R₃, R₇ et R₈ étant tels que définis ci-dessus.

Parmi les composés de formule générale (I) objets de l'invention, un autre groupe de composés peut être décrit, constitué par les composés pour lesquels :
- l'amine cyclique formée par -N-A-L-B- représente une pipérazinyle, une diazépanyle, hexahydro-pyrrolo-pyrazinyle, diaza-bicyclo-heptyle, octahydro-pyrrolo-diazépinyle, diaza-bicyclo-nonyle, hexahydro-pyrrolo-pyrrolyle, octahydro-pyrrolo-pyridinyle, décahydro-naphtyridinyle, éventuellement substituée par un ou plusieurs groupes choisis parmi les groupes, méthyle, éthyle, isopropyle, butyle, cyclopropyle, cyclohexyle, hydroxyéthyle, (hydroxyméthyl)propyle, (hydroxyméthyl)butyle, méthoxyéthyle, fluoroéthyle, trifluoroéthyle, acétyle, isobutyryle, benzyle ;
   R₂, R₃, R₇ et R₈ étant tels que définis ci-dessus.

Parmi les composés de formule générale (I) objets de l'invention, un autre groupe de composés peut être décrit, constitué par les composés pour lesquels :
- l'amine cyclique formée par -N-A-L-B- représente une pipérazin-1-yle, 3-méthylpipérazin-1-yle, 4-méthylpipérazin-1-yle, 3,3-diméthylpipérazin1-yle, 3,4-diméthylpipérazin-1-yle, *cis*-3,5-diméthylpipérazin-1-yle, 4-(2-hydroxyéthyl)pipérazin-1-yle, 4-(2-méthoxyéthyl)pipérazin-1-yle, 4-(2-fluoroéthyl)pipérazin-1-yle, 4-(2,2,2-trifluoroéthyl)pipérazin-1-yle, 4-cyclopropylpipérazin-1-yle, 4-isopropylpipérazin-1-yle, 4-(2-hydroxy-2-méthylpropyl)pipérazin-1-yle, *4-n-*butylpipérazin-1-yle, 4-(3-hydroxy-3-méthyl-butyl)pipérazin-1-yle, cyclohexylpipérazin-1-yle, 4-acétyl-pipérazin-1-yle, 4-isobutyryl-pipérazin-1-yle, (+/-)-hexahydropyrrolo[1,2-a]pyrazin-2-yle, (*S*)-hexahydropyrrolo[1,2-*a*]pyrazin-2-yle, (1*S*,4*S*)-2,5-diaza-bicyclo[2.2.1]hept-2-yle, 1(*S*,4*S*)-5-benzyl-2,5-diazabicyclo[2.2.1]hept-2-yle, (1*S*,4*S*)-5-(2-hydroxyéthyl)-2,5-diazabicyclo[2.2.1]hept-2-yle, 5-isopropyl-2,5-diaza[2.2.1]hept-2-yle, 4-méthyl-[1,4]diazépan-1-yle, (+/-)-octahydropyrrolo[1,2-*d*][1,4]diazépin-3-yle, 1,4-diaza-bicyclo[3.3.2]non-4-yle, (+/-)-3,6-diaza-bicyclo[3.2.0]hept-3-yle, hexahydropyrrolo[3,4-*b*]pyrrol-5(1*H*)-yle, hexahydropyrrolo[3,4-c]pyrrol-2(1*H*)-yle, 5-benzyl-hexahydropyrrolo[3,4-c]pyrrol-2(1*H*)-yle, 5-cyclopropyl-hexahydropyrrolo[3,4-c]pyrrol-2(1*H*)-yle, 5-isopropyl-hexahydropyrrolo[3,4-c]p*y*rrol-2(1*H*)-yle, octahydro-6*H*-pyrrolo[3,4-*b*]pyridin-6-yle, (+/-)-(*cis*)-décahydro[2,6]-naphthyridin-2-yle ;
   R₂, R₃, R₇ et R₈ étant tels que définis ci-dessus.

Parmi les composés de formule générale (I) objets de l'invention, un autre groupe de composés est constitué par les composés pour lesquels :
- A représente un groupe C₁₋₇-alkylène ;
- B représente un groupe C₁₋₇-alkylène;
- L représente un atome de carbone substitué par deux groupes Rₑ₂ ;
   les atomes de carbone de A et de B étant éventuellement substitués par un ou plusieurs groupes R_{f} identiques ou différents l'un de l'autre ;
- Deux Rₑ₂ forment, avec l'atome de carbone qui les porte, un groupe pyrrolidine, pipéridine, morpholine ;
- R_{f} représente un groupe C₁₋₆-alkyle ;
   R₂, R₃, R₇ et R₈ étant tels que définis ci-dessus.

Parmi les composés de formule générale (I) objets de l'invention, un autre groupe de composés peut être décrit, constitué par les composés pour lesquels :
l'amine cyclique formée par -N-A-L-B- représente une diaza-spiro-nonyle, diaza-spiro-décyle, diaza-spiro-undécyle, oxa-diaza-spiro-undécyle ;
   R₂, R₃, R₇ et R₈ étant tels que définis ci-dessus.

Parmi les composés de formule générale (I) objets de l'invention, un autre groupe de composés peut être décrit, constitué par les composés pour lesquels :
l'amine cyclique formée par -N-A-L-B- représente une (+/-)-2,7-diaza-spiro[4.4]non-2-yle, (+/)-2,8-diaza-spiro-[4.5]déc-2-yle, (+/-)-2,7-diaza-spiro-[4.5]déc-2-yle, (+/-)-2,8-diaza-spiro-[4.5]déc-8-yle, (+/-)-2,7-diaza-spiro-[4.5]déc-7-yle, 3,9-diaza-spiro-[5.5]undéc-3-yle, 2,9-diaza-spiro-[5.5]undéc-9-yle, (+/-)-2,8-diaza-spiro-[5.5]undéc-2-yle, 1-oxa-4,9-diaza-spiro[5.5]undéc-9-yle ;
   R₂, R₃, R₇ et R₈ étant tels que définis ci-dessus.

Parmi les composés de formule générale (I) objets de l'invention, un autre groupe de composés est constitué par les composés pour lesquels :
- R₂ représente un groupe phényle éventuellement substitué par un ou plusieurs substituants choisis parmi les atomes de fluor, de chlore et le groupe méthyle ;
- R₃ représente un atome d'hydrogène ;
- l'amine cyclique formée par -N-A-L-B- représente une (+/-)-3-diméthylamino-pyrrolidin-1-yle, 4-(pyrrolidin-1-yl)-pipéridin-1-yle, 4-(morpholin-4-yl)-pipéridin-1-yle, 4-(2,6-diméthyl-morpholin-4-yl)-pipéridin-1-yle, 4-diméthylaminopipéridin-1-yle, 4-(3-hydroxy-pyrrolidin-1-yl)-pipéridin-1-yle, 4-(3-fluoro-pyrrolidin-1-yl)-pipéridin-1-yle ;
- R₇ et R₈ représentent un atome d'hydrogène.

Parmi les composés de formule générale (I) objets de l'invention, un autre groupe de composés peut être décrit, constitué par les composés pour lesquels :
- R₂ représente un groupe phényle éventuellement substitué par un ou plusieurs substituants choisis parmi les parmi les atomes de fluor et de chlore et les groupes méthyle, méthoxy, trifluorométhyle ;
- R₃ représente un atome d'hydrogène ou un groupe méthyle, méthoxy, -NH₂, méthyl-amino, diméthyl-amino ;
- l'amine cyclique formée par -N-A-L-B- représente une pipérazin-1-yle, 3-méthylpipérazin-1-yle, 4-méthylpipérazin-1-yle, 3,3-diméthylpipérazin1-yle, 3,4-diméthylpipérazin-1-yle, *cis*-3,5-diméthylpipérazin-1-yle, 4-(2-hydroxyéthyl)pipérazin-1-yle, 4-(2-méthoxyéthyl)pipérazin-1-yle, 4-(2-fluoroéthyl)pipérazin-1-yle, 4-(2,2,2-trifluoroéthyl)pipérazin-1-yle, 4-cyclopropylpipérazin-1-yle, 4-isopropylpipérazin-1-yle, 4-(2-hydroxy-2-méthylpropyl)pipérazin-1-yle, *4-n-*butylpipérazin-1-yle, 4-(3-hydroxy-3-méthyl-butyl)pipérazin-1-yle, cyclohexylpipérazin-1-yle, 4-acétyl-pipérazin-1-yle, 4-isobutyryl-pipérazin-1-yle, (+/-)-hexahydropyrrolo[1,2-a]pyrazin-2-yle, (*S*)-hexahydropyrrolo[1,2-a]pyrazin-2-yle, (1*S*,4*S*)-2,5-diaza-bicyclo[2.2.1]hept-2-yle, 1(*S*,4*S*)-5-benzyl-2,5-diazabicyclo[2.2.1]hept-2-yle, (1*S*,4*S*)-5-(2-hydroxyéthyl)-2,5-diazabicyclo[2.2.1]hept-2-yle, 5-isopropyl-2,5-diaza[2.2.1]hept-2-yle, 4-méthyl-[1,4]diazépan-1-yle, (+/-)-octahydropyrrolo[1,2-*d*][1,4]diazépin-3-yle, 1,4-diaza-bicyclo[3.3.2]non-4-yle, (+/-)-3,6-diaza-bicyclo[3.2.0]hept-3-yle, hexahydropyrrolo[3,4-*b*]pyrrol-5(1*H*)-yle, hexahydropyrrolo[3,4-c]pyrrol-2(1*H*)-yle, 5-benzyl-hexahydropyrrolo[3,4-*c*]pyrrol-2(1*H*)-yle, 5-cyclopropyl-hexahydropyrrolo[3,4-c]pyrrol-2(1*H*)-yle, 5-isopropyl-hexahydropyrrolo[3,4-c]pyrrol-2(1*H*)-yle, octahydro-6*H*-pyrrolo[3,4-b]pyridin-6-yle, (+/-)-(*cis*)-décahydro[2,6]-naphthyridin-2-yle, 3-éthyl-pipérazin-1-yle, 3,3-diéthyl-pipérazin-1-yle, 3-fluorométhyl-pipérazin-1-yle, 4-(3-hydroxyméthyl)pipérazin-1-yle, 3-(1-hydroxy-1-méthyl-éthyl)-pipérazin-1-yle, 3-isopropyl-pipérazin-1-yle, (1*R*,5*R*)-3,6-diaza-bicyclo[3.2.0]hept-2-yle, 5-méthyl-2,5-diaza-bicyclo[2.2.1]hept-2-yl,1-((1*S*,4*S*)-(2,5-diaza-bicyclo[2.2.1]hept-2-yl)-2-méthyl-propan-2-ol, 3,6-diaza-bicyclo[3.1.1]hept-3-yle, 5-méthyl-hexahydro-pyrrolo[3,4-c]pyrrol-2(1*H*)-yle, (+/-)-hexahydro-pyrrolo[3,4-*b*]pyrrol-5(1H)-yle, (+)-hexahydro-pyrrolo[3,4-b]pyrrol-5(1*H*)-yle, (-)-hexahydro-pyrrolo[3,4-*b*]pyrrol-5(1H)-yle, (4aS,7aS)-octahydro-pyrrolo[3,4-*b*]pyridin-6-yle, 6,9-diaza-spiro[4.5]déc-9-yle ;
- R₇ et R₈ représentent, indépendamment l'un de l'autre un atome d'hydrogène ou un groupe méthyle.

Parmi les composés de formule générale (I) objets de l'invention, un autre groupe de composés est constitué par les composés pour lesquels :
- R₂ représente un groupe phényle éventuellement substitué par un ou plusieurs substituants choisis parmi les atomes de fluor et de chlore ;
- R₃ représente un atome d'hydrogène ;
- l'amine cyclique formée par -N-A-L-B- représente une (+/-)-3-diméthylamino-pyrrolidin-1-yle, 4-(pyrrolidin-1-yl)-pipéridin-1-yle, 4-(morpholin-4-yl)-pipéridin-1-yle, 4-(2,6-diméthyl-morpholin-4-yl)-pipéridin-1-yle, 4-diméthylaminopipéridin-1-yle, 4-(3-hydroxy-pyrrolidin-1-yl)-pipéridin-1-yle ;
- R₇ et R₈ représentent un atome d'hydrogène.

Parmi les composés de formule générale (I) objets de l'invention, un autre groupe de composés peut être décrit, constitué par les composés pour lesquels :
- R₂ représente un groupe phényle éventuellement substitué par un ou plusieurs substituants choisis parmi les parmi les atomes de fluor et de chlore et les groupes méthyle, méthoxy, trifluorométhyle ;
- R₃ représente un atome d'hydrogène ou un groupe méthyle, méthoxy, -NH₂, diméthyl-amino ;
- l'amine cyclique formée par -N-A-L-B- représente une pipérazin-1-yle, 3-méthylpipérazin-1-yle, 4-méthylpipérazin-1-yle, 3,3-diméthylpipérazin1-yle, 3,4-diméthylpipérazin-1-yle, *cis*-3,5-diméthylpipérazin-1-yle, 4-(2-hydroxyéthyl)pipérazin-1-yle, 4-(2-méthoxyéthyl)pipéraziny1-yle, 4-(2-fluoroéthyl)pipérazin-1-yle, 4-(2,2,2-trifluoroéthyl)pipérazin-1-yle, 4-cyclopropylpipérazin-1-yle, 4-isopropylpipérazin-1-yle, 4-(2-hydroxy-2-méthylpropyl)pipérazin-1-yle, 4-*n-*butylpipérazin-1-yle, 4-(3-hydroxy-3-méthyl-butyl)pipérazin-1-yle, 4-cyclohexylpipérazin-1-yle, 4-acétyl-pipérazin-1-yle, 4-isobutyryl-pipérazin-1-yle, (+/-)-3,6-diaza-bicyclo[3.2.0]hept-3-yle (+/-)-hexahydropyrrolo[1,2-*a*]pyrazin-2-yle, (*S*)-hexahydropyrrolo[1,2-a]pyrazin-2-yle, (1*S*,4*S*)-2,5-diaza-bicyclo[2.2.1]hept-2-yle, (1*S*,4*S*)-5-benzyl-2,5-diazabicyc|o[2.2.1]hept-2-yle, 5-isopropyl-2,5-diaza[2.2.1]hept-2-yle, 4-méthyl-[1,4]diazépan-1-yle, (+/-)-octahydropyrrolo[1,2-*d*][1,4]diazépin-2-yle, 1,4-diaza-bicyclo[3.3.2]non-4-yle, hexahydropyrrolo[3,4-*b*]pyrrol-5(1H)-yle, hexahydropyrrolo[3,4-*c*]pyrrol-2(1*H*)-yle, 5-benzyl-hexahydropyrrolo[3,4-*c*]pyrrol-2(1H)-yle, 5-cyclopropyl-hexahydropyrrolo[3,4-c]pyrrol-2(1*H*)-yle, 5-isopropyl-hexahydropyrrolo[3,4-c]pyrrol-2(1*H*)-yle, octahydro-6*H*-pyrrolo[3,4-*b*]pyridin-6-yle, (+/-)-(cis)-décahydro[2,6]-naphthyridin-2-yle ;
- R₇ et R₈ représentent, indépendamment l'un de l'autre un atome d'hydrogène ou un groupe méthyle.

Parmi les composés de formule générale (I) objets de l'invention, un autre groupe de composés peut être décrit, constitué par les composés pour lesquels :
- R₂ représente un groupe phényle éventuellement substitué par un ou plusieurs substituants choisis parmi les parmi les atomes de fluor et de chlore ;
- R₃ représente un atome d'hydrogène ou un groupe méthyle, méthoxy, -NH₂ ;
- l'amine cyclique formée par -N-A-L-B- représente (+/-)-2,7-diaza-spiro[4.4]non-2-yle, (+/-)-2,8-diaza-spiro-[4.5]déc-2-yle, (+/-)-2,7-diaza-spiro-[4.5]déc-2-yle, (+/-)-2,8-diaza-spiro-[4.5]déc-8-yle, (+/-)-2,7-diaza-spiro-[4.5]déc-7-yle, 3,9-diaza-spiro-[5.5]undéc-3-yle, 2,9-diaza-spiro-[5.5]undéc-9-yle, (+/-)-2,8-diaza-spiro-[4.5]undéc-2-yle, 1-oxa-4,9-diaza-spiro[5.5]undéc-9-yle ;
- R₇ et R₈ représentent un atome d'hydrogène.

Parmi les composés.de formule générale (I) objets de l'invention, on peut notamment citer les composés suivants:
2-Phényl-6-pipérazin-1-yl-3-pyridin-4-yl-imidazo[1,2-*b*]pyridazine ;
2-(4-Fluoro-phényl)-6-pipérazin-1-yl-3-pyridin-4-yl-imidazo[1,2-*b*]pyridazine
2-(4-Fluoro-phényl)-7,8-diméthyl-6-pipérazin-1-yl-3-pyridin-4-yl-imidazo[1,2-*b*]pyridazine
4-[2-(4-Fluoro-phényl)-6-pipérazin-1-yl-imidazo[1,2-*b*]pyridazin-3-yl]-pyridin-2-ylamine ;
2-(4-Fluoro-phényl)-3-(2-méthoxy-pyridin-4-yl)-6-pipérazin-1-yl-imidazo[1,2-*b*]pyridazine ;
2-(4-Chloro-phényl)-6-pipérazin-1-yl-3-pyridin-4-yl-imidazo[1,2-*b*]pyridazine ;
2-(4-Chloro-phényl)-7-méthyl-6-pipérazin-1-yl-3-pyridin-4-yl-imidazo[1,2-*b*]pyridazine ;
2-(4-Chloro-phényl)-8-méthyl-6-pipérazin-1-yl-3-pyridin-4-yl-imidazo[1,2-*b*]pyridazine ;
2-(3,5-Diméthyl-phényl)-6-pipérazin-1-yl-3-pyridin-4-yl-imidazo[1,2-*b*]pyridazine ;
2-(3,5-Difluoro-phényl)-6-pipérazin-1-yl-3-pyridin-4-yl-imidazo[1,2-*b*]pyridazine ;
2-(3,5-Difluoro-phényl)-3-(2-méthyl-pyridin-4-yl)-6-pipérazin-1-yl-imidazo[1,2-*b*]pyridazine ;
2-(3,4-Difluoro-phényl)-6-pipérazin-1-yl-3-pyridin-4-yl-imidazo[1,2-*b*]pyridazine ;
2-(3,5-Dichloro-phényl)-6-pipérazin-1-yl-3-pyridin-4-yl-imidazo[1,2-*b*]pyridazine ;
2-(3,5-Dichloro-phényl)-3-(2-méthyl-pyridin-4-yl)-6-pipérazin-1-yl-imidazo[1,2-*b*]pyridazine ;
(+/-)-6-(3-Méthyl-pipérazin-1-yl)-2-phényl-3-pyridin-4-yl-imidazo[1,2-*b*]pyridazine ;
6-((*R*)-3-Méthyl-pipérazin-1-yl)-2-phényl-3-pyridin-4-yl-imidazo[1,2-b]pyridazine ;
6-((*R*)-3-Méthyl-pipérazin-1-yl)-3-(2-méthyl-pyridin-4-yl)-2-phényl-imidazo[1,2-*b*]pyridazine ;
6-((*S*)-3-Méthyl-pipérazin-1-yl)-2-phényl-3-pyridin-4-yl-imidazo[1,2-*b*]pyridazine ;
2-(3-Fluoro-phényl)-6-((*R*)-3-méthyl-pipérazin-1-yl)-3-pyridin-4-yl-imidazo[1,2-*b*]pyridaziné ;
2-(4-Fluoro-phényl)-6-(3-méthyl-pipérazin-1-yl)-3-pyridin-4-yl-imidazo[1,2-b]pyridazine ;
2-(4-Fluoro-phényl)-6-((*R*)-3-méthyl-pipérazin-1-yl)-3-pyridin-4-yl-imidazo[1,2-*b*]pyridazine ;
2-(4-Fluoro-phényl)-6-((*S*)-3-méthyl-pipérazin-1-yl)-3-pyridin-4-yl-imidazo[1,2-*b*]pyridazine ;
2-(4-Fluoro-phényl)-3-(2-méthoxy-pyridin-4-yl)-6-((*R*)-3-méthyl-pipérazin-1-yl)-imidazo[1,2-*b*]pyridazine ;
2-(3,4-Difluoro-phényl)-6-((*R*)-3-méthyl-pipérazin-1-yl)-3-pyridin-4-yl-imidazo[1,2-*b*]pyridazine ;
6-(3,3-Diméthyl-pipérazin-1-yl)-2-phényl-3-pyridin-4-yl-imidazo[1,2-*b*]pyridazine ;
6-(3,3-Diméthyl-pipérazin-1-yl)-2-(4-fluoro-phényl)-3-pyridin-4-yl-imidazo[1,2-*b*]pyridazine ;
6-(3,3-Diméthyl-pipérazin-1-yl)-2-(4-fluoro-phényl)-3-(2-méthoxy-pyridin-4-yl)-imidazo[1,2-*b*]pyridazine;
2-(3,5-Diméthyl-phényl)-6-(3,3-diméthyl-pipérazin-1-yl)-3-pyridin-4-yl-imidazo[1,2-*b*]pyridazine
2-(3,5-Diméthyl-phényl)-6-(3,3-diméthyl-pipérazin-1-yl)-3-(2-méthyl-pyridin-4-yl)-imidazo[1,2-*b*]pyridazine ;
4-[2-(3,5-Diméthyl-phényl)-6-(3,3-diméthyl-pipérazin-1-yl)-imidazo[1,2-*b*]pyridazin-3-yl]-pyridin-2-ylamine ;
2-(3,5-Difluoro-phényl)-6-(3,3-diméthyl-pipérazin-1-yl)-3-pyridin-4-yl-imidazo[1,2-*b*]pyridazine ;
2-(3,5-Difluoro-phényl)-6-(3,3-diméthyl-pipérazin-1-yl)-3-(2-méthyl-pyridin-4-yl)-imidazo[1,2-*b*]pyridazine ;
4-[2-(3,5-Difluoro-phényl)-6-(3,3-diméthyl-pipérazin-1-yl)-imidazo[1,2-*b*]pyridazin-3-yl]-pyridin-2-ylamine ;
2-(3,5-Dichloro-phényl)-6-(3,3-diméthyl-pipérazin-1-yl)-3-pyridin-4-yl-imidazo[1,2-*b*]pyridazine ;
2-(3,5-Dichloro-phényl)-6-(3,3-diméthyl-pipérazin-1-yl)-3-(2-méthyl-pyridin-4-yl)-imidazo[1,2-*b*]pyridazine ;
(+/-)-6-(3,4-Diméthyl-pipérazin-1-yl)-2-(4-fluoro-phényl)-3-pyridin-4-yl-imidazo[1,2-*b*]pyridazine ;
6-((*cis*)-3,5-Diméthyl-pipérazin-1-yl)-2-phényl-3-pyridin-4-yl-imidazo[1,2-b]pyridazine ;
6-((*cis*)-3,5-Diméthyl-pipérazin-1-yl)-3-(2-méthyl-pyridin-4-yl)-2-phényl-imidazo[1,2-*b*]pyridazine ; ,
6-((*cis*)-3,5-Diméthyl-pipérazin-1-yl)-2-(3-fluoro-phényl)-3-pyridin-4-yl-imidazo[1,2-*b*]pyridazine ;
6-((*cis*)3,5-Diméthyl-pipérazin-1-yl)-2-(4-fluoro-phényl)-3-pyridin-4-yl-imidazo[1,2-*b*]pyridazihe ;
6-((*cis*)-3,5-Diméthyl-pipérazin-1-yl)-2-(4-fluoro-phényl)-3-(2-méthoxy-pyridin-4-yl)-imidazo[1,2-*b*]pyridazine ;
2-(3,5-Diméthyl-phényl)-6-((*cis*)-3,5-diméthyl-pipérazin-1-yl)-3-pyridin-4-yl-imidazo[1,2-*b*]pyridazine ;
2-(3,4-Difluoro-phényl)-6-((*cis*)-3,5-diméthyl-pipérazin-1-yl)-3-pyridin-4-yl-imidazo[1,2-*b*]pyridazine ;
2-(4-Chloro-phényl)-6-((*cis*)-3,5-diméthyl-pipérazin-1-yl)-7-méthyl-3-pyridin-4-yl-imidazo[1,2-*b*]pyridazine ;
6-((*cis*)-3,5-Diméthyl-pipérazin-1-yl)-2-(4-fluoro-phényl)-8-méthyl-3-pyridin-4-yl-imidazo[1,2-*b*]pyridazine ;
2-(2-Chloro-phényl)-6-(4-méthyl-pipérazin-1-yl)-3-pyridin-4-yl-imidazo[1,2-*b*]pyridazine ;
2-(3-Chloro-phényl)-6-(4-méthyl-pipérazin-1-yl)-3-pyridin-4-yl-imidazo[1,2-*b*]pyridazine ;
2-(4-Chloro-phényl)-6-(4-méthyl-pipérazin-1-yl)-3-pyridin-4-yl-imidazo[1,2-*b*]pyridazine ;
2-(3-Fluoro-phényl)-6-(4-méthyl-pipérazin-1-yl)-3-pyridin-4-yl-imidazo[1,2-*b*]pyridazine ;
2-(4-Fluoro-phényl)-6-(4-méthyl-pipérazin-1-yl)-3-pyridin-4-yl-imidazo[1,2-*b*]pyridazine ;
2-(3,4-Difluoro-phényl)-6-(4-méthyl-pipérazin-1-yl)-3-pyridin-4-yl-imidazo[1,2-*b*]pyridazine ;
2-(3-Méthoxy-phényl)-6-(4-méthyl-pipérazin-1-yl)-3-pyridin-4-yl-imidazo[1,2-*b*]pyridazine ;
2-(4-Méthoxy-phényl)-6-(4-méthyl-pipérazin-1-yl)-3-pyridin-4-yl-imidazo[1,2-*b*]pyridazine ;
6-(4-Méthyl-pipérazin-1-yl)-3-pyridin-4-yl-2-p-tolyl-imidazo[1,2-*b*]pyridazine ;
6-(4-Méthyl-pipérazin-1-yl)-3-pyridin-4-yl-2-(4-trifluorométhyl-phényl)-imidazo[1,2-*b*]pyridazine ;
2-(4-Fluoro-phényl)-6-(4-méthyl-pipérazin-1-yl)-3-(2-méthyl-pyridin-4-yl)-imidazo[1,2-*b*]pyridazine ;
4-[2-(4-Fluoro-phényl)-6-(4-méthyl-pipérazin-1-yl)-imidazo[1,2-*b*]pyridazin-3-yl]-pyridin-2-lamine;
{4-[2-(4-Fluoro-phényl)-6-(4-méthyl-pipérazin-1-yl)-imidazo[1,2-*b*]pyridazin-3-yl]-pyridin-2-yl}-diméthyl-amine ;
2-(4-Fluoro-phényl)-3-(2-méthoxy-pyridin-4-yl)-6-(4-méthyl-pipérazin-1-yl)-imidazo[1,2-*b*]pyridazine ;
6-(4-Éthyl-pipérazin-1-yl)-2-(4-fluoro-phényl)-3-pyridin-4-yl-imidazo[1,2-*b*]pyridazine ;
2-(3,5-Diméthyl-phényl)-6-(4-éthyl-pipérazin-1-yl)-3-pyridin-4-yl-imidazo[1,2-*b*]pyridazine ;
2-[4-(2-Phényl-3-pyridin-4-yl-imidazo[1,2-*b*]pyridazin-6-yl)-pipérazin-1-yl]-éthanol ;
2-{4-[2-(3-Fluoro-phényl)-3-pyridin-4-yl-imidazo[1,2-*b*]pyridazin-6-yl]-pipérazin-1-yl}-éthanol ;
2-{4-[2-(4-Fluoro-phényl)-3-pyridin-4-yl-imidazo[1,2-*b*]pyridazinf-yl]-pipérazin-1-yl}-éthanol ;
2-{4-[2-(4-Fluoro-phényl)-3-(2-méthoxy-pyridin-4-yl)-imidazo[1,2-*b*]pyridazin-6-yl]-pipérazin-1-yl}-éthanol ;
2-{4-[2-(3,4-Difluoro-phényl)-3-pyridin-4-yl-imidazo[1,2-*b*]pyridazin-6-yl]-pipérazin-1-yl}-éthanol ;
2-{4-[2-(4-Chloro-phényl)-3-pyridin-4-yl-imidazo[1,2-*b*]pyridazin-6-yl]-pipérazin-1-yl}-éthanol ;
2-{4-[3-(2-Amino-pyridin-4-yl)-2-(4-chloro-phényl)-imidazo[1,2-b]pyridazin-6-yl]-pipérazin-1-yl}-éthanol ;
2-(4-Chloro-phényl)-6-[4-(2-méthoxy-éthyl)-pipérazin-1-yl]-3-pyridin-4-yl-imidazo[1,2-*b*]pyridazine ;
6-[4-(2-Fluoro-éthyl)-pipérazin-1-yl]-2-(4-fluoro-phényl)-3-pyridin-4-yl-imidazo[1,2-*b*]pyridazine ;
6-[4-(2-Fluoro-éthyl)-pipérazin-1-yl]-2-(4-fluoro-phényl)-3-(2-méthoxy-pyridin-4-yl)-imidazo[1,2-*b*]pyridazine ;
2-Phényl-3-pyridin-4-yl-6-[4-(2,2,2-trifluoro-éthyl)-pipérazin-1-yl]-imidazo[1,2-*b*]pyridazine ;
2-(4-Fluoro-phényl)-3-pyridin-4-yl-6-[4-(2,2,2-trifluoro-éthyl)-pipérazin-1-yl]-imidazo[1,2-*b*]pyridazine ;
6-(4-Cyclopropyl-pipérazin-1-yl)-2-(4-fluoro-phényl)-3-pyridin-4-yl-imidazo[1,2-*b*]pyridazine ;
6₋(4-Cyclopropyl-pipérazin-1-yl)-2-(4-fluoro-phényl)-3-(2-méthoxy-pyridin-4-yl)-imidazo[1,2-*b*]pyridazine ;
6-(4-Isopropyl-pipérazin-1-yl)-3-(2-méthoxy-pyridin-4-yl)-2-phényl-imidazo[1,2-*b*]pyridazine ;
2-(3-Fluoro-phényl)-6-(4-isopropyl-pipérazin-1-yl)-3-pyridin-4-yl-imidazo[1,2-*b*]pyridazine ;
2-(4-Fluoro-phényl)-6-(4-isopropyl-pipérazin-1-yl)-3-pyridin-4-yl-imidazo[1,2-*b*]pyridazine ;
2-(4-Fluoro-phényl)-6-(4-isopropyl-pipérazin-1-yl)-3-(2-méthoxy-pyridin-4-yl)-imidazo[1,2-*b*]pyridazine ;
2-(3,4-Difluoro-phényl)-6-(4-isopropyl-pipérazin-1-yl)-3-pyridin-4-yl-imidazo[1,2-*b*]pyridazine ;
4-[2-(4-Chloro-phényl)-6-(4-isopropyl-pipérazin-1-yl)-imidazo[1,2-*b*]pyridazin-3-yl]-pyridin-2-ylamine ;
2-Méthyl-1-[4-(2-phényl-3-pyridin-4-yl-imidazo[1,2-*b*]pyridazin-6-yl)-pipérazin-1-yl]-propan-2-ol ;
1-{4-[2-(3-Fluoro-phényl)-3-pyridin-4-yl-imidazo[1,2-*b*]pyridazin-6-yl]-pipérazin-1-yl}-2-méthyl-propan-2-ol ;
1-{4-[2-(4-Fluoro-phényl)-3-(2-méthoxy-pyridin-4-yl)-imidazo[1,2-*b*]pyridazin-6-yl]-piperazin-1-yl}-2-méthyl-propan-2-ol ;
6-(4-Butyl-pipérazin-1-yl)-2-(4-chloro-phényl)-3-pyridin-4-yl-imidazo[1,2-*b*]pyridazine ;
4-{4-[2-(4-Fluoro-phényl)-3-(2-méthoxy-pyridin-4-yl)-imidazo[1,2-*b*]pyridazin-6-yl]-pipérazin-1-yl}-2-méthyl-butan-2-ol ;
6-(4-Cyclohexyl-pipérazin-1-yl)-2-(4-fluoro-phényl)-3-pyridin-4-yl-imidazo[1,2-*b*]pyridazine ;
1-{4-[2-(4-Fluoro-phényl)-3-pyridin-4-yl-imidazo[1,2-*b*]pyridazin-6-yl]-pipérazin-1-yl}-éthanone ;
1-{4-[2-(4-Fluoro-phényl)-3-pyridin-4-yl-imidazo[1,2-*b*]pyridazin-6-yl]-pipérazin-1-yl}-2-méthyl-propan-1-one ;
(+/-)-2-(4-Fluoro-phényl)-6-(hexahydro-pyrrolo[1,2-*a*]pyrazin-2-yl)-3-pyridin-4-yl-imidazo[1,2-*b*]pyridazine ;
2-(4-Fluoro-phényl)-6-(S)-hexahydro-pyrrolo[1,2-*a*]pyrazin-2-yl-3-(2-méthoxy-pyridin-4-yl)-imidazo[1,2-b]pyridazine ;
6-((1*S*,4*S*)-2,5-Diaza-bicyclo[2.2.1]hept-2-yl)-2-phényl-3-pyridin-4-yl-imidazo[1,2-b]pyridazine ;
6-((1*S*,4*S*)-5-Benzyl-2,5-diaza-bicyclo[2.2.1]hept-2-yl)-2-(3-fluoro-phényl)-3-pyridin-4-yl-imidazo[1,2-*b*]pyridazine ;
6-((1S,4S)-2,5-Diaza-bicyclo[2.2.1]hept-2-yl)-2-(4-fluoro-phéhyl)-3-pyridin-4-yl-imidazo[1,2-*b*]pyridazine ;
6-(1S,4S)-5-Benzyl-2,5-diaza-bicyclo[2.2.1]hept-2-yl)-2-(4-fluoro-phényl)-3-pyridin-4-yl-imidazo[1,2-*b*]pyridazine ;
6-((1*S*,4*S*)-2,5-Diaza-bicyclo[2.2.1]hept-2-yl)-2-(4-fluoro-phényl)-3-(2-méthoxy-pyridin-4-yl)-imidazo[1,2-*b*]pyridazine ;
2-{(1*S*,4*S*)-5-[2-(4-Fluoro-phényl)-3-(2-méthoxy-pyridin-4-yl)-imidazo[1,2-*b*]pyridazin-6-yl]-2,5-diaza-bicyclo[2.2.1]hept-2-yl}-éthanol ;
2-(4-Fluoro-phényl)-6-(5-isopropyl-2,5-diaza-bicyclo[2.2.1]hept-2-yl)-3-(2-méthoxy-pyridin-4-yl)-imidazo[1,2-b]pyridazine ;
2-(4-Fluoro-phényl)-6-(4-méthyl-[1,4]diazépan-1-yl)-3-pyridin-4-yl-imidazo[1,2-b]pyridazine ;
(+/-)-3-[2-(4-Fluoro-phényl)-3-pyridin-4-yl-imidazo[1,2-*b*]pyridazin-6-yl]-octahydro-(1H)pyrrolo[1,2-*d*][1,4]diazépine ;
(+/-)-4-[2-(4-Fluoro-phényl)-3-pyridin-4-yl-imidazo[1,2-*b*]pyridazin-6-yl]-1,4-diaza-bicyclo[3.2.2]nonane ;
(+/-)-4-[2-(3,5-Diméthyl-phényl)-3-pyridin-4-yl-imidazo[1,2-*b*]pyridazin-6-yl]-1,4-diaza-bicyclo[3.2.2]nonane ;
(+/-)-3,6-diaza-bicyclo[3.2.0]hept-3-yl-2-phényl-3-pyridin-4-yl-imidazo[1,2-b]pyridazine ;
(+/-)-3,6-(Hexahydro-pyrrolo[3,4-*b*]pyrrol-5(1H)-yl)-2-phényl-3-pyridin-4-yl-imidazo[1,2-*b*]pyridazine ;
(+/-)-6-(Hexahydro-pyrrolo[3,4-*b*]pyrrol-5(1*H*)-yl)-3-(2-méthyl-7pyridin-4-yl)-2-phényl-imidazo[1,2-*b*]pyridazine ;
6-Hexahydro-pyrrolo[3,4-*c*]pyrrol-2(1*H*)-yl-2-phényl-3-pyridin-4-yl-imidazo[1,2-b]pyridazine ;
6-Hexahydro-pyrrolo[3,4-*c*]pyrrol-2(1*H*)-yl-3-(2-méthyl-pyridin-4-yl)-2-phényl-imidazo[1,2-*b*]pyridazine ;
2-(3-Fluoro-phényl)-6-hexahydro-pyrrolo[3,4-*c*]pyrrol-2(1*H*)-yl-3-(2-méthyl-pyridin-4-yl)-imidazo[1,2-*b*]pyridazine ;
2-(4-Fluoro-phényl)-6-hexahydro-pyrrolo[3,4-*c*]pyrrol-2(1*H*)-yl-3-pyridin-4-yl-imidazo[1,2-*b*]pyridazine ;
2-(4-Fluoro-phényl)-6-hexahydro-pyrrolo[3,4-*c*]pyrrol-2(1*H*)-yl-3-(2-méthyl-pyridin-4-yl)-imidazo[1,2-*b*]pyridazine ;
4-[2-(4-Chloro-phényl)-6-(hexahydro-pyrrolo[3,4-*c*]pyrrol-2(1*H*)-yl)-imidazo[1,2-*b*]pyridazin-3-yl]-pyridin-2-ylamine ;
2-(4-Fluoro-phényl)-6-(hexahydro-pyrrolo[3,4-*c*]pyrrol-2(1*H*)-yl)-3-(2-méthoxy-pyridin-4-yl)-imidazo[1,2-*b*]pyridazine ;
4-[6-(5-Benzyl-hexahydro-pyrrolo[3,4-*c*]pyrrol-2(1*H*)-yl)-7,8-diméthyl-2-(4-fluoro-phényl)-imidazo[1,2-*b*]pyridazin-3-yl]-pyridin-2-ylamine ;
4-[2-(4-Fluoro-phényl)-6-hexahydro-pyrrolo[3,4-*c*]pyrrol-2(1*H*)-yl-7,8-diméthyl-imidazo[1,2-*b*]pyridazin-3-yl]-pyridin-2-ylamine ;
6-(5-Cyclopropyl-hexahydro-pyrrolo[3,4-c]pyrrol-2(1*H*)-yl)-2-(4-fluoro-phényl)-3-pyridin-4-yl-imidazo[1,2-*b*]pyridazine ;
2-(4-Fluoro-phényl)-6-(5-isopropyl-hexahydro-pyrrolo[3,4-c]pyrrol-2(1*H*)-yl)-3-pyridin-4-yl-imidazo[1,2-*b*]pyridazine ;
2-(4-Fluoro-phényl)-6-(5-isopropyl-hexahydro-pyrrolo[3,4-*c*]pyrrol-2-(1*H*)yl)-3-(2-méthyl-pyridin-4-yl)-imidazo[1,2-*b*]pyridazine ;
2-(4-Fluoro-phényl)-6-(5-isopropyl-hexahydro-pyrrolo[3,4-c]pyrrol-2(1*H*)-yl)-3-(2-méthoxy-pyridin-4-yl)-imidazo[1,2-*b*]pyridazine
(+/-)-(*cis*)-6-(Octahydro-6*H*-pyrrolo[3,4-*b*]pyridin-6-yl)-2-phényl-3-pyridin-4-yl-imidazo[1,2-*b*]pyridazine ;
(+/-)-(*cis*)-3-(2-Méthyl-pyridin-4-yl)-6-(octahydro-6*H*-pyrrolo[3,4-*b*]pyridin-6-yl)-2-phényl-imidazo[1,2-*b*]pyridazine ;
(+/-)-(*cis*)-2-[2-(4-Fluoro-phényl)-3-pyridin-4-yl-imidazo[1,2-*b*]pyridazin-6-yl]-décahydro-[2,6]naphthyridine ;
(+/-)-6-(2,7-Diaza-spiro[4.4]non-2-yl)-2-(3-fluoro-phényl)-3-pyridin-4-yl-imidazo[1,2-*b*]pyridazine ;
(+/-)-6-(2,7-Diaza-spiro[4.4]non-2-yl)-2-(4-fluoro-phényl)-3-pyridin-4-yl-imidazo[1,2-*b*]pyridazine ;
(+/-)-6-(2,8-Diaza-spiro[4.5]déc-2-yl)-2-(4-fluoro-phényl)-3-pyridin-4-yl-imidazo[1,2-*b*]pyridazine ;
(+/-)-6-(2,7-Diaza-spiro[4.5]déc-2-yl)-2-(4-fluoro-phényl)-3-pyridin-4-yl-imidazo[1,2-*b*]pyridazine ;
(+/-)-6-(2,8-Diaza-spiro[4.5]déc-8-yl)-2-(4-fluoro-phényl)-3-pyridin-4-yl-imidazo[1,2-*b*]pyridazine ;
(+/-)-6-(2,7-Diaza-spiro[4.5]déc-7-yl)-2-(4-fluoro-phényl)-3-pyridin-4-yl-imidazo[1,2-*b*]pyridazine ;
3-[2-(4-Fluoro-phényl)-3-pyridin-4-yl-imidazo[1,2-*b*]pyridazin-6-yl]-3,9-diaza-spiro[5.5]undécane ;
9-(2-Phényl-3-pyridin-4-yl-imidazo[1,2-*b*]pyridazin-6-yl)-2,9-diaza-spiro[5.5]undécane ;
9-[3-(2-Méthyl-pyridin-4-yl)-2-phényl-imidazo[1,2-*b*]pyridazin-6-yl]-2,9-diaza-spiro[5.5]undécane ;
9-[2-(3-Fluoro-phényl)-3-(2-méthyl-pyridin-4-yl)-imidazo[1,2-*b*]pyridazin-6-yl]-2,9-diaza-spiro[5.5]undécane ;
9-[2-(4-Fluoro-phényl)-3-pyridin-4-yl-imidazo[1,2-*b*]pyridazin-6-yl]-2,9-diaza-spiro[5.5]undécane ;
9-[2-(4-Fluoro-phényl)-3-(2-méthyl-pyridin-4-yl)-imidazo[1,2-*b*]pyridazin-6-yl]-2,9-diaza-spiro[5.5]undécane ;
4-[2-(4-Chloro-phényl)-6-(2,9-diaza-spiro[5.5]undéc-9-yl)-imidazo[1,2-*b*]pyridazin-3-yl]-pyridin-2-ylamine ;
9-[2-(4-Fluoro-phényl)-3-(2-méthoxy-pyridin-4-yl)-imidazo[1,2-*b*]pyridazin-6-yl]-2,9-diaza-spiro[5.5]undécane ;
2-[2-(4-Fluoro-phényl)-3-(pyridin-4-yl)-imidazo[1,2-*b*]pyridazin-6-yl]-2,9-diaza-spiro[5.5]undécane
2-[2-(4-Fluoro-phényl)-3-(pyridin-4-yl)-imidazo[1,2-*b*]pyridazin-6-yl]-2,8-diaza-spiro[5.5]undécane
9-[2-(phényl)-3-(pyridin-4-yl)-imidazo[1,2-*b*]pyridazin-6-yl]-1-oxa-4,9-diaza-spiro[5.5]undécane ;
9-[2-(4-Fluoro-phényl)-3-(pyridin-4-yl)-imidazo[1,2-*b*]pyridazin-6-yl]-1-oxa-4,9-diaza-spiro[5.5]undécane ;
9-[2-(4-Fluoro-phényl)-3-(2-méthoxy-pyridin-4-yl)-imidazo[1,2-*b*]pyridazin-6-yl]-1-oxa-4,9-diaza-spiro[5.5]undécane ;
4-{[2-(4-Chloro-phényl)-3-(2-méthoxy-pyridin-4-yl)-imidazo[1,2-*b*]pyridazin-6-yl]-1-oxa-4,9-diaza-spiro[5.5]undéc-9-yl}-2-ylamine ;
(+/-)-{1-[2-(4-Fluoro-phényl)-3-pyridin-4-yl-imidazo[1,2-*b*]pyridazin-6-yl]-pyrrolidin-3-yl}-diméthyl-amine ;
2-(3,5-Diméthyl-phényl)-3-pyridin-4-yl-6-(4-pyrrolidin-1-yl-pipéridin-1-yl)-imidazo[1,2-*b*]pyridazine ;
6-(4-Morpholin-4-yl-pipéridin-1-yl)-2-phényl-3-pyridin-4-yl-imidazo[1,2-*b*]pyridazine ;
6-[4-(2,6-Diméthyl-morpholin-4-yl)-pipéridin-1-yl]-2-phényl-3-pyridin-4-yl-imidazo[1,2-*b*]pyridazine ;
{1-[2-(4-Fluoro-phényl)-3-pyridin-4-yl-imidazo[1,2-*b*]pyridazin-6-yl]-pipéridin-4-yl}-diméthyl-amine ;
2-Phényl-3-pyridin-4-yl-6-(4-pyrrolidin-1-yl-pipéridin-1-yl)-imidazo[1,2-*b*]pyridazine 2-(4-Fluoro-phényl)-3-pyridin-4-yl-6-(4-pyrrolidin-1-yl-pipéridin-1-yl)-imidazo[1,2-*b*]pyridazine ;
2-(4-Fluoro-phényl)-3-(2-méthoxy-pyridin-4-yl)-6-(4-pyrrolidin-1-yl-pipéridin-1-yl)-imidazo[1,2-*b*]pyridazine ;
(*R*)-1-{1-[2-(4-Fluoro-phényl)-3-pyridin-4-yl-imidazo[1,2-*b*]pyridazin-6-yl]-pipéridin-4-yl}-pyrrolidin-3-ol ;
6-(4-Morpholin-4-yl-pipéridin-1-yl)-2-phényl-3-pyridin-4-yl-imidazo[1,2-*b*]pyridazine ;
2-(3-Fluoro-5-méthyl-phényl)-6-((*R*)-3-méthyl-pipérazin-1-yl)-3-pyridin-4-yl-imidazo[1,2-b]pyridazine ;
{4-[2-(4-Fluoro-phényl)-6-((*R*)-3-méthyl-pipérazin-1-yl)-imidazo[1,2-*b*]pyridazin-3-yl]-pyridin-2-yl}-méthyl-amine ;
6-(3,3-Diméthyl-pipérazin-1-yl)-2-(4-fluoro-phényl)-7-méthyl-3-pyridin-4-yl-imidazo[1,2-*b*]pyridazine ;
6-(3,3-Diméthyl-pipérazin-1-yl)-2-(4-fluoro-phényl)-8-méthyl-3-pyridin-4-yl-imidazo[1,2-*b*]pyridazine ;
6-(3,3-Diméthyl-pipérazin-1-yl)-2-(3-fluoro-5-méthyl-phényl)-3-pyridin-4-yl-imidazo[1,2-*b*]pyridazine ;
{4-[6-(3,3-Diméthyl-pipérazin-1-yl)-2-(4-fluoro-phényl)-imidazo[1,2-*b*]pyridazin-3-yl]-pyridin-2-yl}-méthyl-amine ;
6-((*R*)-3-Éthyl-pipérazin-1-yl)-2-(4-fluorophényl)-3-pyridin-4-yl-imidazo[1,2-*b*]pyridazine ;
6-(3,3-Diéthyl-pipérazin-1-yl)-2-(4-fluoro-phényl)-3-pyridin-4-yl-imidazo[1,2-*b*]pyridazine ;
(+/-)-6-(3-Fluorométhyl-pipérazin-1-yl)-2-(4-fluoro-phényl)-3-pyridin-4-yl-imidazo[1,2-*b*]pyridazine ;
(+/-)-{4-[2-(4-Fluoro-phényl)-3-pyridin-4-yl-imidazo[1,2-*b*]pyridazin-6-yl]-pipérazin-2-yl}-methanol ;
2-{4-[2-(3-Fluoro-5-méthyl-phényl)-3-pyridin-4-yl-imidazo[1,2-*b*]pyridazin-6-yl]-pipérazin-1-yl}-éthanol ;
2-{4-[2-(4-Fluoro-phényl)-3-(2-méthyl-amino-pyridin-4-yl)-imidazo[1,2-*b*]pyridazin-6-yl]-pipérazin-1-yl}-ethanol ;
1-{4-[2-(4-Fluoro-phényl)-3-pyridin-4-yl-imidazo[1,2-*b*]pyridazin-6-yl]-pipérazin-1-yl}-2-méthyl-propan-2-ol ;
1-{4-[2-(4-Fluoro-phényl)-8-méthyl-3-pyridin-4-yl-imidazo[1,2-*b*]pyridazin-6-yl]-pipérazin-1-yl}-2-méthyl-propan-2-ol ;
1-{4-[2-(4-Fluoro-phényl)-7-méthyl-3-pyridin-4-yl-imidazo[1,2-*b*]pyridazin-6-yl]-pipérazin-1-yl}-2-méthyl-propan-2-ol ;
1-{4-[2-(4-Fluoro-phényl)-3-(2-méthyl-amino-pyridin-4-yl)-imidazo[1,2-*b*]pyridazin-6-yl]-pipérazin-1-yl}-2-méthyl-propan-2-ol ;
2-{(*R*)-4-[2-(4-Fluoro-phényl)-3-pyridin-4-yl-imidazo[1,2-*b*]pyridazin-6-yl]-pipérazin-2-yl}-propan-2-ol ;
2-(4-Fluoro-phényl)-6-((*R*)-3-isopropyl-pipérazin-1-yl)-3-pyridin-4-yl-imidazo[1,2-*b*]pyridazine ;
{4-[2-(4-Fluoro-phényl)-6-((*R*)-3-isopropyl-pipérazin-1-yl)-imidazo[1,2-*b*]pyridazin-3-yl]-pyridin-2-yl}-méthyl-amine ;
2-(3-Fluoro-phényl)-6-(4-isopropyl-pipérazin-1-yl)-3-(2-méthoxy-pyridin-4-yl)-imidazo[1,2-*b*]pyridazine ;
2-(4-Fluoro-phényl)-6-(4-isopropyl-pipérazin-1-yl)-7-méthyl-3-pyridin-4-yl-imidazo[1,2-*b*]pyridazine ;
2-(4-Fluoro-phényl)-6-(4-isopropyl-pipérazin-1-yl)-8-méthyl-3-pyridin-4-yl-imidazo[1,2-*b*]pyridazine ;
{4-[2-(4-Fluoro-phényl)-6-(4-isopropyl-pipérazin-1-yl)-imidazo[1,2-*b*]pyridazin-3-yl]-pyridin-2-yl}-méthyl-amine ;
2-(3,4-Difluoro-phényl)-6-(4-isopropyl-pipérazin-1-yl)-3-(2-méthoxy-pyridin-4-yl)-imidazo[1,2-*b*]pyridazine ;
2-(3-Fluoro-5-méthyl-phényl)-6-(4-isopropyl-pipérazin-1-yl)-3-pyridin-4-yl-imidazo[1,2-*b*]pyridazine ;
(+/-)-6-(3,6-Diaza-bicyclo[3.2.0]hept-3-yl)-2-phényl-3-pyridin-4-yl-imidazo[1,2-*b*]pyridazine ;
6-(1*S*,4*S*)-2,5-Diaza-bicyclo[2.2.1]hept-2-yl-2-phényl-3-pyridin-4-yl-imidazo[1,2-*b*]pyridazine ;
{4-[(1*S*,4*S*)-6-2,5-Diaza-bicyclo[2.2.1]hept-2-yl-2-(4-fluoro-phényl)-imidazo[1,2-*b*]pyridazin-3-yl]-pyridin-2-yl}-méthyl-amine ;
2-(4-Fluoro-phényl)-6-((1*S*,4*S*)-5-méthyl-2,5-diaza-bicyclo[2.2.1]hept-2-yl)-3-pyridin-4-yl-imidazo[1,2-*b*]pyridazine ;
2-(4-Fluoro-phényl)-7-méthyl-6-((1*S*,4*S*)-5-méthyl-2,5-diaza-bicyclo[2,2.1]hept-2-yl)-3-pyridin-4-yl-imidazo[1,2-*b*]pyridazine ;
2-(4-Fluoro-phényl)-8-méthyl-6-((1*S*,4*S*)-5-méthyl-2,5-diaza-bicyclo[2.2.1]hept-2-yl)-3-pyridin-4-yl-imidazo[1,2-*b*]pyridazine ;
2-(4-Fluoro-phényl)-6-((1*S*,4*S*)-5-méthyl-2,5-diaza-bicyclo[2.2.1]hept-2-yl)-3-(2-méthyl-pyridin-4-yl)-imidazo[1,2-*b*]pyridazine ;
2-Méthyl-1-[(1*S*,4*S*)-5-(2-phényl-3-pyridin-4-yl-imidazo[1,2-*b*]pyridazin-6-yl)-2,5-diaza-bicyclo[2.2.1]hept-2-yl]-propan-2-ol ;
6-(3,6-Diaza-bicyclo[3.1.1]hept-3-yl)-2-(4-fluoro-phényl)-3-pyridin-4-yl-imidazo[1,2-*b*]pyridazine ;
2-(4-Fluoro-phényl)-6-(5-méthyl-hexahydro-pyrrolo[3,4-*c*]pyrrol-2(1*H*)-yl)-3-pyridin-4-yl-imidazo[1,2-b]pyridazine ;
2-(4-Fluoro-phényl)-7-méthyl-6-(5-méthyl-hexahydro-pyrrolo[3,4-*c*]pyrrol-2(1*H*)-yl)-3-pyridin-4-yl-imidazo[1,2-*b*]pyridazine ;
2-(4-Fluoro-phényl)-8-méthyl-6-(5-méthyl-hexahydro-pyrrolo[3,4-*c*]pyrrol-2(1*H*)-yl)-3-pyridin-4-yl-imidazo[1,2-*b*]pyridazine ;
2-(4-Fluoro-phényl)-8-méthyl-6-(-5-méthyl-hexahydro-pyrrolo[3,4-*c*]pyrrol-2(1*H*)-yl)-3-(2-méthyl-pyridin-4-yl)-imidazo[1,2-*b*]pyridazine ;
{4-[2-(4-Fluoro-phényl)-6-(5-méthyl-hexahydro-pyrrolo[3,4-*c*]pyrrol-2(1*H*)-yl)-imidazo[1,2-*b*]pyridazin-3-yl]-pyridin-2-yl}-méthyl-amine ;
2-(4-Fluoro-phényl)-3-(2-méthoxy-pyridin-4-yl)-6-(5-méthyl-hexahydro-pyrrolo[3,4-*c*]pyrrol-2(1*H*)-yl)-imidazo[1,2-*b*]pyridazine ;
2-(4-Fluoro-phényl)-6-(5-méthyl-hexahydro-pyrrolo[3,4-*c*]pyrrol-2(1*H*)-yl)-3-(2-méthyl-pyridin-4-yl)-imidazo[1,2-*b*]pyridazine ;
{4-[2-(4-Fluoro-phényl)-8-méthyl-6-(-5-méthyl-hexahydro-pyrrolo[3,4-*c*]pyrrol-2(1*H*)-yl)-imidazo[1,2-*b*]pyridazin-3-yl]-pyridin-2-yl}-méthyl-amine ;
{4-[2-(4-Fluoro-phényl)-7-méthyl-6-(5-méthyl-hexahydro-pyrrolo[3,4-*c*]pyrrol-2(1*H*)-yl)-imidazo[1,2-*b*]pyridazin-3-yl]-pyridin-2-yl}-méthyl-amine ;
(+/-)-2-(4-Fluoro-phényl)-6-(hexahydro-pyrrolo[3,4-*b*]pyrrol-5(1*H*)-yl)-3-pyridin-4-yl-imidazo[1,2-*b*]pyridazine ;
(+)-2-(4-Fluoro-phényl)-6-hexahydro-pyrrolo[3,4-*b*]pyrrol-5(1*H*)-yl-3-pyridin-4-yl-imidazo[1,2-*b*]pyridazine ;
(-)-2-(4-Fluoro-phényl)-6-hexahydro-pyrrolo[3,4-*b*]pyrrol-5(1*H*)-yl-3-pyridin-4-yl-imidazo[1,2-*b*]pyridazine ;
(+/-)-{4-[2-(4-Fluoro-phényl)-6-(hexahydro-pyrrolo[3,4-*b*]pyrrol-5(1*H*)-yl)-imidazo[1,2-*b*]pyridazin-3-yl]-pyridin-2-yl}-méthyl-amine ;
6-(4a*S*,7a*S*)-octahydro-pyrrolo[3,4-*b*]pyridin-6-yl-2-phényl-3-pyridin-4-yl-imidazo[1,2-*b*]pyridazine ;
3-(2-Méthyl-pyridin-4-yl)-6-(4a*S*,7a*S*)-octahydro-pyrrolo[3,4-*b*]pyridin-6-yl-2-phényl-imidazo[1,2-*b*]pyridazine ;
3-(2-Méthyl-pyridin-4-yl)-6-(4a*R*,7a*R*)-octahydro-pyrrolo[3,4-*b*]pyridin-6-yl-2-phényl-imidazo[1,2-*b*]pyridazine ;
2-(4-Fluoro-phényl)-8-méthyl-6-(4a*S*,7a*S*)-octahydro-pyrrolo[3,4-*b*]pyridin-6-yl-3-pyridin-4-yl-imidazo[1,2-*b*]pyridazine ;
2-(4-Fluoro-phényl)-7-méthyl-6-(4a*S*,7a*S*)-octahydro-pyrrolo[3,4-*b*]pyridin-6-yl-3-pyridin-4-yl-imidazo[1,2-*b*]pyridazine ;
2-(4-Fluoro-phényl)-7-méthyl-6-(4a*R*,7a*R*)-octahydro-pyrrolo[3,4-*b*]pyridin-6-yl-3-pyridin-4-yl-imidazo[1,2-*b*]pyridazine ;
2-(4-Fluoro-phényl)-6-(4a*S*,7a*S*)-octahydro-pyrrolo[3,4-*b*]pyridin-6-yl-3-pyridin-4-yl-imidazo[1,2-*b*]pyridazine ;
2-(4-Fluoro-phényl)-3-(2-méthyl-pyridin-4-yl)-6-(4a*S*,7a*S*)-octahydro-pyrrolo[3,4-*b*]pyridin-6-yl-imidazo[1,2-*b*]pyridazine ;
{4-[2-(4-Fluoro-phényl)-6-(octahydro-pyrrolo[3,4-*b*]pyridin-6-yl)-imidazo[1,2-*b*]pyridazin-3-yl]-pyridin-2-yl}-méthyl-amine ;
6-(4a*R*,7a*R*)-Octahydro-pyrrolo[3,4-*b*]pyridin-6-yl-2-phényl-3-pyridin-4-yl-imidazo[1,2-*b*]pyridazine ;
2-(4-Fluoro-phényl)-6-(4a*R*,7a*R*)-octahydro-pyrrolo[3,4-*b*]pyridin-6-yl-3-pyridin-4-yl-imidazo[1,2-*b*]pyridazine ;
2-(4-Fluoro-phényl)-3-(2-méthyl-pyridin-4-yl)-6-(4a*R*,7a*R*)-octahydro-pyrrolo[3,4-*b*]pyridin-6-yl-imidazo[1,2-*b*]pyridazine ;
2-(4-Fluoro-phényl)-8-méthyl-6-(4a*R*,7a*R*)-octahydro-pyrrolo[3,4-*b*]pyridin-6-yl-3-pyridin-4-yl-imidazo[1,2-*b*]pyridazine ;
6-(2,7-Diaza-spiro[4.4]non-2-yl)-2-(4-fluoro-phényl)-8-méthyl-3-pyridin-4-yl-imidazo[1,2-*b*]pyridazine ;
6-(6,9-Diaza-spiro[4.5]déc-9-yl)-2-(4-fluoro-phényl)-3-pyridin-4-yl-imidazo[1,2-*b*]pyridazine ;
(+/-)-2-[2-(4-Fluoro-phényl)-8-méthyl-3-pyridin-4-yl-imidazo[1,2-*b*]pyridazin-6-yl]-2,8-diaza-spiro[5.5]undécane ;
9-[2-(4-Fluoro-phényl)-8-méthyl-3-pyridin-4-yl-imidazo[1,2-*b*]pyridazin-6-yl]-2,9-diaza-spiro[5.5]undécane ;
{4-[6-(2,9-Diaza-spiro[5.5]undéc-9-yl)-2-(4-fluoro-phényl)-imidazo[1,2-*b*]pyridazin-3-yl]-pyridin-2-yl}-méthyl-amine ;
9-[2-(3,4-Difluoro-phényl)-3-pyridin-4-yl-imidazo[1,2-*b*]pyridazin-6-yl]-2,9-diaza-spiro[5.5]undécane ;
9-[2-(3-Fluoro-5-méthyl-phényl)-3-pyridin-4-yl-imidazo[1,2-*b*]pyridazin-6-yl]-2,9-diaza-spiro[5.5]undécane ;
2-(3-Fluoro-phényl)-3-pyridin-4-yl-6-(4-pyrrolidin-1-yl-pipéridin-1-yl)-imidazo[1,2-, *b*]pyridazine ;
{4-[2-(4-Fluoro-phényl)-6-(4-pyrrolidin-1-yl-pipéridin-1-yl)-imidazo[1,2-*b*]pyridazin-3-yl]-pyridin-2-yl}-méthyl-amine ;
2-(4-Fluoro-phényl)-6-[4-((*R*)-3-fluoro-pyrrolidin-1-yl)-pipéridin-1-yl]-3-pyridin-4-yl-imidazo[1,2-*b*]pyridazine.;
(*R*)-1-[1-(2-Phényl-3-pyridin4-yl-imidazo[1,2-*b*]pyridazin-6-yl)-pipéridin-4-yl]-pyrrolidin-3-ol ;
(*R*)-1-{1-[2-(3-Fluoro-5-méthyl-phényl)-3-pyridin-4-yl-imidazo[1,2-*b*]pyridazin-yl]-pipéridin-4-yl}-pyrrolidin-3-ol.

Le tableau 1 qui suit illustre les structures chimiques et les propriétés physiques de quelques composés selon l'invention.

Dans ce tableau :
- la colonne "PF°C" renseigne les points de fusion des produits en degrés Celsius. "N.D" signifie que le point de fusion est non déterminé,
- dans la colonne "PF°C", « HCl » ou « CF₃COOH» représente un composé sous forme de chlorhydrate ou de trifluoroacétate et le rapport entre parenthèses est le rapport (acide:base),
- la colonne "LC-MS ou (MS)" renseigne le résultat d'analyse des produits par LC-MS (liquid chromatography coupled to Mass Spectroscopy) réalisée sur un appareil Agilent LC-MSD Trap en mode ESI positif ou par MS (Mass Spectroscopy) sur un appareil Autospec M (EBE) en utilisant la technique DCI-NH3.
- « décomp » signifie que le composé présente une décomposition ;
- « CH₃- » siginifie méthyle ;
- « H₃O- » signifie Méthoxy ;
- « NH₂- » signife amino ;
- « CH₃NH- » signifie méthyl-amino ;
- « (CH₃)₂N- » signifie diméthyl-amino ;

**TABLEAU 1**

| **N°** | **-N-A-L-B-** | **R₇** | **R₈** | **R₂** | **R₃** | PF **°C** | M+H |
|---|---|---|---|---|---|---|---|
| 1 | Pipérazin-1-yl | H | H | Phényl | H | N.D | 357 |
| 2 | Pipérazin-1-yl | H | H | 4-Fluorophényl | H | 240-242 | 375 |
| 3 | Pipérazin-1-yl | CH₃- | CH₃- | 4-Fluorophényl | H | 233-235 | 403 |
| 4 | Pipérazin-1-yl | H | H | 4-Fluorophényl | NH₂- | 255 décomp. | 390 |
| 5 | Pipérazin-1-yl | H | H | 4-Fluorophényl | CH₃O- | 227-229 | 405 |
| 6 | Pipérazin-1-yl | H | H | 4-Chlorophényl | H | 238-240 | 391 |
| 7 | Pipérazin-1-yl | CH₃- | H | 4-Chlorophényl | H | 180-182 | 405 |
| 8 | Pipérazin-1-yl | H | CH₃- | 4-Chlorophényl | H | 216-218 | 405 |
| 9 | Pipérazin-1-yl | H | H | 3,5-Diméthylphényl | H | N.D | 385 |
| 10 | Pipérazin-1-yl | H | H | 3,5-Difluorophényl | H | N.D | 393 |
| 11 | Pipérazin-1-yl | H | H | 3,5-Difluorophényl | CH₃- | N.D | 407 |
| 12 | Pipérazin-1-yl | H | H | 3,4-Difluorophényl | H | 183-185 | 393 |
| 13 | Pipérazin-1-yl | H | H | 3,5-Dichlorophényl | H | N.D | 425 |
| 14 | Pipérazin-1-yl | H | H | 3,5-Dichlorophényl | CH₃- | N.D | 439 |
| 15 | (+/-)-3-Méthylpipérazin-1-yl | H | H | Phényl | H | N.D | 371 |
| 16 | (*R*)-3-Méthylpipérazin-1-yl | H | H | Phényl | H | N.D | 371 |
| 17 | (*R*)-3-Méthylpipérazin-1-yl | H | H | Phényl | CH₃- | N.D | 385 |
| 18 | (*S*)-3-Méthylpipérazin-1-yl | H | H | Phényl | H | N.D | 371 |
| 19 | (*R*)-3-Méthylpipérazin-1-yl | H | H | 3-Fluorophényl | H | N.D | 389 |
| 20 | (+/-)-3-Méthylpipérazin-1-yl | H | H | 4-Fluorophényl | H | 197-199 | 389 |
| 21 | (*R*)-3-Méthylpipérazin-1-yl | H | H | 4-Fluorophényl | H | N.D | 389 |
| 22 | (*S*)-3-Méthylpipérazin-1-yl | H | H | 4-Fluorophényl | H | N.D | 389 |
| 23 | (*R*)-3-Méthylpipérazin-1-yl | H | H | 4-Fluorophényl | CH₃O- | 215-218 | 419 |
| 24 | (*R*)-3-Méthylpipérazin-1-yl | H | H | 3,4-Difluorophényl | H | N.D | 407 |
| 25 | 3,3-Diméthylpipérazin-1-yl | H | H | Phényl | H | N.D | 385 |
| 26 | 3,3-Diméthylpipérazin-1-yl | H | H | 4-Fluorophényl | H | 232-235 HCl (3:1) | 403 |
| 27 | 3,3-Diméthylpipérazin-1-yl | H | H | 4-Fluorophényl | CH₃O- | 167-170 | 433 |
| 28 | 3,3-Diméthylpipérazin-1-yl | H | H | 3,5-Diméthylphényl | H | N.D | 413 |
| 29 | 3,3-Diméthylpipérazin-1-yl | H | H | 3,5-Diméthylphényl | CH₃- | N.D | 427 |
| 30 | 3,3-Diméthylpipérazin-1-yl | H | H | 3,5-Diméthylphényl | NH₂- | N.D | 428 |
| 31 | 3,3-Diméthylpipérazin-1-yl | H | H | 3,5-Difluorophényl | H | N.D | 421 |
| 32 | 3,3-Diméthylpipérazin-1-yl | H | H | 3,5-Difluorophényl | Me | N.D | 435 |
| 33 | 3,3-Diméthylpipérazin-1-yl | H | H | 3,5-Difluorophényl | NH₂- | N.D | 436 |
| 34 | 3,3-Diméthylpipérazin-1-yl | H | H | 3,5-Dichlorophényl | H | N.D | 453 |
| 35 | 3,3-Diméthylpipérazin-1-yl | H | H | 3,5-Dichlorophényl | CH₃- | N.D | 467 |
| 36 | (+/-)-3,4-Diméthylpipérazin-1-yl | H | H | 4-Fluorophényl | H | 254-256 | 403 |
| 37 | cis-3,5-Diméthylpipérazin-1-yl | H | H | Phényl | H | N.D | 385 |
| 38 | cis-3,5-Diméthylpipérazin-1-yl | H | H | Phényl | CH₃- | N.D | 399 |
| 39 | cis-3,5-Diméthylpipérazin-1-yl | H | H | 3-Fluorophényl | H | N.D | 403 |
| 40 | cis-3,5-Diméthylpipérazin-1-yl | H | H | 4-Fluorophényl | H | 231-233 | 403 |
| 41 | cis-3,5-Diméthylpipérazin-1-yl | H | H | 4-Fluorophényl | CH₃O- | 173-175 | 433 |
| 42 | cis-3,5-Diméthylpipérazin-1-yl | H | H | 3,5-Diméthylphényl | H | N.D | 413 |
| 43 | cis-3,5-Diméthylpipérazin-1-yl | H | H | 3,4-Difluorophényl | H | N.D | 421 |
| 44 | cis-3,5-Diméthylpipérazin-1-yl | CH₃- | H | 4-Chlorophényl | H | 230-232 | 433 |
| 45 | cis-3,5-Diméthylpipérazin-1-yl | H | CH₃- | 4-Flùorophényl | H | 235-237 | 417 |
| 46 | 4-Méthylpipérazin-1-yl | H | H | 2-Chlorophényl | H | 198-200 | 405 |
| 47 | 4-Méthylpipérazin-1-yl | H | H | 3-Chlorophényl | H | 195-197 | 405 |
| 48 | 4-Méthylpipérazin-1-yl | H | H | 4-Chlorophényl | H | 260-262 | 405 |
| 49 | 4-Méthylpipérazin-1-yl | H | H | 3-Fluorophényl | H | 205-206 | 389 |
| 50 | 4-Méthylpipérazin-1-yl | H | H | 4-Fluorophényl | H | 294-296 | 389 |
| | | | | | | 248-250 | |
| 51 | 4-Méthylpipérazin-1-yl | H | H | 3,4-Difluorophényl | H | 210 | 407 |
| 52 | 4-Méthylpipérazin-1-yl | H | H | 3-Méthoxyphényl | H | 131 | 401 |
| 53 | 4-Méthylpipérazin-1-yl | H | H | 4-Méthoxyphényl | H | 183-185 | 401 |
| 54 | 4-Méthylpipérazin-1-yl | H | H | 4-Méthylphényl | H | 205-206 | 385 |
| 55 | 4-Méthylpipérazin-1-yl | H | H | 4-Trifluoro-méthylphényl | H | 201-203 | 439 |
| 56 | 4-Méthylpipérazin-1-yl | H | H | 4-Fluorophényl | CH₃- | 258-260 | 403 |
| 57 | 4-Méthylpipérazin-1-yl | H | H | 4-Fluorophényl | NH₂- | 235-238 | 404 |
| 58 | 4-Méthylpipérazin-1-yl | H | H | 4-Fluorophényl | (CH₃)₂N- | 180-183 | 432 |
| 59 | 4-Méthylpipérazin-1-yl | H | H | 4-Fluorophényl | CH₃O- | 189-194 | 419 |
| 60 | 4-Éthylpipérazin-1-yl | H | H | 4-Fluorophényl | H | 208-210 | 403 |
| 61 | 4-Éthylpipérazin-1-yl | H | H | 3,5-Diméthylphényl | H | N.D | 413 |
| 62 | 4-(2-hydroxyéthyl)pipérazin-1-yl | H | H | Phényl | H | N.D | 401 |
| 63 | 4-(2-hydroxyéthyl)pipérazin-1-yl | H | H | 3-Fluorophényl | H | N.D | 419 |
| 64 | 4-(2-hydroxyéthyl)pipérazin-1-yl | H | H | 4-Fluorophényl | H | 203-206 | 419 |
| 65 | 4-(2-hydroxyéthyl)pipérazin-1-yl | H | H | 4-Fluoropliényl | CH₃O- | 176-179 | 449 |
| 66 | 4-(2-hydroxyéthyl)pipérazin-1-yl | H | H | 3,4-Difluorophényl | H | N.D | 437 |
| 67 | 4-(2-hydroxyéthyl)pipérazin-1-yl | H | H | 4-Chlorophényl | H | N.D | 435 |
| 68 | 4-(2-hydroxyéthyl)pipérazin-1-yl | H | H | 4-Chlorophényl | NH₂- | 191-193 | 450 |
| 69 | 4-(2-Méthoxyéthyl)pipérazin-1-yl | H | H | 4-Chlorophényl | H | N.D | 449 |
| 70 | 4-(2-Fluoroéthyl)pipérazin-1-yl | H | H | 4-Fluorophényl | H | 183-185 | 421 |
| 71 | 4-(2-Fluoroéthyl)pipérazin-1-yl | H | H | 4-Fluorophényl | CH₃O- | 149-151 | 451 |
| 72 | 4-(2,2,2-Trifluoro-éthyl)pipérazin-1-yl | H | H | Phényl | H | 188-190 | 439 |
| 73 | 4-(2,2,2-Trifluoro-éthyl)pipérazin-1-yl | H | H | 4-Fluorophényl | H | 188-191 | 457 |
| | | | | | | 184-186 | |
| 74 | 4-Cyclopropylpipérazin-1-yl | H | H | 4-Fluorophényl | H | 205-208 | 415 |
| 75 | 4-Cyclopropylpipérazin-1-yl | H | H | 4-Fluorophényl | CH₃O- | 194-196 | 445 |
| 76 | 4-Isopropylpipérazin-1-yl | H | H | Phényl | CH₃O- | 157-159 | 429 |
| 77 | 4-Isopropylpipérazin-1-yl | H | H | 3-Fluorophényl | H | N.D | 417 |
| 78 | 4-Isopropylpipérazin-1-yl | H | H | 4-Fluorophényl | H | 185-187 | 417 |
| 79 | 4-Isopropylpipérazin-1-yl | H | H | 4-Fluorophényl | CH₃O- | 153-156 | 447 |
| 80 | 4-Isopropylpipérazin-1-yl | H | H | 3,4-Difluorophényl | H | N.D | 435 |
| 81 | 4-Isopropylpipérazin-1-yl | H | H | 4-Chlorophényl | NH₂- | 236-239 | 448 |
| 82 | 4-(2-Hydroxy-2-méthyl-propyl)pipérazin-1-yl | H | H | Phényl | H | N.D | 429 |
| 83 | 4-(2-Hydroxy-2-méthyl-propyl)pipérazin-1-yl | H | H | 3-Fluorophényl | H | N.D | 447 |
| 84 | 4-(2-Hydroxy-2-méthyl-propyl)pipérazin-1-yl | H | H | 4-Fluorophényl | CH₃O- | 176-178 | 477 |
| 85 | 4-n-Butylpipérazin-1-yl | H | H | 4-Chlorophényl | H | N.D | 447 |
| 86 | 4-(3-Hydroxy-3-méthyl-butyl)pipérazin-1-yl | H | H | 4-Fluorophényl | CH₃O- | 151-154 | 491 |
| 87 | 4-Cyclohexylpipérazin-1-yl | H | H | 4-Fluorophényl | H | 197-199 | 457 |
| 88 | 4-Acétyl-pipérazin-1-yl | H | H | 4-Fluorophényl | H | 231-233 | 417 |
| 89 | 4-Isobutyryl-pipérazin-1-yl | H | H | 4-Fluorophényl | H | 197-200 | 445 |
| 90 | (+/-)-Hexahydro-pyrrolo[1,2-a]pyrazin-2-yl | H | H | 4-Fluorophényl | H | 223-225 | 415 |
| | 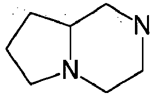 | | | | | | |
| 91 | (*S*)-Hexahydro-pyrrolo[1,2-*a*]pyrazin-2-yl | H | H | 4-Fluorophényl | CH₃O- | 187-189 | 445 |
| | 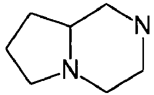 | | | | | | |
| 92 | (1*S*,4*S*)-2,5-diaza-bicyclo[2.2.1]hept-2-yl | H | H | Phényl | H | N.D | 369 |
| | 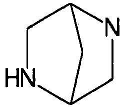 | | | | | | |
| 93 | (1*S*,4*S*)-5-Benzyl-2,5-diaza-bicyclo[2.2.1]hept-2-yl | H | H | 3-Fluorophényl | H | N.D | 477 |
| 94 | (1*S*,4*S*)-2,5-diaza-bicyclo[2.2.1]hept-2-yl | H | H | 4-Fluorophényl | H | 192-196 | 387 |
| 95 | (1*S*,4*S*)-5-Benzyl-2,5-diaza-bicyclo[2.2.1]hept-2-yl | H | H | 4-Fluorophényl | H | 182-184 | 477 |
| 96 | (1*S*,4*S*)-2,5-diaza-bicyclo[2.2.1]hept-2-yl | H | H | 4-Fluorophényl | CH₃O- | 164-167 | 417 |
| 97 | (1*S*,4*S*)-5-(2-Hydroxy-éthyl)-2,5-diaza-bicyclo[2.2.1]hept-2-yl | H | H | 4-Fluorophényl | CH₃O- | 154-156 | 461 |
| 98 | 5-Isopropyl-2,5-diaza-bicyclo[2.2.1]hept-2-yl | H | H | 4-Fluorophényl | CH₃O- | 204-206 | 459 |
| 99 | 4-Méthyl-[1,4]diazépan-1-yl | H | H | 4-Fluorophényl | H | 138-140 | 403 |
| 100 | (+/-)-Octahydro-1*H*-pyrrolo[1,2-*d*][1,4]diazépin-3-yl | H | H | 4-Fluorophényl | H | 166-168 | 429 |
| | 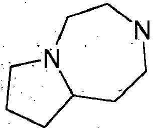 | | | | | | |
| 101 | (+/-)-1,4-Diaza-bicyclo[3.2.2]non-4-yl | H | H | 4-Fluorophényl | H | 286-288 | 415 |
| | 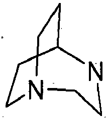 | | | | | | |
| 102 | (+/-)-1,4-Diaza-bicyclo[3,2.2]non-4-yl | H | H | 3,5-Diméthylphényl | H | N.D | 425 |
| 103 | (+/-)-3,6-diaza-bicyclo[3.2.0]hept-3-yl | H | H | Phénol | H | 212-218 | 370 |
| | 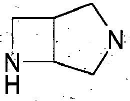 | | | | | | |
| 104 | (+/-)-Hexahydro-pyrrolo[3,4-*b*]pyrrol-5(1*H*)-yl | H | H | Phényl | H | 181-183 | 383 |
| | 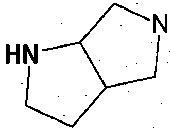 | | | | | | |
| 105 | Hexahydro-pyrrolo[3,4-*b*]pyrrol-5(1*H*)-yl | H | H | Phényl | CH₃- | 170-172 | 397 |
| 106 | Hexahydro-pyrrolo[3,4-*c*]pyrrol-2(1*H*)-yl | H | H | Phényl | H | 199-201 | 383 |
| | 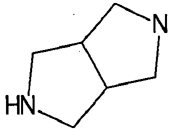 | | | | | | |
| 107 | Hexahydro-pyrrolo[3,4-*c*]pyrrol-2(1*H*)-yl | H | H | Phényl | CH₃- | 214-217 | 397 |
| 108 | Hexahydro-pyrrolo[3,4-*c*]pyrrol-2(1*H*)-yl | H | H | 3-Fluorophényl | CH₃- | 217-219 | 415 |
| 109 | Hexahydro-pyrrolo[3,4-*c*]pyrrol-2(1*H*)-yl | H | H | 4-Flyorophényl | H | 246-250 | 401 |
| | | | | | | 195-198 | |
| 110 | Hexahydro-pyrrolo[3,4-*c*]pyrrol-2(1*H*)-yl | H | H | 4-Fluorophényl | CH₃- | 200-202 | 415 |
| 111 | Hexahydro-pyrrolo[3,4-c]pyrrol-2(1*H*)-yl | H | H | 4-Chlorophényl | NH₂- | 267-270 | 432 |
| 112 | Hexahydro-pyrrolo[3,4-c]pyrrol-2(1*H*)-yl | H | H | 4-Fluorophényl | CH₃O- | 126-129 | 431 |
| 113 | 5-Benzyl-hexahydro-pyrrolo[3,4-*c*]pyrrol-2(1*H*)-yl | CH₃- | CH₃- | 4-Fluorophényl | NH₂- | 113 | |
| 114 | Hexahydro-pyrrolo[3,4-c]pyrrol-2(1*H*)-yl | CH₃- | CH₃- | 4-Fluorophényl | NH₂- | 254-256 | 444 |
| 115 | 5-Cyclopropyl-hexahydro-pyrrolo[3,4-c]pyrrol-2(1*H*)-yl | H | H | 4-Fluorophényl | H | 204-206 | 441 |
| 116 | 5-Isopropyl-hexahydro-pyrrolo[3,4-*c*]pyrrol-2(1*H*)-yl | H | H | 4-Fluorophényl | H | 200-202 | 443 |
| 117 | 5-Isopropyl-hexahydro-pyrrolo[3,4-*c*]pyrrol-2(1*H*)-yl | H | H | 4-Fluorophényl | CH₃- | 197-199 | 457 |
| 118 | 5-Isopropyl-hexahydro-pyrrolo[3,4-*c*]pyrrol-2(1*H*)-yl | H | H | 4-Fluorophényl | CH₃O- | 208-211 | 473 |
| 119 | (+/-)-(*cis*)-Octahydro-6*H*-pyrrolo[3,4-*b*]pyridin-6-yl | H | H | Phényl | H | 212-215 | 397 |
| | 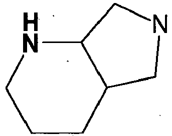 | | | | | | |
| 120 | (+/-)-(*cis*)-Octahydro-6*H*-pyrrolo[3,4-*b*]pyridin-6-yl | H | H | Phényl | CH₃- | 208-210 | 411 |
| 121 | (+/-)-(*cis*)-Décahydro-[2,6]naphthyridin-2-yl | H | H | 4-Fluorophényl | H | 231-234 (3HCl) | 429 |
| | 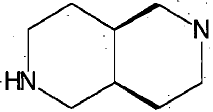 | | | | | | |
| 122 | (+/-)-2,7-Diaza-spiro[4.4]non-2-yl | H | H | 3-Fluorophényl | H | N.D | 415 |
| | 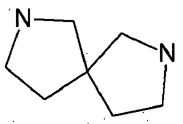 | | | | | | |
| 123 | (+/-)-2,7-Diaza-spiro[4.4]non-2-yl | H | H | 4-Fluorophényl | H | 204-206 | 415 |
| 124 | (+/-)-2,8-Diaza-spiro[4.5]déc-2-yl | H | H | 4-Fluorophényl | H | 198-200 | 429 |
| | 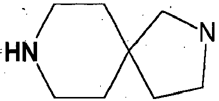 | | | | | | |
| 125 | (+/-)-2,7-Diaza-spiro[4.5]déc-2-yl | H | H | 4-Fluorophényl | H | 228-230 | 429 |
| | 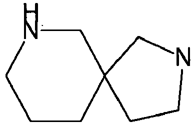 | | | | | | |
| 126 | (+/-)-2,8-Diaza-spiro[4.5]déc-8-yl | H | H | 4-Fluorophényl | H | 223-225 | 429 |
| | 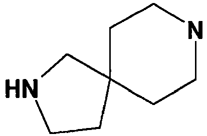 | | | | | | |
| 127 | (+/-)-2,7-Diaza-spiro[4.5]déc-7-yl | H | H | 4-Fluorophényl | H | 162-165 | 429 |
| | 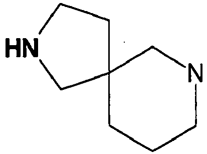 | | | | | | |
| 128 | 3,9-Diaza-spiro[5.5]undéc-3-yl | H | H | 4-Fluorophényl | H | 230-232 | 443 |
| | 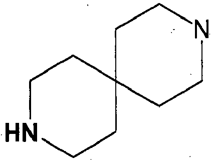 | | | | | | |
| 129 | 2,9-Diaza-spiro[5.5]undéc-9-yl | H | H | Phényl | H | 173-176 | 425 |
| | 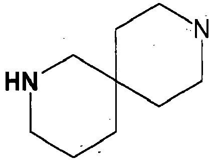 | | | | | | |
| 130 | 2,9-Diaza-spiro[5.5]undéc-9-yl | H | H | Phényl | CH₃- | 175-179 | 439 |
| 131 | 2,9-Diaza-spiro[5.5]undéc-9-yl | H | H | 3-Fluorophényl | CH₃- | 189-191 | 457 |
| 132 | 2,9-Diaza-spiro[5.5]undéc-9-yl | H | H | 4-Fluorophényl | H | 253-255 | 443 |
| 133 | 2,9-Diaza-spiro[5.5]undéc-9-yl | H | H | 4-Fluorophényl | CH₃- | 228-230 | 457 |
| 134 | 2,9-Diaza-spiro[5.5]undéc-9-yl | H | H | 4-Chlorophényl | NH₂- | 255-258 | 474 |
| | | | | | | 240-243 | |
| 135 | 2,9-Diaza-spiro[5.5]undéc-9-yl | H | H | 4-Fluorophényl | CH₃O- | 191-194 | 473 |
| 136 | 2,9-Diaza-spiro[5.5]undéc-2-yl | H | H | 4-Fluorophényl | H | 248-250 | 443 |
| | 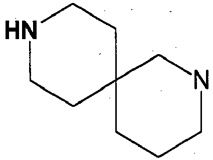 | | | | | | |
| 137 | (+/-)-2,8-Diaza-spiro[5.5]undéc-2-yl | H | H | 4-Fluorophényl | H | 218-220 | 443 |
| | 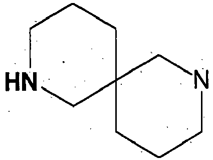 | | | | | | |
| 138 | 1-Oxa-4,9-diaza-spiro[5.5]undéc-9-yl | H | H | Phényl | H | N.D. | 427 |
| | 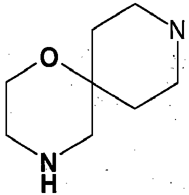 | | | | | | |
| 139 | 1-Oxa-4,9-diaza-spiro[5.5]undéc-9-yl | H | H | 4-Fluorophényl | H | 219-221 | 445 |
| 140 | 1-Oxa-4,9-diaza-spiro[5.5]undéc-9-yl | H | H | 4-Fluorophényl | CH₃O- | 198-200 | 475 |
| 141 | 1-Oxa-4,9-diaza-spiro[5.5]undéc-9-yl | H | H | 4-Chlorophényl | NH₂- | 241-244 | 476 |
| 142 | (+/-)-3-Diméthylamino-pyrrolidin-1-yl | H | H | 4-Fluorophényl | H | 184-186 | 403 |
| 143 | 4-(Pyrrolidin-1-yl)-pipéridin-1-yl | H | H | 3,5-Diméthylphényl | H | N.D. | 453 |
| 144 | 4-(Morpholin-4-yl)-pipéridin-1-yl | H | H | Phényl | H | N.D. | 441 |
| 145 | 4-(2,6-Diméthyl-morpholin-4-yl)-pipéridin-1-yl | H | H | Phényl | H | N.D. | 469 |
| 146 | 4-Diméthylaminopipéridin-1-yl | H | H | 4-Fluorophényl | H | 193-195 | 417 |
| 147 | 4-Pyrrolidin-1-yl-pipéridin-1-yl | H | H | Phényl | H | 165.0-165.5 | 425 |
| 148 | 4-Pyrrolidin-1-yl-pipéridin-1-yl | H | H | 4-Fluorophényl | H | 210-212 | 443 |
| 149 | 4-Pyrrolidin-1-yl-pipéridin-1-yl | H | H | 4-Fluorophényl | CH₃O- | 161-164 | 473 |
| 150 | 4-((*R*)-3-Hydroxy-pyrrolidin-1-yl)-pipéridin-1-yl | H | H | 4-Fluorophényl | H | 203-205 | 459 |
| 151 | 4-Morpholin-4-yl-pipéridin-1-yl | H | H | Phényl | H | N.D. | 441 |
| 152 | (*R*)-3-méthyl-pipérazin-1-yl | H | H | 3-Fluoro-5-méthyl- phényl | H | 216-219 | 403 |
| 153 | (*R*)-3-méthyl-pipérazin-1-yl | H | H | 4-Fluoro-phényl | CH₃NH- | 232-234 | 418 |
| 154 | 3,3-Diméthyl-pipérazin-1-yl | CH₃- | H | 4-Fluoro-phényl | H | 224-226 | 417 |
| 155 | 3,3-Diméthyl-pipérazin-1-yl | H | CH₃- | 4-Fluoro-phényl | H | 224-226 et 222-224 | 417 |
| 156 | 3,3-Diméthyl-pipérazin-1-yl | H | H | 3-Fluoro-5-méthyl- phényl | H | 218-221 | 417 |
| 157 | 3,3-Diméthyl-pipérazin-1-yl | H | H | 4-Fluoro-phényl | CH₃NH- | 235-237 | 432 |
| 158 | (*R*)-3-Éthyl-pipérazin-1-yl | H | H | 4-Fluoro-phényl | H | 186-188 | 403 |
| 159 | 3,3-Diéthyl-pipérazin-1-yl | H | H | 4-Fluoro-phényl | H | 182-184 | 431 |
| 160 | (+/-)-3-Fluorométhyl-pipérazin-1-yl | H | H | 4-Fluoro-phényl | H | 198-200 | 407 |
| 161 | (+/-)-(3-Hydroxyméthyl)-pipérazin-1-yl | H | H | 4-Fluoro-phényl | H | 215-219 | 405 |
| 162 | 4-(2-Hydroxyéthyl)pipérazin-1-yl | H | H | 3-Fluoro-5-méthyl- phényl | H | 187-189 | 433 |
| 163 | 4-(2-hydroxyéthyl)pipérazin-1-yl | H | H | 4-Fluoro-phényl | CH₃NH- | 207-209 | 448 |
| 164 | 4-(2-Hydroxy-2-méthyl-propyl)pipérazin-1-yl | H | H | 4-Fluoro-phényl | H | 212-214 | 447 |
| 165 | 4-(2-Hydroxy-2-méthyl-propyl)pipérazin-1-yl | H | CH₃- | 4-Fluoro-phényl | H | 205-207 | 461 |
| 166 | 4-(2-Hydroxy-2-méthyl-propyl)pipérazin-1-yl | CH₃- | H | 4-Fluoro-phényl | H | 219-221 | 461 |
| 167 | 4-(2-Hydroxy-2-méthyl-propyl)pipérazin-1-yl | H | H | 4-Fluoro-phényl | CH₃NH- | 182-184 | 476 |
| 168 | (*R*)-3-(1-hydroxy-1-méthyl-éthyl)-pipérazin-1-yl | H | H | 4-Fluoro-phényl | H | 184-186 | 433 |
| 169 | (*R*)-3-Isopropylpipérazin-1-yl | H | H | 4-Fluorophényl | H | 179-181 | 417 |
| 170 | (*R*)-3-Isopropyl-pipérazin-1-yl | H | H | 4-Fluoro-phényl | CH₃NH- | 172-174 | 446 |
| 171 | 4-Isopropyl-pipérazin-1-yl | H | H | 3-Fluoro-phényl | CH₃O- | 124-126 | 447 |
| 172 | 4-Isopropyl-pipérazin-1-yl | CH₃- | H | 4-Fluoro-phényl | H | 210-212 | 431 |
| 173 | 4-Isopropyl-pipérazin-1-yl | H | CH₃- | 4-Fluoro-phényl | H | 195-197 | 431 |
| 174 | 4-Isopropyl-pipérazin-1-yl | H | H | 4-Fluoro-phényl | CH₃NH- | 207-209 | 446 |
| 175 | 4-Isopropylpipérazin-1-yl | H | H | 3,4-Difluorophényl | CH₃O- | 136-138 | 465 |
| 176 | 4-Isopropyl-pipérazin-1-yl | H | H | 3-Fluoro-5-méthyl- phényl | H | 161-163 | 431 |
| 177 | (+/-)-3,6-Diaza-bicyclo[3.2.0]hept-2-yl | H | H | Phényl | H | 212-128 | 369 |
| 178 | (1*S*,4*S*)-2,5-Diaza-bicyclo[2.2.1]hept-2-yl | H | H | Phényl | H | N.D. | 369 |
| 179 | (1*S*,4*S*)-2_{;}5-Diaza-bicyclo[2.2.1]hept-2-yl | H | H | 4-Fluoro-phényl | CH₃NH- | 191-193 | 416 |
| 180 | (1*S*,4*S*)-5-Méthyl-2,5-diaza-bicyclo[2.2.1]hept-2-yl | H | H | 4-Fluoro-phényl | H | 178-180 et 181-189 | 401 |
| 181 | (1*S*,4*S*)-5-Méthyl-2,5-diaza-bicyclo[2,2.1]hept-2-yl | CH₃- | H | 4-Fluoro-phényl | H | 185-187 | 415 |
| 182 | (1*S*,4*S*)-5-Méthyl-2,5-diaza-bicyclo[2.2.1]hept-2-yl | H | CH₃- | 4-Fluoro-phényl | H | 212-214 | 415 |
| 183 | (1*S*,4*S*)-5-Méthyl-2,5-diaza-bicyclo[2.2.1]hept-2-yl | H | H | 4-Fluoro-phényl | CH₃- | 156-158 | 415 |
| 184 | 1-(1*S*,4*S*)-(2,5-Diaza-bicyclo[2.2.1]hept-2-yl)-2-méthyl-propan-2-ol | H | H | Phényl | H | ND CF₃COOH (3 :1) | 441 |
| | 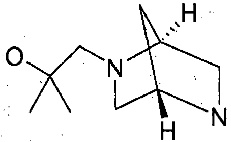 | | | | | | |
| 185 | 3,6-Diaza-bicyclo[3.1.1]hept-3-yl | H | H | 4-Fluoro-phényl | H | 244-246 | 387 |
| 186 | 5-Méthyl-hexahydro-pyrrolo[3,4-c]pyrrol-2(1*H*)-yle | H | H | 4-Fluoro-phényl | H | 166-169 | 415 |
| 187 | 5-Méthyl-hexahydro-pyrrolo[3,4-*c*]pyrrol-2(1*H*)-yle | CH₃- | H | 4-Fluoro-phényl | H | 164-166 | 429 |
| 188 | 5-Méthyl-hexahydro-pyrrolo[3,4-*c*]pyrrol-2(1*H*)-yle | H | CH₃- | 4-Fluoro-phényl | H | 235-238 | 429 |
| 189 | 5-Méthyl-hexahydro-pyrrolo[3,4-*c*]pyrrol-2(1*H*)-yle | H | CH₃- | 4-Fluoro-phényl | CH₃- | 219-221 | 443 |
| 190 | 5-Méthyl-hexahydro-pyrrolo[3,4-*c*]pyrrol-2(1*H*)-yle | H | H | 4-Fluoro-phényl | CH₃NH- | 211-213 | 444 |
| 191 | 5-Méthyl-hexahydro-pyrrolo[3,4-*c*]pyrrol-2(1*H*)-yle | H | H | 4-Fluoro-phényl | CH₃O- | 205-207 | 445 |
| 192 | 5-Méthyl-hexahydro-pyrrolo[3,4-*c*]pyrrol-2(1*H*)-yle | H | H | 4-Fluoro-phényl | CH₃- | 173-178 | 429 |
| 193 | 5-Méthyl-hexahydro-pyrrolo[3,4-*c*]pyrrol-2(1*H*)-yle | H | CH₃- | 4-Fluoro-phényl | CH₃NH- | 190-192 | 458 |
| 194 | 5-Méthyl-hexahydro-pyrrolo[3,4-*c*]pyrrol-2(1*H*)-yle | CH₃- | H | 4-Fluoro-phényl | CH₃NH- | 211-213 | 458 |
| 195 | (+-/-)-Hexahydro-pyrrolo[3,4-*b*]-ylpyrrol-5(1*H*)-yl | H | H | 4-Fluoro-phényl | H | 237-239 | 401 |
| 196 | (+)-Hexahydro-pyrrolo[3,4-*b*]pyrrol-5(1*H*)-yl | H | H | 4-Fluoro-phényl | H | 244-246 | 401 |
| 197 | (-)-Hexahydro-pyrrolo[3,4-*b*]pyrrol-5(1*H*)-yl | H | H | 4-Fluoro-phényl | H | 246-248 | 401 |
| 198 | (+/-)-Hexahydro-pyrrolo[3,4-*b*]pyrrol-5(1*H*)-yl | H | H | 4-Fluoro-phényl | CH₃NH- | 189-191 | 430 |
| 199 | (4a*S*,7a*S*)-Octahydro-pyrrolo[3,4-*b*]pyridin-6-yl | H | H | Phényl | H | 237-239 | 397 |
| 200 | (4a*S*,7a*S*)-Octahydro-pyrrolo[3,4-*b*]pyridin-6-yl | H | H | Phényl | CH₃- | 203-206 | 411 |
| 201 | (4a*R*,7a*R*)-Octahydro-pyrrolo[3,4-*b*]pyridin-6-yl | H | H | Phényl | CH₃- | 199-201 | 411 |
| 202 | (4a*S*,7a*S*)-Octahydro-pyrrolo[3,4-*b*]pyridin-6-yl | H | CH₃- | 4-Fluoro-phényl | H | 217-219 | 429 |
| 203 | (4a*S*,7a*S*)-Octahydro-pyrrolo[3,4-*b*]pyridin-6-yl | CH₃- | H | 4-Fluoro-phényl | H | 234-236 | 429 |
| 204 | (4a*R*,7a*R*)-Octahydro-pyrrolo[3,4-*b*]pyridin-6-yl | CH₃- | H | 4-Fluoro-phényl | H | 229-231 | 429 |
| 205 | (4a*S*,7a*S*)-Octahydro-pyrrolo[3,4-*b*]pyridin-6-yl | H | H | 4-Fluoro-phényl | H | 238-240 | 415 |
| 206 | (4a*S*,7a*S*)-Octahydro-pyrrolo[3,4-*b*]pyridin-6-yl | H | H | 4-Fluoro-phényl | CH₃- | 219-221 | 429 |
| 207 | (+/-)-Octahydro-pyrrolo[3,4-*b*]pyridin-6-yl | H | H | 4-Fluoro-phényl | CH₃NH- | 244-246 | 444 |
| 208 | (4a*R*,7a*R*)-Octahydro-pyrrolo[3,4-*b*]pyridin-6-yl | H | H | Phényl | H | 234-236 | 297 |
| 209 | (4a*R*,7a*R*)-Octahydro-pyrrolo[3,4-*b*]pyridin-6-yl | H | H | 4-Fluoro-phényl | H | 222-224 | 415 |
| 210 | (4a*R*,7a*R*)-Octahydro-pyrrolo[3,4-*b*]pyridin-6-yl | H | H | 4-Fluoro-phényl | CH₃- | 220-222 | 429 |
| 211 | (4a*R*,7a*R*)-Octahydro-pyrrolo[3,4-*b*]pyridin-6-yl | H | CH₃- | 4-Fluoro-phényl | H | 219-221 | 429 |
| 212 | (+/-)-2,7-Diaza-spiro[4.4]non-2-yl | H | CH₃- | 4-Fluoro-phényl | H | 219-222 | 429 |
| 213 | 6,9-Diaza-spiro[4.5]déc-9-yl | H | H | 4-Fluoro-phényl | H | 209-211 | 429 |
| 214 | (+/-)-2,8-Diaza-spiro[5.5]undéc-2-yl | H | CH₃- | 4-Fluoro-phényl | H | 215-218 | 457 |
| 215 | 2,9-Diaza-spiro[5.5]undéc-9-yl | H | CH₃- | 4-Fluoro-phényl | H | 242-245 | 457 |
| 216 | 2,9-Diaza-spiro[5.5]undéc-9-yl | H | H | 4-Fluoro-phényl | CH₃NH- | 238-241 | 472 |
| 217 | 2,9-Diaza-spiro[5:5]undéc-9-yl | H | H | 3,4-Difluorophényl | H | 227-229 | 461 |
| 218 | 2,9-Diaza-spiro[5.5]undéc-9-yl | H | H | 3-Fluoro-5-méthyl- phényl | H | 172-175 | 457 |
| 219 | 4-Pyrrolidin-1-yl-pipéridin-1-yl | H | H | 3-Fluoro-phényl | H | N.D. | 443 |
| 220 | 4-Pyrrolidin-1-yl-pipéridin-1-yl | H | H | 4-Fluoro-phényl | CH₃NH- | 217-219 | 472 |
| 221 | 4-((*R*)-3-fluoro-pyrrolidin-1-yl)-pipéridin-1-yl | H | H | 4-Fluorophényl | H | 208-210 | 461 |
| 221 | 4-((*R*)-3-Hydroxy-pyrrolidin-1-yl)-pipéridin-1-yl | H | H | Phényl | H | 154-158 | 441 |
| 223 | 4-((*R*)-3-Hydroxy-pyrrolidin-1-yl)-pipéridin-1-yl | H | H | 3-Fluoro-5-méthyl-phényl | H | 178-183 | 473 |

L'invention a également pour objet un procédé de préparation des composés de l'invention de formule (I).

Conformément à l'invention, on peut préparer les composés de formule générale (I) selon le procédé général décrit dans le schéma 1 ci-après.

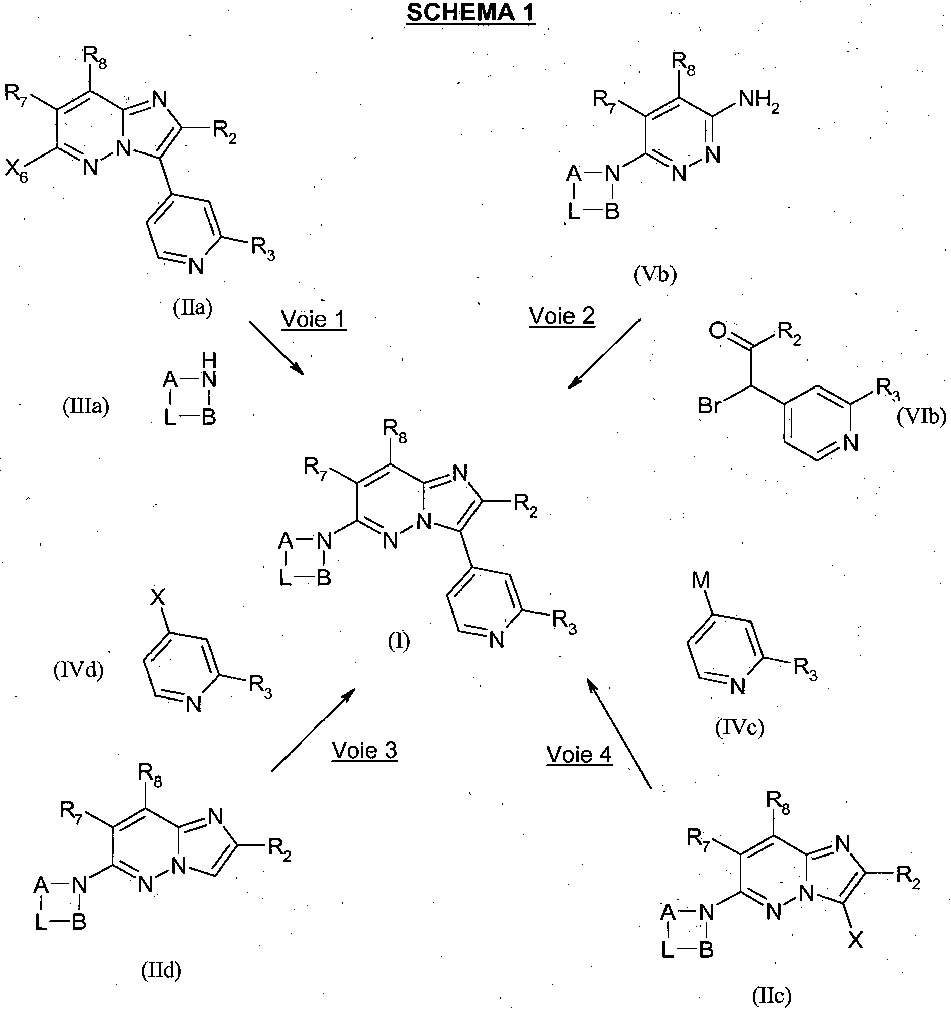

Dans ce qui suit, on entend par groupe partant un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un mésyle, tosyle, triflate, acétyle, etc. Des exemples de groupes partants ainsi que des références pour leurs préparations sont données dans « Advances in Organic Chemistry », J. March, 3rd Edition, Wiley Interscience, p. 310-316.

### Schéma 1, voie 1 : Introduction de l'amine

De manière générale et comme illustré dans le schéma 1, les dérivés de 6-amino-3-pyridin-4-ylimidazo[1,2-b]pyridazine de formule générale (I) dans laquelle R₂, R₃, A, L, B, R₇ et R₈ sont tels que définis ci-dessus peuvent être préparés à partir d'un dérivé de 3-pyridine-4-ylimidazo[1,2-*b*]pyridazine de formule générale (IIa), dans laquelle R₂, R₃, R₇ et R₈ sont tels que définis ci-dessus et X₆ représente un groupe partant tel qu'un halogène par traitement au moyen d'une aminé de formule générale (IIIa) dans laquelle A, L et B sont tels que définis précédement. Cette réaction peut être effectuée par chauffage des réactifs dans un solvant polaire tel que le diméthylsulfoxyde ou les alcools aliphatiques, par exemple le pentanol.

### Schéma 1, voie 2 : Construction de l'hétérocycle

Les dérivés de 6-amino-3-pyridin-4-ylimidazo[1,2-*b*]pyridazine de formule générale (I) dans laquelle R₂, R₃, A, L, B, R₇ et R₈ sont tels que définis ci-dessus peuvent également être préparés par condensation entre un dérivé de pyridazin-3-ylamine de formule générale (Vb) dans laquelle A, L, B, R₇ et R₈ sont tels que définis ci-dessus et un dérivé de 2-bromo-2-(pyridin-4-yl)-éthan-1-one de formule générale (Vlb) dans laquelle R₂ et R₃ sont tels que définis ci-dessus.

La réaction peut être effectuée par chauffage des réactifs dans un solvant polaire tel que les alcools aliphatiques, par exemple l'éthanol ou le butanol.
0

### Schéma 1, voie 3 : Introduction de la pyridine - C-H arylation métallocatalysée

Les dérivés de 6-amino-3-pyridin-4-ylimidazo[1,2-*b*]pyridazine de formule générale (I) dans laquelle R₂, R₃, A, L, B, R₇ et R₈ sont tels que définis ci-dessus peuvent également être préparés à partir d'un dérivé de 6-aminoimidazo[1,2-*b*]pyridazine de formule générale (IId) dans laquelle R₂, A, L, B, R₇ et R₈ sont tels que définis ci-dessus, par C-H arylation métallocatalysée avec un dérivé de 4-iodopyridine de formule générale (IVd) dans laquelle R₃ est tel que défini ci-dessus et X représente un atome d'iode. Ce couplage peut être réalisé en présence d'un catalyseur tel que l'acétate de Palladium, d'une base minérale telle que le carbonate de potassium et dans un solvant polaire aprotique tel que le diméthylformamide.

### Schéma 1, Voie 4 : Introduction de la pyridine - couplage de type « Stille » ou « Suzuki »

Les dérivés de 6-amino-3-pyridin-4-ylimidazo[1,2-*b*]pyridazine de formule générale (I) dans laquelle R₂, R₃, A, L, B, R₇ et R₈ sont tels que définis ci-dessus peuvent également être préparés à partir d'un dérivé 6-amino-3-iodo- ou 3-bromo-imidazo[1,2-*b*]pyridazine de formule générale (IIc), dans laquelle R₂, A, L, B, R₇ et R₈ sont tels que définis ci-dessus et X représente un atome de brome ou d'iode par couplage selon les condition de Stille ou de Suzuki avec un stannane ou un boronate de pyridine de formule générale (IVc) dans laquelle R₃ est tel que défini ci-dessus et M représente un groupe trialkylstannyle, le plus fréquemment un groupement tributylstannyle ou un groupe dihydroxyboryle ou dialkyloxyboryle, le plus fréquemment un groupe 4,4,5,5-tétraméthyl-1,3,3,2-dioxaborolan-2-yle.

Les couplages selon la méthode de Stille sont par exemple réalisés par chauffage en présence d'un catalyseur tel que le tétrakis(triphény)phosphine)palladium, d'iodure de cuivre dans un solvant tel que le N,N-diméthylacétamide.

Les couplages selon la méthode de Suzuki sont par exemple réalisés par chauffage en présence d'un catalyseur tel que le 1,1'-bis (diphénylphosphino) ferrocènedichloropalladium, d'une base minérale telle que le carbonate de césium dans un mélange de solvant tels que le tétrahydrofurane et l'eau.

### Stratégies Particulières de synthèse

### 1. Stratégie de synthèse lorsque R₃ = H, alkyle

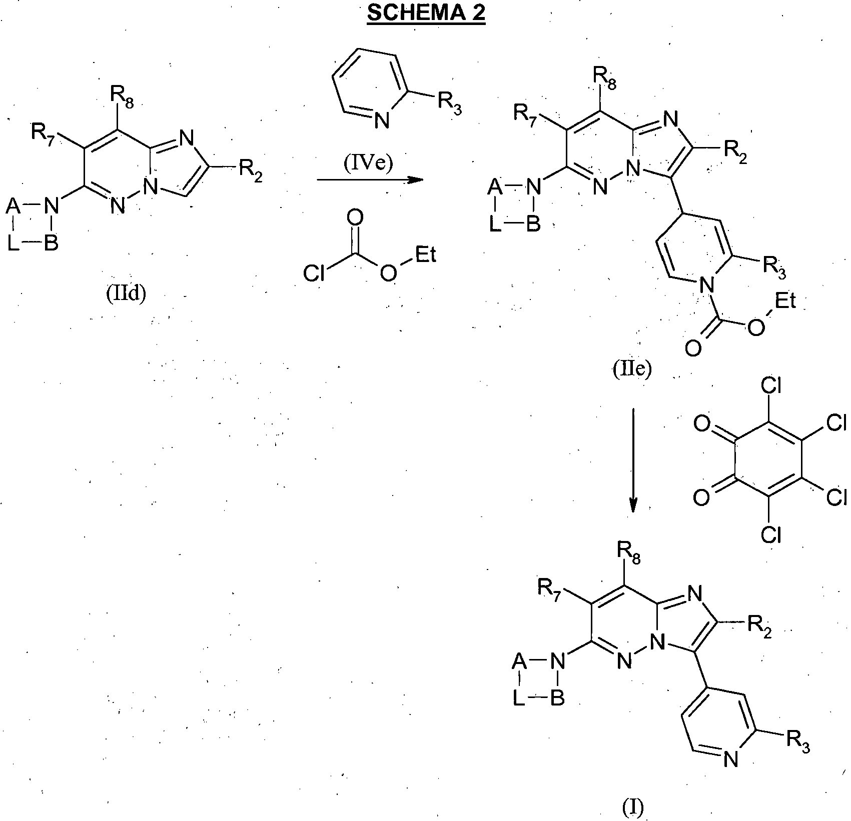

De manière spécifique, selon le schéma 2 les dérivés de 6-amino-3-pyridin-4-ylimidazo[1,2-*b*]pyridazine de formule générale (I) dans laquelle R₂, A, L, B, R₇ et R₈ sont tels que définis ci-dessus et dans laquelle R₃ représente un atome d'hydrogène ou un groupe C₁₋₃-alkyle, peuvent être préparés en deux étapes à partir d'un dérivé de 6-aminoimidazo[1,2-*b*]pyridazine de formule générale (IId) tel que défini précédement.

Ainsi, la réaction d'un dérivé de 6-aminoimidazo[1,2-*b*]pyridazine de formule générale (IId) sur un mélange d'un dérivé de pyridine de formule générale (IVe) dans laquelle R₃ représente un atome d'hydrogène ou un groupe C₁₋₃-alkyle, et de chloroformiate d'alkyle, par exemple le chloroformiate d'éthyle, conduit au dérivé de formule générale (IIe) dans laquelle R₂, A, L, B, R₇ et R₈ sont tels que définis ci-dessus et dans laquelle R₃ représente un atome d'hydrogène ou un groupe C₁₋₃-alkyle. Le dérivé de formule générale (IIe) est ensuite oxydé au moyen d'ortho-chloranile dans un solvant tel que le toluène pour conduire aux dérivés de 6-amino-3-pyridin-4-ylimidazo[1,2-*b*]pyridazine de formule générale (I) dans laquelle R₂, A, L, B, R₇ et R₈ sont tels que définis ci-dessus et dans laquelle R₃ représente un atome d'hydrogène ou un groupe C₁₋₃-alkyle.

### 2. Stratégies de synthèse lorsque R₃ = NH₂, NH-C₁₋₃ alkyle, N(-C₁₋₃ alkyle)(C₁₋₃ alkyle), hydroxyle, C₁₋₃ alkyloxy.

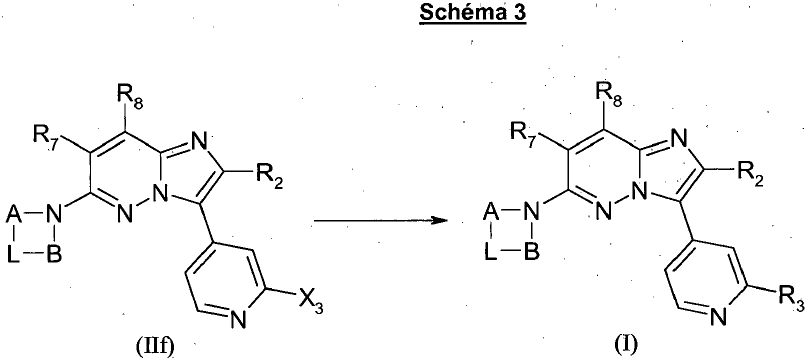

Selon le schéma 3, les dérivés de 6-amino-3-pyridin-4-ylimidazo[1,2-*b*]pyridazine de formule générale (I) dans laquelle R₂, A, L, B, R₇ et R₈ sont tels que définis ci-dessus et où R₃ représente un groupe amino, C₁₋₃-monoalkylamine, ou di-C₁₋₃ alkylamine, un groupe hydroxyle ou C₁₋₃ alkyloxy peuvent être préparés à partir d'un dérivé de 6-amino-3-pyridin-4-ylimidazo[1,2-b]pyridazine de formule générale (IIf) dans laquelle R₂, A, L, B, R₇ et R₈ sont tels que définis ci-dessus et où X₃ représente un atome d'halogène ou un groupement partant.
- Dans le cas où R₃ représente un groupe amino, C₁₋₃ monoalkylamine ou di-C₁₋₃ alkylamine, la réaction peut être réalisée par substitution nucléophile au moyen de l'amine primaire ou secondaire correspondante dans un solvant aprotique tel que la *N*-méthylpyrrolidone.
- Dans le cas où R₃ représente un groupe amino, la réaction peut également être réalisée en deux étapes par couplage avec la benzhydrylidène-amine en présence d'un catalyseur tel que le tris(dibenzylidèneacétone)dipalladium(0), d'une base telle que le tert-butoxyde de sodium et d'un ligand phosphinique tel que le (+/-)-2,2'-bis(diphénylphosphino)-1,1'-binaphtyle puis hydrolyse du groupement benzhydrylidene au moyen d'acide tel que l'acide chlorhydrique.
- Dans le cas où R₃ représente un groupe hydroxyle, la réaction peut être réalisée par chauffage en présence de soude ou de potasse dans un solvant tel que le *tertio-*butanol.
- Dans le cas où R₃ représente un groupe C₁₋₃ alkoxy, la réaction peut être réalisée par traitement au moyen du C₁₋₃ alkoxylate de sodium ou de potassium correspondant dans un solvant tel que la *N*-méthylpyrrolidone.

### Synthèse des précurseurs

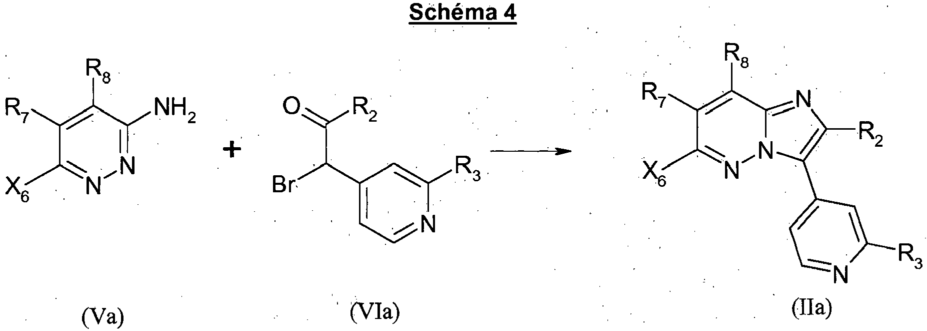

Selon le schéma 4, les dérivés 3-pyridin-4-ylimidazo[1,2-*b*]pyridazine de formule générale (IIa), dans laquelle R₂, R₃, R₇ et R₈ sont tels que définis ci-dessus et X₆ représente un groupe partant tel qu'un halogène, peuvent être préparés par condensation entre un dérivé de pyridazin-3-ylamine de formule générale (Va) dans laquelle R₇ et R₈ sont tels que définis ci-dessus et X₆ représente un halogène ou un groupe partant et un dérivé de 2-brome-2-(pyridin-4-yl)-éthan-1-one de formule générale (Via) dans laquelle R₂ et R₃ sont tels que définis ci-dessus.

La réaction peut être effectuée par chauffage des réactifs dans un solvant polaire tel que les alcools aliphatiques, par exemple l'éthanol ou le butanol.

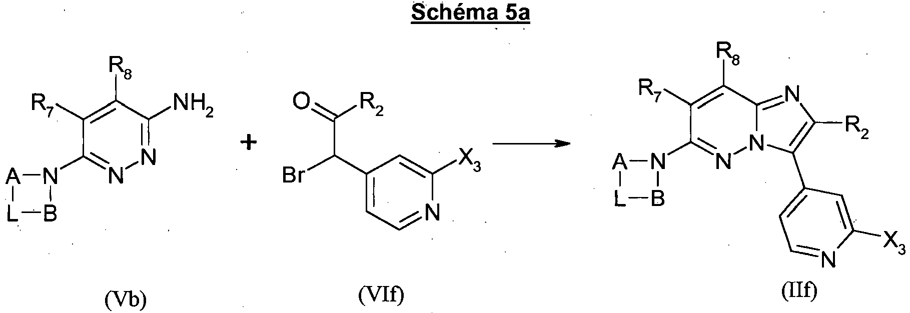

Selon le schéma 5a, les dérivés 3-pyridin-4-ylimidazo[1,2-b]pyridazine de formule générale (IIf), dans laquelle R₂, A, L, B, R₇ et R₈ sont tels que définis ci-dessus et X₃ représente un groupe partant tel qu'un halogène peuvent être préparés par condensation entre un dérivé de pyridazin-3-ylamine de formule générale (Vb) dans laquelle A, L, B, R₇ et R₈ sont tels que définis ci-dessus et un dérivé de 2-bromo-2-(pyridin-4-yl)-éthan-1-one de formule générale (VIf) dans laquelle R₂ est tel que défini ci-dessus et X₃ représente un halogène ou un groupe partant.

Cette réaction peut être effectuée par chauffage des réactifs dans un solvant polaire tel que les acides aliphatiques, par exemple l'éthanol ou le butanol.

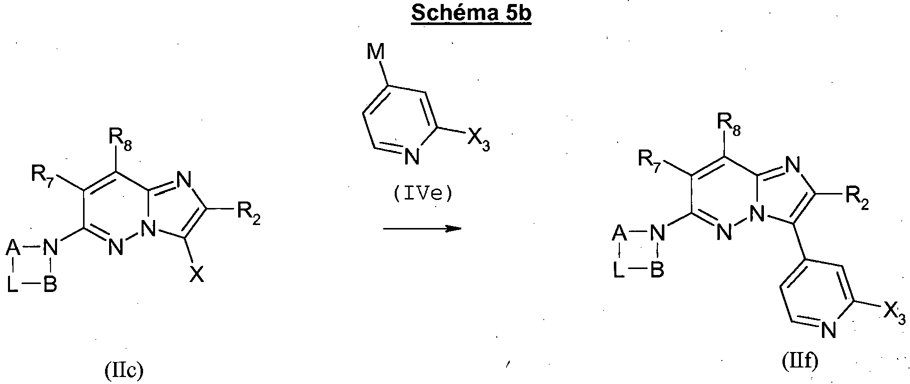

Selon le schéma 5b, les dérivés 3-pyridin-4-ylimidazo[1,2-*b*]pyridazine de formule générale (IIf) tels que définis ci-dessus peuvent également être obtenus à partir d'un dérivé 6-amino-3-iodo- ou 3-bromo-imidazo[1,2-*b*]pyridazine de formule générale (IIc), dans laquelle R₂, A, L, B, R₇ et R₈ sont tels que définis ci-dessus et X représente un atome de brome ou d'iode par couplage selon les conditions de Stille ou de Suzuki avec un stannane ou un boronate de pyridine de formule générale (IVe) dans laquelle X₃ représente un halogène ou un groupe partant et M représente un groupe trialkylstannyle, le plus fréquemment un groupement tributylstannyle ou un groupe dihydroxyboryle ou dialkyloxyboryle, le plus fréquemment un groupe 4,4,5,5-tétraméthyl-1,3,3,2-dioxaborolan-2-yle.

Les couplages selon la méthode de Stille sont par exemple réalisés par chauffage en présence d'un catalyseur tel que le tétrakis(triphénylphosphine)palladium, d'iodure de cuivre dans un solvant tels que le N,N-diméthylacétamide.

Les couplages selon la méthode de Suzuki sont par exemple réalisés par chauffage en présence d'un catalyseur tel que le 1,1'-bis (diphénylphosphino) ferrocènedichloropalladium, d'une base minérale telle que le carbonate de césium dans un mélange de solvants tels que le tétrahydrofurane et l'eau.

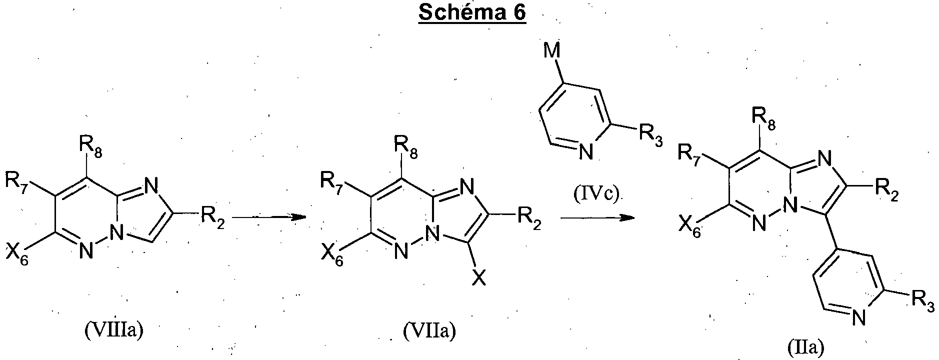

Selon le schéma 6, les dérivés 3-pyridin-4-ylimidazo[1,2-*b*]pyridazine de formule générale (IIa), dans laquelle R₂, R₃, R₇ et R₈ sont tels que définis ci-dessus et X₆ représente un groupe partant tel qu'un halogène peuvent également être préparés en deux étapes à partir d'un dérivé d'imidazo[1,2-*b*]pyridazine de formule générale (VIIIa) dans laquelle R₂, R₇ et R₈ sont tels que définis ci-dessus et X₆ représente un groupe partant.

La bromation ou l'iodation d'un dérivé d'imidazo[1,2-*b*]pyridazine de formule générale (VIIIa), dans laquelle R₂, R₇ et R₈ sont tels que définis ci-dessus et X₆ représente un atome d'halogène ou un groupe partant conduit à un dérivé de 3-bromo- ou iodoimidazo[1,2-*b*]pyridazine de formule générale (VIIa), dans laquelle R₂, R₇ et R₈ sont tels que définis ci-dessus et X₆ représente un halogène ou un groupe partant et X représente un atome de brome ou d'iode. Cette réaction peut être réalisée au moyen de N-bromo- ou iodosuccinimide ou de monochlorure d'iode dans un solvant polaire tel que l'acétonitrile, le tétrahydrofurane, le méthanol oul le chloroforme.

Le dérivé de 3-bromo ou iodoimidazo[1,2-*b*]pyridazine de formule générale (VIIa) obtenu est ensuite couplé régiosélectivement selon les méthodes de Stille ou de Suzuki avec un stannane ou un boronate de pyridine de formule générale (IVc) dans laquelle R₃ est tel que défini ci-dessus et M représente un groupe trialkylstannyle, le plus fréquemment un groupement tributylstannyle ou un groupe dihydroxyboryle ou dialkyloxyboryle, le plus fréquemment un groupe 4,4,5,5-tétraméthyl-1,3,2-dioxaborolan-2-yle.

Les couplages selon la méthode de Stille sont par exemple réalisés par chauffage en présence d'un catalyseur tel que le tétrakis(triphénylphosphine)palladium, d'iodure de cuivre dans un solvant tels que le diméthylacétamide.

Les couplages selon la méthode de Suzuki sont par exemple réalisés par chauffage en présence d'un catalyseur tel que le 1,1,-bis(diphénylphosphino)ferrocènedichloropalladium, d'une base minérale telle que le carbonate de césium dans un mélange de solvants tels que le tétrahydrofurane et l'eau.

Les dérivés d'imidazo[1,2-*b*]pyridazine de formule générale (VIIIa) dans laquelle R₂, R₇ et R₈ sont tels que définis ci-dessus et X₆ représente un groupe partant sont connus ou peuvent être préparés par analogie avec des méthodes décrites dans la littérature (Abignente, Enrico ; Caprariis, Paolo de ; Patscot, Rosaria ; Sacchi, Antonella ; J. Heterocycl. Chem.; 23; 1986; 1031-1034 ; Barlin, Gordon B.; Davies, Les P.; Ireland, Stephen J.; Ngu, Maria M. L.; Zhang, Jiankuo ; Aust. J. Chem.; EN; 45; 4; 1992; 731-749 ; Mourad, Alaa E.; Wise, Dean S.;

Townsend, Leroy B.; J. Heterocycl. Chem.; 30; 5; 1993; 1365-1372 ; Pollak et al.; Tetrahedron; 24; 1968; 2623 ; Hervet, Maud ; Galtier, Christophe ; Enguehard, Cécile; Gueiffier, Alain ; Debouzy, Jean-Claude ; Journal of Heterocyclic Chemistry (2002), 39(4), 737-742).

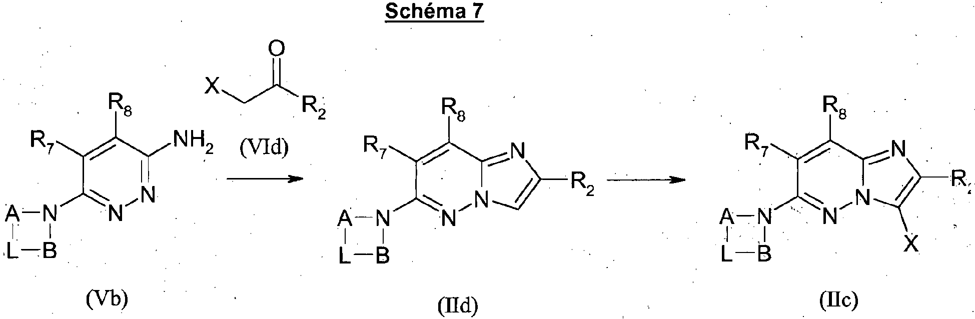

Selon le schéma 7, les dérivés de 6-amino-3-pyridin-4-ylimidazo[1,2-*b*]pyridazine de formule générale (IIc) dans laquelle R₂, A, L, B, R₇ et R₈ sont tels que définis ci-dessus et où X représente un atome de brome ou d'iode sont préparés par bromation ou l'iodation d'un dérivé partir 6-amino-3-pyridin-4-ylimidazo[1,2-*b*]pyridazine de formule générale (IId) dans laquelle R₂, A, L, B, R₇ et R₈ sont tels que définis ci-dessus.

Cette réaction peut être réalisée au moyen de *N*-bromo- ou iodosuccinimide ou d'iode monochlorure dans un solvant polaire aprotique tel que l'acétonitrile ou le tétrahydrofurane.

Les dérivés de 6-amino-3-pyridin-4-ylimidazo[1,2-*b*]pyridazine de formule générale (IId) dans laquelle R₂, A, L, B, R₇ et R₈ sont tels que définis ci-dessus sont connus ou peuvent être préparés par analogie avec des méthodes décrites dans la littérature (par exemple, Watanabe et al.; Synthesis; 1977; 761 ; Jurgee et al.; J. Heterocycl. Chem.; 12; 1975; 253,255. ; Werbel,L.M.; Zamora,M.L.; J. Heterocycl. Chem.; 2; 1965; 287-290 ; Yoneda et al.; Chem. Pharm. Bull.; 12; 1964; 1351,1353,1354 ; Tomoyasu; lizawa, Yuji; Okonogi, Kenji; Miyake, Akio; J. Antibiot.; 53; 10; 2000; 1053 - 1070).

Ils sont usuellement préparés par condensation entre un dérivé de pyridazin-3-ylamine de formule générale (Vb) dans laquelle A, L, B, R₇ et R₈ sont tels que définis ci-dessus et un dérivé de 2-bromo, chloro- ou iodoéthan-1-one de formule générale (Vld) dans laquelle R₂ est tel que défini ci-dessus.

La réaction peut être effectuée par chauffage des réactifs dans un solvant polaire tel que l'éthanol ou le butanol.

Dans les schémas de synthèse qui précédent, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

### Groupes protecteurs

Pour les composés de formule générale (I), (IIc), (IId), (IIe), (IIf), (IIIa), (Vb) telles que définies ci-dessus et dans le cas où le groupement N-A-L-B comporte une fonction amine primaire ou secondaire, cette fonction peut éventuellement être protégée lors de la synthèse par des groupements protecteurs connus de l'homme de l'art, par exemple un benzyle ou un t-butyloxycarbonyle.

Pour les composés de formule générale (I), (IIa), (IVc), (IVd), (VIa), (VIb), telles que définies ci-dessus et dans le cas où le groupement R₃ comporte une fonction amine primaire ou secondaire, cette fonction peut éventuellement être protégée par des groupements protecteurs connus de l'homme de l'art, par exemple un benzyle ou un t-butyloxycarbonyle. Les produits de structure générale (I) tels que définis ci dessus sont obtenus selon les procédés décrits après une étape finale supplémentaire de déprotection du groupement protecteur selon les conditions usuelles connues de l'Homme de l'art.

Les exemples suivants décrivent la préparation de certains composés conformes à l'invention. Ces exemples ne font qu'illustrer la présente invention. Les numéros des composés exemplifiés renvoient à ceux donnés dans le tableau ci-après, qui illustre les structures chimiques et les propriétés physiques de quelques composés selon l'invention.

### Exemple 1 (composé n° 50) : 2-(4-Fluoro-phényl)-6-(4-méthyl-pipérazin-1-yl)-3-pyridin-4-yl-imidazo[1,2-b]pyridazine.

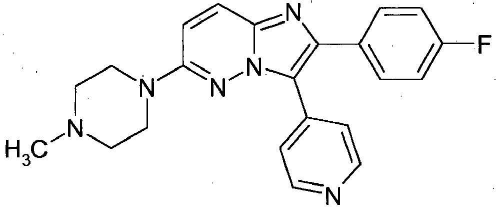

### a) Synthèse selon le procédé du schéma 1, voie 4 :

### Etape 1.1 a. 2-(4-Fluoro-phényl)-6-(4-méthyl-pipérazin-1-yl)-imidazo[1,2-b]pyridazine

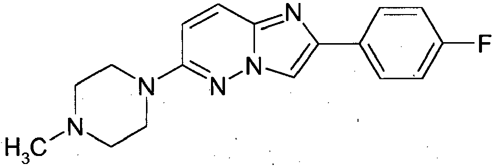

Après chauffage d'une suspension de 32 g (97,4 mmoles) de 6-chloro-2-(4-fluoro-phényl)-imidazo[1,2-*b*]pyridazine (CAS : 2069-47-8) dans 200 ml 1-méthyl-pipérazine à 160°C pendant 16 heures, le mélange est versé dans 1,5 L d'eau. Le précipité jaunâtre formé est séparé par filtration et rincé avec de l'isopropanol froid puis de l'éther diisopropylique. On isole ainsi 30 g de poudre beige après séchage sous vide.
PF : 190°C
RMN ¹H (CDCl₃) δ : 7,85 (s; 1H), 7,8 (m, 2H), 7,60 (d, 1H), 7,05 (pseudo t, 2H), 6,75 (d, 1H), 3,45 (m, 4H), 2,50 (m, 4H), 2,30 (s, 3H) ppm.

### Etape 1.2a. 2-(4-Fluoro-phényl)-3-iodo-6-(4-méthyl-pipérazin-1-yl)-imidazo[1,2-b]pyridazine

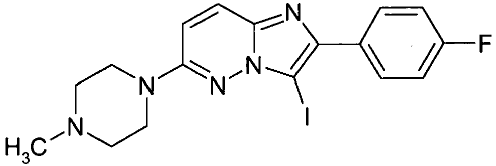

A une solution refroidie vers 5°C de 31,0 g (99,6 mmoles) de 2-(4-fluoro-phényl)-6-(4-méthyl-pipérazin-1-yl)-imidazo[1,2-b]pyridazine dans le chloroforme, on additionne rapidement au goutte à goutte une solution de 64,7 g (398 mmoles) de monochlorure d'iode dans 150 mL de méthanol On observe une légère exothermicité et la formation d'un précipite au cours de l'addition. Après retour à la température ambiante et 30 minutes d'agitation, le mélange est versé dans 2 L d'une solution aqueuse à 5% de thiosulphate de sodium saturée en bicarbonate de sodium. Après une forte agitation jusqu'à décoloration, le produit est extrait avec du chloroforme. La phase organique est séparée, séchée sur sulfate de sodium, filtrée et concentrée sous pression réduite pour conduire à un solide brun. Celui ci est trituré dans un mélange de 400 mL d'éther diisopropylique et de 50 mL d'isopropanol au reflux. Après refroidissement, on isole 40 g de poudre beige par filtration sur fritté et séchage sous vide.
PF : 180°C
RMN ¹H (CDCl₃) δ : 8,05 (pseudo dd; 2H), 7,65 (d, 1H), 7,15 (pseudo t, 2H), 6,85 (d, 1H), 3,65 (m, 4H), 2,55 (m, 4H), 2,35 (s, 3H) ppm.

### Etape 1.3a. 2-(4-Fluoro-phényl)-6-(4-méthyl-pipérazin-1-yl)-3-pyridin-4-yl-imidazo[1,2-b]pyridazine

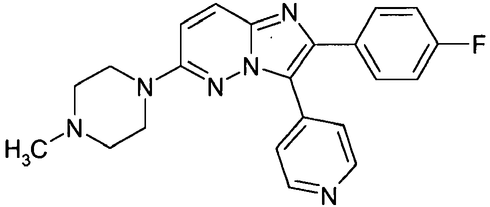

A une suspension de 2,80 g (6,40 mmoles) de 2-(4-fluoro-phényl)-3-iodo-6-(4-méthyl-pipérazin-1-yl)-imidazo[1,2-*b*]pyridazine dans 200 mL d'un mélange de diméthoxyéthane et d'eau (9:1), on additionne 1,36 g (12,8 mmoles) de bicarbonate de sodium et 0,95 g (7,7 mmoles) d'acide (pyridin-4-yl)boronique. Après barbotage d'un courant d'argon pendant quelques instants, on ajoute 0,21 g (0,26 mmoles) de complexe de 1,1'-bis(diphénylphosphinoferrocènedichloro palladium (II) et de dichlorométhane (PdCl₂(dppf).CH₂Cl₂) et la réaction est portée au reflux sous argon pour 18 heures. On ajoute alors à nouveau 0,95 g (7,7 mmoles) d'acide (pyridin-4-yl)boronique, 0,21 g (0,26 mmole) de 1,1'-bis(diphénylphosphinoferrocènedichloro palladium (II) dichlorométhane ainsi que 10 mL d'eau. Le chauffage est poursuivit pendant 24 heures. Le solvant est alors évaporé sous pression réduite et le résidu brun est trituré avec de l'acide chlorhydrique aqueux 3N puis filtré sur Büchner. La phase aqueuse est lavée deux fois avec de l'éther diéthylique puis neutralisée précautionneusement au moyen d'ammoniaque aqueux dilué glacé jusqu'à pH basique. Le produit est extrait avec du chloroforme, la phase organique séchée sur sulfate de sodium, filtrée et le solvant évaporé pour donner 2,5 g poudre jaunâtre.

Le produit est purifié par chromatographie sur gel de silice en éluant avec un mélange de dichlorométhane de méthanol et d'ammoniaque (96/4/0,4) pour donner 2,3 g de poudre blanchâtre. Le produit est recristallisé dans un mélange de 130 mL d'acétonitrile et de 10 à 30 mL d'isopropanol.

On isole 1,1 g de poudre blanchâtre.
PF : 248 - 250°C
RMN ¹H (CDCl₃) δ : 8,65 (d; 2H), 7,85 (d, 1 H), 7,5-7,7 (m, 4H), 7,05 (pseudo t, 2H), 6,95 (d, 1H), 3,55 (m, 4H), 2,55 (m, 4H), 2,40 (s, 3H) ppm.

### b) Synthèse selon le procédé du schéma 2 :

### Etape 1.1b. 4-[2-(4-Fluoro-phényl)-6-(4-méthy)-pipérazin-1-yl)-imidazo[1,2-b]pyridazin-3-yl]-4H-pyridine-1-carboxylate d'éthyle

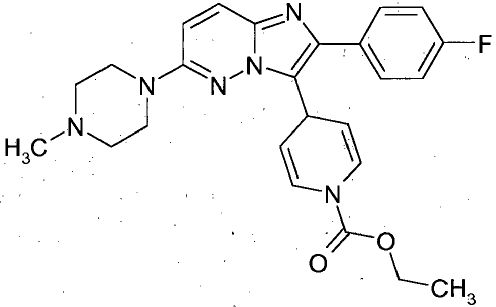

A une suspension refroidie vers 0°C de 3,24 g (10,4 mmoles) de 2-(4-fluoro-phényl)-6-(4-méthyl-pipérazin-1-yl)-imidazo[1,2-*b*]pyridazine dans 7,5 mL de pyridine, on additionne goutte à goutte une solution de 5,0 mL (52 mmoles) de chloroformiate d'éthyle. Après retour à la température ambiante, on additionne en deux fois 10 mL (104 mmoles) de chloroformiate d'éthyle et 15 mL de pyridine.

Après 18 heures, le mélange est dilué avec 50 mL de dichlorométhane et on additionne en trois fois 20 mL (208 mmoles) de chloroformiate d'éthyle en 5 heures. On ajoute ensuite 350 mL d'eau, la phase organique est séparée et lavée deux fois avec de l'eau, séchée sur sulfate de sodium et concentrée sous pression réduite pour conduire à 4,5 g de solide brunâtre. Ce dernier est trituré dans de l'éther diéthylique pour donner 3,5 g de cristaux beiges. Le solide est dissout dans du dichlorométhane et purifié par chromatographie sur colonne de gel de silice en éluant avec un mélange de 10% de méthanol dans le dichlorométhane pour donner 2,95 g de cristaux jaune pâle après trituration dans un l'éther diéthylique, filtration sur fritté et séchage sous vide.
PF : 179,9°C
RMN ¹H (DMSOd₆) δ : 7,80 (d; 2H), 7,65 (m, 2H), 7,2 (m, 3H), 6,95 (d, 1H), 4,8-5,0 (m, 3H), 4,25 (q, 2H), 3,40 (m, 4H), 2,40 (m, 4H), 2,20 (s, 3H), 1,30 (t, 3H) ppm.

### Etape 1.2b. 2-(4-Fluoro-phényl)-6-(4-méthyl-pipérazin-1-yl)-3-pyridin-4-yl-imidazo[1,2-b]pyridazine

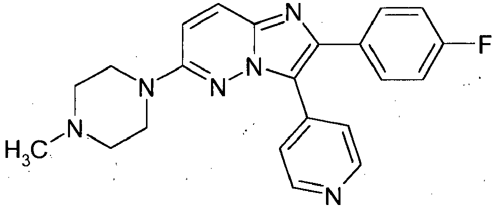

A une suspension de 1,00 g (2,16 mmoles) 4-[2-(4-fluoro-phényl)-6-(4-méthyl-pipérazin-1-yl)-imidazo[1,2-*b*]pyridazin-3-yl]-4*H*-pyridine-1-carboxylate d'éthyle dans 35 mL de toluène, on additionne une solution de 0,65 g (2,6 mmoles) d'ortho-chloranil dans 6 mL de toluène. Après 6 heures de réaction, on additionne 60 mL de soude 1N et le produit est extrait avec de l'acétate d'éthyle. La phase organique est lavée avec de l'eau saturée en chlorure de sodium, séchée sur sulfate de sodium et le solvant est évaporé sous pression réduite. On isole ainsi 1,2 g de solide qui est purifié sur colonne de gel de silice en éluant avec un gradient par palier de 3 à 15% de méthanol dans le dichlorométhane. Le produit obtenu est lavé avec de l'éther diisopropylique et recristallisé dans l'alcool isopropylique pour donner 0,47 g de cristaux après filtration et séchage sous pression réduite.
PF : 294 - 296°C
RMN ¹H, (CDCl₃) δ : 8,65 (d; 2H), 7,85 (d, 1H), 7,5-7,7 (m, 4H), 7,05 (pseudo t, 2H), 6,95 (d, 1H), 3,55 (m, 4H), 2,55 (m, 4H), 2,40 (s, 3H) ppm.

### c) Synthèse selon le schéma 1, voie 3 :

### Etape 1.1c. 2-(4-Fluoro-phényl)-6-(4-méthyl-pipérazin-1-yl)-3-pyridin-4-yl-imidazo[1,2-b]pyridazine

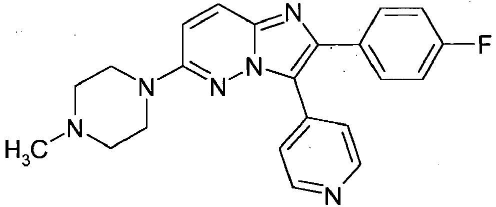

Le mélange de 0,90 g (2,89 mmoles) de 2-(4-fluoro-phényl)-6-(4-méthyl-pipérazin-1-yl)-imidazo[1,2-*b*]pyridazine (Synthesis, 2001, 4, 595-600), de 0,71 g (3,5 mmoles) de 4-iodopyridine, de 0,032 g (0,14 mmole) d'acétate de palladium et de 0,48 g (3,5 mmoles) de carbonate de potassium dans 18 ml de diméthylformamide est chauffé à 135 °C pendant 18 heures. Après refroidissement le milieu réactionnel est versé dans de l'eau et le produit est extrait avec de l'acétate d'éthyle. La phase organique est lavée avec de l'eau, séchée sur sulfate de sodium et le solvant est évaporé sous pression réduite. Le résidu est purifié sur colonne de gel de silice en éluant avec un mélange de dichlorométhane, de méthanol et d'ammoniaque (95/5/0,5) pour donner 0,5 g de cristaux après recristallisation dans l'acétonitrile, filtration et séchage sous pression réduite.
PF : 250°C
RMN ¹H (CDCl₃) δ : 8,65 (d, 2H), 7,85 (d, 1H), 7,5-7,7 (m, 4H), 7,05 (pseudo t, 2H), 6,95 (d, 1 H), 3,55 (m, 4H), 2,55 (m, 4H), 2,40 (s, 3H) ppm.

### Exemple 2 (composé n°3) :2-(4-Fluoro-phényl)-7,8-diméthyl-6-pipérazin-1-yl-3-pyridin-4-yl-imidazo[1,2-b]pyridazine

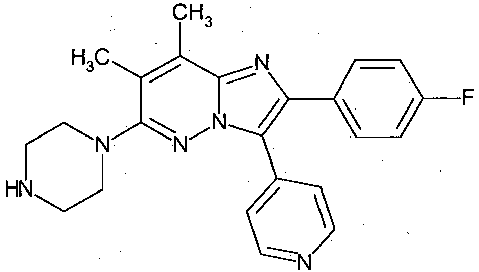

### Synthèse selon le schéma 1, voie 4 :

### Etape 2.1. 6-Chloro-2-(4-fluoro-phényl)-7,8-diméthyl-imidazo[1,2-b]pyridazine

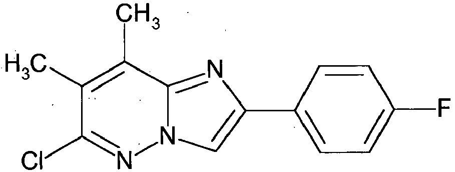

Une solution de 13 g (85 mmoles) de 3-amino-6-chloro-4,5-diméthyl-pyridazine et 23 g (107 mmoles) de 2-bromo-1-(4-fluoro-phényl)-éthanone dans 130 mL d'éthanol est chauffée à reflux pendant 16 heures. Après refroidissement, le solvant est évaporé sous pression réduite et le résidu est repris avec du chloroforme. La phase organique est lavée avec de l'ammoniaque dilué, séchée sur sulfate de sodium, concentrée sous pression réduite pour conduire à un solide brun. Celui-ci est trituré dans de l'acétone pour conduire à 19,2 g de poudre beige.
Rendement 84%
PF : 172 - 174°C
RMN ¹H (CDCl₃) δ : 8,10 (s, 1H), 7,95 (m, 2H), 7,15 (pseudo t, 2H), 2,70 (s, 3H), 2,45 (s, 3H) ppm.

### Etape 2.2. 2-(4-Fluoro-phényl)-7,8-diméthyl-6-pipérazin-1-yl-imidazo[1,2-b]pyridazine

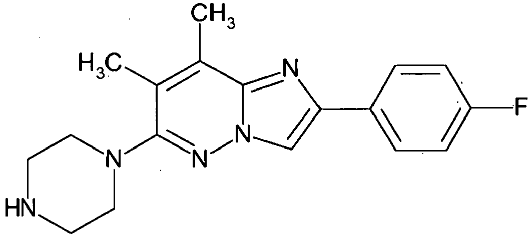

Un mélange de 1,4 g (5,08 mmoles) de 6-chloro-2-(4-fluoro-phényl)-7,8-diméthyl-imidazo[1,2-*b*]pyridazine et de 8,7 g (100 mmoles) de pipérazine est chauffé à 150°C pendant 3 heures dans un réacteur. Le milieu est alors versé dans de l'eau et le précipité formé est isolé par filtration. Le produit est ensuite recristallisé dans un mélange d'éther diisopropylique et d'isopropanol pour donner 1,15 g de poudre blanche.
Rendement : 70%.
RMN ¹H (CDCl₃) δ : 8,00 (s; 1 H), 7,95 (pseudo dd, 2H), 7,15 (pseudo t, 2H), 3,15 (m, 8H), 2,65 (s, 3H), 2,35 (s, 3H) ppm.

### Etape 2.3. 2-(4-Fluoro-phényl)-3-iodo-7,8-diméthyl-6-pipérazin-1-yl-imidazo[1,2-b]pyridazine

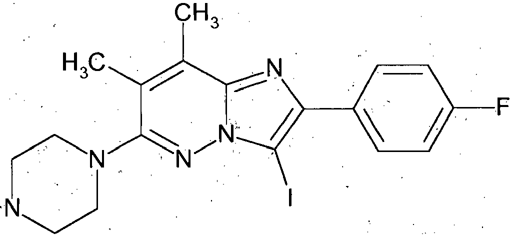

A une solution refroidie à 0°C de 1,15 g (3,53 mmoles) de 2-(4-fluoro-phényl)-7,8-diméthyl-6-pipérazin-1-yl-imidazo[1,2-*b*]pyridazine dans 20 mL de dichlorométhane, on ajoute goutte à goutte une solution de 2,29 g (14,1 mmoles) de monochlorure d'iode dans 5 mL de méthanol et le milieu est agité pendant 30 minutes à température ambiante. On additionne ensuite une solution à 5% de thiosulfate de sodium et l'on basifie le milieu par addition d'hydrogénocarbonate de sodium.

Le produit est extrait avec du dichlorométhane la phase organique séchée sur sulfate de sodium, filtrée et le solvant évaporé pour donner 0,82 g poudre jaune après cristallisation dans l'éther diéthylique et séchage.
Rendement: 51%.
RMN ¹H (CDCl₃) δ : 8,15 (pseudo dd, 2H), 7,35 (pseudo t, 2H), 3,15 (m, 4H), 2,95 (m, 4H), 2,5 (s, 3H), 2,30 (s, 3H) ppm.

### Etape 2.4. 4-[2-(4-Fluoro-phényl)-3-iodo-7,8-diméthyl-imidazo[1,2-b]pyridazin-6-yl]-pipérazine-1-carboxylate de tertio-butyle

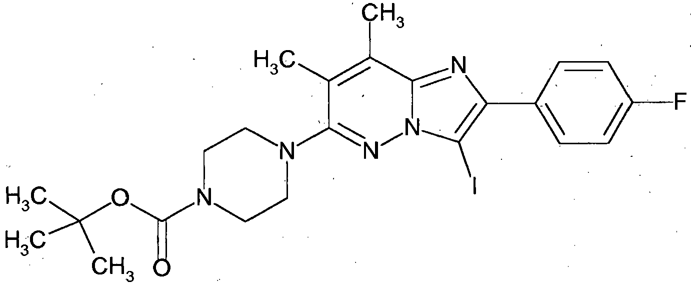

Une solution de 0,70 g (1,55 mmole) de 2-(4-fluoro-phényl)-3-iodo-7,8-diméthyl-6-pipérazin-1-yl-imidazo[1,2-*b*]pyridazine et de 19 mg (0,16 mmole) de diméthylaminopyridine dans 10 mL de tétrahydrofurane est traitée avec 0,41 g (1,9 mmole) de carbonate de di-*tertio*-butyle pendant 1 heure. Le solvant est alors évaporé et le solide obtenu est recristallisé dans l'acétonitrile. On isole ainsi 0,67 g de produit après séchage.
Rendement: 78%.
RMN ¹H (CDCl₃) δ : 8,00 (pseudo dd, 2H), 7,10 (pseudo t, 2H), 3,60 (m, 4H), 3,15 (m, 4H), 2,55 (s, 3H), 2,25 (s, 3H), 1,40 (s, 9H) ppm.

### Etape 2.5. 4-[2-(4-Fluoro-phényl)-7,8-diméthyl-3-pyridin-4-yl-imidazo[1,2-b]pyridazin-6-yl]-pipérazine-1-carboxylate de tertio-butyle

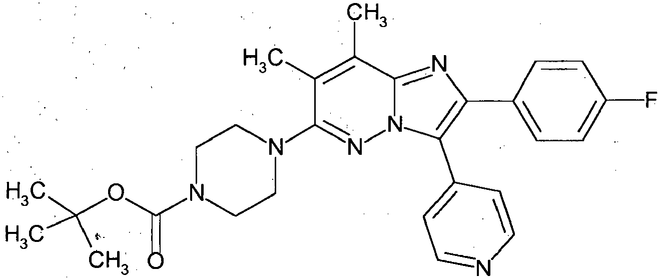

A un mélange de 0,66 g (1,2 mmole) de 4-[2-(4-fluoro-phényl)-3-iodo-7,8-diméthyl-imidazo[1,2-*b*]pyridazin-6-yl]-pipérazine-1-carboxylate de *tertio*-butyle dans 15 mL d'un mélange de tétrahydrofurane et d'eau (9:1), on additionne 1,17 g (3,6 mmole) de carbonate de césium et 0,31 g (1,4 mmoles) d'acide (pyridin-4-yl) boronique. Après barbotage d'un courant d'argon pendant quelques instants, on ajoute 88 mg (0,11 mmole) de complexe de 1,1'-bis(diphénylphosphinoferrocènedichloro-palladium (II) et de dichlorométhane (PdCl₂(dppf).CH₂Cl₂) et la réaction est portée au reflux sous argon pendant 18 heures. Le mélange est alors versé dans de l'eau et le produit est extrait avec du dichlorométhane la phase organique séchée sur sulfate de sodium, filtrée et le solvant évaporé pour donner un solide marron. Le produit est purifié par chromatographie sur gel de silice en éluant avec un mélange d'acétate d'éthyle et de cyclohexane (3/7) pour donner 0,44 g de poudre blanche.
Rendement : 73%
PF : 231 - 233°C
RMN ¹H (CDCl₃) δ : 8,55 (pseudo d, 2H), 7,55-7,75 (m, 4H), 7,00 (pseudo t, 2H), 3,55 (m, 4H), 3,05 (m, 4H), 2,60 (s, 3H), 2,25 (s, 3H), 1,40 (s, 9H) ppm.

### Etape 2.6. 2-(4-Fluoro-phényl)-7,8-diméthyl-6-pipérazin-1-yl-3-pyridin-4-yl-imidazo[1,2-b]pyridazine

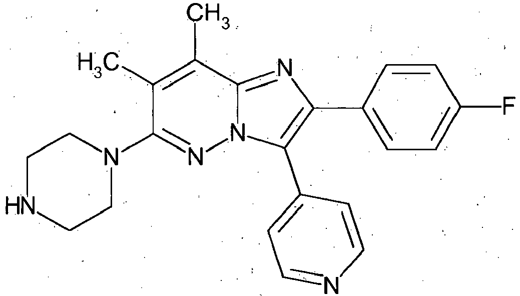

A une solution de 0,43 g (0,86 mmoles) de 4-[2-(4-fluoro-phényl)-7,8-diméthyl-3-pyridin-4-yl-imidazo[1,2-*b*]pyridazin-6-yl]-pipérazine-1-carboxylate de *tertio*-butyle dans 5 mL de dichlorométhane à 0°C on ajoute 0,64 mL (8,6 mmoles) d'acide trifluoroacétique goutte à goutte. Après 4 h d'agitation à température ambiante on additionne à nouveau 0,64 mL (8,6 mmoles) d'acide trifluoroacétique et on laisse la réaction 18 heures. Le solvant est ensuite éliminé sous pression réduite et le résidu est repris avec de l'eau. La phase aqueuse résultante est lavée avec de l'éther puis basifiée par addition d'une solution aqueuse d'hydrogénocarbonate de sodium. Le produit est extrait avec du dichlorométhane et la phase organique séchée sur sulfate de sodium, filtrée et le solvant évaporé pour donner 0,285 g de poudre blanche.
Rendement 83%
PF : 233 - 235°C
RMN ¹H (CDCl₃) δ : 8,60 (pseudo d, 2H), 7,55-7,75 (m, 4H), 7,05(pseudo t, 2H), 3,00-3,2 (m, 4H), 2,65 (s, 3H), 2,35 (s, 3H) ppm.

### Exemple 3 ( composé n°58) : {4-[2-(4-Fluoro-phényl)-6-(4-méthyl-pipérazin-1-yl)-imidazo[1,2-b]pyridazin-3-yl]-pyridin-2-yl}-diméthyl-amine

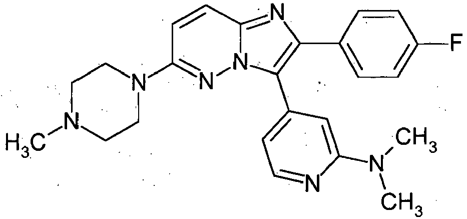

### Synthèse selon le schéma 3 :

### Etape 3.1. 6-(4-Benzyl-pipérazin-1-yl)-pyridazin-3-ylamine

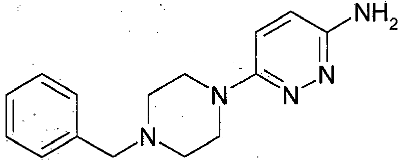

48,9 g (278 mmoles) de 1-benzylpipérazine et 12,0 g (92,6 mmoles) de 3-amino-6-chloropyridazine sont chauffés à 160°C pendant 1 heure. L'huile marron obtenue est versée dans 500 mL d'une solution de bicarbonate de sodium aqueuse et le produit est extrait avec du dichlorométhane. La phase organique est séchée puis concentrée sous pression réduite. L'huile obtenue est triturée dans l'éther diéthylique et 20,5 g de solide sont isolés après filtration et séchage.
Rendement : 82%.
RMN ¹H (CDCl₃) δ : 7,45-7,65 (m, 6H), 7,20 (s, 1H), 5,5 (massif large, 2H) 3,80 (s, 2H), 3,60-3,75 (m, 4H), 2,80-2,85 (m, 4H) ppm.

### Etape 3.2. 2-(4-Fluoro-phényl)-3-(2-fluoro-pyridin-4-yl)-6-(4-méthyl-pipérazin-1-yl)-imidazo[1,2-b]pyridazine

### Synthèse selon le schéma 5a :

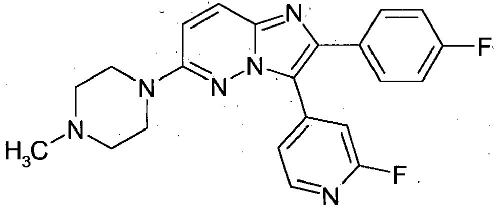

Un mélange de 0,77 g (2,5 mmoles) de 2-bromo-1-(4-fluoro-phényl)-2-(2-fluoro-pyridin-4-yl)-éthanone (CAS : 302839-10-7) et de 0,57 g (3,0 mmoles) de 6-(4-méthyl-pipérazin-1-yl)-pyridazin-3-ylamine (CAS : 66346-94-9) dans 15 ml d'éthanol est porté au reflux pendant 1 h 30. Après refroidissement, le milieu est repris avec du chloroforme et lavé avec une solution aqueuse de bicarbonate de sodium saturée.

La phase organique est ensuite séchée sur sulfate de sodium puis concentrée sous pression réduite pour conduire à un solide orangé.

Le solide obtenu, est purifié par chromatographie sur gel de silice (7 g) en éluant avec un mélange de dichlorométhane de méthanol et d'ammoniaque (95/5/0,5). Le produit obtenu est cristallisé dans l'acétonitrile au reflux pour conduire à 0,76 g de poudre blanche après filtration et séchage.
PF : 250 - 255°C
RMN ¹H (CDCl₃) δ : 8,20 (d, 1H), 7,80 (d, 1H), 7,60 (m, 2H), 7,4 (m, 2H), 7,10 (m, 2H), 6,95 (d, 1H), 3,60 (m, 4H), 2,60 (m, 4H), 2,40 (s, 3H) ppm.

### Etape 3.3. {4-[2-(4-Fluoro-phényl)-6-(4-méthyl-pipérazin-1-yl)-imidazo[1,2-b]pyridazin-3-yl]-pyridin-2-yl}-diméthyl-amine

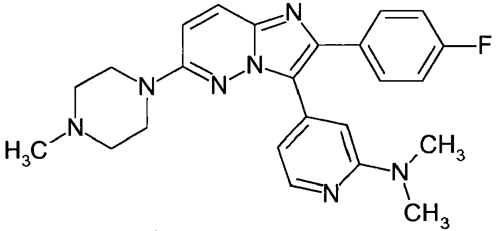

Dans un autoclave, on introduit 0,10 g (0,25 mmole) de 2-(4-fluoro-phényl)-3-(2-fluoro-pyridin-4-yl)-6-(4-méthyl-pipérazin-1-yl)-imidazo[1,2-b]pyridazine et 10 mL de diméthylamine. Le mélange est chauffé à 150°C pendant une nuit puis refroidit et versé dans de l'eau. Le produit est extrait avec du chlorforme, la phase organique séchée sur sulfate de sodium puis concentrée sous pression réduite pour conduire à un solide. Celui-ci est cristallisé et recristallisé dans l'acétonitrile pour conduire à 0,028 g de poudre blanche après refroidissement, filtration et séchage.
PF: 180 - 183°C
RMN ¹H (CDCl₃) δ : 8,15 (d, 1H), 7,70 (d, 1H), 7,70 (m, 2H), 6,95(m, 2H), 6,80 (m, 2H), 6,70 (d, 1 H), 3,50 (m, 4H), 3,00 (s, 6H), 2,50 (m, 4H), 2,30 (s, 3H) ppm.

### Exemple 4 (composé n°59) : 2-(4-Fluoro-phényl)-3-(2-méthoxy-pyridin-4-yl)-6-(4-méthyl-pipérazin-1-yl)-imidazo[1,2-b]pyridazine

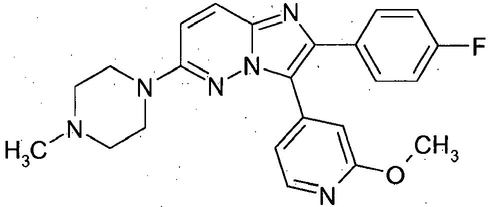

### Synthèse selon le schéma 3 :

A une solution sous argon de 0,10 g (0,25 mmole) de 2-(4-fluoro-phényl)-3-(2-fluoro-pyridin-4-yl)-6-(4-méthyl-pipérazin-1-yl)-imidazo[1,2-*b*]pyridazine dans la N-méthyl-pyrrolidone, on ajoute 0,65 mL (3,4 mmoles) de méthylate de sodium à 30% en poids dans le méthanol. Après 4 jours d'agitation à température ambiante, le milieu est versé dans 200 mL d'eau et le produit est extrait avec de l'acétate d'éthyle. La phase organique est séchée sur sulfate de sodium puis concentrée sous pression réduite pour conduire à un solide jaune pâle. Le solide obtenu, est purifié par chromatographie sur gel de silice (7 g) en éluant avec un mélange de dichlorométhane de méthanol et d'ammoniaque (95/05/0,5). Le produit obtenu est cristallisé dans l'acétonitrile au reflux pour conduire à 0,04 g de poudre blanche après refroidissement, filtration et séchage.
PF : 189 - 194°C
RMN ¹H (CDCl₃) δ : 8,20 (d, 1H), 7,80 (d, 1H), 8,6 (m, 2H), 7,15-6,95 (m, 4H), 6,90 (d, 1H), 4,00 (s, 3H), 3,55 (m, 4H), 2,55 (m, 4H), 2,40 (s, 3H) ppm.

### Exemple 5 (composé n°4) : 4-[2-(4-Fluoro-phényl)-6-pipérazin-1-yl-imidazo[1,2-b]pyridazin-3-yl]-pyridin-2-ylamine

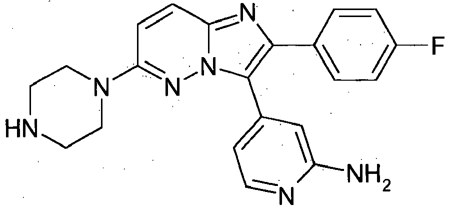

### Etape 5.1. 2-(4-Fluoro-phényl)-6-pipérazin-1-yl-imidazo[1,2-b]pyridazine

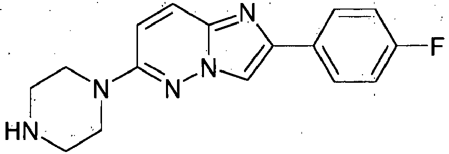

Dans un réacteur, contenant 94 g (823 mmoles) de 1-formylpipérazine 98% à 135°C, on additionne 26,5 g (107 mmoles) de 2-(4-fluoro-phényl)-6-chloro-imidazo[1,2-*b*]pyridazine puis le réacteur est fermé et porté à 135°C pendant 2 h 30.

Le réacteur est alors refroidi et le milieu réactionnel est versé dans 1,5 L d'eau. Le solide jaunâtre est isolé par filtration et repris dans 700 mL de tétrahydrofurane. On ajoute ensuite 800 mL d'acide sulfurique 4N et la suspension est portée au reflux pendant une nuit.

Le mélange est filtré à chaud et le filtrat est partiellement concentré sous pression réduite. Cette phase aqueuse est lavée 2 fois avec de l'éther diéthylique puis alcalinisée au moyen d'une solution d'ammoniaque refroidie. Le précipité formé est agité pendant 30 minutes et isolé par filtration sur fritté et lavage avec de l'eau. Le solide est repris avec du chloroforme et la phase organique obtenue est lavée avec de l'eau, séchée sur sulfate de sodium puis concentrée sous pression réduite pour conduire à un solide jaune-brun. On isole 21,5 g de poudre jaune pâle par clairçage dans un mélange de 200 mL d'éther diisopropylique et de 10 mL d'isopropanol au reflux puis filtration à froid et séchage.
PF : 200 - 203°C
Rendement: 61%.
RMN ¹H (CDCl₃) δ : 8,05 (s, 1H), 7,85 (m, 2H), 7,65 (d, 1H), 7,05 (m, 2H), 6,94 (d, 1H), 3,40 (s, 4H), 2,85 (s, 4H) ppm.

### Etape 5.2. 4-[2-(4-Fluoro-phényl)-imidazo[1,2-b]pyridazin-6-yl]-pipérazine-1-carboxylate de tertio-butyle

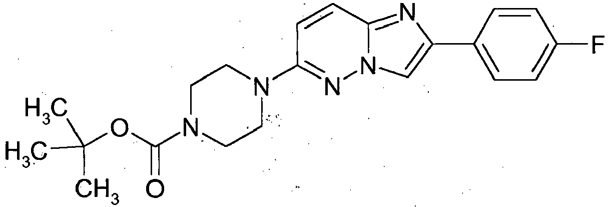

A une solution de 21,5 g (72,3 mmoles) de 2-(4-fluoro-phényl)-f-pipérazin-1-yl-imidazo[1,2-*b*]pyridazine et de 0,44 g (3,6 mmole) de diméthylaminopyridine dans 500 mL de chloroforme, on additionne goutte à goutte, une solution d'anhydride *tert*-butylique en solution dans 100 mL de chloroforme Après 30 min à d'agitation à température ambiante, le milieu est versé dans une solution aqueuse saturée en d'hydogénocarbonate de sodium. La phase organique obtenue est lavée avec de l'eau, séchée sur sulfate de sodium puis concentrée sous pression réduite pour conduire à un solide brun. Enfin, on isole 28,0 g de poudre beige après trituration dans un mélange de 200 mL d'éther diisopropylique et de 10 mL d'isopropanol au reflux puis filtration à froid et séchage.
Rendement: 97%.
RMN ¹H (CDCl₃) δ : 7,78-8,00 (m, 3H), 7,80 (d, 1 H), 7,15 (pseudo t, 1H) ,6,85 (d, 1H), 3,45-3,7 (m, 8H), 1,5 (s, 9H) ppm.

### Etape 5.3. 4-[2-(4-Fluoro-phényl)-3-iodo-imidazo[1,2-b]pyridazin-6-yl]-pipérazine-1-carboxylate de tertio-butyle

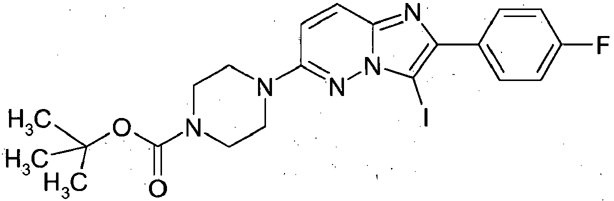

A une solution de 28;0 g (70,5 mmoles) de 4-[2-(4-fluoro-phényl)-imidazo[1,2-*b*]pyridazin-6-yl]-pipérazine-1-carboxylate de *tertio*-butyle dans 800 ml de tétrahydrofurane refroidie vers 0°C on additionne 17,4 g (77,5 mmoles) de N-iodosuccinimide. Après 18 heures d'agitation à température ambiante, le solvant est évaporé sous pression réduite.
Le solide rouge-orangé obtenu est trituré dans 1,5 L d'eau
et séparé par filtration. Le solide est ensuite repris avec du chloroforme et la phase organique obtenue est lavée avec 1 L d'une solution à 5% de thiosulphate de sodium, séchée sur sulfate de sodium puis concentrée sous pression réduite pour conduire à un solide brun.

On isole enfin 32,8 g de poudre jaune pâle par clairçage dans un mélange de 300 mL d'éther diisopropylique et de 50 mL d'isopropanol au reflux puis filtration à froid et séchage.
Rendement : 89%
RMN ¹H (CDCl₃) δ : 7,85 (pseudo q, 2H), 7,60 (d, 1 H), 7,05 (pseudo t, 1H) ,6,75 (d, 1H), 3,5 (m, 8H), 1,4 (s, 9H) ppm.

### Etape 5.4. 4-[3-(2-Chloro-pyridin-4-yl)-2-(4-fluoro-phényl)-imidazo[1,2-b]pyridazin-6-yl]-pipérazine-1-carboxylate de tertio-butyle (selon le schéma 5b)

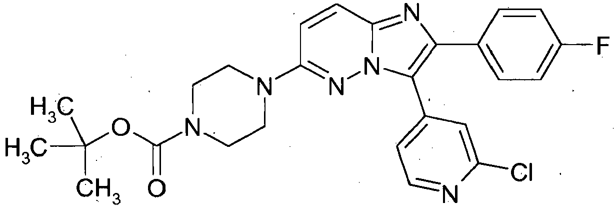

A une suspension de 1,25 g (2,39 mmoles) de 4-[2-(4-fluoro-phényl)-3-iodo-imidazo[1,2-*b*]pyridazin-6-yl]-pipérazine-1-carboxylate de *tertio*-butyle dans un mélange tétrahydrofurane et d'eau, on additionne 2,33 g (7,17 mmoles) de carbonate de césium et 0,45 g (2,9 mmoles) d'acide 2-chloropyridine-4-boronique. Après barbotage d'un courant d'argon pendant quelques instants, on ajoute 0,18 g (0,21 mmole) de 1,1'-bis(diphénylphosphinoferrocènedichloro)palladium (II) (PdCl₂(dppf)) et la réaction est portée au reflux sous argon pour 18 heures. Le solvant est ensuite chassé sous pression réduite, le résidu est repris avec du chloroforme et la phase organique obtenue est lavée avec de l'eau, séchée sur sulfate de sodium, filtrée et le filtrat concentré sous pression réduite. Le solide brun obtenu est purifié par chromatographie sur gel de silice (50 g) en éluant avec un mélange de dichlorométhane de méthanol et d'ammoniaque (95/5/0,5). Le produit obtenu est cristallisé dans 20 ml d'acétonitrile au reflux pour conduire à 0,95 g de poudre blanche après refroidissement, filtration et séchage.
Rendement: 78%
RMN ¹H (CDCl₃) δ : 8,25 (d, 1 H), 7,70 (d, 1 H), 7,60 (s, 1 H), 7,45 (pseudo q, 2H), 7,25 (d, 1 H), 6,95 (pseudo t, 2H), 6,80 (d, 1 H), 3,25-3,50 (m, 8H), 1,35 (s, 9H) ppm.

### Etape 5.5. 4-[3-[2-(Benzhydrylidène-amino)-pyridin-4-yl]-2-(4-fluoro-phényl)-imidazo[1,2-b]pyridazin-6-yl]-pipérazine-1-carboxylate de tertio-butyle Synthèse selon le schéma 3 :

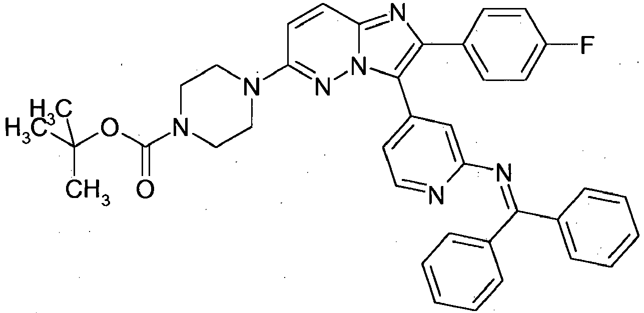

A une suspension de 0,95 g (1,9 mmole) de 4-[3-(2-chloro-pyridin-4-yl)-2-(4-fluoro-phényl)-imidazo[1,2-*b*]pyridazin-6-yl]-pipérazine-1-carboxylate de *tertio*-butyle et 0.41 g (2,2 mmoles) de benzhydrylidéne-amine dans 100 mL de toluène anhydre, on additionne sous argon 0,25 g (2,6 mmoles) de tert-butoxyde de sodium, 46 mg (0,075 mmole) de (+/-)-2,2'-bis(diphénylphosphino)-1,1'-binaphtyle.

Après barbotage d'un courant d'argon pendant quelques instants, on ajouté 34 mg (0,037 mmole) de tris(dibenzylidèneacétone)dipalladium(0) et la réaction est portée au reflux sous argon pour 18 heures. Le milieu est filtré à chaud et le solvant est ensuite chassé sous pression réduite. Le résidu est repris avec du chloroforme et la phase organique obtenue est lavée avec de l'eau, séchée sur sulfate de sodium, filtrée et le filtrat concentré sous pression réduite. Le solide brun obtenu, est purifié par chromatographie sur gel de silice (50 g) en éluant avec un mélange de dichlorométhane dé méthanol et d'ammoniaque (95/2/0,2). Le produit obtenu est cristallisé dans un mélange de 20 mL d'acétonitrile et de 5 mL n-butanol au reflux pour conduire à 0,80 g de poudre blanche après refroidissement, filtration et séchage.
Rendement : 65%
RMN ¹H (CDCl₃) δ : 8,25 (d, 1 H), 7,70 (m, 3H), 7,05-7,45 (m), 6,85-6,95 (m, 4H), 6,80 (d, 1H), 3,55-3,30 (m, 8H), 1,40 (s, 9H) ppm.

### Etape 5.6. 4-[2-(4-Fluoro-phényl)-6-pipérazin-1-yl-imidazo[1,2-b]pyridazin-3-yl]-pyridin-2-ylamine

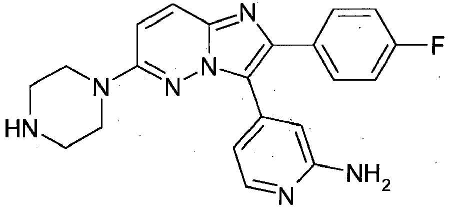

Une suspension de 0,80 (1,22 mmole) de 4-[3-[2-(benzhydrylidène-amino)-pyridin-4-yl]-2-(4-fluoro-phényl)-imidazo[1,2-*b*]pyridazin-6-yl]-pipérazine-1-carboxylate de *tertio*-butyle dans 70 mL d'acide chlorhydrique aqueux est portée à 80°C pendant environ 1 h 30. Après refroidissement, la phase aqueuse est lavée deux fois avec de l'éther diéthylique, puis alcalinisée par addition d'ammoniaque aqueux glacé. Le produit est extrait avec du chloroforme et la phase organique obtenue est lavée avec de l'eau, séchée sur sulfate de sodium, filtrée et le filtrat concentré sous pression réduite. Le solide brun obtenu, est purifié par chromatographie sur gel de silice (35 g) en éluant avec un mélange de dichlorométhane de méthanol et d'ammoniaque (90/10/1). Le produit obtenu est cristallisé dans 20 mL d'acétonitrile au reflux pour conduire à 0,38 g de poudre blanche après refroidissement, filtration et séchage
PF : 255°C décomposition
RMN ¹H (CDCl₃) δ : 8,05 (d, 1H), 7,80 (d, 1H), 7,60-7,70 (m, 2H), 6,85-7,15 (m, 4H), 6,80 (s, 1 H), 4,45 (massif large, 2H), 3,40-3,60 (m, 4H), 2,95-3,10 (m, 4H) ppm.

### Exemple 6 ( composé n°113) : 4-[2-(4-Fluoro-phényl)-6-[5-benzyl-(hexahydro-pyrrolo[3,4-c]pyrrol-2(1H)-yl)]1-7,8-diméthyl-imidazo[1,2-b]pyridazin-3-yl]-pyridin-2-ylamine.

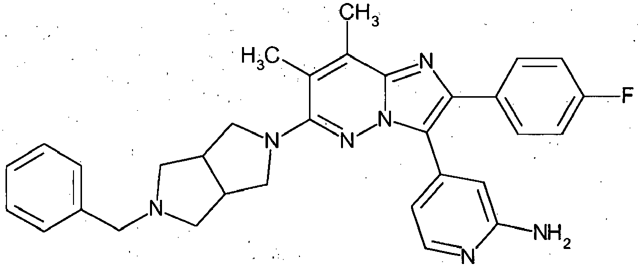

### Synthèse selon le schéma 1, voie 4 :

### Etape 6.1. [4-(4,4,5,5-tétraméthyl-1,3,2-dioxaborolan-2-yl)pyridin-2-yl]carbamate de tert-butyle

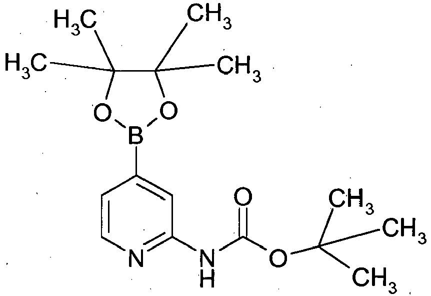

A une solution de 6,76 (24,8 mmoles) de (4-bromo-pyridin-2-yl)carbamate de *tert*-butyle (Deady, Leslie W.; Korytsky, Olga L.; Rowe, Jeffrey E.; Aust. J. Chem.; 35; 10; 1982; 2025-2034) dans 150 mL de diméthylformamide, on additionne 8,0 g (81 mmoles) d'acétate de potassium préalablement séché à 130 °C et 6,9 g (27 mmoles) de bis(pinacolato)diboron. On fait ensuite barbotter un courant d'argon pendant quelques instant et on ajoute 1,2 g ( 1,5 mmoles) de 1,1'-bis(diphénylphosphino)ferrocènedichloropalladium (II). Le mélange est agité à 80°C sous argon, pendant 2 heures puis vesé sur une solution saturée aqueuse de chlorure dammonium. Le produit est extrait avec de l'acétate d'éthyle, la phase organique est séchée sur sulfate de sodium et le solvant est chassé sous pression réduite. Le résidu est trituré dans 300 mL d'éther diisopropylique à reflux et l'insolube est séparé par filtration. Le filtrat est refroidit, partiellement concentré sous pression réduite. Après addition de 70 mL d'hexane, le précipité formé est isolé par filtration pour conduire à 4,2 g de solide orangé après séchage.
Rendement : 53%
PF : 188 -193°C
RMN ¹H (CDCl₃) δ : 8,15 (m, 2H), 7,65 (s large, 1H), 7,15 (d, 1H), 1,40 (s, 9H), 1,20 (s, 12H) ppm.

### Etape 6.2. 6-(5-Benzyl-hexahydro-pyrrolo[3,4-c]pyrrol-2-yl)-2-(4-fluoro-phényl)-7,8-diméthyl-imidazo[1,2-b]pyridazine

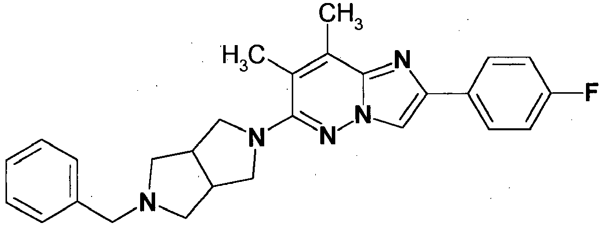

Un mélange de 3,00 (10,9 mmoles) de 6-chloro-2-(4-fluoro-phényl)-7,8-diméthyl-imidazo[1,2-*b*]pyridazine et de 6,6 g (33 mmoles) de 2-benzyl-octahydro-pyrrolo[3,4-*c*]pyrrole dans 20 ml de pentanol est chauffé à 150°C pendant 2 jours dans un réacteur. Le milieu est alors versé dans une solution aqueuse d'acide chlorhydrique 1N. La phase aqueuse est lavée avec de l'acétate d'éthyle puis basifiée au moyen de soude.

Le produit est extrait avec du dichlorométhane, la phase organique est séchée sur sulfate de sodium puis concentrée sous pression réduite. L'huile jaune obtenue est chromatographiée sur gel de silice en éluant avec du dichlorométhane contenant 3% de méthanol et 0,3% d'ammoniaque. Le produit est ensuite cristallisé dans l'éther diisopropylique pour donner 3,2 g de poudre légèrement jaune.
PF : 115-117°C
RMN ¹H (CDCl₃) δ : 7,85 (m; 3H), 7,30 (m, 5H), 7,05 (pseudo t, 2H), 3,60 (s, 2H), 3,30 (m, 2H), 3,10 (m, 2H), 2,80 (m, 4H), 2,55 (s, 3H), 2,35 (m, 2H), 2,25 (s, 3H) ppm.

### Etape 6.3. 6-(5-Benzyl-hexahydro-pyrrolo[3,4-c]pyrrol-2-yl)-2-(4-fluoro-phényl)-3-iodo-7,8-diméthyl-imidazo[1,2-b]pyridazine

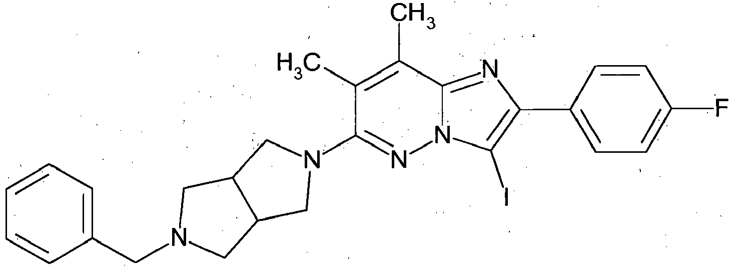

A une solution refroidie à 0°C de 3,2 g (7,3 mmoles) de 6-(5-benzyl-hexahydro-pyrrolo[3,4-*c*]pyrrol-2-yl)-2-(4-fluoro-phényl)-7,8-diméthyl-imidazo[1,2-*b*]pyridazine dans 20 mL de chloroforme, on ajoute goutte à goutte une solution de 1,8 g (11 mmoles) de monochlorure d'iode dans 3 mL de méthanol et le milieu est agité pendant une heure à température ambiante. On basifie ensuite le milieu par addition d'une solution aqueuse d'hydrogénocarbonate de sodium et on additionne ensuite une solution à 5% de thiosulfate de sodium jusqu'à décoloration

Le produit est extrait avec du chloroforme, la phase organique séchée sur sulfate de sodium, filtrée et le solvant évaporé pour donner 3,2 g poudre jaune après cristallisation dans l'éther diéthylique et séchage.
RMN ¹H (DMSOd₆) δ : 8,35 (m; 2H), 7,65 (m, 7H), 3,80 (s, 2H), 3,65 (m, 2H), 3,40 (m, 2H), 3,07 (m, 2H), 2,90 (m, 2H), 2,65 (m, 2H), 2,5 (s, 3H) ppm.

### Etape 6.4. {4-[6-(5-Benzyl-hexahydro-pyrrolo[3,4-c]pyrrol-2-yl)-2-(4-fluoro-phényl)-7,8-diméthyl-imidazo[1,2-b]pyridazin-3-yl]-pyridin-2-y l}-carbamate de tertio-butyle

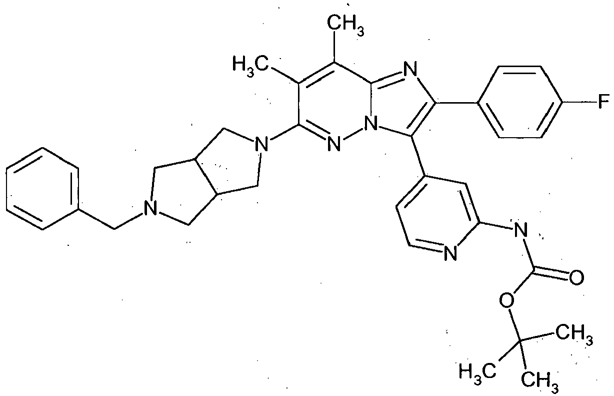

A une solution de 3,60 g (6,34 mmoles) de 6-(5-benzyl-hexahydro-pyrrolo[3,4-*c*]pyrrol-2-yl)-2-(4-fluoro-phényl)-3-iodo-7,8-diméthyl-imidazo[1,2-*b*]pyridazine dans 15 mL d'un mélange de tétrahydrofurane et d'eau (9:1), on additionne 6,2 g (19 mmoles) de carbonate de césium et 2,4 g ( 7,6 mmoles) de [4-(4,4,5,5-tétraméthyl-1,3,2-dioxaborolan-2-yl)pyridin-2-yl]carbamate de *tert*-butyle. Après barbotage d'un courant d'argon pendant quelques instants, on ajoute 0,47 mg (0,57 mmole) de complexe de 1,1'-bis(diphénylphosphinoferrocènedichloropalladium (II) et de dichlorométhane (PdCl₂(dppf).CH₂Cl₂) et la réaction est portée au reflux sous argon pendant 5 heures. Le mélange est alors versé dans 250 mL d'eau et le produit est extrait avec de l'acétate d'éthyle, la phase organique séchée sur sulfate de sodium, filtrée et le solvant évaporé pour donner un solide marron. Le produit est purifié par chromatographie sur gel de silice en éluant avec un mélange de dichlorométhane, de méthanol et d'ammoniaque (95:5:05) pour donner 2,91 g de solide beige.
PF : 214-216°C
RMN ¹H (CDCl₃) δ : 8,70 (s, 1 H), 8,20 (d; 1 H), 7,92 (s, 1 H), 7,7 (m, 2H), 7,3-7,4 (m, 4H), 7,1 (m, 3H), 3,70 (s, 2H), 3,35 (m, 4H), 3,0 (m, 4H), 2,70 (m, 4H), 2,70 (s, 3H), 2,35 (s+m, 3+2H), 1,55 (s, 9H) ppm.

### Etape 6.5. 4-[6-(5-Benzyl-hexahydro-pyrrolo[3,4-c]pyrrol-2-yl)-2-(4-fluoro-phényl)-7,8-diméthyl-imidazo[1,2-b]pyridazin-3-yl]-pyridin-2-ylamine

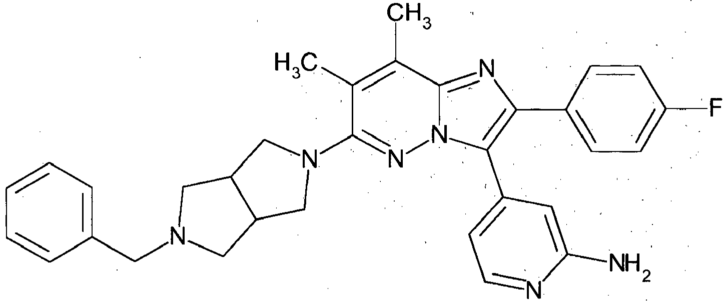

Une solution de 2,9 g (4,58 mmoles) de {4-[6-(5-benzyl-hexahydro-pyrrolo[3,4-*c*]pyrrol-2-yl)-2-(4-fluoro-phényl)-7,8-diméthyl-imidazo[1,2-*b*]pyridazin-3-yl]-pyridin-2-yl}-carbamate de *tertio*-butyle dans 30 mL de dichlorométhane, on additionne 6,8 mL d'acide trifluoroacétique et la réaction est agitée pendant 2 heures.

Le milieu est versé dans 200 mL d'eau et le mélange est basifié par addition d'ammoniaque. La phase organique est séparée, séchée sur sulfate de sodium et concentrée à sec sous pression réduite. On isole ainsi 2,3 g de solide gommeux blanc.
PF : 113°C
RMN ¹H (CDCl₃) δ : 8,10 (d, 1H), 7,70 (m; 2H), 7,3 (m, 4H), 7,1 (pseudo t, 2H), 7,00 (d, 1H), 6,90 (s, 1 H), 4,40 (s large, 2H), 3,65 (s, 2H), 3,3 (m, 4H), 2,9 (m, 4H), 2,65 (s, 3H), 2,35 (s+m, 3+2H), 1,55 (s, 9H) ppm.

### Exemple 7 (composé n°114) : 4-[2-(4-Fluoro-phényl)-6-(hexahydro-pyrrolo[3,4-c]pyrrol-2-yl)-7,8-diméthyl-imidazo[1,2-b]pyridazin-3-yl]-pyridin-2-ylamine

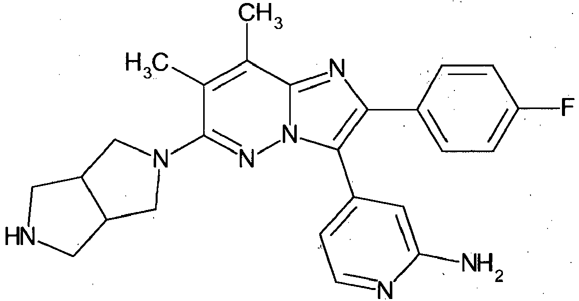

A une solution de 2,30 g (4,3 mmoles) de 4-[6-(5-benzyl-hexahydro-pyrrolo[3,4-*c*]pyrrol-2-yl)-2-(4-fluoro-phényl)-7,8-diméthyl-imidazo[1,2-*b*]pyridazin-3-yl]-pyridin-2-ylamine dans 150 mL de méthanol, on additionne 4 g (64 mmoles) de formiate d'ammonium et 1 g de palladium sur charbon à 10% contenant 50% d'humidité. Le mélange est agité à reflux pendant 2 heures puis le solvant est éliminé sous pression réduite. Le résidu est repris avec de l'eau et la phase aqueuse résultante est basifiée au moyen de soude aqueuse 1N. Le produit est extrait avec du chloroforme, la phase organique lavée avec de l'eau, séchée sur sulfate de sodium, filtrée et le solvant évaporé pour donner une huile orangée. Après purification par chromatographie sur colonne de gel de silice en éluant avec un mélange de dichlorométhane de méthanol et d'ammoniaque (87:13:1,3) pour donner 1,23 g de poudre blanche après cristallisation dans l'éther et séchage sous presssion réduite.
PF : 254-256°C
RMN ¹H (DMSOd₆) δ : 7,95 (d, 1 H), 7,60 (pseudo dd; 2H), 7,20 (pseudo t, 2H), 6,65 (s, 1 H), 6,65 (d, 1H), 5,95 (s large, 1H), 3,3 (m, 6H), 2,8-3,1 (mol 4H), 2,8-2,25 (m), 2,25 (s, 3H),ppm.

### Exemple 8 (composé n°111) : 4-[2-(4-Chloro-phényl)-6-(hexahydro-pyrrolo[3,4-c]pyrrol-2(1H)-yl)-imidazo[1,2-b]pyridazin-3-yl]-pyridin-2-ylamine

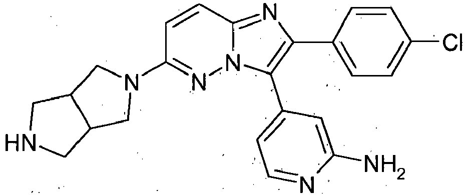

### Procédé de synthèse selon le schéma 1, voie 1 :

### Etape 8.1. 6-Chloro-2-(4-chloro-phényl)-3-iodo-imidazo[1,2-b]pyridazine (selon schéma 6)

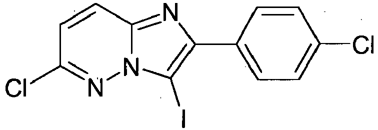

A une solution refroidie à 0°C de 3,00 g (11,1 mmoles) de 6-chloro-2-(4-chloro-phényl)-3-iodo-imidazo[1,2-*b*]pyridazine (CAS : 1844-56-0) dans 55 mL de chloroforme, on additionne goutte à goutte rapide une solution de 2,70 g (16,7 mmoles) de monochlorure d'iode dans 15 mL de chloroforme. Après retour à la température ambiante et 3 heures d'agitation, on additionne encore 0,75 g (4,6 mmoles) de monochlorure d'iode et la réaction est agitée une heure supplémentaire. Le mélange est ensuite traité avec une solution aqueuse à 5% de thiosulfate de sodium. Le produit est extrait avec du dichlorométhane, La phase organique est séchée par filtration sur cartouche filtrante hydrophobe et concentrée sous pression réduite. Le résidu est trituré dans de l'acétonitrile, le solide est isolé par filtration. On isole 3,8 g de poudre beige après séchage sous vide.
PF : 201-203°C
RMN ¹H (CDCl₃) δ : 8,20 (d; 1H), 8,10 (d, 2H), 8,6 (d, 2H), 7,45 (d, 1H) ppm.

### Etape 8.2. {4-[6-Chloro-2-(4-chloro-phényl)-imidazo[1,2-b]pyridazin-3-yl]-pyridin-2-yl}-carbamate de tert-butyle

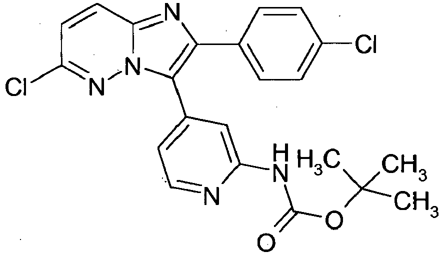

A une suspension 3 g (7,31 mmoles) de 6-chloro-2-(4-chloro-phényl)-3-iodo-imidazo[1,2-*b*]pyridazine dans 183 mL d'un mélange de tétrahydrofurane et d'eau (9:1), on additionne 7,1 g (22 mmoles) de carbonate de césium et 2,90 g (8,8 mmoles) [4-(4,4,5,5-tétraméthyl-1,3,2-dioxaborolan-2-yl)pyridin-2-yl]carbamate de *tert*-butyle. Après barbotage d'un courant d'argon pendant quelques instants, on ajoute 0,54 g (0,66 mmoles) de 1,1'-bis(diphénylphosphinoferrocènedichloro palladium (II) et la réaction est portée au reflux sous argon pour 18 heures. Après filtration sur filtre Whatman et sur célite, le fitrat est alors concentré sous pression réduite pour donner 7,0 g de résidu brun. Le résidu est repris avec de l'eau, le produit est extrait avec du dichlorométhane, la phase organique séchée sur sulfate de sodium, filtrée et le solvant évaporé pour donner 3,5 g poudre sombre.

Le produit est purifié par chromatographie sur gel de silice en éluant avec un mélange de dichlorométhane et d'acétate d'éthyle (100:0 à 80:20) pour donner 1,8 g de cristaux beiges après cristallisation dans l'éther diisopropylique et séchage sous pression réduite.
PF : 212-214°C
RMN ¹H (DMSOd₆) δ : 9,9 (s; 1 H), 8,4 (d, 1 H), 8,3 (d, 1 H), 7,95 (s, 1 H), 7,60 (d, 2H), 7,45 (m, 3H), 7,15 (d, 1H), 4,40 (s, 9H) ppm.

### Etape 8.3. 4-[6-Chloro-2-(4-chloro-phényl)-imidazo[1,2-b]pyridazin-3-yl]-pyridin-2-ylamine

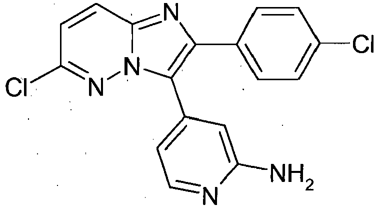

A une suspension 0,76 g (1,67 mmoles) de {4-[6-chloro-2-(4-chloro-phényl)-imidazo[1,2-*b*]pyridazin-3-yl]-pyridin-2-yl}-carbamate de *tert*-butyle dans 8 mL de dichlorométhane, on additionne 4 mL (48 mmoles) d'acide chlorhydrique. Après 2 heures d'agitation à température ambiante, le solvant est évaporé sous pression réduite, le résidu huileux est repris avec de l'ammoniaque et le produit est extrait avec du dichlorométhane. La phase organique est filtrée sur cartouche hydrophobe et concentrée sous pression réduite Le solide obtenu est trituré avec de l'éther diisopropylique pour donner 0,56 g de solide après filtration et séchage sous pression réduite.
PF : 269-271 °C
RMN ¹H (DMSOd₆) δ : 8,25 (d; 1 H), 8,05 (d, 1 H), 7,65 (d, 1 H), 7,45 (m, 3H), 6,65 (m, 2H), 6,1 (s large, 2H) ppm.

### Etape 8.4. 5-[3-(2-Amino-pyridin-4-yl)-2-(4-chloro-phényl)-imidazo[1,2-b]pyridazin-6-yl]-hexahydro-pyrrolo[3,4-c]pyrrole-2-carboxylate de tert-butyle

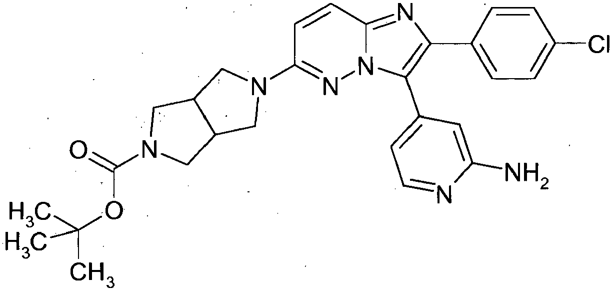

Un mélange de 0,15 g (0,42 mmole) de 4-[6-chloro-2-(4-chloro-phényl)-imidazo[1,2-*b*]pyridazin-3-yl]-pyridin-2-ylamine et de 0,36 g (1,7 mmole) d'hexahydro-pyrrolo[3,4-c]pyrrole-2-carboxylate de *tert*-butyle dans 7 mL de pentanol est agité à 135°C pendant 20 heures. Le solvant est alors évaporé sous pression réduite, le résidu huileux est chromatographié sur colonne de gel de silice en éluant avec un gradient par palier de méthanol et d'ammoniaque dans le dichlorométhane (0:0:100 à 2:1:98). On isole 0,16 g de produit après trituration dans l'éther diisopropylique, filtration et séchage sous pression réduite.
RMN ¹H (DMSOd₆) δ : 7,95 (d; 1 H), 7,85 (d, 1H), 7,55 (d, 2H), 7,40 (d, 2H), 6,90 (d, 1 H), 6,70 (s, 1 H), 6,55 (d, 1 H), 5,95 (s large, 2H), 2,9-3,7 (m), 1,35 (s, 9H) ppm.

### Etape 8.5. 4-[2-(4-Chloro-phényl)-6-(hexahydro-pyrrolo[3,4-c]pyrrol-2-yl)-imidazo[1,2-b]pyridazin-3-yl]-pyridin-2-ylamine

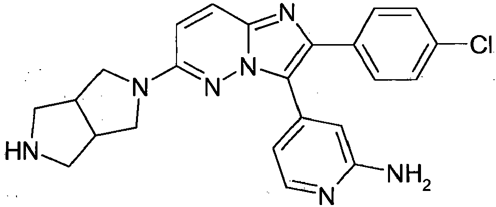

A une solution de 0,16 g (0,29 mmoles) de 5-[3-(2-amino-pyridin-4-yl)-2-(4-chloro-phényl)-imidazo[1,2-*b*]pyridazin-6-yl]-hexahydro-pyrrolo[3,4-*c*]pyrrole-2-carboxylate de *tert*-butyle dans 3 mL de dichlorométhane, on additionne 1,5 mL (18 mmoles) d'acide chlorhydrique concentré. Après 1 heure d'agitation à température ambiante, le solvant est évaporé sous pression réduite, le résidu huileux est repris avec de l'ammoniaque et le produit est extrait avec du dichlorométhane. La phase organique est filtrée sur cartouche hydrophobe et concentrée sous pression réduite. Le solide obtenu est trituré avec de l'éther diisopropylique pour donner 0,091 g de solide après filtration et séchage sous pression réduite.
PF : 267-270°C
RMN ¹H (DMSOd₆) δ : 7,95 (d; 1 H), 7,85 (d, 1 H), 7,60 (d, 2H), 7,40 (d, 2H), 6,95 (d, 1 H), 6,70 (s, 1 H), 6,60 (d, 1 H), 5,95 (s large, 2H), 3,6 (m, 2H), 3,4-3,1 (m), 2,7-3,95 (m, 4H), 2,6 (d, 2H) ppm.

### Exemple 9 (composé n° 65) : 2-{4-[2-(4-Fluoro-phényl)-3-(2-méthoxy-pyridin-4-yl) imidazo[1,2-b]pyridazin-6-yl]-pipérazin-1-yl}-éthanol

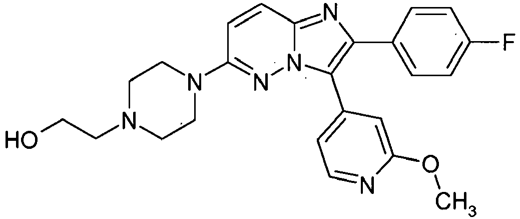

### Synthèse selon le schéma 1, voie 1 :

Etape 9.1. 6-Chloro-2-(4-fluoro-phényl)-3-iodo-imidazo[1,2-*b*]pyridazine

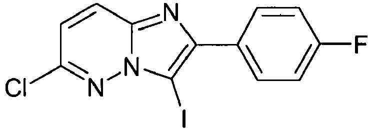

A une solution refroidie à 0°C de 5,20 g (21,0 mmoles) de 6-chloro-2-(4-fluoro-phényl)-imidazo[1,2-*b*]pyridazine (numéro CAS : 244081-70-7) dans 130 mL de chloroforme, on additionne goutte à goutte rapide une solution de 6,61 g (40,9 mmoles) de monochlorure d'iode dans 40 mL de chloroforme. Après retour à la température ambiante et 4 heures d'agitation, le mélange est traité avec une solution aqueuse à 5% de thiosulfate de sodium. Le produit est extrait avec du dichlorométhane, La phase organique est séchée par filtration sur cartouche filtrante hydrophobe et concentré sous pression réduite. Le résidu est trituré dans de l'acétonitrile, le solide est isolé après filtration et rinçage avec de l'éther diisopropylique. On isole 5,7 g de poudre beige après séchage sous vide.
PF : 215°C
RMN ¹H (DMSOd₆) δ : 8,20 (m; 3H), 7,40 (m, 3H) ppm.

### Etape 9.2. 6-Çhloro-2-(4-fluoro-phényl)-3-(2-méthoxy-pyridin-4-yl)imidazo[1,2-b]pyridazine (selon schéma 6)

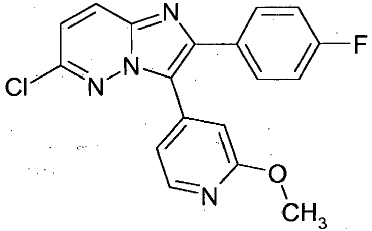

A une suspension de 5,7 g (14,8 mmoles) de 6-chloro-2-(4-fluoro-phényl)-3-iodo-imidazo[1,2-*b*]pyridazine dans 370 mL d'un mélange de tétrahydrofurane et d'eau (9:1), on additionne 14,7 g (44,4 mmoles) de carbonate de césium et 2,77 g (17,8 mmoles) d'acide (2-méthoxy-pyridin-4-yl)boronique. Après barbotage d'un courant d'argon pendant quelques instants, on ajoute 0,98 g (1,2 mmoles) de complexe de 1,1'-bis(diphénylphosphinoferrocènedichloro palladium (II) et de dichlorométhane (PdCl₂(dppf).CH₂Cl₂) et la réaction est portée au reflux sous argon pour 4 heures. Le milieu est alors concentré sous pression réduite pour donner un résidu noir.

Le résidu est repris avec 200 mL d'eau, le produit est extrait avec 500 mL de dichlorométhane, la phase organique séchée par passage sur filtre hydrophobe, filtrée et le solvant évaporé pour donner 7 g de résidu. Le produit est purifié par chromatographie sur gel de silice en éluant avec un mélange de dichlorométhane et d'acétate d'éthyle (100:0 à 70:30) pour donner 3,5 g de cristaux beige après cristallisation dans l'éther diisopropylique et séchage sous pression réduite.
PF : 184°C
RMN ¹H (DMSOd₆) δ : 8,30 (m, 2H), 7,60 (m, 2H), 7,45 (d, 1 H), 7,20 (pseudo t, 2H), 7,05 (m, 2H), 2,90 (s, 3H) ppm.

### Etape 9.3. 2-{4-[2-(4-Fluoro-phényl)-3-(2-méthoxy-pyridin-4-yl) imidazo[1,2-b]pyridazin-6-yl]-pipérazin-1-yl}-éthanol

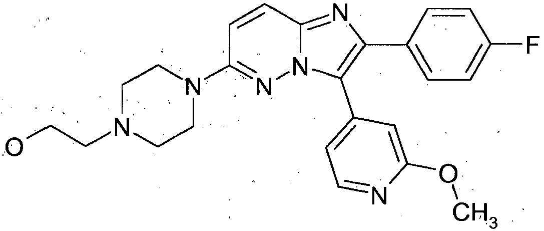

Un mélange de 0,25 g (0,70 mmole) de 6-chloro-2-(4-fluoro-phényl)-3-(2-méthoxy-pyridin-4-yl)imidazo[1,2-*b*]pyridazine et de 0,37 g (2,8 mmoles) de 2-(pipérazin-1-yl)éthanol dans 6 mL de pentanol est agité à 135°C pendant 18 heures. Le solvant est alors évaporé sous pression réduite, le résidu huileux est chromatographié sur colonne de gel de silice en éluant avec un gradient par palier de méthanol et d'ammoniaque dans le dichlorométhane (0/0/100 à 2/1/98). On isole 0,136 g de produit après trituration dans l'éther diisopropylique, filtration et séchage sous pression réduite.
PF : 176-179°C
RMN ¹H (CDCl₃) δ : 8,20 (d; 1H), 7,80 (d, 1H), 7,60 (d, 2H), 7,00-7,15 (m, 4H), 4,00 (s, 3H), 3,70 (m, 2H), 3,55 (m, 4H), 2,7 (m, 6H) ppm.

### Exemple 10 (composé n° 130) : 9-[3-(2-Méthyl-pyridin-4-yl)-2-phényl-imidazo[1,2-b]pyridazin-6-yl]-2,9-diaza-spiro[5.5]undécane

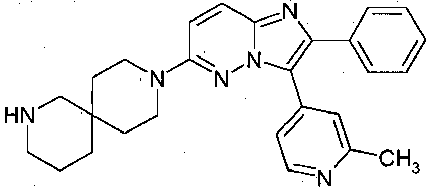

### Procédé de synthèse selon le schéma 1, voie 1 :

### Etape 10.1. 6-Chloro-2-phényl-3-iodo-imidazo[1,2-b]pyridazine

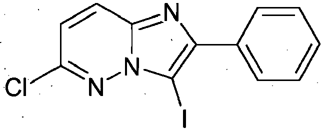

A une solution refroidie à 0°C de 10,4 g (45,4 mmoles) de 6-chloro-2-phényl-imidazo[1,2-*b*]pyridazine (numéro CAS : 1844-53-7) dans 500 mL de chloroforme, on additionne goutte à goutte rapide une solution de 11,1 g (68,1 mmoles) de monochlorure d'iode dans 100 mL de chloroforme. Après retour à la température ambiante et 1 heure d'agitation, le mélange est traité avec une solution aqueuse à 5% de thiosulphate de sodium. Le produit est extrait avec du dichlorométhane, la phase organique est séchée sur sulfate de sodium et concentrée sous pression réduite. Le résidu est trituré dans 150 ml d'acétonitrile contenant quelques ml d'alccol isopropylique et le solide est isolé après filtration. On isole 15 g de poudre jaune après séchage sous vide.
PF : 207-212°C
RMN ¹H (DMSOd₆) δ: 8,15 (dd; 2H), 7,90 (d, 1H)), 7,5 (d, 3H)), 7,15 (d, 1H) ppm.

### Etape 10.2. 6-Chloro-2-phényl-3-(2-méthyl-pyridin-4-yl)imidazo[1,2-b]pyridazine (selon schéma 6)

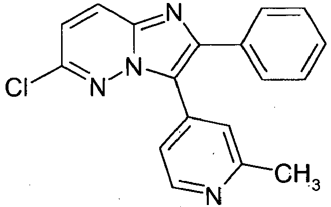

A une suspension 4,33 g (14,8 mmoles) de 6-chloro-2-phényl-3-iodo-imidazo[1,2-*b*]pyridazine dans 200 mL d'un mélange de tétrahydrofurane et d'eau (9:1), on additionne 11,9 g (36,5 mmoles) de carbonate de césium et 2 g (14,6 mmoles) d'acide (2-méthyl-pyridin-4-yl)boronique. Après barbotage d'un courant d'argon pendant quelques instants, on ajoute 0,89 g (1,1 mmoles) de complexe de 1,1'-bis(diphénylphosphinoferrocènedichloro palladium (II) et de dichlorométhane (PdCl₂(dppf).CH₂Cl₂) et la réaction est portée au reflux sous argon pour 18 heures. Le milieu est alors concentré sous pression réduite pour donner un résidu noir.

Le résidu est repris avec de l'acide chlorhydrique 1 N et la solution est filtrée sur buchner. La phase aqueuse est lavée avec de l'éther diéthylique puis la phase aqueuse est basifiée au moyen d'ammoniaque.

Le produit est alors extrait avec du dichlorométhane, la phase organique séchée sur sulfate de sodium puis concentrée sous pression réduite pour donner 3,3 g de résidu jaunâtre. Le produit est purifié par chromatographie sur gel de silice en éluant avec un mélange de dichlorométhane de méthanol et d'ammoniaque (98/2/0,2)) pour donner 2,65 g de solide jaune pâle après séchage sous pression réduite.
PF : 183-188°C
RMN ¹H (DMSOd₆) δ : 8,60 (d, 1H), 8,05 (d, 1H), 7,7 (m, 1H), 7,5 (m, 5H), 7,20 (d, 1H), 2,60 (s, 3H) ppm.

### Etape 10.3. 9-[3-(2-Méthyl-pyridin-4-yl)-2-phényl-imidazo[1,2-b]pyridazin-6-yl]-2,9-diaza-spiro[5.5]undécane

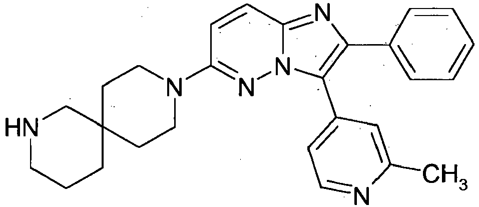

Un mélange de 0,80 g (2,5 mmole) de 6-chloro-2-phényl-3-(2-méthyl-pyridin-4-yl)imidazo[1,2-*b*]pyridazine et de 2,2 g (7,5 mmoles) de 2,9-diaza-spiro[5.5]undécane-2-carboxylate de *tert*-butyle dans 8 mL de pentanol est agité à 150°C pendant 40 heures.

Le mélange est alors versé 30 ml d'acide chlorhydrique aqueux 3N et agité pendant 2 heures. La phase aqueuse est lavée avec de l'éther diéthylique puis la phase aqueuse est basifiée au moyen d'ammoniaque.

Le produit est alors extrait avec du dichlorométhane, la phase organique séchée sur sulfate de sodium puis concentrée sous pression réduite pour donner 0,76 g de solide. Le produit est cristallisé dans 30 ml d'acétonitrile pour donner 0,577 g de produit après séchage sous pression réduite.
PF : 175-179°C
RMN ¹H (CDCl₃) δ : 8,50 (d; 1H), 7,75 (d, 1H), 7,65 (m, 2H), 7,50 (s, 1 H), 7,30-7,45 (m, 4H), 6,90 (d, 1 H), 3,50 (t, 4H), 2,85 (m, 2H), 2,75 (s, 2H), 2,55 (s, 3H), 1,6-1,75 (m, 6H) ppm.

### Exemple 11 (composé n° 147) : 2-Phényl-3-pyridin-4-yl-6-(4-pyrrolidin-1-yl-pipéridin-1-yl)-imidazo[1,2-b]pyridazine

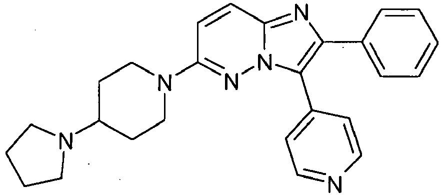

### Procédé de synthèse selon le schéma 1, voie 2 :

### Etape 11.1. 2-Bromo-1-phényl-2-pyridin-4-yl-éthanone

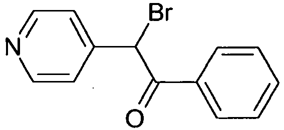

A une solution de 36 g (182 mmoles) de 1-phényl-2-pyridin-4-yl-éthanone dans 350 ml d'acide acétique, on additionne successivement à température ambiante 28 ml (210 mmole) d'une solution à 30 % en poids d'acide bromhydrique dans l'acide acétique et 10,1 ml (197 mmoles) de brome en solution dans 50 ml d'acide acétique. La solution est chauffée à 60°C pendant une heure puis refroidie. Le produit est précipité par addition d'éther di-éthylique et on isole 63 g de solide jaune après séchage.
RMN ¹H (DMSOd₆) δ : 8,80 (d; 2H), 8,10 (d, 2H), 7,90 (d, 2H), 7,70 (m, 1 H), 7,60 (m, 2H), 7,25 (s, 1H) ppm.

### Etape 11.2. 6-Chloro-2-phényl-3-pyridin-4-yl-imidazo[1,2-b]pyridazine

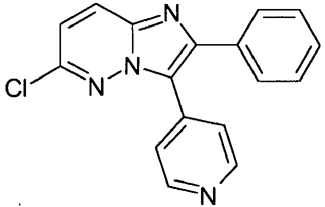

Un mélange de 13 g (36,5 mmoles) de 2-bromo-1-phényl-2-pyridin-4-yl-éthanone (CAS : 741633-76-1) et de 18,9 g (146 mmoles) de 6-chloro-pyridazin-3-ylamine dans 200 ml d'éthanol est porté à 90°C pendant 5 h 30. Après refroidissement, le solvant est évaporé sous pression réduite et le milieu est repris avec 50 ml d'eau et le produit extrait avec de l'acétate d'éthyle. Les phases organiques sont séchées sur sulfate de magnésium puis concentrées sous pression réduite.

Le solide obtenu est re-cristallisé dans 50 ml de méthanol pour conduire à 4,0 g de cristaux jaunes après séchage.
RMN ¹H (CDCl₃) δ : 8,80 (d, 2H), 8,10 (d, 1H), 7,55-7,80 (m, 4H), 7,50 (m, 2H), 7,35 (s, 1H), 7,20 (d, 1H) ppm.

### Etape 11.3. 2-Phényl-3-pyridin-4-yl-6-(4-pyrrolidin-1-yl-pipéridin-1-yl)-imidazo[1,2-b]pyridazine

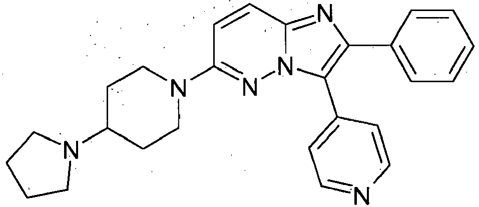

Un mélange de 4,03 g (13,1 mmole) de 6-chloro-2-phényl-3-pyridin-4-yl-imidazo[1,2-*b*]pyridazine et de 4,4 g (29 mmoles) de 4-pyrrolidin-1-yl-pipéridine dans 50 mL de éthanol est chauffé à 155°C pendant 4 heures.

Après refroidissement, le solvant est évaporé sous pression réduite
et le résidu est chromatographié sur gel de silice en éluant avec un mélange de dichlorométhane et de méthanol (93:7). Le produit est ensuite recristallisé dans 40 ml de méthanol pour donner 1,5 g de cristaux jaunes après séchage sous pression réduite.
PF : 165,0-165,5°C
RMN ¹H (CDCl₃) δ : 8,65 (d; 2H), 7,80 (d, 1H), 7,65 (m, 4H), 7,35 (m, 3H), 6,95 (d, 1H), 4,10 (m, 2H), 3,00 (m, 2H), 2,60 (m, 4H), 2,55 (m, 1H), 2,05 (m, 2H), 1,85 (m, 4H), 1,65 (m, 2H) ppm.

### Exemples biologiques

La capacité des composés de l'invention à inhiber la phosphorylation de la caséine par les caséine kinase 1 epsilon et delta peut être évaluée selon la procédure décrite dans le document US20050131012.

### Dosage sur Plaque-Filtre-d'ATP-³³P pour le criblage des inhibiteurs de CK1epsilon :

On mesure l'effet des composés pour inhiber la phosphorylation de la caséine par l'enzyme caséine kinase 1 epsilon (CK1 epsilon) en utilisant un dosage de la caséine par filtration d'ATP-³³P in vitro.

La Caséine Kinase 1 epsilon (0,58 mg/ml) est obtenue par des procédés de fermentation et de purification effectués selon des méthodes bien connues de l'homme du métier ou peut également être obtenue auprès d'Invitrogen Corporation™ (human CK1 epsilon).

Les composés sont testés à cinq concentrations différentes de manière à générer des CI₅₀, c'est à dire la concentration à laquelle un composé est capable d'inhiber l'activité enzymatique de 50%, ou bien l'inhibition en % à une concentration de 10 micromolaires.

On prépare des plaques Falcon à fond en « U » en placant 5 µL de solutions des composés selon l'invention aux concentrations de 10, 1, 0,1, 0,01 ou 0,001 µM dans différents puits. Les solutions des composés selon l'invention à ces différentes concentrations sont préparées par dilution dans un tampon d'essai (Tris 50 mM pH 7,5, MgCl2 10 M, DTT 2 mM et EGTA 1 mM) d'une solution mère dans le DMSO à la concentration de 10 mM. Ensuite, on additionne 5 µL de caséine déphosphorylée à la concentration finale de 0,2 µg/µL, 20 µL de CK1 epsilon à la concentration finale de 3 ng/µL, et 20 µL d'ATP-³³P à la concentration finale de 0,02 µCi/µL mélangée avec de l'ATP froide (10 µM final - environ 2×106 CPM par puits). Le volume total final d'essai par puits est égal à 50 µL.

La plaque d'essai Falcon^{®} à fond en « U » citée ci-dessus est agitée au vortex, puis incubée à la température ambiante pendant 2 heures. Après 2 heures, la réaction est arrêtée par addition d'une solution glacée de 65 µL d'ATP froid (2 mM) préparée dans du tampon d'essai.

On transfère ensuite 100 µL du mélange réactionnel de la plaque Falcon^{®} à fond en U dans des plaques de filtration MAPH Millipore^{®}, préalablement imprégnées avec 25 µL de TCA glacé à 100 %

Les plaques de filtration MAPH Millipore sont agitées doucement et on les laisse au repos à la température ambiante pendant au moins 30 minutes pour précipiter les protéines.

Après 30 minutes, les plaques de filtration sont séquentiellement lavées et filtrées avec 2×150 µL de TCA à 20%, 2×150 µL de TCA à 10% et 2×150 µL de TCA à 5% (6 lavages au total par plaque/900 µL par puits).

On laisse les plaques sécher pendant une nuit à la température ambiante. Ensuite, on ajoute 40 µL de liquide de scintillation Microscint-20 Packard^{®} par puits et les plaques sont fermées de manière étanche. On mesure alors le rayonnement émis par chaque puits pendant 2 minutes dans un compteur à scintillation Topcount NXT Packard^{®} où les valeurs de CPM /puits sont mesurées.

On détermine l'inhibition en % de la capacité de l'enzyme à phosphoryler le substrat (caséine) pour chaque concentration de composé testé. Ces données d'inhibition exprimées en % sont utilisées pour calculer la valeur de CI50 pour chaque composé comparativement aux contrôles.

Les études cinétiques ont déterminé la valeur de K_{M} pour ATP comme étant de 21 µM dans ce système d'essai.

Dans ces conditions, les composés les plus actifs de l'invention présentent des CI50 (concentration inhibant de 50 % l'activité enzymatique de la Caséine Kinase 1 Epsilon ou Caséine Kinase 1 Delta) comprises entre 1 nM et 200 nM.

Le tableau 2 ci-dessous présente les CI₅₀ d'inhibition de la phosphorylation de la Caséine Kinase 1 Epsilon pour quelques composés selon l'invention.

**Tableau 2**

| *Composé N°* | CK1 epsilon CI₅₀ (nM) |
|---|---|
| 1 | 3 |
| 3 | 1 |
| 30 | 60 |
| 147 | 22 - 116 |
| 153 | 8 |

La capacité des composés de l'invention à inhiber la phosphorylation de la caséine par les caséines kinases 1 epsilon et delta peut être évaluée en utilisant un test de fluorescence FRET (« transfert d'énergie entre molécules fluorescentes », de l'anglais « Fluorescence Résonance Energy Transfert ») à partir du kit « Z'Lyte^{™} kinase assay Kit » (référence PV3670 ; Invitrogen Corporation™) selon les instructions du fournisseur.

Les Caséines Kinases 1 utilisées sont obtenues chez Invitrogen Corporation (human CK1 epsilon PV3500 et human CK1 delta PV3665).

Un peptide substrat, marqué à ses deux extrémités par un groupe fluorophore donneur (la coumarine) et un groupe fluorophore accepteur (la fluoresceine) constituant un système FRET est phosphorylé en présence d'ATP par la caséine kinases 1 epsilon ou delta en présence de concentrations croissantes de composés de l'invention.

Le mélange est traité au moyen d'une protéase site spécifique coupant spéciquement le peptide substrat pour former deux fragments fluorescents présentant un grand ratio d'émission par fluoresence.

La fluorescence observée est donc reliée à la capacité des produits de l'invention à inhiber la phosphorylation du peptide substrat par la caséine kinase 1 epsilon ou de la caséine kinase 1 delta.

Les composés de l'invention sont mis en solution à des concentrations différentes à partir d'une solution mère à 10 mM dans le DMSO diluée dans un tampon contenant 50 mM HEPS, pH 7,5, 1 mMEGTA, 0,01% Brij-35, 10 mM MgCl pour la caséine kinase 1 epsilon et supplémenté avec Trizma Base (50 mM), pH 8,0 et NaN3 (0,01% finaux) pour la caséine kinase 1 delta.

La phosphsphorylation du peptide substrat SER/THR 11 obtenu chez Invitrogen Corporation™ est réalisée à la concentration finale de 2 µM. La concentration en ATP est de 4 fois le K_{M}, celui-ci étant de 2 µM pour la caséine kinase 1 epsilon et de 4 µM pour la caséine kinase 1 delta.

La mesure de la fluorescence émise est mesurée aux longueurs d'onde de 445 et 520 nm (exitation à 400 nm).

Dans ces conditions, les composés les plus actifs de l'invention présentent des CI50 (concentration inhibant de 50 % l'activité enzymatique de la Caséine Kinase 1 Epsilon ou Caséine Kinase 1 Delta) comprises entre 1 nM et 200 nM.

Le tableau 3 ci-dessous présente les CI₅₀ d'inhibition de la phosphorylation de la Caséine Kinase 1 Delta pour quelques composés selon l'invention.

**Tableau 3**

| *Composé N°* | CK1 delta CI₅₀ (nM) |
|---|---|
| 31 | 91 - 93 |
| 50 | 5 |
| 81 | 59 - 110 |
| 147 | 50 - 53 |

Il apparaît donc que les composés selon l'invention ont une activité inhibitrice sur l'enzyme Caséine Kinase 1 epsilon ou Caséine Kinase 1 delta.

### Protocoles expérimentaux de dosage circadien cellulaire

Des cultures de fibroblastes Mper1-luc Rat-1 (P2C4) ont été réalisées en divisant les cultures tous les 3-4 jours (environ 10-20 % de confluence) sur des flacons de culture de tissus en polystyrène dégazés de 150 cm² (Falcon® # 35-5001) et maintenues en milieu de croissance [EMEM (Cellgro #10-010-CV) ; sérum bovin foetal à 10 % (FBS; Gibco #16000-044) ; et 50 I.U./mL de pénicilline-streptomycine (Cellgro #30-001-CI)] à 37°C et sous CO₂ 5 %.

Des cellules issues de cultures de fibroblastes Rat-1 à 30-50 % de confluence telle que décrite ci-dessus ont été co-transfectées avec des vecteurs contenant le marqueur de sélection pour la résistance à la Zéocine pour une transfection stable et un gène rapporteur de la luciférase dirigé par le promoteur mPer-1. Après 24 à 48 heures, les cultures ont été divisées sur des plaques de 96 puits et maintenues en milieu de croissance additionné de 50-100 µg/mL de Zéocine (Invitrogen^{®} #45-0430) pendant 10-14 jours. Les transfectants stables résistant à la Zéocine ont été évalués pour l'expression du rapporteur en ajoutant au milieu de croissance de la luciférine 100 µM (Promega^{®} #E1603^{®}) et en dosant l'activité de la luciférase sur un compteur à scintillation TopCount^{®} (Packard Modèle #C384V00). Les clones de cellule Rat-1 exprimant aussi bien la résistance à la Zéocine que l'activité de la luciférase dirigée par mPer1 ont été synchronisés par choc au sérum avec du sérum de cheval à 50 % [HS (Gibco^{®} #16050-122)] et l'activité du rapporteur circadien a été évaluée. Le clone P2C4 de fibroblastes Mper1-luc Rat-1 a été sélectionné pour l'essai du composé.

Des fibroblastes Mper1-luc Rat-1 (P2C4) à 40-50 % de confluence obtenus selon le protocole décrit précédemment ont été étalés sur des plaques de culture de tissu opaques de 96 puits (Perkin Elmer^{®} #6005680). Les cultures sont maintenues en milieu de croissance additionné de 100 µg/mL de Zéocine (Invitrogen #45-0430). jusqu'à ce qu'elles aient atteint 100 % de confluence (48-72 h). Les cultures ont ensuite été synchronisées avec 100 µL de milieu de synchronisation [EMEM (Cellgro #10-010-CV) ; 100 I.U. /mL de pénicilline-streptomycine (Cellgro #30-001-C1) ; HS à 50% (Gibco #16050-122)] pendant 2 heures à 37°C et sous CO₂ 5%. Après synchronisation, les cultures ont été rincées avec 100 µL d'EMEM (Cellgro #10-010-CV) pendant 10 minutes à température ambiante. Après rinçage, le milieu a été remplacé par 300 µL de milieu indépendant de CO₂ [CO₂I (Gibco #18045-088) ; L-glutamine 2 mM (Cellgro #25-005-C1) ; 100 U.I./mL de pénicilline-streptomycine (Cellgro #30-001-C1) ; luciférine 100 µM (Promega #E 1603)]. Les composés de l'invention testés pour les effets circadiens ont été ajoutés à du milieu indépendant de CO₂ dans du DMSO à 0,3 % (concentration finale): Les cultures ont été fermées immédiatement de manière étanche avec du film TopSeal-A^{®} (Packard #6005185) et transférées pour la mesure de l'activité de luciférase.

Après synchronisation, les plaques d'essai ont été maintenues à 37°C dans une étuve de culture de tissu (Forma Scientific Modèle #3914). L'activité de luciférase In Vivo a été estimée en mesurant l'émission relative de lumière sur un compteur à scintillation TopCount (Packard Modèle #C384V00).

L'analyse de périodes a été effectuée soit en déterminant l'intervalle entre les minimums d'émission relative de lumière sur plusieurs jours ou par transformation de Fourier.

Lès deux méthodes ont produit une estimation de période pratiquement identique sur une gamme de périodes circadiennes. La puissance est rapportée en CE Delta (t+1h), qui est présentée comme la concentration micromolaire efficace qui a induit un prolongement de la période de 1 heure. Les données ont été analysées par ajustement d'une courbe hyperbolique aux données exprimées en changement de période (ordonnée) en fonction de la concentration du composé à tester (abscisse) dans le logiciel XLfit™ et la CE Delta (t+1 h) a été interpolée à partir de cette courbe.

Le tableau 4 ci-dessous présente les CE Delta (t+1 h) pour quelques composés selon l'invention.

**Tableau 4**

| *Composé N°* | CE Delta (t+1h) (nM) |
|---|---|
| 50 | 28 |
| 115 | 2 |
| 146 | 39 |
| 153 | 2-9 |

En inhibant les enzymes CK1epsilon et/ou de CK1delta, les composés objets de l'invention modulent la rythmicité circadienne, et peuvent être utiles pour le traitement des désordres liés au rythme circadien.

Les composés selon l'invention peuvent notamment être utilisés pour la préparation d'un médicament destiné à prévenir ou à traiter les désordres du sommeil ; les troubles du rythme circadien, tels que notamment ceux dus au décalage horaire, au travail posté.

Parmi les troubles du sommeil, on distingue notamment les troubles primaires du sommeil tels.que la dyssomnie (par exemple l'insomnie primaire), la parasomnie, l'hypersomnie (par exemple la somnolence excessive), la narcolepsie, les troubles du sommeil liés à l'apnée du sommeil, les troubles du sommeil liés au rythme circadien et les dyssomnies non spécifiées par ailleurs, les troubles du sommeil associés à des troubles médicaux/psychiatriques.

Les composés objets de l'invention provoquent également un déplacement de la phase circadienne et une telle propriété peut être utile dans le cadre d'une monothérapie ou une thérapie combinée potentielle cliniquement efficace pour les troubles de l'humeur.

Parmi les troubles de l'humeur, on distingue notamment les troubles dépressifs (dépression unipolaire), les troubles bipolaires, les troubles de l'humeur dus à une affection médicale générale ainsi que les troubles de l'humeur induits par des substances pharmacologiques. Parmi les troubles bipolaires, on distingue notamment les troubles bipolaires et troubles bipolaires II, dont notamment les troubles affectifs saisonniers.

Les composés objets de l'invention modulant la rythmicité circadienne, peuvent être utiles dans le traitement des troubles anxieux et dépressifs dus en particulier à une altération sur la sécrétion de CRF.

Parmi les troubles dépressifs, on distingue notamment les troubles dépressifs majeurs, troubles dysthymiques, les troubles dépressifs non spécifiés par ailleurs.

Les composés objets de l'invention modulant la rythmicité circadienne, peuvent être utiles pour la préparation d'un médicament destiné à traiter les maladies liées à la dépendance à des substances d'abus telles que la cocaine, la morphine, la nicotine, l'éthanol, le cannabis.

Les composés selon l'invention peuvent donc être utilisés pour la préparation de médicaments, en particulier de médicaments inhibiteurs de la caséine kinase 1 epsilon et/ou de la caséine kinase 1 delta.

Ainsi, selon un autre de ses aspects, l'invention a pour objet des médicaments qui comprennent un composé de formule (I), ou un sel d'addition de ce dernier à un acide pharmaceutiquement acceptable ou encore un hydrate ou un du composé de formule (I).

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention ou un sel pharmaceutiquement acceptable, un hydrate ou solvate dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intraveineuse, topique, locale, intra trachéale, intra nasale, transdermique ou rectale, le principe actif de formule (I) ci-dessus, ou son sel, solvate ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intra trachéale, intraoculaire, intra nasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Par voie orale, la dose de principe actif administrée par jour peut atteindre 0,1 à 20 mg/kg, en une ou plusieurs prises.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. Composé répondant à la formule générale (I) 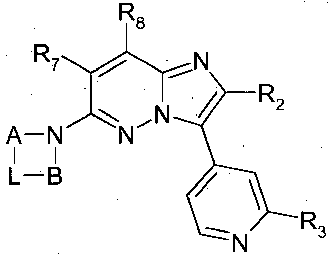 dans laquelle
- R₂ représente un groupe phényle éventuellement substitué par un ou plusieurs substituants choisis parmi les atomes d'halogène et les groupes C₁₋₆alkyle, C₁₋₆alkyloxy, C₁₋₆ fluoroalkyle;
- R₃ représente un atome d'hydrogène ou un groupe C₁₋₃ alkyle, -NR₄R₅, hydroxyle ou C₁₋₄ alkyloxy ;
- A représente un groupe C₁₋₇-alkylène éventuellement substitué par un ou deux groupes Rₐ ;
- B représente un groupe C₁₋₇-alkylène éventuellement substitué par un groupe R_{b} ;
- L représente soit un atome d'azote éventuellement substitué par un groupe R_{c} ou R_{d}, soit un atome de carbone substitué par un groupe Rₑ₁ et un groupe R_{d} ou deux groupes Rₑ₂ ;
les atomes de carbone de A et de B étant éventuellement substitués par un ou plusieurs groupes R_{f} identiques ou différents l'un de l'autre ;
Lorsqu'ils sont présents, Rₐ, R_{b} et R_{c} sont définis en combinaison comme suit :
soit deux groupes Rₐ forment ensemble un groupe C₁₋₆-alkylène ;
soit Rₐ et R_{b} forment ensemble une liaison ou un groupe C₁₋₆-alkylène;
soit Rₐ et R_{c} forment ensemble une liaison ou un groupe C₁₋₆-alkylène ;
soit R_{b} et R_{c} forment ensemble une liaison ou un groupe C₁₋₆-alkylène ;
R_{d} représente un groupe choisi parmi l'atome d'hydrogène et les groupes C₁₋₆-alkyle, C₃₋₇-cycloalkyle, C₃₋₇-cycloalkyl-C₁₋₆-alkyle, C₁₋₆-alkylthio-C₁₋₆-alkyle, C₁₋₆-alkyloxy-C₁₋₆-alkyle, C₁₋₆-fluoroalkyle, benzyle, C₁₋₆-acyle, hydroxy-C₁₋₆-alkyle ;
Rₑ₁ représente un groupe -NR₄R₅ ou une monoamine cyclique comportant éventuellement un atome d'oxygène, la monoamine cyclique étant éventuellement substituée par un ou plusieurs substituants choisis parmi l'atome de fluor et les groupes C₁₋₆-alkyle, C₁₋₆-alkyloxy, hydroxyle ;
Les deux Rₑ₂ forment, avec l'atome de carbone qui les porte, un groupe pyrrolidine, pipéridine, morpholine éventuellement substitué par un ou plusieurs groupes R_{f} identiques ou différents l'un de l'autre ;
R_{f} représente un groupe C₁₋₆-alkyle, C₃₋₇-cycloalkyle, C₃₋₇-cycloalkyle-C₁₋₆-alkyle, C₁₋₆-alkyloxy-C₁₋₆-alkyle, hydroxy-C₁₋₆-alkyle, C₁₋₆-fluoroalkyle ou benzyle ;
R₄ et R₅ représentent, indépendamment l'un de l'autre un atome d'hydrogène ou un groupe C₁₋₄ alkyle, C₃₋₇-cycloalkyle, C₃₋₇-cycloalkyle-C₁₋₆-alkyle ;
- R₇ et R₈ représentent, indépendamment l'un de l'autre, un atome d'hydrogène ou un groupe un groupe C₁₋₆-alkyle ;
à l'état de base ou de sel d'addition à un acide.

2. Composé de formula générale (I) selon la revendication 1, **caractérisé en ce que** :
R₃ représente un groupement choisi parmi un atome d'hydrogène, un groupe C₁₋₃ alkyle, C₁₋₄ alkyloxy, -NR₄R₅ ; R₄ et R₅ représentent un atome d'hydrogène ou un groupe C₁₋₄-alkyle.

3. Composé de formule générale (I) selon la revendication 2, **caractérisé en ce que** :
R₃ représente un groupement choisi parmi un atome d'hydrogène, un groupe méthyle et méthoxy.

4. Composé de formule générale (I) selon la revendication 2, **caractérisé en ce que** :
R₃ représente un groupement choisi parmi -NH₂, méthyl-amino, diméthyl-amino.

5. Composé de formule générale (I) selon l'une quelconque des revendications 1 à 4, **caractérisé en ce que** :
R₇ et R₈ représentent, indépendamment l'un de l'autre un atome d'hydrogène ou un groupe méthyle.

6. Composé de formule générale (I) selon l'une quelconque des revendications 1 à 5, **caractérisé en ce que** :
- A représente un groupe C₁₋₇-alkylène ;
- B représente un groupe C₁₋₇-alkylène ;
- L représente un carbone substitué par un groupe Rₑ₁ et un groupe R_{d} ;
- R_{d} représente un atome d'hydrogène ;
- Rₑ₁ représente un groupe NR₄R₅, où R₄ et R₅ représentent indépendamment l'un de l'autre un groupe C₁₋₄-alkyle ; ou bien Rₑ₁ représente une monoamine cyclique comportant éventuellement un atome d'oxygène, la monoamine étant éventuellement substituée par un ou plusieurs groupements choisis parmi l'atome de fluor et les groupes hydroxyle, C₁₋₆-alkyle.

7. Composé de formule générale (I) selon l'une quelconque des revendications 1 à 5, **caractérisé en ce que** :
- A représente un groupe C₁₋₇-alkylène éventuellement substitué par un ou deux groupes Rₐ ;
- B représente un groupe C₁₋₇-alkylène éventuellement substitué par un groupe R_{b} ;
- L représente un atome d'azote éventuellement substitué par un groupe R_{c} ou R_{d} ;
les atomes de carbone de A et de B étant éventuellement substitués par un ou plusieurs groupes R_{f} identiques ou différents l'un de l'autre ;
Lorsqu'ils sont présents, Rₐ, R_{b} et R_{c} sont définis en combinaison comme suit :
- soit deux groupes Rₐ forment ensemble un groupe C₁₋₆-alkylène,
- soit Rₐ et R_{b} forment ensemble une liaison ou un groupe C₁₋₆-alkylène;
- soit Rₐ et R_{c} forment ensemble une liaison ou un groupe C₁₋₆-alkylène ;
- soit R_{b} et R_{c} forment ensemble une liaison ou un groupe C₁₋₆-alkylène ;
- R_{d} représente un substituant choisi parmi un groupe C₁₋₆-alkyle, C₃₋₇-cycloalkyle, C₃₋₇-cycloalkyle-C₁₋₆-alkyle, hydroxy-C₁₋₆-alkyle, C₁₋₆-alkyloxy-C₁₋₆-alkyle, C₁₋₆-fluoroalkyle, benzyle, C₁₋₆-acyle ;
- R_{f} représente un groupe C₁₋₆-alkyle ;

8. Composé de formule générale (I) selon l'une quelconque des revendications 1 à 5, **caractérisé en ce que** :
- A représente un groupe C₁₋₇-alkylène ;
- B représente un groupe C₁₋₇-alkylène ;
- L représente un atome de carbone substitué par deux groupes Rₑ₂ ;
les atomes de carbone de A et de B étant éventuellement substitués par un ou plusieurs groupes R_{f} identiques ou différents l'un de l'autre ;
- Deux Rₑ₂ forment, avec l'atome de carbone qui les porte, un groupe pyrrolidine, pipéridine, morpholine ;
- R_{f} représente un groupe C₁₋₆-alkyle.

9. Composé de formule générale (I) selon selon l'une quelconque des revendications 1 à 5, **caractérisé en ce que** :
- R₂ représente un groupe phényle éventuellement substitué par un ou plusieurs substituants choisis parmi les atomes de fluor, de chlore et le groupe méthyle ;
- R₃ représente un atome d'hydrogène ;
l'amine cyclique formée par -N-A-L-B- représente une (+/-)-3-diméthylamino-pyrrolidin-1-yle, 4-(pyrrôlidin-1-yl)-pipéridin-1-yle, 4-(morpholin-4-yl)-pipéridin-1-yle, 4-(2,6-diméthyl-morpholin-4-yl)-pipéridin-1-yle, 4-diméthylaminopipéridin-1-yle,4-(3-hydroxy-pyrrolidin-1-yl)-pipéridin-1-yle, 4-(-3-fluoro-pyrrolidin-1-yl)-pipéridin-1-yle ;
- R₇ et R₈ représentent un atome d'hydrogène.

10. Composé de formule générale (I) selon l'une quelconque des revendications 1 à 5, **caractérisé en ce que** :
- R₂ représente un groupe phényle éventuellement substitué par un ou plusieurs substituants choisis parmi les parmi les atomes de fluor et de chlore et les groupes méthyle, methoxy, trifluorométhyle ;
- R₃ représente un atome d'hydrogène ou un groupe méthyle, méthoxy, -NH₂, méthyl-amino, diméthyl-amino ;
- l'amine cyclique formée par -N-A-L-B- représente une pipérazin-1-yle, 3-méthylpipérazin-1-yle, 4-méthylpipérazin-1-yle, 3,3-diméthylpipérazin1-yle, 3,4-diméthylpipérazin-1-yle, *cis*-3,5-diméthylpipérazin-1-yle, 4-(2-hydroxyéthyl)pipérazin-1-yle, 4-(2-méthoxyéthyl)pipérazin-1-yle, 4-(2-fluoroéthyl)pipérazin-1-yle, 4-(2,2,2-trifluoroéthyl)pipérazin-1-yle, 4-cyclopropylpipérazin-1-yle, 4-isopropylpipérazin-1-yle, 4-(2-hydroxy-2-méthylpropyl)pipérazin-1-yle, 4-n-butylpipérazin-1-yle, 4-(3-hydroxy-3-méthyl-butyl)pipérazin-1-yle, cyclohexylpipérazin-1-yle, 4-acétyl-pipérazin-1-yle, 4-isobutyryl-pipérazin-1-yle, (+/-)-hexahydropyrrolo[1,2-a]pyrazin-2-yle, (S)-hexahydropyrrolo[1,2-a]pyrazin-2-yle, (1S,4S)-2,5-diaza-bicyclo[2.2.1]hept-2-yle, 1 (S,4S)-5-benzyl-2,5-diazabicyclo[2.2.1]hept-2-yle, (1S,4S)-5-(2-hydroxyéthyl)-2,5-diazabicyclo[2.2.1]hept-2-yle, 5-isopropyl-2,5-diaza[2.2.1]hept-2-yle, 4-méthyl-[1,4]diazépan-1-yle, (+/-)-octahydropyrrolo[1,2-d][1,4]diazépin-3-yle, 1,4-diaza-bicyclo[3.3.2]non-4-yle, (+/-)-3,6-diaza-bicyclo[3.2.0]hept-3-yle, hexahydropyrrolo[3,4-b]pyrrol-5(1H)-yle, hexahydropyrrolo[3,4-c]pyrrol-2(1*H*)-yle, 5-benzyl-hexahydropyrrolo[3,4-c]pyrrol-2(1*H*)-yle, 5-cyclopropyl-hexahydropyrrolo[3,4-c]pyrrol-2(1H)-yle, 5-isopropyl-hexahydropyrrolo[3,4-c]pyrrol-2(1*H*)-yle, octahydro-6*H-*pyrrolo[3,4-b]pyridin-6-yle, (+/-)-(cis)-décahydro[2,6]-naphthyridin-2-yle, 3-éthyl-pipérazin-1-yle, 3,3-diéthyl-pipérazin-1-yle, 3-fluorométhyl-pipérazin-1-yle, 4-(3-hydroxyméthyl)pipérazin-1-yle, 3-(1-hydroxy-1-méthyl-éthyl)-pipérazin-1-yle, 3-isopropyl-pipérazin-1-yle, (1R,5R)-3,6-diaza-bicyclo[3.2.0]hept-2-yle, 5-méthyl-2,5-diaza-bicyclo[2.2.1]hept-2-yl, 1-((1S,4S)-(2,5-diaza-bicyclo[2.2.1]hept-2-yl)-2-méthyl-propan-2-ol, 3,6-diaza-bicyclo[3.1.1]hept-3-yle, 5-méthyl-hexahydro-pyrrolo[3,4-c]pyrrol-2(1H)-yle, (+/-)-hexahydro-pyrrolo[3,4-b]pyrrol-5(1H)-yle, (+)-hexahydro-pyrrolo[3,4-b]pyrrol-5(1*H*)-yle, (-)-hexahydro-pyrrolo[3,4-b]pyrrol-5(1*H*)-yle, (4aS,7aS)-octahydro-pyrrolo[3,4-b]pyridin-6-yle, 6,9-diaza-spiro[4.5]déc-9-yle :
- R₇ et R₈ représentent, indépendamment l'un de l'autre un atome d'hydrogène ou un groupe méthyle.

11. Composé de formule générale (I) selon selon l'une quelconque des revendications 1 à 5, **caractérisé en ce que** :
- R₂ représente un groupe phényle éventuellement substitué par un ou plusieurs substituants choisis parmi les parmi les atomes de fluor et de chlore ;
- R₃ représente un atome d'hydrogène ou un groupe méthyle, méthoxy, -NH₂ ;
- l'amine cyclique formée par -N-A-L-B- représente une (+/-)-2,7-diaza-spiro[4.4]non-2-yle, (+/-)-2,8-diaza-spiro-[4.5]déc-2-yle, (+/-)-2,7-diaza-spiro-[4.5]déc-2-yle, (+/-)-2,8-diaza-spiro-[4.5]déc-8-yle, (+/-)-2,7-diaza-spiro-[4.5]déc-7-yle, 3,9-diaza-spiro-[5.5]undéc-3-yle, 2,9-diaza-spiro-[5.5]undéc-9-yle, (+/-)-2,8-diaza-spiro-[5.5]undéc-2-yle, 1-oxa-4,9-diaza-spiro[5.5]undéc-9-yle.
- R₇ et R₈ représentent un atome d'hydrogène.

12. Composé de formule générale (I) selon selon la revendication 1, **caractérisé en ce qu'**il est choisi parmi les composés suivants :
2-Phényl-6-pipérazin-1-yl-3-pyridin-4-yl-imidazo[1,2-*b*]pyridazine ;
2-(4-Fluoro-phényl)-6-pipérazin-1-yl-3-pyridin-4-yl-imidazo[1,2-*b*]]pyridazine ;
2-(4-Fluoro-phényl)-7,8-diméthyl-6-pipérazin-1-yl-3-pyridin-4-yl₋imidazo[1,2-*b*]pyridazine ;
4-[2-(4-Fluoro-phényl)-6-pipérazin-1-yl-imidazo[1,2-*b*]pyridazin-3-yl]-pyridin-2-ylamine ;
2-(4-Fluoro-phényl)-3-(2-méthoxy-pyridin-4-yl)-6-pipérazin-1-yl-imidazo[1,2-*b*]pyridazine ;
2-(4-Chloro-phényl)-6-pipérazin-1-yl-3-pyridin-4-yl-imidazo[1,2-*b*]pyridazine ;
2-(4-Chloro-phényl)-7-méthyl-6-pipérazin-1-yl-3-pyridin-4-yl-imidazo[1,2-*b*]pyridazine ;
2-(4-Chloro-phényl)-8-méthyl-6-pipérazin-1-yl-3-pyridin-4-yl-imidazo[1,2-*b*]pyridazine ;
2-(3,5-Diméthyl-phényl)-6-pipérazin-1-yl-3-pyridin-4-yl-imidazo[1,2-*b*]pyridazine ;
2-(3,5-Difluoro-phényl)-6-pipérazin-1-yl-3-pyridin-4-yl-imidazo[1,2-*b*]pyridazine ;
2-(3,5-Difluoro-phényl)-3-(2-méthyl-pyridin-4-yl)-6-pipérazin-1-yl-imidazo[1,2-*b*]pyridazine ;
2-(3,4-Difluoro-phényl)-6-pipérazin-1-yl-3-pyridin-4-yl-imidazo[1,2-*b*]pyridazine ;
2-(3,5-Dichloro-phényl)-6-pipérazin-1-yl-3-pyridin-4-yl-imidazo[1,2-*b*]pyridazine ;
2-(3,5-Dichloro-phényl)-3-(2-méthyl-pyridin-4-yl)-6-pipérazin-1-yl-imidazo[1,2-*b*]pyridaz,ine ;
(+/-)-(3-Méthyl-pipérazin-1-yl)-2-phényl-3-pyridin-4-yl-imidazo[1,2-*b*]pyridazine ;
6-((*R*)-3-Méthyl-pipérazin-1-yl)-2-phényl-3-pyridin-4-yl-imidazo[1,2-*b*]pyridazirie ;
6-((*R*)-3-Méthyl-pipérazin-1-yl)-3-(2-méthyl-pyridin-4-yl)-2-phényl-imidazo[1,2-*b*]pyridazine ;
6-((*S*)-3-Méthyl-pipérazin-1-yl)-2-phényl-3-pyridin-4-yl-imidazo[1,2-*b*]pyridazine ;
2-(3-Fluoro-phényl)-6-((*R*)-3-méthyl-pipérazin-1-yl)-3-pyridin-4-yl-imidazo[1,2-*b*]pyridazine ;
2-(4-Fluoro-phényl)-6-(3-méthyl-pipérazin-1-yl)-3-pyridin-4-yl-imidazo[1,2-*b*]pyridazine ;
2-(4-Fluoro-phényl)-6-((*R*)-3-méthyl-pipérazin-1-yl)-3-pyridin-4.-yl-imidazo[1,2-*b*]pyridazine ;
2-(4-Fluoro-phényl)-6-((*S*)-3-méthyl-pipérazin-1-yl)-3-pyridin-4-yl-imidazo[1,2-*b*]pyridazine ;
2-(4-Fluoro-phényl)-3-(2-méthoxy-pyridin-4-yl)-6-((*R*)-3-méthyl-pipérazin-1-yl)-imidazo[1,2-*b*]pyridazine ;
2-(3,4-Difluoro-phényl)-6-((*R*)-3-méthyl-pipérazin-1-yl)-3-pyridin-4-yl-imidazo[1,2-*b*]pyridazine ;
6-(3,3-Diméthyl-pipérazin-1-yl)-2-phényl-3-pyridin-4-yl-imidazo[1,2-*b*]pyridazine ;
6-(3,3-Diméthyl-pipérazin-1-yl)-2-(4-fluoro-phényl)-3-pyridin-4-yl-imidazo[1,2-*b*]pyridazine ;
6-(3,3-Diméthyl-pipérazin-1-yl)-2-(4-fluoro-phényl)-3-(2-méthoxy-pyridin-4-yl)-imidazo[1,2-*b*]pyridazine ;
2-(3,5-Diméthyl-phényl)-6-(3,3-diméthyl-pipérazin-1-yl)-3-pyridin-4-yl-imidazo[1,2-*b*]pyridazine ;
2-(3,5-Diméthyl-phényl)-6-(3,3-diméthyl-pipérazin-1-yl)-3-(2-méthyl-pyridin-4-yl)-imidazo[1,2-*b*]pyridazine ;
4₋[2-(3,5-Diméthyl-phényl)-6-(3,3-diméthyl-pipérazin-1-yl)-imidazo[1,2-*b*]pyridazin-3-yl]-pyridin-2-ylamine ;
2-(3,5-Difluoro-phényl)-6-(3,3-diméthyl-pipérazin-1-yl)-3-pyridin-4-yl-imidazo[1,2-*b*]pyridazine ;
2-(3,5-Difluoro-phényl)-6-(3,3-diméthyl-pipérazin-1-yl)-3-(2-méthyl-pyridin-4-yl)-imidazo[1,2-*b*]pyridazine ;
4-[2-(3,5-Difluoro-phényl)-6-(3,3-diméthyl-pipérazin-1-yl)-imidazo[1,2-*b*]pyridazin-3-yl]-pyridin-2-ylamine ;
2-(3,5-Dichloro-phényl)-6-(3,3-diméthyl-pipérazin-1-yl)-3-pyridin-4-yl-imidazo[1,2-*b*]pyridazine ;
2-(3,5-Dichloro-phényl)-6-(3,3-diméthyl-pipérazin-1-yl)-3-(2-méthyl-pyridin-4-yl)-imidazo[1,2-*b*]pyridazine ;
(+/-)-6 -(3,4-Diméthyl-pipérazin-1-yl)-2-(4-fluoro-phényl)-3-pyridin-4-yl-imidazo[1,2-*b*]pyridazine ;
6-((*cis*)-3,5-Diméthyl-pipérazin-1-yl)-2-phényl-3-pyridin-4-yl-imidazo[1,2-*b*]pyridazine ;
6-((*cis*)-3,5-Diméthyl-pipérazin-1-yl)-3-(2-méthyl-pyridin-4-yl)-2-phényl-imidazo[1,2-*b*]pyridazine ;
6-((*cis*)-3,5-Diméthyl-pipérazin-1-yl)-2-(3-fluoro-phényl)-3-pyridin-4-yl-imidazo[1,2-*b*]pyridazine ;
6-((*cis*)3,5-Diméthyl-pipérazin-1-yl)-2-(4-fluoro-phényl)-3-pyridin-4-yl-imidazo[1,2-6]pyridazine ;
6-((*cis*)-3,5-Diméthyl-pipérazin-1-yl)-2-(4-fluoro-phényl)-3-(2-méthoxy-pyridin-4-yl)-imidazo[1,2-*b*]pyridazine ;
2-(3,5-Diméthyl-phényl)-76-((*cis*)-3,5-diméthyl-pipérazin-1-yl)-3-pyridin-4-yl-imidazo[1,2-*b*]pyridazine ;
2-(3,4-Difluoro-phényl)-6-((*cis*)-3,5-diméthyl-pipérazin-1-yl)-3-pyridin-4-yl-imidazo[1,2-*b*]pyridazine ;
2-(4-Chloro-phényl)-6-((*cis*)-3,5-diméthyl-pipérazin-1-yl)-7-méthyl-3-pyridin-4-yl-imidazo[1,2-*b*]pyridazine ;
6-((*cis*)-3,5-Diméthyl-pipérazin-1-yl)-2-(4-fluoro-phényl)-8-méthyl-3-pyridin-4-yl-imidazo[1,2-*b*]pyridazine ;
2-(2-Chloro-phényl)-6-(4-méthyl-pipérazin-1-yl)-3-pyridin-4-yl-imidazo[1,2-*b*]pyridazine ;
2-(3-Chloro-phényl)-6-(4-méthyl-pipérazin-1-yl)-3-pyridin-4-yl-imidazo[1,2-*b*]pyridazine ;
2-(4-Chloro-phényl)-6-(4₋méthyl-pipérazin-1-yl)-3-pyridin-4-yl-imidazo[1,2-*b*]pyridazine ;
2-(3-Fluoro-phényl)-6-(4-méthyl-pipérazin-1-yl)-3-pyridin-4-yl-imidazo[1,2-*b*]pyridazine ;
2-(4-Fluoro-phényl)-6-(4-méthyl-pipérazin-1-yl)-3-pyridin-4-yl-imidazo[1,2-*b*]pyridazine ;
2-(3,4-Difluoro-phényl)-6-(4-méthyl-pipérazin-1-yl)-3-pyridin-4-yl-imidazo[1,2-*b*]pyridazine ;
2-(3-Méthoxy-phényl)-6-(4-méthyl-pipérazin-1-yl)-3-pyridin-4-yl-imidazo[1,2-*b*]pyridazine ;
2-(4-Méthoxy-phényl)-6-(4-méthyl-pipérazin-1-yl)-3-pyridin-4-yl-imidazo[1,2-*b*]pyridazine ;
6-(4-Méthyl-pipérazin-1-yl)-3-pyridin-4-yl-2-p-tolyl-imidazo[1,2-*b*]pyridazine ;
6-(4-Méthyl-pipérazin-1-yl)-3-pycidin-4-yl-2-(4-trifluorométhyl-phényl)-imidazo[1,2-*b*]pyridazine ;
2-(4-Fluoro-phényl)-6-(4-méthyl-pipérazin-1-yl)-3-(2-méthyl-pyridin-4-yl)-imidazo[1,2-*b*]pyridazine ;
4-[2-(4-Fluoro-phényl)-6-(4-méthyl-pipérazin-1-yl)-imidazo[1,2-b]pyridazin-3-yl]-pyridin-2-ylamine ;
{4-[2-(4-Fluoro-phényl)-6-(4-méthyl-pipérazin-1-yl)-imidazo[1,2-*b*]pyridazin-3-yl]-pyridin-2-yl}-diméthyl-amine ;
2-(4-Fluoro-phényl)-3-(2-méthoxy-pyridin-4-yl)-6-(4-méthyl-pipérazin-1-yl)-imidazo[1,2-*b*]pyridazine ;
6-(4-Éthyl-pipérazin-1-yl)-2-(4-fluoro-phényl)-3-pyridin-4-yl-imidazo[1,2-*b*]pyridazine ;
2-(3,5-Dirnéthyl-phényl)-6-(4-éthyl-pipérazin-1-yl)-3-pyridin-4-yl-imidazo[1,2-*b*]pyridazine ;
2-[4-(2-Phényl-3-pyridin-4-yl-imidazo[1,2-*b*]pyridazin-6-yl)-pipérazin-1-yl]-éthanol ;
2-{4-[2-(3-Fluoro-phényl)-3-pyridin-4-yl-imidazo[1,2-*b*]pyridazin-6-yl]-pipérazin-1-yl}éthanol ;
2-{4-[2-(4-Fluoro-phényl)-3-pyridin-4-yl-imidazo[1,2-*b*]pyridazin-6-yl]-pipérazin-1-yl}-éthanol
2-{4-[2-(4-Fluoro-phényl)-3-(2-méthoxy-pyridin-4-yl)-imidazo[1,2-*b*]pyridazin-6-yl]-pipérazin-1-yl}-éthanol ;
2-{4-[2-(3,4-Difluoro-phényl)-3-pyridin-4-yl-imidazo[1,2-*b*]pyridazin-yl]-pipérazin-1-yl}-éthanol ;
2-{4-[2-(4-Chloro-phényl)-3-pyridin-4-yl-imidazo[1,2-*b*]pyridazin-6-yl]-pipérazin-1-yl}-éthanol ;
2-{4-[3-(2-Amino-pyridin-4-yl)-2-(4-chloro-phényl)-imidazo[1,2-*b*]pyridazin-6-yl]-pipérazin-1-yl}-éthanol ;
2-(4-Chloro-phényl)-6-[4-(2-méthoxy-éthyl)-pipérazin-1-yl]-3-pyridin-4-yl-imidazo[1,2-*b*]pyridazine ;
6-[4-(2-Fluoro-éthyl)-pipérazin-1-yl]-2-(4-fluoro-phényl)-3-pyridin-4-yl-imidazo[1,2-*b*]pyrïdazine ;
6-[4-(2-Fluoro-éthyl)-pipérazin-1-yl]-2-(4-fluoro-phényl)-3-(2-méthoxy-pyridin-4-yl)-imidazo[1,2-*b*]pyridazine ;
2-Phényl-3-pyridin-4-yl-6-[4-(2,2,2-trifluoro-éthyl)-pipérazin-1-yl]-imidazo[1,2-*b*]pyridazine ;
2-(4-Fluoro-phényl)-3-pyridin-4-yl-6-[4-(2,2,2-trifluoro-éthyl)-pipérazin-1-yl]-imidazo[1,2-*b*]pyridazine ;
6-(4-Cyclopropyl-pipérazin-1-yl)-2-(4-fluoro-phényl)-3-pyridin-4-yl-imidazo[1,2-*b*]pyridazine ;
6-(4-Cyclopropyl-pipérazin-1-yl)-2-(4-fluoro-phényl)-3-(2-méthoxy-pyridin-4-yl)-imidazo[1,2-*b*]pyridazine ;
6-(4-Isopropyl-pipérazin-1-yl)-3-(2-méthoxy-pyridin-4-yl)-2-phényl-imidazo[1,2-*b*]pyridazine ;
2-(3-Fluoro-phényl)-6-(4-isopropyl-pipérazin-1-yl)-3-pyridin-4-yl-imidazo[1,2-*b*]pyridazine ;
2-(4-Fluoro-phényl)-6-(4-isopropyl-pipérazin-1-yl)-3-pyridin-4-yl-imidazo[1,2-*b*]pyridazine ;
2-(4-Fluoro-phényl)-6-(4-isopropyl-pipérazin-1-yl)-3-(2-méthoxy-pyridin-4-yl)-imidazo[1,2-*b*]pyridazine ;
2-(3,4-Difluoro-phényl)-6-(4-isopropyl-pipérazin-1-yl)-3-pyridin-4-yl-imidazo[1,2-*b*]pyridazine ;
4-[2-(4-Chloro-phényl)-6-(4-isopropyl-pipérazin-1-yl)-imidazo[1,2-*b*]pyridazin-3-yl]-pyridin-2-lamine ;
2-Méthyl-1-[4-(2-phényl-3-pyridin-4-yl-imidazo[1,2-*b*]pyridazin-6-yl)-pipérazin-1-yl]-propan-2-ol ;
1-{4-[2-(3-Fluoro-phényl)-3-pyridin-4-yl-imidazo[1,2-*b*]pyridazin-6-yl]-pipérazin-1-yl]-2-méthyl-propan-2-ol ;
1-{4-[2-(4-Fluoro-phényl)-3-(2-méthoxy-pyridin-4-yl)-imidazo[1,2-*b*]pyridazin-6-yl]-pipérazin-1-yl}-2-méthyl-propan-2-ol ;
6-(4-Butyl-pipérazin-1-yl)-2-(4-chloro-phényl)-3-pyridin-4-yl-imidazo[1,2-*b*]pyridazine ;
4-{4-[2-(4-Fluoro-phényl)-3-(2-méthoxy-pyridin-4-yl)-imidazo[1,2-*b*]pyridazin-6-yl]-pipérazin-1-yl}-2-méthyl-butan-2-ol ;
6-(4-Cyclohexyl-pipérazin-1-yl)-2-(4-fluoro-phényl)-3-pyridin-4-yl-imidazo[1,2-*b*]pyridazine ;
1-{4-[2-(4-Fluoro-phényl)-3-pyridin-4-yl-imidazo[1,2-*b*]pyridazin-6-yl]-pipérazin-1-yl}-éthanone ;
1-{4-[2-(4-Fluoro-phényl)-3-pyridin-4-yl-imidazo[1,2-*b*]pyridazin-6-yl]-pipérazin-1-yl}-2-méthyl-propan-1-one ;
(+/-)-2-(4-Fluoro-phényl)-6-(hexahydro-pyrrolo[1,2-*a*]pyrazin-2-yl)-3-pyridin-4-yl-imidazo[1,-2-*b*]pyridazine ;
2-(4-Fluoro-phényl)-6-(*S*)-hexahydro-pyrrolo[1,2-*a*]pyrazin-2-yl-3-(2-méthoxy-pyridin-4-yl)-imidazo[1,2-*b*]pyridazine ;
6-((1*S*,4*S*)-2,5-Diaza-bicyclo[2.2.1]hept-2-yl)-2-phényl-3-pyridin-4-yl-imidazo[1,2-*b*]pyridazine ;
6-((1*S*,4*S*)-5-Benzyl-2,5-diaza-bicyclo[2.2.1]hept-2-yl)-2-(3-fluoro-phényl)-3-pyridin-4-yl-imidazo[1,2-*b*]pyridazine ;
6-((1*S*,4*S*)-2,5-Diaza-bicyclo[2.2.1]hept-2-yl)-2-(4-fluoro-phényl)-3-pyridin-4-yl-imidazo[1,2-*b*]pyridazine ;
6-((1*S*,4*S*)-5-Benzyl-2,5-diaza-bicyclo[2.2.1]hept-2-yl)-2-(4-fluoro-phényl)-3-pyridin-4-yl-imidazo[1,2-*b*]pyridazine ;
6-((1*S*,4*S*)-2,5-Diaza-bicyclo[2.2.1]hept-2-yl)-2-(4-fluoro-phényl)-3-(2-méthoxy-pyridin-4-yl)-imidazo[1,2-*b*]pyridazine ;
2-{(1*S*,4*S*)-5-[2-(4-Fluoro-phényl)-3-(2-méthoxy-pyridin-4-yl)-imidazo[1,2-*b*]pyridazin-6-yl]-2,5-diaza-bicyclo[2.2.1]hept-2-yl}-éthanol ;
2-(4-Fluoro-phényl)-6-(5-isopropyl-2,5-diaza-bicyclo[2.2.1]hept-2-yl)-3-(2-méthoxy-pyridin-4-yl)-imidazo[1,2-*b*]pyridazine ;
2-(4-Fluoro-phényl)-6-(4-méthyl-[1,4]diazépan-1-yl)-3-pyridin-4-yl-imidazo[1,2-*b*]pyridazine ; (+/-)-3-[2-(4-Fluoro-phényl)-3-pyridin-4-yl-imidazo[1,2*-b*]pyridazin-6-yl]-octahydro-(1*H*)pyrrolo[1,2-*d*][1,4]diazépine ;
(+/-)-4-[2-(4-Fluoro-phényl)-3-pyridin-4-yl-imidazo[1,2-*b*]pyridazin-6-yl]-1,4-diaza-bicyclo[3.2.2]nonane ;
(+/-)-4-[2-(3,5-Diméthyl-phényl)-3-pyridin-4-yl-imidazo[1,2-*b*]pyridazin-6-yl]-1,4-diaza-bicyclo[3.2.2]nonane ;
(+/-)-3,6-diaza-bicyclo[3.2.0]hept-3-yl-2-phényl-3-pyridin-4-yl-imidazo[1,2-*b*]pyridazine ;
(+/-)-6-(Hexahydro-pyrrolo[3,4-*b*]pyrrol-5(1*H*)-yl)-2-phényl-3-pyridin-4-yl-imidazo[1,2-*b*]pyridazine ;
(+/-)-6-(Hexahydro-pyrrolo[3,4-*b*]pyrrol-5(1*H*)-yl)-3-(2-méthyl-pyridin-4-yl)-2-phényl-imidazo[1,2-*b*]pyridazine ;
6-Hexahydro-pyrrolo[3,4-*c*]pyrrol-2(1*H*)-yl-2-phényl-3-pyridin-4-yl-imidazo[1,2-*b*]pyridazine ;
6-Hexahydro-pyrrolo[3,4-c]pyrrol-2(1*H*)-yl-3-(2-méthyl-pyridin-4-yl)-2-phényl-imidazo[1,2-*b*]pyridazine ;
2-(3-Fluoro-phényl)-6-hexahydro-pyrrolo[3,4-*c*]pyrrol-2(1*H*)-yl-3-(2-méthyl-pyridin-4-yl)-imidazo[1,2-*b*]pyridazine ;
2-(4-Fluoro-phényl)-6-hexahydro-pyrrolo[3,4-*c*]pyrrol-2(1*H*)-yl-3-pyridin-4-yl-imidazo[1,2-*b*]pyridazine ;
2-(4-Fluoro-phényl)-6-hexahydro-pyrrolo[3,4-*c*]pyrrol-2(1*H*)-yl-3-(2-méthyl-pyridin-4-yl)-imidazo[1,2-*b*]pyridazine ;
4-[2-(4-Chloro-phényl)-6-(hexahydro-pyrrolo[3,4-*c*]pyrrol-2(1*H*)-yl)-imidazo[1,2-*b*]pyridazin-3-yl]-pyridin-2-ylamine ;
2-(4-Fluoro-phényl)-6-(hexahydro-pyrrolo[3,4-*c*]pyrrol-2(1*H*)-yl)-3-(2-méthoxy-pyridin-4-yl)-imidazo[1,2-*b*]pyridazine ;
4-[6-(5-Benzyl-hexahydro-pyrrolo[3,4-*c*]pyrrol-2(1*H*)-yl)-7,8-diméthyl-2-(4-fluoro-phényl)-imidazo[1,2-*b*]pyridazin-3-yl]-pyridin-2-ylamine ;
4-[2-(4-Fluoro-phényl)-6-hexahydro-pyrrolo[3,4-*c*]pyrrol-2(1*H*)-yl-7,8-diméthyl-imidazo[1,2-*b*]pyridazin-3-yl]-pyridin-2-ylamine ;
6-(5-Cyclopropyl-hexahydro-pyrrolo[3,4-*c*]pyrrol-2(1*H*)-yl)-2-(4-fluoro-phényl)-3-pyridin-4-yl-imidazo[1,2-*b*]pyridazine ;
2-(4-Fluoro-phényl)-6-(5-isopropyl-hexahydro-pyrrolo[3,4-*c*]pyrrol-2(1*H*)-yl)-3-pyridin-4-yl-imidazo[1,2-*b*]pyridazine ;
2-(4-Fluoro-phényl)-6-(5-isopropyl-hexahydro-pyrrolo[3,4-*c*]pyrrol-2-(1*H*)yl)-3-(2-méthyl-pyridin-4-yl)-imidâzo[1,2-*b*]pyridazine ;
2-(4-Fluoro-phényl)-6-(5-isopropyl-hexahydro-pyrrolo[3,4-*c*]pyrrol-2(1*H*)-yl)-3-(2-méthoxy-pyridin-4-yl)-imidazo[1,2-*b*]pyridazine
(+/-)-(*ci*s)-6-(Octahydro-6*H*-pyrrolo[3,4-*b*]pyridin-6-yl)-2-phényl-3-pyridin-4-yl-imidazo[1,2-*b*]pyridazine ;
(+/-)-(*cis*)-3-(2-Méthyl-pyridin-4-yl)-6-(octahydro-6*H*-pyrrolo[3,4-*b*]pyridin-6-yl)-2-phényl-imidazo[1,2-*b*]pyridaziné ;
(+/-)-(*cis*)-2-[2-(4-Fluoro-phényl)-3-pyridin-4-yl-imidazo[1,2-*b*]pyridazin-6-yl]-décahydro-[2,6]naphthyridine ;
(+/-)-6-(2,7-Diaza-spiro[4.4]non-2-yl)-2-(3-fluoro-phényl)-3-pyridin-4-yl-imidazo[1,2-*b*]pyridazine ;
(+/-)-6-(2,7-Diaza-spiro[4.4]non-2-yl)-2-(4-fluoro-phényl)-3-pyridin-4-yl-imidazo[1,2-*b*]pyridazine ;
(+/-)-6-(2,8-Diaza-spiro[4.5]déc-2-yl)-2-(4-fluoro-phényl)-3-pyridin-4-yl-imidazo[1,2-*b*]pyridazine ;
(+/-)-6-(2,7-Diaza-spiro[4.5]déc-2-yl)-2-(4-fluoro-phényl)-3-pyridin-4-yl-imidazo[1,2-*b*]pyridazine ;
(+/-)-6-(2,8-Diaza-spiro[4.5]déc-8-yl)-2-(4-fluoro-phényl)-3-pyridin-4-yl-imidazo[1,2-*b*]pyridazine ;
(+/-)-6-(2,7-Diaza-spiro[4.5]déc-7-yl)-2-(4-fluoro-phényl)-3-pyridin-4-yl-imidazo[1,2-*b*]pyridazine ;
3-[2-(4-Fluoro-phényl)-3-pyridin-4-yl-imidazo[1,2-*b*]pyridazin-6-yl]-3,9-diaza-spiro[5.5]undécane ;
9-(2-Phényl-3-pyridin-4-yl-imidazo[1,2-*b*]pyridazin-6-yl)-2,9-diaza-spiro[5.5]undécane ;
9-[3-(2-Méthyl-pyridin-4-yl)-2-phényl-imidazo[1,2-*b*]pyridazin-6-yl]-2,9-diaza-spiro[5.5]undécane ;
9-[2-(3-Fluoro-phényl)-3-(2-méthyl-pyridin-4-yl)-imidazo[1,2-*b*]pyridazin-6-yl]-2,9-diaza-spiro[5.5]undécane ;
9-[2-(4-Fluoro-phényl)-3-pyridin-4-yl-imidazo[1,2-*b*]pyridazin-6-yl]-2,9-diaza-spiro[5.5]undécane ;
9-[2-(4-Fluoro-phényl)-3-(2-méthyl-pyridin-4-yl)-imidazo[1,2-*b*]pyridazin-6-yl]-2,9-diaza-spiro[5.5]undécane ;
4-[2-(4-Chloro-phényl)-6-(2,9-diaza-spiro[5.5]undéc-9-yl)-imidazo[1,2-*b*]pyridazin-3-yl]-pyridin-2-ylamine ;
9-[2-(4-Fluoro-phényl)-3-(2-méthoxy-pyridin-4-yl)-imidazo[1,2-*b*]pyridazin-6-yl]-2,9-diaza-spiro[5.5]undécane ;
2-[2-(4-Fluoro-phényl)-3-(pyridin-4-yl)-imidazo[1,2-*b*]pyridazin-6-yl]-2,9-diaza-spiro[5.5]undécane
2-[2-(4-Fluoro-phényl)-3-(pyridin-4-yl)-imidazo[1,2-*b*]pyridazin-6-yl]-2,8-diaza-spiro[5.5]undécane
9-[2-(phényl)-3-(pyridin-4-yl)-imidazo[1,2-*b*]pyridazin-6-yl]-1-oxa-4,9-diaza-spiro[5.5]undécane ;
9-[2-(4-Fluoro-phényl)-3-(pyridin-4-yl)-imidazo[1,2-*b*]pyridazin-6-yi]-1-oxa-4,9-diaza-spiro[5.5]undécane ;
9-[2-(4-Fluoro-phényl)-3-(2-méthoxy-pyridin-4-yl)-imidazo[1,2-*b*]pyridazin-6-yl]-1-oxa-4,9-diaza-spiro[5:5]undécane ;
4-{[2-(4-Chloro-phényl)-3-(2-méthoxy-pyridin-4-yl)-imidazo[1,2-*b*]pyridazin-6-yl]-1-oxa-4,9-diaza-spiro[5.5]uridéc-9-yl}-2-ylamine ;
(+/-)-{1-[2-(4-Fluoro-phényl)-3-pyridin-4-yl-imidazo[1,2-*b*]pyridazin-6-yl]-pyrrolidin-3-yl}-diméthyl-amine ;
2-(3,5-Diméthyl-phényl)-3-pyridin-4-yl-6-(4-pyrrolidin-1-yl-pipéridin-1-yl)-imidazo[1,2-*b*]pyridazine ;
6-(4-Morpholin-4-yl-pipéridin-1-yl)-2-phényl-3-pyridin-4-yl-imidazo[1,2-*b*]pyridazine ;
6-[4-(2,6-Diméthyl-morpholin-4-yl)-pipéridin-1-yl]-2-phényl-3-pyridin-4-yl-imidazo[1,2-*b*]pyridazine ;
{1-[2-(4-Fluoro-phényl)-3-pyridin-4-yl-imidazo[1,2-*b*]pyridazin-6-yl]-pipéridin-4-yl}-diméthyl-amine;
2-Phényl-3-pyridin-4-yl-6-(4-pyrrolidin-1-yl-pipéridin-1-yl)-imidazo[1,2-*b*]pyridazine ;
2-(4-Fluoro-phényl)-3-pyridin-4-yl-6-(4-pyrrolidin-1-yl-pipéridin-1-yl)-imidazo[1,2-*b*]pycidazine ;
2-(4-Fluoro-phényl)-3-(2-méthoxy-pyridin-4-yl)-6-(4-pyrrolidin-1-yl-pipéridin-1-yl)-imidazo[1,2-*b*]pyridazine ;
(*R*)-1-{1-[2-(4-Fluoro-phényl)-3-pyridin-4-yl-imidazo[1,2-b]pyridazin-6-yl]-pipéridin-4-yl}-pyrrolidin-3-ol ;
6-(4-Morpholin-4-yl-pipéridin-1-yl)-2-phényl-3-pyridin-4-yl-imidazo[1,2-*b*]pyridazine ;
2-(3-Fluoro-5-méthyl-phényl)-6-((*R*)-3-méthyl-pipérazin-1-yl)-3-pyridin-4-yl-imidazo[1,2-*b*]pyridazine ;
{4-[2-(4-Fluoro-phényl)-6-((*R*)-3-méthyl-pipérazin-1-yl)-imidazo[1,2-b]pyridazin-3-yl]-pyridin-2-yl}-méthyl-amine ;
6-(3,3-Diméthyl-pipérazin-1-yl)-2-(4-fluoro-phényl)-7-méthyl-3-pyndin-4-yl-imidazo[1,2-*b*]pyridazine ;
6-(3,3-Dirüéthyl-pipérazin-1-yl)-2-(4-fluoro-phényl)-8-méthyl-3-pyridin-4-yl-imidazo[1,2-*b*]pyridazine ;
6-(3,3-Diméthyl-pipérazin-1-yl)-2-(3-fluoro-5-méthyl-phényl)-3-pyridin-4-yl-imidazo[1,2-*b*]pyridazine ;
{4-[6-(3,3-Diméthyl-pipérazin-1-yl)-2-(4-fluoro₋phényl)-imidazo[1,2-*b*]pyridazin-3-yl]-pyridin-2-yl}-méthyl-amine ;
6-((*R*)-3-Éthyl-pipérazin-1-yl)₋2-(4-fluoro-phényl)-3-pyridin-4-yl-imidazo[1,2-*b*]pyridazine ;
6-(3,3-Diéthyl-pipérazin-1-yl)-2-(4-fluoro-phényl)-3-pyridin-4-yl-imidazo[1,2-*b*]pyridazine ;
(+/-)-6-(3-Fluorométhyl-pipérazin-1-yl)-2-(4-fluoro-phényl)-3-pyridin-4-yl-imidazo[1,2-*b*]pyridazine ;
(+/-)-{4-[2-(4-Fluoro-phényl)-3-pyridin-4-yl-imidazo[1,2-*b*]pyridazin-6-yl]-pipérazin-2-yl}-methanol ;
2-{4-[2-(3-Fluoro-5-méthyl-phényl)-3-pyridin-4-yl-imidazo[1,2-*b*]pyridazin-6-yl]-pipérazin-1-yl}-éthanol ;
2-{4-[2-(4-Fluoro-phényl)-3-(2₋méthyl-amino-pyridin-4-yl)=imidazo[1,2-*b*]pyridazin-F-yl]-pipérazin-1-yl}-ethanol ;
1-{4-[2-(4-Fluoro-phényl)-3-pyridin-4-yl-imidazo[1,2-*b*]pyridazin-6-yl]-pipérazin-1-yl}-2-méthyl-propan-2-ol ;
1-{4-[2-(4-Fluoro-phényl)-8-méthyl-3-pyridin-4-yl-imidazo[1,2-*b*]pyridazin-6-yl]-pipérazin-1-yl}-2-méthyl-propan-2-ol ;
1-{4-[2-(4-Fluoro-phényl)-7-méthyl-3-pyridin-4-yl-imidazo[1,2-*b*]pyridazin-6-yl]-pipérazin-1-yl}-2-méthyl-propan-2-ol ;
1-{4-[2-(4-Fluoro-phényl)-3-(2-méthyl-amino-pyridin-4-yl)-imidazo[1,2-*b*]pyridazin-6-yl]-pipérazin-1-yl}-2-méthyl-propan-2-ol ;
2-{(*R*)-4-[2-(4-Fluoro-phényl)-3-pyridin-4-yl-imidazo[1,2-*b*]pyridazin-6-yl]-pipérazin-2-yl}-propan-2-ol ;
2-(4-Fluoro-phényl)-6-((*R*)-3-isopropyl-pipérazin-1-yl)-3-pyridin-4-yl-imidazo[1,2-*b*]pyridazine ;
{4-[2-(4-Fluoro-phényl)-6-((*R*)-3-isopropyl-pipérazin-1-yl)-imidazo[1,2-*b*]pyridazin-3-yl]-pyridin-2-yl}-méthyl-amine ;
2-(3-Fluoro-phényl)-6-(4-isopropyl-pipérazin-1-yl)-3-(2-méthoxy-pyridin-4-yl)-imidazo[1,2-*b*]pyridazine ;
2-(4-Fluoro-phényl)-6-(4-isopropyl-pipérazin-1 -yl)-7-méthyl-3-pyridin-4-yl-imidazo[1,2-*b*]pyridazine ;
2-(4-Fluoro-phényl)-6-(4-isopropyl-pipérazin-1-yl)-8-méthyl-3-pyridin-4-yl-imidazo[1,2-*b*]pyridazine ;
{4-[2-(4-Fluoro-phényl)-6-(4-isopropyl-pipérazin-1-yl)-imidazo[1,2-*b*]pyridazin-3-yl]-pyridin-2-yl}-méthyl-amine ;
2-(3,4-Difluoro-phényl)-6-(4-isopropyl-pipérazin-1-yl)-3-(2-méthoxy-pyridin-4-yl)-imidazo[1,2-*b*]pyridazine
2-(3-Fluoro-5-méthyl-phényl)-6-(4-isopropyl-pipérazin-1-yl)-3-pyridin-4-yl-imidazo[1,2-*b*]pyridazine ;
(+/-)-6-(3,6-Diaza-bicyclo[3.2.0]hept-3-yl)-2-phényl-3-pyridin-4-yl-imidazo[1,2-*b*]pyridazine ;
6-(1*S*,4*S*)-2,5-Diaza-bicyclo[2.2.1]hept-2-yl-2-phényl-3-pyridin-4-yl-imidazo[1,2-*b*]pyridazine ;
{4-[(1*S*,4*S*)-6-2,5-Diaza-bicyclo[2.2.1]hept-2-yl-2-(4-fluoro-phényl)-imidazo[1,2-*b*]pyridazin-3-yl]-pyridin-2-yl}-méthyl-amine ;
2-(4-Fluoro-phényl)-6-((1*S*,4*S*)-5-méthyl-2,5-diaza-bicyclo[2.2.1]hept-2-yl)-3=pyridin-4-yl-imidazo[1,2-*b*]pyridazine ;
2-(4-Fluoro-phényl)-7-méthyl-6-((1*S*,4*S*)-5-méthyl-2,5-diaza-bicyclo[2.2.1]hept-2-yl)-3-pyridin-4-yl-imidazo[1,2-*b*]pyridazine ;
2-(4-Fluoro-phényl)-8-méthyl-6-((1*S*,4*S*)-5-méthyl-2,5-diaza-bicyclo[2.2.1]hept-2-yl)-3-pyridin-4-yl-imidazo[1,2-*b*]pyridazine ;
2-(4-Fluoro-phényl)-6-((1*S*,4*S*)-5-méthyl-2,5-diaza-bicyclo[2.2.1]hept-2-yl)-3-(2-méthyl-pyridin-4-yl)-imidazo[1,2-*b*]pyridazine ;
2-Méthyl-1-[(1*S*,4*S*)-5-(2-phényl-3-pyridin-4-yl-imidazo[1,2-*b*]pyridazin-6-yl)-2,5-diazabicyclo[2.2.1]hept-2-yl]-propan-2-ol ;
6-(3,6-Diaza-bicyclo[3.1.1]hept-3-yl)-2-(4-fluoro-phényl)-3-pyridin-4-yl-imidazo[1,2-*b*]pyridazine ;
2-(4-Fluoro-phényl)-6-(5-méthyl-hexahydro-pyrrolo[3,4-*c*]pyrrol-2(1*H*)-yl)-3-pyridin-4-yl-imidazo[1,2-*b*]pyridazine ;
2-(4-Fluoro-phényl)-7-méthyl-6-(5-méthyl-hexahydro-pyrrolo[3,4-*c*]pyrrol-2(1*H*)-yl)-3-pyridin-4-yl-imidazo[1,2-*b*]pyridazine ;
2-(4-Fluoro-phényl)-8-méthyl-6-(5-méthyl-hexahydro-pyrrolo[3,4-c]pyrrol-2(1*H*)-yl)-3-pyridin-4-yl-imidazo[1,2-*b*]pyridazine ;
2-(4-Fluoro-phényl)-8-méthyl-6-(-5-méthyl-hexahydro-pyrrolo[3,4-c]pyrrol-2(1*H*)-yl)-3-(2-méthyl-pyridin-4-yl)-imidazo[1,2-*b*]pyridazine ;
{4-[2-(4-Fluoro-phényl)-6-(5-méthyl-hexahydro-pyrrolo[3,4-*c*]pyrrol-2(1*H*)-yl)-imidazo[1,2-*b*]pyridazin-3-yl]-pyridin-2-yl}-methyl-amine ;
2-(4-Fluoro-phényl)-3-(2-méthoxy-pyridin-4-yl)-6-(5-méthyl-hexahydro-pyrrolo[3,4-*c*]pyrrol-2(1*H*)-yl)-imidazo[1,2-*b*]pyridazine ;
2-(4-Fluoro-phényl)-6-(5-méthyl-hexahydro-pyrrolo[3,4-c]pyrrol-2(1*H*)-yl)-3-(2-méthyl-pyridin-4-yl)-imidazo[1,2-*b*]pyridazine ;
{4-[2-(4-Fluoro-phényl)-8-méthyl-6-((3a*R*,6a*S*)-5-méthyl-hexahydro-pyrrolo[3,4-*c*]pyrrol-2(1*H*)-yl)-imidazo[1,2-*b*]pyridazin-3-yl]-pyridin-2-yl}-méthyl-amine ;
{4-[2-(4-Fluoro-phényl)-7-méthyl-6-(5-méthyl-hexahydro-pyrrolo[3,4-*c*]pyrrol-2(1*H*)-yl)-imidazo[1,2-*b*]pyridazin-3-yl]-pyridin-2-yl}-méthyl-amine ;
(+/-)-2-(4-Fluoro-phényl)-6-(hexahydro-pyrrolo[3,4-*b*]pyrrol-5(1*H*)-yl)-3-pyridin-4-yl-imidazo[1,2-*b*]pyridazine ;
(+)-2-(4-Fluoro-phényl)-6-hexahydro-pyrrolo[3,4-*b*]pyrrol-5(1*H*)-yl-3-pyridin-4-yl-imidazo(1,2-*b*]pyridazine ;
(-)-2-(4-Fluoro-phényl)-6-héxahydro-pyrrolo[3,4-*b*]pyrrol-5(1*H*)-yl-3-pyridin-4-yl-imidazo[1,2-*b*]pyridazine ;
(+/-)-(4-[2-(4-Fluoro-phényl)-6-(hexahydro-pyrrolo[3,4-*b*]pyrro)-5(1*H*)-yl)-imidazo[1,2-*b*]pyridazin3-yl]-pyridin-2-yl}-méthyl-amine ;
6-(4a*S*,7a*S*)-octahydro₋pyrrolo[3,4-*b*]pyridinf-yl-2-phényl-3-pyridin-4-yl-imidazo[1,2-*b*]pyridazine ;
3-(2-Méthyl-pyridin-4-yl)-6-(4a*S*,7a*S*)-octahydro-pyrrolo[3,4-*b*]pyridin-6-yl-2-phényl-imidazo[1,2-*b*]pyridazine ;
3-(2-Méthyl-pyridin-4-yl)-6-(4a*R*,7a*R*)-octahydro-pyrrolo[3,4-*b*]pyridin-6-yl-2-phényl-imidazo[1,2-*b*]pyridazine ;
2-(4-Fluoro-phényl)-8-méthyl-6-(4a*S*,7a*S*)-octahydro-pyrrolo[3,4-*b*]pyridin-6-yl-3-pyridin-4-yl-imidazo[1,2-*b*]pyridazine ;
2-(4-Fluoro-phényl)-7-méthyl-6-(4a*S*,7a*S*)-octahydro-pyrrolo[3,4-*b*]pyridin-6-yl-3-pyridin-4-yl-imidazo[1,2-*b*]pyridazine ;
2-(4-Fluoro-phënyl)-7-méthyl-6-(4a*R*,7a*R*)-octahydro-pyrrolo[3,4-*b*]pyridin-6-yl-3-pyridin-4-yl-imidazo[1,2-*b*]pyridazine ;
2-(4-Fluoro-phényl)-6-(4aS,7aS)-octahydro-pyrrolo[3,4-b]pyridin-6-yl-3-pyridin-4-yl-imidazo[1,2-*b*]pyridazine ;
2-(4-Fluoro-phényl)-3-(2-méthyl-pyridin-4-yl)-6-(4a*S*,7a*S*)-octahydro-pyrrolo[3,4-*b*]pyridin-6-yl-imidazo[1,2-*b*]pyridazine ;
{4-[2-(4-Fluoro-phényl)-6-(octahydro-pyrrolo[3,4-*b*]pyridin-6-yl)-imidazo[1,2,-*b*]pyridazin-3-yl]-pyridin-2-yl}-méthyl-amine ;
6-(4a*R*,7a*R*)-Octahydro-pyrrolo[3,4-*b*]pyridin-6-yl-2-phényl-3-pyridin-4-yl-imidazo[1,2-*b*]pyridazine ;
2-(4-Fluoro-phényl)-6-(4a*R*,7a*R*)-octahydro-pyrrolo[3,4-*b*]pyridin-6-yl-3-pyridin-4-yl-imidazo[1,2-*b*]pyridazine ;
2-(4-Fluoro-phényl)-3-(2-méthyl-pyridin-4-yl)-6-(4a*R*,7a*R*)-octahydro-pyrrolo[3,4-b]pyridin-6-yl-imidazo[1,2-*b*]pyridazine ;
2-(4-Fluoro-phényl)-8-méthyl-6-(4a*R*,7a*R*)-octahydro-pyrrolo[3,4-*b*]pyridin-6-yl-3-pyridin-4-yl-imidazo[1,2-*b*]pyridazine ;
6-(2,7-Diaza-spiro[4.4]non-2-yl)-2-(4-fluoro-phényl)-8-méthyl-3-pyridin-4-yl-imidazo[1,2-*b*]pyridazine ;
6-(6,9-Diaza-spiro[4.5]dèc-9-yl)-2-(4-fluoro-phényl)-3-pyridin-4-yl-imidazo[1,2-*b*]pyridazine ;
(+/-)-2-[2-(4-Fluoro-phényl)-8-méthyl-3-pyridin-4-yl-imᵢdazo[1,2-*b*]pyridazin-6-yl]-2,8-diaza-spiro[5.5]undécane ;
9-[2-(4-Fluoro-phényl)-8-méthyl-3-pyridin-4-yl-imidazo[1,2-*b*]pyridazin-6-yl]-2,9-diaza-spiro[5.5]undécane ;
{4-[6-(2,9-Diaza-spiro[5.5]undéc-9-yl)-2-(4-fluoro-phényl)-imidazo[1,2-*b*]pyridazin-3-yl]-pyridin-2-yl}-méthyl-amine ;
9-[2-(3,4-Difluoro-phényl)-3-pyridin-4-yl-imidazo[1,2-*b*]pyridazin-6-yl]-2,9-diaza-spiro[5.5]undécane ;
9-[2-(3-Fluoro-5-méthyl-phényl)-3-pyridin-4-yl-imidazo[1,2-*b*]pyridazin-6-yl]-2,9-diaza-spiro[5.5]undécane ;
2-(3-Fluoro-phényl)-3-pyridin-4-yl-6-(4-pyrrolidin-1-yl-pipéridin-1-yl)-imidazo[1,2-*b*]pyridazine ;
{4-[2-(4-Fluoro-phényl)-6-(4-pyrrolidin-1-yl-pipéridin-1-yl)-imidazo[1,2-*b*]pyridazin-3-yl]-pyridin-2-yl}-méthyl-amine ;
2-(4-Fluoro-phényl)-6-[4-((*R*)-3-fluoro-pyrrolidin-1-yl)-pipéridin-1-yl]-3-pyridin-4-yl-imidazo[1,2-*b*]pyridazine;
(*R*)-1-[1-(2-Phényl-3-pyridin-4-yl-imidazo[1,2-*b*]pyridazin-6-yl)-pipéridin-4-yl]-pyrrolidin-3-ol ;
(R)-1-{1-[2-(3-Fluoro-5-méthyl-phényl)-3-pyridin-4-yl-imidazo[1,2-*b*]pyridazin-6-yl]-piperidin-4-yl}-pyrrolidin-3-ol.

13. Procédé de préparation d'un composé de formule (I) selon la revendication 1, **caractérisé en ce que** l'on fait réagir un composé de formule générale (IIa) 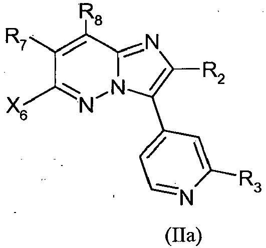 dans laquelle dans laquelle R₂, R₃, R₇ et R₈ sont tels que définis selon la revendication 1 et X₆ représente un groupe partant, avec une amine de formule générale (IIIa) 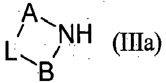 dans laquelle A, L et B sont tels que défini selon la revendication 1, dans un solvant polaire.

14. Procédé de préparation d'un composé de formule (I) selon la revendication 1, **caractérisé en ce que** l'on fait réagir un composé de formule générale (Vb) 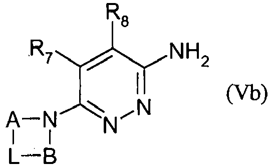 dans laquelle A, L, B, R₇ et R₈ sont tels que définis selon la revendication 1, avec un composé de formule générale (Vlb) 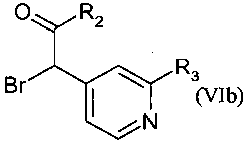 dans laquelle R₂ et R₃ sont tels que définis selon la revendication 1, dans un solvant polaire.

15. Procédé de préparation d'un composé de formule (I) selon la revendication 1, **caractérisé en ce que** l'on fait réagir un composé de formule générale (IId) 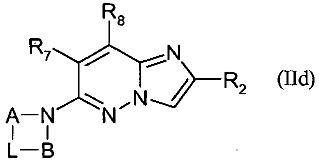 dans laquelle R₂, A, L, B, R₇ et R₈ sont tels que définis selon la revendication 1, avec un composé de formule générale (IVd) 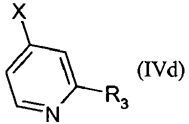 dans laquelle R₃ est tel que défini selon selon la revendication 1 et X représente un atome d'iode, en présence d'un catalyseur, d'une base minérale et dans un solvant polaire aprotique.

16. Procédé de préparation d'un composé de formule (I) selon la revendication 1, **caractérisé en ce que** l'on fait réagir un composé de formule générale (IIc) 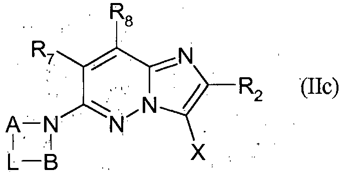 dans laquelle R₂, A, L, B, R₇ et R₈ sont tels que définis selon la revendication 1 et X représente un atome d'iode ou de brome, avec un composé de formule générale (IVc) 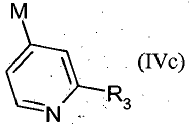 dans laquelle R₃ est tel que défini selon la revendication 1 et M représente un groupe trialkylstannyle, un groupe dihydroxyboryle ou dialkyloxyboryle.

17. Procédé de préparation d'un composé de formule (I) selon la revendication 1, dans laquelle R₃ représente un atome d'hydrogène ou un groupe C₁₋₃-alkyle, **caractérisé en ce que** :
a) on fait réagir un composé de formule générale (IId) 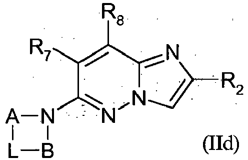 dans laquelle R₂, A, L, B, R₇ et R₈ sont tels que définis selon la revendication 1, avec un mélange de composé de formule (IVe) 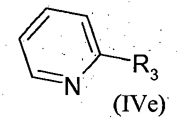 dans laquelle R₃ représente un atome d'hydrogène ou un groupe C₁₋₃-alkyle, en présence de chloroformiate d'alkyle, pour obtenir un composé de fomule (IIe) 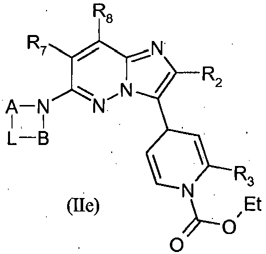 dans laquelle R₂, A, L, B, R₇ et R₈ sont tels que définis selon la revendication 1 et dans laquelle R₃ représente un atome d'hydrogène ou un groupe C₁₋₃-alkyle ;
b) on fait réagir le produit de formule (IIe) obtenu à l'étape b) avec un agent d'oxydation pour obtenir un composé de formule générale (I) selon la revendication 1 et dans laquelle R₃ représente un atome d'hydrogène ou un groupe C₁₋₃-alkyle.

18. Médicament, **caractérisé en ce qu'**il comprend un composé de formule (I) selon l'une quelconque des revendications 1 à 12, à l'état de base ou de sel d'addition à un acide pharmaceutiquement acceptable.

19. Composition pharmaceutique, **caractérisée en ce qu'**elle comprend un composé de formule (I) selon l'une quelconque des revendications 1 à 12, à l'état de base ou de sel d'addition à un acide pharmaceutiquement acceptable, ainsi qu'au moins un excipient pharmaceutiquement acceptable.

20. Composé de formule générale (I) selon l'une quelconque des revendications 1 à 12 pour son utilisation pour le traitement ou la prévention des désordres du sommeil, des troubles du rythme circadien.

21. Composé de formule générale (I) selon l'une quelconque des revendications 1 à 12 pour son utilisation pour le traitement ou la prévention des maladies liées à la dépendance à des substances d'abus.

## Patentansprüche

1. Verbindung der allgemeinen Formel (I) 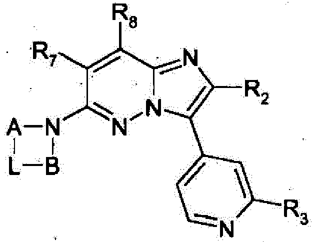 wobei
- R₂ eine Phenyl-Gruppe, gegebenenfalls substituiert durch einen oder mehrere Substituenten, ausgewählt aus Halogenatomen und C₁₋₆-Alkyl-, C₁₋₆-Alkyloxy-, C₁₋₆-Fluoralkyl-Gruppen, bedeutet;
- R₃ ein Wasserstoffatom oder eine C₁₋₃-Alkyl-, -NR₄R₅-Hydroxyl- oder C₁₋₄-Akyloxy-Gruppe bedeutet;
- A eine C₁₋₇-Alkylen-Gruppe, gegebenenfalls substituiert durch eine oder zwei Rₐ-Gruppen, bedeutet;
- B eine C₁₋₇-Alkylen-Gruppe, gegebenenfalls substituiert durch eine R_{b}-Gruppe, bedeutet;
- L entweder ein Stickstoffatom, gegebenenfalls substituiert durch eine R_{c}- oder R_{d}-Gruppe, oder ein Kohlenstoffatom, substituiert durch eine Rₑ₁-Gruppe und eine R_{d}-Gruppe oder zwei Rₑ₂-Gruppen, bedeutet;
wobei die Kohlenstoffatome von A und von B gegebenenfalls durch eine oder mehrere identische oder voneinander verschiedene R_{f}-Gruppen substituiert sind;
wobei, wenn sie vorhanden sind, die Rₐ, R_{b} und R_{c} in folgender Kombination definiert sind:
entweder zwei Rₐ-Gruppen bilden gemeinsam eine C₁₋₆-Alkylen-Gruppe;
oder R₃ und R_{b} bilden gemeinsam eine Bindung oder eine C₁₋₆-Alkylen-Gruppe;
oder R₃ und R_{c} bilden gemeinsam eine Bindung oder eine C₁₋₆-Alkylen-Gruppe;
oder R_{b} und R_{c} bilden gemeinsam eine Bindung oder eine C₁₋₆-Alkylen-Gruppe;
R_{d} eine Gruppe, ausgewählt aus einem Wasserstoffatom und C₁₋₆-Alkyl-, C₃₋₇-Cycloalkyl-, C₃₋₇-Cycloalkyl-C₁₋₆-alkyl-, C₁₋₆-Alkylthio-C₁₋₆-alkyl-, C₁₋₆-Alkoxy-C₁₋₆-alkyl-, C₁₋₆-Fluoralkyl-, Benzyl-, C₁₋₆-Acyl-, Hydroxy-C₁₋₆-alkyl-Gruppen, bedeutet;
Rₑ₁ eine -NR₄R₅-Gruppe oder ein cyclisches Monoamin, gegebenenfalls umfassend ein Sauerstoffatom, bedeutet, wobei das cyclische Monoamin gegebenenfalls substituiert ist durch einen oder mehrere Substituenten, ausgewählt aus einem Fluoratom und C₁₋₆-Alkyl-, C₁₋₆-Alkyloxy-, HydroxylGruppen;
die beiden Rₑ₂, mit dem Kohlenstoffatom, das diese trägt, eine Pyrrolidin-, Piperidin-, Morpholin-Gruppe, gegebenenfalls substituiert durch eine oder mehrere identische oder voneinander verschiedene R_{f}-Gruppen, bedeuten;
R_{f} eine C₁₋₆-Alkyl-, C₃₋₇-Cycloalkyl-, C₃₋₇-Cycloalkyl-C₁₋₆-alkyl-, C₁₋₆-Alkyloxy-C₁₋₆-alkyl-, Hydroxy-C₁₋₆-alkyl-, C₁₋₆-Fluoralkyl- oder BenzylGruppe bedeutet;
R₄ und R₅ unabhängig voneinander ein Wasserstoffatom oder eine C₁₋₄-Alkyl-, C₃₋₇-Cycloalkyl-, C₃₋₇Cycloalkyl-C₁₋₆-alkyl-Gruppe bedeuten;
- R₇ und R₈ unabhängig voneinander ein Wasserstoffatom oder eine C₁₋₆-Alkyl-Gruppe bedeuten;
in dem Zustand einer Base oder eines Säureadditionssalzes.

2. Verbindung der allgemeinen Formel (I) nach Anspruch 1, **dadurch gekennzeichnet, dass**:
R₃ eine Gruppe, ausgewählt aus einem Wasserstoffatom, einer C₁₋₃-Alkyl-, C₁₋₄-Alkyloxy-, -NR₄R₅-Gruppe, bedeutet, wobei R₄ und R₅ ein Wasserstoffatom oder eine C₁₋₄-Alkyl-Gruppe bedeuten.

3. Verbindung der allgemeinen Formel (I) nach Anspruch 2, **dadurch gekennzeichnet, dass**:
R₃ eine Gruppe, ausgewählt aus einem Wasserstoffatom, einer Methyl- und Methoxy-Gruppe, bedeutet.

4. Verbindung der allgemeinen Formel (I) nach Anspruch 2, **dadurch gekennzeichnet, dass**:
R₃ eine Gruppe, ausgewählt aus -NH₂, Methylamino und Dimethylamino, bedeutet.

5. Verbindung der allgemeinen Formel (I) nach einem der Ansprüche 1 bis 4, **dadurch gekennzeichnet, dass**:
R₇ und R₈ unabhängig voneinander ein Wasserstoffatom oder eine Methyl-Gruppe bedeuten.

6. Verbindung der allgemeinen Formel (I) nach einem der Ansprüche 1 bis 5, **dadurch gekennzeichnet, dass**:
- A eine C₁₋₇-Alkylen-Gruppe bedeutet;
- B eine C₁₋₇-Alkylen-Gruppe bedeutet;
- L einen Kohlenstoff, substituiert durch eine Rₑ₁-Gruppe und eine R_{d}-Gruppe, bedeutet;
- R_{d} ein Wasserstoffatom bedeutet;
- Rₑ₁ eine NR₄R₅-Gruppe bedeutet, wobei R₄ und R₅ unabhängig voneinander eine C₁₋₄-Alkyl-Gruppe bedeuten; oder Rₑ₁ auch ein cyclisches Monoamin, gegebenenfalls umfassend ein Sauerstoffatom, bedeutet, wobei das Monoamin gegebenenfalls durch eine oder mehrere Gruppen, ausgewählt aus einem Fluoratom und Hydroxyl-, C₁₋₆-Alkyl-Gruppen, substituiert ist.

7. Verbindung der allgemeinen Formel (I) nach einem der Ansprüche 1 bis 5, **dadurch gekennzeichnet, dass**:
- A eine C₁₋₇-Alkylen-Gruppe, gegebenenfalls substituiert durch eine oder zwei Rₐ-Gruppen, bedeutet;
- B eine C₁₋₇-Alkylen-Gruppe, gegebenenfalls substituiert durch eine R_{b}-Gruppe, bedeutet;
- L ein Stickstoffatom, gegebenenfalls substituiert durch eine R_{c}- oder R_{d}-Gruppe, bedeutet;
wobei die Kohlenstoffatome von A und von B gegebenenfalls durch eine oder mehrere identische oder voneinander verschiedene R_{f}-Gruppen substituiert sind;
wobei, wenn sie vorhanden sind, die Rₐ, R_{b} und R_{c} in folgender Kombination definiert sind:
- entweder zwei Rₐ-Gruppen bilden gemeinsam eine C₁₋₆-Alkylen-Gruppe;
- oder R₃ und R_{b} bilden gemeinsam eine Bindung oder eine C₁₋₆-Alkylen-Gruppe;
- oder R₃ und R_{c} bilden gemeinsam eine Bindung oder eine C₁₋₆-Alkylen-Gruppe;
- oder R_{b} und R_{c} bilden gemeinsam eine Bindung oder eine C₁₋₆-Alkylen-Gruppe;
- R_{d} einen Substituenten, ausgewählt aus einer C₁₋₆-Alkyl-, C₃₋₇-Cycloalkyl-, C₃₋₇-Cycloalkyl-C₁₋₆-alkyl-, Hydroxy-C₁₋₆-alkyl-, C₁₋₆-Alkyloxy-C₁₋₆-alkyl-, C₁₋₆-Fluoralkyl-, Benzyl-, C₁₋₆-Acyl-Gruppe, bedeutet;
- R_{f} eine C₁₋₆-Alkyl-Gruppe bedeutet.

8. Verbindung der allgemeinen Formel (I) nach einem der Ansprüche 1 bis 5, **dadurch gekennzeichnet, dass**:
- A eine C₁₋₇-Alkylen-Gruppe bedeutet;
- B eine C₁₋₇-Alkylen-Gruppe bedeutet;
- L ein Kohlenstoffatom, substituiert durch zwei Rₑ₂-Gruppen, bedeutet;
wobei die Kohlenstoffatome von A und von B gegebenenfalls durch eine oder mehrere identische oder voneinander verschiedene R_{f}-Gruppen substituiert sind;
- zwei Rₑ₂ mit dem Kohlenstoffatom, das diese trägt, eine Pyrrolidin-, Piperidin-, Morpholin-Gruppe bilden;
- R_{f} eine C₁₋₆-Alkyl-Gruppe bedeutet.

9. Verbindung der allgemeinen Formel (I) nach einem der Ansprüche 1 bis 5, **dadurch gekennzeichnet, dass**:
- R₂ eine Phenyl-Gruppe, gegebenenfalls substituiert durch einen oder mehrere Substituenten, ausgewählt aus Fluor-, Chloratomen und einer Methyl-Gruppe, bedeutet;
- R₃ ein Wasserstoffatom bedeutet;
das cyclische Amin, das durch -N-A-L-B- gebildet wird, ein (+/-)-3-Dimethylaminopyrrolidin-1-yl, 4-(Pyrrolidin-1-yl)-piperidin-1-yl, 4-(Morpholin-4-yl)-piperidin-1-yl, 4-(2,6-Dimethylmorpholin-4-yl)-piperidin-1-yl, 4-Dimethylaminopiperidin-1-yl, 4-(3-Hydroxypyrrolidin-1-yl)-piperidin-1-yl, 4-(3-Fluorpyrrolidin-1-yl)-piperidin-1-yl bedeutet;
- R₇ und R₈ ein Wasserstoffatom bedeuten.

10. Verbindung der allgemeinen Formel (I) nach einem der Ansprüche 1 bis 5, **dadurch gekennzeichnet, dass**:
- R₂ eine Phenyl-Gruppe, gegebenenfalls substituiert durch einen oder mehrere Substituenten, ausgewählt aus Fluor- und Chloratomen und Methyl-, Methoxy-, Trifluormethyl-Gruppen, bedeutet;
- R₃ ein Wasserstoffatom oder eine Methyl-, Methoxy-, --NH2-, Methylamino-, Dimethylamino-Gruppe bedeutet;
- das cyclische Amin, das durch -N-A-L-B-gebildet wird, ein Piperazin-1-yl, 3-Methylpiperazin-1-yl, 4-Methylpiperazin-1-yl, 3,3-Dimethylpiperazin-1-yl, 3,4-Dimethylpiperazin-1-yl, *cis*-3,5-Dimethylpiperazin-1-yl, 4-(2-Hydroxyethyl)-piperazin-1-yl, 4-(2-Methoxyethyl)-piperazin-1-yl, 4-(2-Fluorethyl)-piperazin-1-yl, 4-(2,2,2-Trifluorethyl)-piperazin-1-yl, 4-Cyclopropylpiperazin-1-yl, 4-Isopropylpiperazin-1-yl, 4-(2-Hydroxy-2-methylpropyl)-piperazin-1-yl, 4-n-Butylpiperazin-1-yl, 4-(3-Hydroxy-3-methylbutyl)-piperazin-1-yl, Cyclohexylpiperazin-1-yl, 4-Acetylpiperazin-1-yl, 4-Isobutyrylpiperazin-1-yl, (+/-)-Hexahydropyrrolo[1,2-a]pyrazin-2-yl, (S)-Hexahydropyrrolo[1,2-a]pyrazin-2-yl, (1S,4S)-2,5-Diazabicyclo[2.2.1]hept-2-yl, 1(S,4S)-5-Benzyl-2,5-diazabicyclo[2.2.1]hept-2-yl, (1S,4S)-5- (2-Hydroxyethyl)-2,5-diazabicyclo[2.2.1]hept-2-yl, 5-Isopropyl-2,5-diaza[2.2.1]hept-2-yl, 4-Methyl[1,4]diazepan-1-yl, (+/-)-Octahydropyrrolo[1,2-d][1,4]-diazepin-3-yl, 1,4-Diazabicyclo[3.3.2]non-4-yl, (+/-)-3,6-Diazabicyclo[3.2.0]hept-3-yl, Hexahydropyrrolo[3,4-b]pyrrol-5(1H)-yl, Hexahydropyrrolo[3,4-c]pyrrol-2(1H)-yl, 5-Benzylhexahydropyrrolo[3,4-c]pyrrol-2(1H)-yl, 5-Cyclopropylhexahydropyrrolo[3,4-c]pyrrol-2(1H)-yl, 5-Isopropylhexahydropyrrolo[3,4-c]pyrrol-2(1H)-yl, Octahydro-6H-pyrrolo[3,4-b]pyridin-6-yl, (+/-)-(*cis*)-Decahydro[2,6]naphthyridin-2-yl, 3-Ethylpiperazin-1-yl, 3,3-Diethylpiperazin-1-yl, 3-Flourmethylpiperazin-1-yl, 4-(3-Hydroxymethyl)-piperazin-1-yl, 3-(1-Hydroxy-1-methylethyl)-piperazin-1-yl, 3-Isopropylpiperazin-1-yl, (1R,5R)-3,6-Diazabicyclo[3.2.0]hept-2-yl, 5-Methyl-2,5-diazabicyclo[2.2.1]hept-2-yl, 1-((1S,4S)-(2,5-Diazabicyclo[2.2.1]hept-2-yl)-2-methylpropan-2-ol,3,6-Diazabicyclo[3.1.1]hept-3-yl, 5-Methylhexahydropyrrolo[3,4-c]pyrrol-2(1H)-yl, (+/-)-Hexahydropyrrolo[3,4-b]pyrrol-5(1H)-yl, (+)-Hexahydropyrrolo[3,4-b]pyrrol-5(1H)-yl, (-)-Hexahydropyrrolo[3,4-b]pyrrol-5(1H)-yl, (4aS,7aS)-Octahydropyrrolo[3,4-b]pyridin-6-yl, 6,9-Diazaspiro[4.5]dec-9-yl bedeutet;
- R₇ und R₈ unabhängig voneinander ein Wasserstoffatom oder eine Methyl-Gruppe bedeuten.

11. Verbindung der allgemeinen Formel (I) nach einem der Ansprüche 1 bis 5, **dadurch gekennzeichnet, dass**:
- R₂ eine Phenyl-Gruppe, gegebenenfalls substituiert durch einen oder mehrere Substituenten, ausgewählt aus Fluor- und Chloratomen, bedeutet;
- R₃ ein Wasserstoffatom oder eine Methyl-, Methoxy-, -NH₂-Gruppe bedeutet;
- das cyclische Amin, das durch -N-A-L-B-gebildet wird, ein (+/-)-2,7-Diazaspiro[4.4]non-2-yl, (+/-)-2,8-Diazaspiro[4.5]dec-2-yl, (+/-)-2,7-Diazaspiro[4.5]dec-2-yl, (+/-)-2,8-Diazaspiro[4.5]dec-8-yl, (+/-)-2,7-Diazaspiro[4.5]dec-7-yl, 3,9-Diazaspiro[5.5]undec-3-yl, 2,9-Diazaspiro[5.5]undec-9-yl, (+/-)-2,8-Diazaspiro[5.5]undec-2-yl, 1-Oxa-4,9-diazaspiro[5.5]undec-9-yl bedeutet,
- R₇ und R₈ ein Wasserstoffatom bedeuten.

12. Verbindung der allgemeinen Formel (I) nach Anspruch 1, **dadurch gekennzeichnet, dass** diese aus den folgenden Verbindungen ausgewählt ist:
2-Phenyl-6-piperazin-1-yl-3-pyridin-4-ylimidazo[1,2-b]pyridazin;
2-(4-Fluorphenyl)-6-piperazin-1-yl-3-pyridin-4-ylimidazo[1,2-b]pyridazin;
2-(4-Fluorphenyl)-7,8-dimethyl-6-piperazin-1-yl-3-pyridin-4-ylimidazo[1,2-b]pyridazin;
4-[2-(4-Fluorphenyl)-6-piperazin-1-ylimidazo[1,2-b]pyridazin-3-yl]-pyridin-2-ylamin;
2-(4-Fluorphenyl)-3-(2-methoxypyridin-4-yl)-6-piperazin-1-ylimidazo[1,2-b]pyridazin;
2-(4-Chlorphenyl)-6-piperazin-1-yl-3-pyridin-4-ylimidazo[1,2-b]pyridazin;
2-(4-Chlorphenyl)-7-methyl-6-piperazin-1-yl-3-pyridin-4-ylimidazo[1,2-b]pyridazin;
2-(4-Chlorphenyl)-8-methyl-6-piperazin-1-yl-3-pyridin-4-ylimidazo[1,2-b]pyridazin;
2-(3,5-Dimethylphenyl)-6-piperazin-1-yl-3-pyridin-4-ylimidazo[1,2-b]pyridazin;
2-(3,5-Difluorphenyl)-6-piperazin-1-yl-3-pyridin-4-ylimidazo[1,2-b]pyridazin;
2-(3,5-Difluorphenyl)-3-(2-methylpyridin-4-yl)-6-piperazin-1-ylimidazo[1,2-b]pyridazin;
2-(3,4-Difluorphenyl)-6-piperazin-1-yl-3-pyridin-4-ylimidazo[1,2-b]pyridazin;
2-(3,5-Dichlorphenyl)-6-piperazin-1-yl-3-pyridin-4-ylimidazo[1,2-b]pyridazin;
2-(3,5-Dichlorphenyl)-3-(2-methylpyridin-4-yl)-6-piperazin-1-ylimidazo[1,2-b]pyridazin;
(+/-)-6-(3-Methylpiperazin-1-yl)-2-phenyl-3-pyridin-4-ylimidazo[1,2-b]pyridazin;
6-((R)-3-Methylpiperazin-1-yl)-2-phenyl-3-pyridin-4-ylimidazo[1,2-b]pyridazin;
6-((R)-3-Methylpiperazin-1-yl)-3-(2-methylpyridin-4-yl)-2-phenylimidazo[1,2-b]pyridazin;
6-((S)-3-Methylpiperazin-1-yl)-2-phenyl-3-pyridin-4-ylimidazo[1,2-b]pyridazin;
2-(3-Fluorphenyl)-6-((R)-3-methylpiperazin-1-yl)-3-pyridin-4-ylimidazo[1,2-b]pyridazin;
2-(4-Fluorphenyl)-6-(3-methylpiperazin-1-yl)-3-pyridin-4-ylimidazo[1,2-b]pyridazin;
2-(4-Fluorphenyl)-6-((R)-3-methylpiperazin-1-yl)-3-pyridin-4-ylimidazo[1,2-b]pyridazin;
2-(4-Fluorphenyl)-6-((S)-3-methylpiperazin-1-yl)-3-pyridin-4-ylimidazo[1,2-b]pyridazin;
2-(4-Fluorphenyl)-3-(2-methoxypyridin-4-yl)-6-((R)-3-methylpiperazin-1-yl)-imidazo[1,2-b]pyridazin;
2-(3,4-Difluorphenyl)-6-((R)-3-methylpiperazin-1-yl)-3-pyridin-4-ylimidazo[1,2-b]pyridazin;
6-(3,3-Dimethylpiperazin-1-yl)-2-phenyl-3-pyridin-4-ylimidazo[1,2-b]pyridazin;
6-(3,3-Dimethylpiperazin-1-yl)-2-(4-fluorphenyl)-3-pyridin-4-ylimidazo[1,2-b]pyridazin;
6-(3,3-Dimethylpiperazin-1-yl)-2-(4-fluorphenyl)-3-(2-methoxypyridin-4-yl)-imidazo[1,2-b]pyridazin;
2-(3,5-Dimethylphenyl)-6-(3,3-dimethylpiperazin-1-yl)-3-pyridin-4-ylimidazo[1,2-b]pyridazin;
2-(3,5-Dimethylphenyl)-6-(3,3-dimethylpiperazin-1-yl)-3-(2-methylpyridin-4-yl)-imidazo[1,2-b]pyridazin;
4-[2-(3,5-Dimethylphenyl)-6-(3,3-dimethylpiperazin-1-yl)-imidazo[1,2-b]pyridazin-3-yl]-pyridin-2-ylamin;
2-(3,5-Difluorphenyl)-6-(3,3-dimethylpiperazin-1-yl)-3-pyridin-4-ylimidazo[1,2-b]pyridazin;
2-(3,5-Difluorphenyl)-6-(3,3-dimethylpiperazin-1-yl)-3-(2-methylpyridin-4-yl)-imidazo[1,2-b]pyridazin;
4-[2-(3,5-Difluorphenyl)-6-(3,3-dimethylpiperazin-1-yl)-imidazo[1,2-b]pyridazin-3-yl]-pyridin-2-ylamin;
2-(3,5-Dichlorphenyl)-6-(3,3-dimethylpiperazin-1-yl)-3-pyridin-4-ylimidazo[1,2-b]pyridazin;
2-(3,5-Dichlorphenyl)-6-(3,3-dimethylpiperazin-1-yl)-3-(2-methylpyridin-4-yl)-imidazo[1,2-b]pyridazin;
(+/-)-6-(3,4-Dimethylpiperazin-1-yl)-2-(4-fluorphenyl)-3-pyridin-4-ylimidazo[1,2-b]pyridazin;
6-((*cis*)-3,5-Dimethylpiperazin-1-yl)-2-phenyl-3-pyridin-4-ylimidazo[1,2-b]pyridazin;
6-((*cis*)-3,5-Dimethylpiperazin-1-yl)-3-(2-methylpyridin-4-yl)-2-phenylimidazo[1,2-b]pyridazin;
6-((*cis*)-3,5-Dimethylpiperazin-1-yl)-2-(3-fluorphenyl)-3-pyridin-4-ylimidazo[1,2-b]pyridazin;
6-((*cis*)-3,5-Dimethylpiperazin-1-yl)-2-(4-fluorphenyl)-3-pyridin-4-ylimidazo[1,2-b]pyridazin;
6-((*cis*)-3,5-Dimethylpiperazin-1-yl)-2-(4-fluorphenyl)-3-(2-methoxypyridin-4-yl)-imidazo[1,2-b]pyridazin;
2-(3,5-Dimethylphenyl)-6-((*cis*)-3,5-dimethylpiperazin-1-yl)-3-pyridin-4-ylimidazo[1,2-b]pyridazin;
2-(3,4-Difluorphenyl)-6-((*cis*)-3,5-dimethylpiperazin-1-yl)-3-pyridin-4-ylimidazo[1,2-b]pyridazin;
2-(4-Chlorphenyl)-6-((*cis*)-3,5-dimethylpiperazin-1-yl)-7-methyl-3-pyridin-4-ylimidazo[1,2-b]pyridazin;
6-((*cis*)-3,5-Dimethylpiperazin-1-yl)-2-(4-fluorphenyl)-8-methyl-3-pyridin-4-ylimidazo[1,2-b]pyridazin;
2-(2-Chlorphenyl)-6-(4-methylpiperazin-1-yl)-3-pyridin-4-ylimidazo[1,2-b]pyridazin;
2-(3-Chlorphenyl)-6-(4-methylpiperazin-1-yl)-3-pyridin-4-ylimidazo[1,2-b]pyridazin;
2-(4-Chlorphenyl)-6-(4-methylpiperazin-1-yl)-3-pyridin-4-ylimidazo[1,2-b]pyridazin;
2-(3-Fluorphenyl)-6-(4-methylpiperazin-1-yl)-3-pyridin-4-ylimidazo[1,2-b]pyridazin;
2-(4-Fluorphenyl)-6-(4-methylpiperazin-1-yl)-3-pyridin-4-ylimidazo[1,2-b]pyridazin;
2-(3,4-Difluorphenyl)-6-(4-methylpiperazin-1-yl)-3-pyridin-4-ylimidazo[1,2-b]pyridazin;
2-(3-Methoxyphenyl)-6-(4-methylpiperazin-1-yl)-3-pyridin-4-ylimidazo[1,2-b]pyridazin;
2-(4-Methoxyphenyl)-6-(4-methylpiperazin-1-yl)-3-pyridin-4-ylimidazo[1,2-b]pyridazin;
6-(4-Methylpiperazin-1-yl)-3-pyridin-4-yl-2-p-tolylimidazo[1,2-b]pyridazin;
6-(4-Methylpiperazin-1-yl)-3-pyridin-4-yl-2-(4-trifluormethylphenyl)-imidazo[1,2-b]pyridazin;
2-(4-Fluorphenyl)-6-(4-methylpiperazin-1-yl)-3-(2-methylpyridin-4-yl)-imidazo[1,2-b]pyridazin;
4-[2-(4-Fluorphenyl)-6-(4-methylpiperazin-1-yl)-imidazo[1,2-b]pyridazin-3-yl]-pyridin-2-ylamin;
{4-[2-(4-Fluorphenyl)-6-(4-methylpiperazin-1-yl)-imidazo[1,2-b]pyridazin-3-yl]-pyridin-2-yl}-dimethylamin;
2-(4-Fluorphenyl)-3-(2-methoxypyridin-4-yl)-6-(4-methylpiperazin-1-yl)-imidazo[1,2-b]pyridazin;
6-(4-Ethylpiperazin-1-yl)-2-(4-fluorphenyl)-3-pyridin-4-ylimidazo[1,2-b]pyridazin;
2-(3,5-Dimethylphenyl)-6-(4-ethylpiperazin-1-yl)-3-pyridin-4-ylimidazo[1,2-b]pyridazin;
2-[4-(2-Phenyl-3-pyridin-4-ylimidazo[1,2-b]pyridazin-6-yl)-piperazin-1-yl]-ethanol;
2-{4-[2-(3-Fluorphenyl)-3-pyridin-4-ylimidazo[1,2-b]pyridazin-6-yl]-piperazin-1-yl}-ethanol;
2-{4-[2-(4-Fluorphenyl)-3-pyridin-4-ylimidazo[1,2-b]pyridazin-6-yl]-piperazin-1-yl}-ethanol;
2-{4-[2-(4-Fluorphenyl)-3-(2-methoxypyridin-4-yl)-imidazo[1,2-b]pyridazin-6-yl]-piperazin-1-yl}-ethanol;
2-{4-[2-(3,4-Difluorphenyl)-3-pyridin-4-ylimidazo[1,2-b]pyridazin-6-yl]-piperazin-1-yl}-ethanol;
2-{4-[2-(4-Chlorphenyl)-3-pyridin-4-ylimidazo[1,2-b]pyridazin-6-yl]-piperazin-1-yl}-ethanol;
2-{4-[3-(2-Aminopyridin-4-yl)-2-(4-chlorphenyl)-imidazo[1,2-b]pyridazin-6-yl]-piperazin-1-yl}-ethanol;
2-(4-Chlorphenyl)-6-[4-(2-methoxyethyl)-piperazin-1-yl]-3-pyridin-4-ylimidazo[1,2-b]pyridazin;
6-[4-(2-Fluorethyl)-piperazin-1-yl]-2-(4-fluorphenyl)-3-pyridin-4-ylimidazo[1,2-b]pyridazin;
6-[4-(2-Fluorethyl)-piperazin-1-yl]-2-(4-fluorphenyl)-3-(2-methoxypyridin-4-yl)-imidazo[1,2-b]pyridazin;
2-Phenyl-3-pyridin-4-yl-6-[4-(2,2,2-trifluorethyl)-piperazin-1-yl]-imidazo[1,2-b]pyridazin;
2-(4-Fluorphenyl)-3-pyridin-4-yl-6-[4-(2,2,2-trifluorethyl)-piperazin-1-yl]-imidazo[1,2-b]pyridazin;
6-(4-Cyclopropylpiperazin-1-yl)-2-(4-fluorphenyl)-3-pyridin-4-ylimidazo[1,2-b]pyridazin;
6-(4-Cyclopropylpiperazin-1-yl)-2-(4-fluorphenyl)-3-(2-methoxypyridin-4-yl)-imidazo[1,2-b]pyridazin;
6-(4-Isopropylpiperazin-1-yl)-3-(2-methoxypyridin-4-yl)-2-phenylimidazo[1,2-b]pyridazin;
2-(3-Fluorphenyl)-6-(4-isopropylpiperazin-1-yl)-3-pyridin-4-ylimidazo[1,2-b]pyridazin;
2-(4-Fluorphenyl)-6-(4-isopropylpiperazin-1-yl)-3-pyridin-4-ylimidazo[1,2-b]pyridazin;
2-(4-Fluorphenyl)-6-(4-isopropylpiperazin-1-yl)-3-(2-methoxypyridin-4-yl)-imidazo[1,2-b]pyridazin;
2-(3,4-Difluorphenyl)-6-(4-isopropylpiperazin-1-yl)-3-pyridin-4-ylimidazo[1,2-b]pyridazin;
4-[2-(4-Chlorphenyl)-6-(4-isopropylpiperazin-1-yl)-imidazo[1,2-b]pyridazin-3-yl]-pyridin-2-ylamin;
2-Methyl-1-[4-(2-phenyl-3-pyridin-4-ylimidazo[1,2-b]pyridazin-6-yl)-piperazin-1-yl]-propan-2-ol;
1-{4-[2-(3-Fluorphenyl)-3-pyridin-4-ylimidazo[1,2-b]pyridazin-6-yl]-piperazin-1-yl}-2-methylpropan-2-ol;
1-{4-[2-(4-Fluorphenyl)-3-(2-methoxypyridin-4-yl)-imidazo[1,2-b]pyridazin-6-yl]-piperazin-1-yl}-2-methylpropan-2-ol;
6-(4-Butylpiperazin-1-yl)-2-(4-chlorphenyl)-3-pyridin-4-ylimidazo[1,2-b]pyridazin;
4-{4-[2-(4-Fluorphenyl)-3-(2-methoxypyridin-4-yl)-imidazo[1,2-b]pyridazin-6-yl]-piperazin-1-yl}-2-methylbutan-2-ol;
6-(4-Cyclohexylpiperazin-1-yl)-2-(4-fluorphenyl)-3-pyridin-4-ylimidazo[1,2-b]pyridazin;
1-{4-[2-(4-Fluorphenyl)-3-pyridin-4-ylimidazo[1,2-b]pyridazin-6-yl]-piperazin-1-yl}-ethanon;
1-{4-[2-(4-Fluorphenyl)-3-pyridin-4-ylimidazo[1,2-b]pyridazin-6-yl]-piperazin-1-yl}-2-methylpropan-1-on;
(+/-)-2-(4-Fluorphenyl)-6-(hexahydropyrrolo[1,2-a]pyrazin-2-yl)-3-pyridin-4-ylimidazo[1,2-b]pyridazin;
2-(4-Fluorphenyl)-6-(S)-hexahydropyrrolo[1,2-a]pyrazin-2-yl)-3-(2-methoxypyridin-4-yl)-imidazo[1,2-b]pyridazin;
6-((1S,4S)-2,5-Diazabicyclo[2.2.1]hept-2-yl)-2-phenyl-3-pyridin-4-ylimidazo[1,2-b]pyridazin;
6-((1S,4S)-5-Benzyl-2,5-diazabicyclo[2.2.1]hept-2-yl)-2-(3-fluorphenyl)-3-pyridin-4-ylimidazo[1,2-b]pyridazin;
6-((1S,4S)-2,5-Diazabicyclo[2.2.1]hept-2-yl)-2-(4-fluorphenyl)-3-pyridin-4-ylimidazo[1,2-b]pyridazin;
6-((1S,4S)-5-Benzyl-2,5-diazabicyclo[2.2.1]hept-2-yl)-2-(4-fluorphenyl)-3-pyridin-4-ylimidazo[1,2-b]pyridazin;
6-((1S,4S)-2,5-Diazabicyclo[2.2.1]hept-2-yl)-2-(4-fluorphenyl)-3-(2-methoxypyridin-4-yl)-imidazo[1,2-b]pyridazin;
2-{(1S,4S)-5-[2-(4-Fluorphenyl)-3-(2-methoxypyridin-4-yl)-imidazo[1,2-b]pyridazin-6-yl]-2,5-diazabicyclo[2.2.1]hept-2-yl}-ethanol;
2-(4-Fluorphenyl)-6-(5-isopropyl-2,5-diazabicyclo[2.2.1]hept-2-yl)-3-(2-methoxypyridin-4-yl)-imidazo[1,2-b]pyridazin;
2-(4-Fluorphenyl)-6-(4-methyl[1,4]diazepan-1-yl)-3-pyridin-4-ylimidazo[1,2-b]pyridazin;
(+/-)-3-[2-(4-Fluorphenyl)-3-pyridin-4-ylimidazo[1,2-b]pyridazin-6-yl]-octahydro(1H)-pyrrolo[1,2-d][1,4]diazepin;
(+/-)-4-[2-(4-Fluorphenyl)-3-pyridin-4-ylimidazo[1,2-b]pyridazin-6-yl]-1,4-diazabicyclo[3.2.2]nonan;
(+/-)-4-[2-(3,5-Dimethylphenyl)-3-pyridin-4-ylimidazo[1,2-b]pyridazin-6-yl]-1,4-diazabicyclo[3.2.2]nonan;
(+/-)-3,6-Diazabicyclo[3.2.0]hept-3-yl-2-phenyl-3-pyridin-4-ylimidazo[1,2-b]pyridazin;
(+/-)-6-Hexahydropyrrolo[3,4-b]pyrrol-5(1H)-yl)-2-phenyl-3-pyridin-4-ylimidazo[1,2-b]pyridazin;
(+/-)-6-Hexahydropyrrolo[3,4-b]pyrrol-5(1H)-yl)-3-(2-methylpyridin-4-yl)-2-phenylimidazo[1,2-b]pyridazin;
6-Hexahydropyrrolo[3,4-c]pyrrol-2(1H)-yl-2-phenyl-3-pyridin-4-ylimidazo[1,2-b]pyridazin;
6-Hexahydropyrrolo[3,4-c]pyrrol-2(1H)-yl-3-(2-methylpyridin-4-yl)-2-phenylimidazo[1,2-b]pyridazin;
2-(3-Fluorphenyl)-6-hexahydropyrrolo[3,4-c]pyrrol-2(1H)-yl-3-(2-methylpyridin-4-yl)-imidazo[1,2-b]pyridazin;
2-(4-Fluorphenyl)-6-hexahydropyrrolo[3,4-c]pyrrol-2(1H)-yl-3-pyridin-4-ylimidazo[1,2-b]pyridazin;
2-(4-Fluorphenyl)-6-hexahydropyrrolo[3,4-c]pyrrol-2(1H)-yl-3-(2-methylpyridin-4-yl)-imidazo[1,2-b]pyridazin;
4-[2-(4-Chlorphenyl)-6-(hexahydropyrrolo[3,4-c]pyrrol-2(1H)-yl)-imidazo[1,2-b]pyridazin-3-yl]-pyridin-2-ylamin;
2-(4-Fluorphenyl)-6-(hexahydropyrrolo[3,4-c]pyrrol-2(1H)-yl)-3-(2-methoxypyridin-4-yl)-imidazo[1,2-b]pyridazin;
4-[6-(5-Benzylhexahydropyrrolo[3,4-c]pyrrol-2(1H)-yl)-7,8-dimethyl-2-(4-fluorphenyl)-imidazo[1,2-b]pyridazin-3-yl]-pyridin-2-ylamin;
4-[2-(4-Fluorphenyl)-6-hexahydropyrrolo[3,4-c]pyrrol-2(1H)-yl-7,8-dimethylimidazo[1,2-b]pyridazin-3-yl]-pyridin-2-ylamin;
6-(5-Cyclopropylhexahydropyrrolo[3,4-c]pyrrol-2(1H)-yl)-2-(4-fluorphenyl)-3-pyridin-4-ylimidazo[1,2-b]pyridazin;
2-(4-Fluorphenyl)-6-(5-isopropylhexahydropyrrolo[3,4-c]pyrrol-2(1H)-yl)-3-pyridin-4-ylimidazo[1,2-b]pyridazin;
2-(4-Fluorphenyl)-6-(5-isopropylhexahydropyrrolo[3,4-c]pyrrol-2(1H)-yl)-3-(2-methylpyridin-4-yl)-imidazo[1,2-b]pyridazin;
2-(4-Fluorphenyl)-6-(5-isopropylhexahydropyrrolo[3,4-c]pyrrol-2(1H)-yl)-3-(2-methoxypyridin-4-yl)-imidazo[1,2-b]pyridazin;
(+/-)-(*cis*)-6-(Octahydro-6H-pyrrolo[3,4-b]pyridin-6-yl)-2-phenyl-3-pyridin-4-ylimidazo[1,2-b]pyridazin;
(+/-)-(*cis*)-3-(2-Methylpyridin-4-yl)-6-(octahydro-6H-pyrrolo[3,4-b]pyridin-6-yl)-2-phenylimidazo[1,2-b]pyridazin;
(+/-)-(*cis*)-2-[2-(4-Fluorphenyl)-3-pyridin-4-ylimidazo[1,2-b]pyridazin-6-yl]-decahydro[2,6]naphthyridin;
(+/-)-6-(2,7-Diazaspiro[4.4]non-2-yl)-2-(3-fluorphenyl)-3-pyridin-4-ylimidazo[1,2-b]pyridazin;
(+/-)-6-(2,7-Diazaspiro[4.4]non-2-yl)-2-(4-fluorphenyl)-3-pyridin-4-ylimidazo[1,2-b]pyridazin;
(+/-)-6-(2,8-Diazaspiro[4.5]dec-2-yl)-2-(4-fluorphenyl)-3-pyridin-4-ylimidazo[1,2-b]pyridazin;
(+/-)-6-(2,7-Diazaspiro[4.5]dec-2-yl)-2-(4-fluorphenyl)-3-pyridin-4-ylimidazo[1,2-b]pyridazin;
(+/-)-6-(2,8-Diazaspiro[4.5]dec-8-yl)-2-(4-fluorphenyl)-3-pyridin-4-ylimidazo[1,2-b]pyridazin;
(+/-)-6-(2,7-Diazaspiro[4.5]dec-7-yl)-2-(4-fluorphenyl)-3-pyridin-4-ylimidazo[1,2-b]pyridazin;
3-[2-(4-Fluorphenyl)-3-pyridin-4-ylimidazo[1,2-b]pyridazin-6-yl]-3,9-diazaspiro[5.5]undecan;
9-(2-Phenyl-3-pyridin-4-ylimidazo[1,2-b]pyridazin-6-yl)-2,9-diazaspiro[5.5]undecan;
9-[3-(2-Methylpyridin-4-yl)-2-phenylimidazo[1,2-b]pyridazin-6-yl]-2,9-diazaspiro[5.5]undecan;
9-[2-(3-Fluorphenyl)-3-(2-methylpyridin-4-yl)-imidazo[1,2-b]pyridazin-6-yl]-2,9-diazaspiro[5.5]undecan;
9-[2-(4-Fluorphenyl)-3-pyridin-4-ylimidazo[1,2-b]pyridazin-6-yl]-2,9-diazaspiro[5.5]undecan;
9-[2-(4-Fluorphenyl)-3-(2-methylpyridin-4-yl)-imidazo[1,2-b]pyridazin-6-yl]-2,9-diazaspiro[5.5] undecan;
4-[2-(4-Chlorphenyl)-6-(2,9-diazaspiro[5.5]undec-9-yl)-imidazo[1,2-b]pyridazin-3-yl]-pyridin-2-ylamin;
9-[2-(4-Fluorphenyl)-3-(2-methoxypyridin-4-yl)-imidazo[1,2-b]pyridazin-6-yl]-2,9-diazaspiro[5.5] undecan;
2-[2-(4-Fluorphenyl)-3-(pyridin-4-yl)-imidazo[1,2-b]pyridazin-6-yl]-2,9-diazaspiro[5.5] undecan;
2-[2-(4-Fluorphenyl)-3-(pyridin-4-yl)-imidazo[1,2-b]pyridazin-6-yl]-2,8-diazaspiro[5.5] undecan;
9-[2-(Phenyl)-3-(pyridin-4-yl)-imidazo[1,2-b]pyridazin-6-yl]-1-oxa-4,9-diazaspiro[5.5] undecan;
9-[2-(4-Fluorphenyl)-3-(pyridin-4-yl)-imidazo[1,2-b]pyridazin-6-yl]-1-oxa-4,9-diazaspiro[5.5] undecan;
9-[2-(4-Fluorphenyl)-3-(2-methoxypyridin-4-yl)-imidazo[1,2-b]pyridazin-6-yl]-1-oxa-4,9-diazaspiro[5.5] undecan;
4-{[2-(4-Chlorphenyl)-3-(2-methoxypyridin-4-yl)-imidazo[1,2-b]pyridazin-6-yl]-1-oxa-4,9-diazaspiro[5.5]undec-9-yl}-2-ylamin;
(+/-)-{1-[2-(4-Fluorphenyl)-3-pyridin-4-ylimidazo[1,2-b]pyridazin-6-yl]-pyrrolidin-3-yl}-dimethylamin;
2-(3,5-Dimethylphenyl)-3-pyridin-4-yl-6-(4-pyrrolidin-1-ylpiperidin-1-yl)-imidazo[1,2-b]pyridazin;
6-(4-Morpholin-4-ylpiperidin-1-yl)-2-phenyl-3-pyridin-4-ylimidazo[1,2-b]pyridazin;
6-[4-(2,6-Dimethylmorpholin-4-yl)-piperidin-1-yl]-2-phenyl-3-pyridin-4-ylimidazo[1,2-b]pyridazin;
{1-[2-(4-Fluorphenyl)-3-pyridin-4-ylimidazo[1,2-b]pyridazin-6-yl]-piperidin-4-yl}-dimethylamin;
2-Phenyl-3-pyridin-4-yl-6-(4-pyrrolidin-1-ylpiperidin-1-yl)-imidazo[1,2-b]pyridazin;
2-(4-Fluorphenyl)-3-pyridin-4-yl-6-(4-pyrrolidin-1-ylpiperidin-1-yl)-imidazo[1,2-b]pyridazin;
2-(4-Fluorphenyl)-3-(2-methoxypyridin-4-yl)-6-(4-pyrrolidin-1-ylpiperidin-1-yl)-imidazo[1,2-b]pyridazin;
(R)-1-{1-[2-(4-Fluorphenyl)-3-pyridin-4-ylimidazo[1,2-b]pyridazin-6-yl]-piperidin-4-yl}-pyrrolidin-3-ol;
6-(4-Morpholin-4-yl-piperidin-1-yl)-2-phenyl-3-pyridin-4-ylimidazo[1,2-b]pyridazin;
2-(3-Fluor-5-methylphenyl)-6-((R)-3-methylpiperazin-1-yl)-3-pyridin-4-ylimidazo[1,2-b]pyridazin;
{4-[2-(4-Fluorphenyl)-6-((R)-3-methylpiperazin-1-yl)-imidazo[1,2-b]pyridazin-3-yl]-pyridin-2-yl}-methylamin;
6-(3,3-Dimethylpiperazin-1-yl)-2-(4-fluorphenyl)-7-methyl-3-pyridin-4-ylimidazo[1,2-b]pyridazin;
6-(3,3-Dimethylpiperazin-1-yl)-2-(4-fluorphenyl)-8-methyl-3-pyridin-4-ylimidazo[1,2-b]pyridazin;
6-(3,3-Dimethylpiperazin-1-yl)-2-(3-fluor-5-methylphenyl)-3-pyridin-4-ylimidazo[1,2-b]pyridazin;
{4-[6-(3,3-Dimethylpiperazin-1-yl)-2-(4-fluorphenyl)-imidazo[1,2-b]pyridazin-3-yl]-pyridin-2-yl}-methylamin;
6-((R)-3-Ethylpiperazin-1-yl)-2-(4-fluorphenyl)-3-pyridin-4-ylimidazo[1,2-b]pyridazin;
6-(3,3-Diethylpiperazin-1-yl)-2-(4-fluorphenyl)-3-pyridin-4-ylimidazo[1,2-b]pyridazin;
(+/-)-6-(3-Fluormethylpiperazin-1-yl)-2-(4-fluorphenyl)-3-pyridin-4-ylimidazo[1,2-b]pyridazin;
(+/-)-{4-[2-(4-Fluorphenyl)-3-pyridin-4-ylimidazo[1,2-b]pyridazin-6-yl]-piperazin-2-yl}-methanol;
2-{4-[2-(3-Fluor-5-methylphenyl)-3-pyridin-4-ylimidazo[1,2-b]pyridazin-6-yl]-piperazin-1-yl}-ethanol;
2-{4-[2-(4-Fluorphenyl)-3-(2-methylaminopyridin-4-yl)-imidazo[1,2-b]pyridazin-6-yl]-piperazin-1-yl}-ethanol;
1-{4-[2-(4-Fluorphenyl)-3-pyridin-4-ylimidazo[1,2-b]pyridazin-6-yl]-piperazin-1-yl}-2-methylpropan-2-ol;
1-{4-[2-(4-Fluorphenyl)-8-methyl-3-pyridin-4-ylimidazo[1,2-b]pyridazin-6-yl]-piperazin-1-yl}-2-methylpropan-2-ol;
1-{4-[2-(4-Fluorphenyl)-7-methyl-3-pyridin-4-ylimidazo[1,2-b]pyridazin-6-yl]-piperazin-1-yl}-2-methylpropan-2-ol;
1-{4-[2-(4-Fluorphenyl)-3-(2-methylaminopyridin-4-yl)-imidazo[1,2-b]pyridazin-6-yl]-piperazin-1-yl}-2-methylpropan-2-ol;
2-{(R)-4-[2-(4-Fluorphenyl)-3-pyridin-4-ylimidazo[1,2-b]pyridazin-6-yl]-piperazin-2-yl}-propan-2-ol;
2-(4-Fluorphenyl)-6-((R)-3-isopropylpiperazin-1-yl)-3-pyridin-4-ylimidazo[1,2-b]pyridazin;
{4-[2-(4-Fluorphenyl)-6((R)-3-isopropylpiperazin-1-yl)-imidazo[1,2-b]pyridazin-3-yl]-pyridin-2-yl}-methylamin;
2-(3-Fluorphenyl)-6-(4-isopropylpiperazin-1-yl)-3-(2-methoxypyridin-4-yl)-imidazo[1,2-b]pyridazin;
2-(4-Fluorphenyl)-6-(4-isopropylpiperazin-1-yl)-7-methyl-3-pyridin-4-ylimidazo[1,2-b]pyridazin;
2-(4-Fluorphenyl)-6-(4-isopropylpiperazin-1-yl)-8-methyl-3-pyridin-4-ylimidazo[1,2-b]pyridazin;
{4-[2-(4-Fluorphenyl)-6-(4-isopropylpiperazin-1-yl)-imidazo[1,2-b]pyridazin-3-yl]-pyridin-2-yl}-methylamin;
2-(3,4-Difluorphenyl)-6-(4-isopropylpiperazin-1-yl)-3-(2-methoxypyridin-4-yl)-imidazo[1,2-b]pyridazin;
2-(3-Fluor-5-methylphenyl)-6-(4-isopropylpiperazin-1-yl)-3-pyridin-4-ylimidazo[1,2-b]pyridazin;
(+/-)-6-(3,6-Diazabicyclo[3.2.0]hept-3-yl)-2-phenyl-3-pyridin-4-ylimidazo[1,2-b]pyridazin;
6-(1S,4S)-2,5-Diazabicyclo[2.2.1]hept-2-yl-2-phenyl-3-piridin-4-ylimidazo[1,2-b]pyridazin;
{4-[(1S,4S)-6-2,5-Diazabicyclo[2.2.1]hept-2-yl-2-(4-fluorphenyl)-imidazo[1,2-b]pyridazin-3-yl]-pyridin-2-yl}-methylamin;
2-(4-Fluorphenyl)-6-((1S,4S)-5-methyl-2,5-diazabicyclo[2.2.1]hept-2-yl)-3-pyridin-4-ylimidazo[1,2-b]pyridazin;
2-(4-Fluorphenyl)-7-methyl-6-((1S,4S)-5-methyl-2,5-diazabicyclo[2.2.1]hept-2-yl)-3-pyridin-4-ylimidazo[1,2-b]pyridazin;
2-(4-Fluorphenyl)-8-methyl-6-((1S,4S)-5-methyl-2,5-diazabicyclo[2.2.1]hept-2-yl)-3-pyridin-4-ylimidazo[1,2-b]pyridazin;
2-(4-Fluorphenyl)-6-((1S,4S)-5-methyl-2,5-diazabicyclo[2.2.1]hept-2-yl)-3-(2-methylpyridin-4-yl)-imidazo[1,2-b]pyridazin;
2-Methyl-1-[(1S,4S)-5-(2-phenyl-3-pyridin-4-yl-imidazo[1,2-b]pyridazin-6-yl)-2,5-diazabicyclo[2.2.1]hept-2-yl]-propan-2-ol;
6-(3,6-Diazabicyclo[3.1.1]hept-3-yl)-2-(4-fluorphenyl)-3-pyridin-4-ylimidazo[1,2-b]pyridazin;
2-(4-Fluorphenyl)-6-(5-methylhexahydropyrrolo[3,4-c]pyrrol-2(1H)-yl)-3-pyridin-4-ylimidazo[1,2-b]pyridazin;
2-(4-Fluorphenyl)-7-methyl-6-(5-methylhexahydropyrrolo[3,4-c]pyrrol-2(1H)-yl)-3-pyridin-4-ylimidazo[1,2-b]pyridazin;
2-(4-Fluorphenyl)-8-methyl-6-(5-methylhexahydropyrrolo[3,4-c]pyrrol-2(1H)-yl)-3-pyridin-4-ylimidazo[1,2-b]pyridazin;
2-(4-Fluorphenyl)-8-methyl-6-(5-methylhexahydropyrrolo[3,4-c]pyrrol-2(1H)-yl)-3-(2-methylpyridin-4-yl)-imidazo[1,2-b]pyridazin;
{4-[2-(4-Fluorphenyl)-6-(5-methylhexahydropyrrolo[3,4-c]pyrrol-2(1H)-yl)-imidazo[1,2-b]pyridazin-3-yl]-pyridin-2-yl}-methylamin;
2-(4-Fluorphenyl)-3-(2-methoxypyridin-4-yl)-6-(5-methylhexahydropyrrolo[3,4-c]pyrrol-2(1H)-yl)-imidazo[1,2-b]pyridazin;
2-(4-Fluorphenyl)-6-(5-methylhexahydropyrrolo[3,4-c]pyrrol-2(1H)-yl)-3-(2-methylpyridin-4-yl)-imidazo[1,2-b]pyridazin;
{4-[2-(4-Fluorphenyl)-8-methyl-6-((3aR,6aS)-5-methylhexahydropyrrolo[3,4-c]pyrrol-2(1H)-yl)-imidazo[1,2-b]pyridazin-3-yl]-pyridin-2-yl}-methylamin;
{4-[2-(4-Fluorphenyl)-7-methyl-6-(5-methylhexahydropyrrolo[3,4-c]pyrrol-2(1H)-yl)-imidazo[1,2-b]pyridazin-3-yl]-pyridin-2-yl}-methylamin;
(+/-)-2-(4-Fluorphenyl)-6-(hexahydropyrrolo[3,4-b]pyrrol-5(1H)-yl)-3-pyridin-4-ylimidazo[1,2-b]pyridazin;
(+)-2-(4-Fluorphenyl)-6-hexahydropyrrolo[3,4-b]pyrrol-5(1H)-yl-3-pyridin-4-ylimidazo[1,2-b]pyridazin;
(-)-2-(4-Fluorphenyl)-6-hexahydropyrrolo[3,4-b]pyrrol-5(1H)-yl-3-pyridin-4-ylimidazo[1,2-b]pyridazin;
(+/-)-{4-[2-(4-Fluorphenyl)-6-hexahydropyrrolo[3,4-b]pyrrol-5(1H)-yl)-imidazo[1,2-b]pyridazin-3-yl]-pyridin-2-yl}-methylamin;
6-(4aS,7aS)-Octahydropyrrolo[3,4-b]pyridin-6-yl-2-phenyl-3-pyridin-4-ylimidazo[1,2-b]pyridazin;
3-(2-Methylpyridin-4-yl)-6-(4aS,7aS)-octahydropyrrolo[3,4-b]pyridin-6-yl-2-phenylimidazo[1,2-b]pyridazin;
3-(2-Methylpyridin-4-yl)-6-(4aR,7aR)-octahydropyrrolo[3,4-b]pyridin-6-yl-2-phenylimidazo[1,2-b]pyridazin;
2-(4-Fluorphenyl)-8-methyl-6-(4aS,7aS)-octahydropyrrolo[3,4-b]pyridin-6-yl-3-pyridin-4-ylimidazo[1,2-b]pyridazin;
2-(4-Fluorphenyl)-7-methyl-6-(4aS,7aS)-octahydropyrrolo[3,4-b]pyridin-6-yl-3-pyridin-4-ylimidazo[1,2-b]pyridazin;
2-(4-Fluorphenyl)-7-methyl-6-(4aR,7aR)-octahydropyrrolo[3,4-b]pyridin-6-yl-3-pyridin-4-ylimidazo[1,2-b]pyridazin;
2-(4-Fluorphenyl)-6-(4aS,7aS)-octahydropyrrolo[3,4-b]pyridin-6-yl-3-pyridin-4-ylimidazo[1,2-b]pyridazin;
2-(4-Fluorphenyl)-3-(2-methylpyridin-4-yl)-6-(4aS,7aS)-octahydropyrrolo[3,4-b]pyridin-6-ylimidazo[1,2-b]pyridazin;
{4-[2-(4-Fluorphenyl)-6-octahydropyrrolo[3,4-b]pyridin-6-yl)-imidazo[1,2-b]pyridazin-3-yl]-pyridin-2-yl}-methylamin;
6-(4aR,7aR)-Octahydropyrrolo[3,4-b]pyridin-6-yl-2-phenyl-3-pyridin-4-ylimidazo[1,2-b]pyridazin;
2-(4-Fluorphenyl)-6-(4aR,7aR)-octahydropyrrolo[3,4-b]pyridin-6-yl-3-pyridin-4-ylimidazo[1,2-b]pyridazin;
2-(4-Fluorphenyl)-3-(2-methylpyridin-4-yl)-6-(4aR,7aR)-octahydropyrrolo[3,4-b]pyridin-6-ylimidazo[1,2-b]pyridazin;
2-(4-Fluorphenyl)-8-methyl-6-(4aR,7aR)-octahydropyrrolo[3,4-b]pyridin-6-yl-3-pyridin-4-ylimidazo[1,2-b]pyridazin;
6-(2,7-Diazaspiro[4.4]non-2-yl)-2-(4-fluorphenyl)-8-methyl-3-pyridin-4-ylimidazo[1,2-b]pyridazin;
6-(6,9-Diazaspiro[4.5]dec-9-yl)-2-(4-fluorphenyl)-3-pyridin-4-ylimidazo[1,2-b]pyridazin;
(+/-)-2-[2-(4-Fluorphenyl)-8-methyl-3-pyridin-4-ylimidazo[1,2-b]pyridazin-6-yl]-2,8-diazaspiro[5.5] undecan;
9-[2-(4-Fluorphenyl)-8-methyl-3-pyridin-4-ylimidazo[1,2-b]pyridazin-6-yl]-2,9-diazaspiro[5.5] undecan;
{4-[6-(2,9-Diazaspiro[5.5]undec-9-yl)-2-(4-fluorphenyl)-imidazo[1,2-b]pyridazin-3-yl]-pyridin-2-yl}-methylamin;
9-[2-(3,4-Difluorphenyl)-3-pyridin-4-ylimidazo[1,2-b]pyridazin-6-yl]-2,9-diazaspiro[5.5] undecan;
9-[2-(3-Fluor-5-methylphenyl)-3-pyridin-4-ylimidazo[1,2-b]pyridazin-6-yl]-2,9-diazaspiro[5.5] undecan;
2-(3-Fluorphenyl)-3-pyridin-4-yl-6-(4-pyrrolidin-1-yl-piperidin-1-yl)-imidazo[1,2-b]pyridazin;
{4-[2-(4-Fluorphenyl)-6-(4-pyrrolidin-1-yl-piperidin-1-yl)-imidazo[1,2-b]pyridazin-3-yl]-pyridin-2-yl}-methylamin;
2-(4-Fluorphenyl)-6-[4-((R)-3-fluorpyrrolidin-1-yl)-piperidin-1-yl]-3-pyridin-4-ylimidazo[1,2-b]pyridazin;
(R)-1-[1-(2-Phenyl-3-pyridin-4-ylimidazo[1,2-b]pyridazin-6-yl)-piperidin-4-yl]-pyrrolidin-3-ol;
(R)-1-{1-[2-(3-Fluor-5-methylphenyl)-3-pyridin-4-ylimidazo[1,2-b]pyridazin-6-yl]-piperidin-4-yl}-pyrrolidin-3-ol.

13. Verfahren zur Herstellung einer Verbindung der Formel (I) nach Anspruch 1, **dadurch gekennzeichnet, dass** eine Verbindung der allgemeinen Formel (IIa) 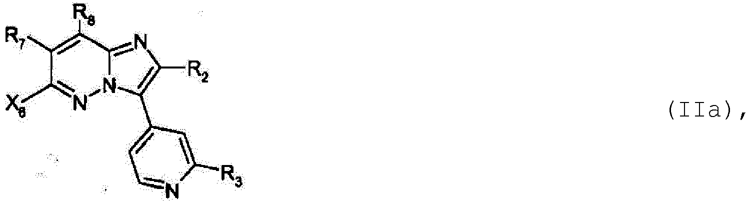 wobei R₂, R₃, R₇ und R₈ wie in Anspruch 1 definiert sind, und X₆ eine Abgangsgruppe bedeutet, mit einem Amin der allgemeinen Formel (IIIa) 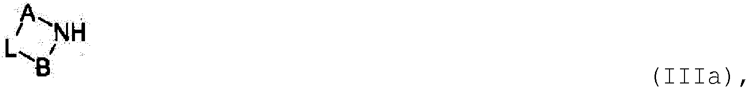 wobei A, L und B wie in Anspruch 1 definiert sind, in einem polaren Lösungsmittel umgesetzt wird.

14. Verfahren zur Herstellung einer Verbindung der Formel (I) nach Anspruch 1, **dadurch gekennzeichnet, dass** eine Verbindung der allgemeinen Formel (Vb) 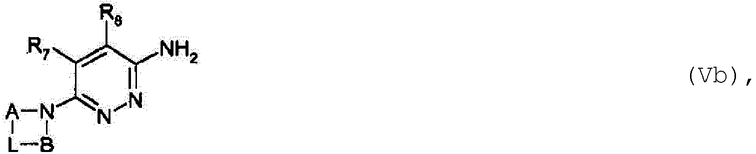 wobei A, L, B, R₇ und R₈ wie in Anspruch 1 definiert sind, mit einer Verbindung der allgemeinen Formel (VIb) 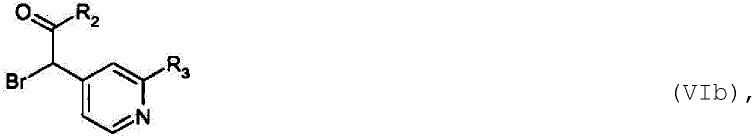 wobei R₂ und R₃ wie in Anspruch 1 definiert sind, in einem polaren Lösungsmittel umgesetzt wird.

15. Verfahren zur Herstellung einer Verbindung der Formel (I) nach Anspruch 1, **dadurch gekennzeichnet, dass** eine Verbindung der allgemeinen Formel (IId) 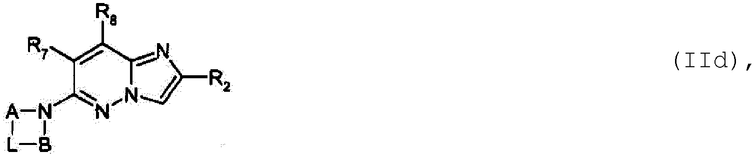 wobei R₂, A, L, B, R₇ und R₈ wie in Anspruch 1 definiert sind, mit einer Verbindung der allgemeinen Formel (IVd) 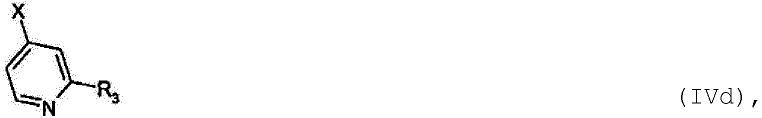 wobei R₃ wie in Anspruch 1 definiert ist, und X ein Iodatom bedeutet, in Anwesenheit eines Katalysators, einer mineralischen Base und in einem polaren aprotischen Lösungsmittel umgesetzt wird.

16. Verfahren zur Herstellung einer Verbindung der Formel (I) nach Anspruch 1, **dadurch gekennzeichnet, dass** eine Verbindung der allgemeinen Formel (IIc) 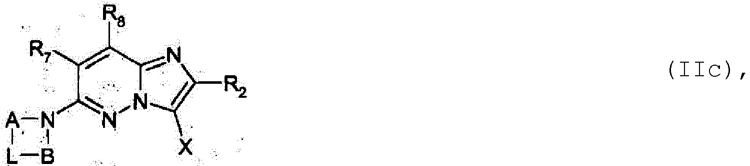 wobei R₂, A, L, B, R₇ und R₈ wie in Anspruch 1 definiert sind, und X ein Iod- oder Bromatom bedeutet, mit einer Verbindung der allgemeinen Formel (IVc) 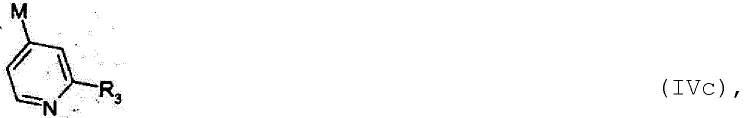 wobei R₃ wie in Anspruch 1 definiert ist, und M eine Trialkylstannyl-Gruppe, eine Dihydroxyboryl- oder Dialkyloxyboryl-Gruppe bedeutet, umgesetzt wird.

17. Verfahren zur Herstellung einer Verbindung der Formel (I) nach Anspruch 1, wobei R₃ ein Wasserstoffatom oder eine C₁₋₃-Alkyl-Gruppe bedeutet, **dadurch gekennzeichnet, dass**:
(a) eine Verbindung der allgemeinen Formel (IId) 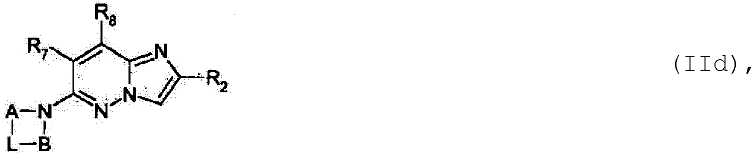 wobei R₂, A, L, B, R₇ und R₈ wie in Anspruch 1 definiert sind, mit einer Mischung einer Verbindung der Formel (IVe) 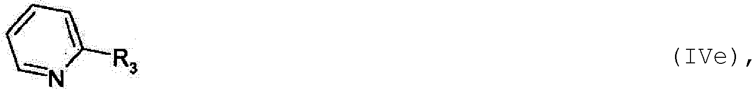 wobei R₃ ein Wasserstoffatom oder eine C₁₋₃-AlkylGruppe bedeutet, in Anwesenheit von Alkylchloroformiat umgesetzt wird, wobei eine Verbindung der Formel (IIe) erhalten wird 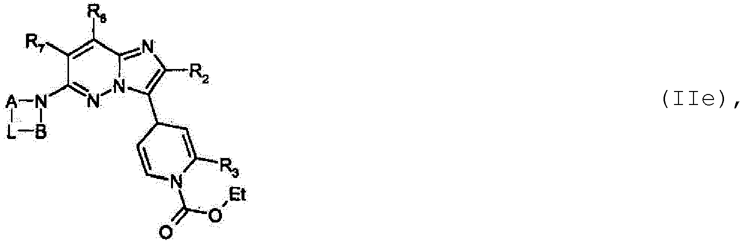 wobei R₂, A, L, B, R₇ und R₈ wie in Anspruch 1 definiert sind, und wobei R₃ ein Wasserstoffatom oder eine C₁₋₃-Alkyl-Gruppe bedeutet;
(b) das in Schritt b) erhaltene Produkt der Formel (IIe) mit einem Oxidationsmittel umgesetzt wird, wobei eine Verbindung der allgemeinen Formel (I) nach Anspruch 1 erhalten wird, und wobei R₃ ein Wasserstoffatom oder eine C₁₋₃-Alkyl-Gruppe bedeutet.

18. Medikament, **dadurch gekennzeichnet, dass** dieses eine Verbindung der Formel (I) nach einem der Ansprüche 1 bis 12 im Zustand einer Base oder eines Additionssalzes einer pharmazeutisch annehmbaren Säure umfasst.

19. Pharmazeutische Zusammensetzung, **dadurch gekennzeichnet, dass** diese eine Verbindung der Formel (I) nach einem der Ansprüche 1 bis 12 im Zustand einer Base oder eines Additionssalzes einer pharmazeutisch annehmbaren Säure sowie mindestens einen pharmazeutisch annehmbaren Exzipienten umfasst.

20. Verbindung der allgemeinen Formel (I) nach einem der Ansprüche 1 bis 12, zu deren Verwendung bei der Behandlung oder der Prävention von Schlafstörungen und Tagesrhythmusstörungen.

21. Verbindung der allgemeinen Formel (I) nach einem der Ansprüche 1 bis 12, zu deren Verwendung bei der Behandlung oder der Prävention von Krankheiten, die mit der Abhängigkeit von Missbrauchsubstanzen verbunden sind.

## Claims

1. Compound corresponding to the general formula (I) 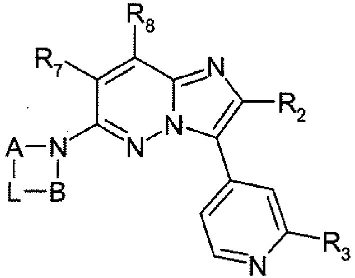 in which
- R₂ represents a phenyl group optionally substituted with one or more substituents chosen from halogen atoms and the groups C₁₋₆ alkyl, C₁₋₆ alkyloxy or C₁₋₆ fluoroalkyl;
- R₃ represents a hydrogen atom or a group C₁₋₃ alkyl,-NR₄R₅, hydroxyl or C₁₋₄ alkyloxy;
- A represents a group C₁₋₇-alkylene optionally substituted with one or two groups R_{a;}
- B represents a group C₁₋₇-alkylene optionally substituted with a group R_{b;}
- L represents either a nitrogen atom optionally substituted with a group R_{c} or R_{d}, or a carbon atom substituted with a group Rₑ₁ and a group R_{d} or two groups Rₑ₂;
the carbon atoms of A and B being optionally substituted with one or more groups R_{f}, which may be identical to or different than each other;
When they are present, Rₐ, R_{b} and R_{c} are defined in combination as follows:
either two groups Rₐ together form a group C₁₋₆-alkylene;
or Rₐ and R_{b} together form a bond or a group C₁₋₆-alkylene;
or Rₐ and R_{c} together form a bond or a group C₁₋₆-alkylene;
or R_{b} and R_{c} together form a bond or a group C₁₋₆-alkylene;
R_{d} represents a group chosen from a hydrogen atom and the groups C₁₋₆-alkyl, C₃₋₇-cycloalkyl, C₃₋₇-cycloalkyl-C₁₋₆-alkyl, C₁₋₆-alkylthio-C₁₋₆-alkyl, C₁₋₆-alkyloxy-C₁₋₆-alkyl, C₁₋₆-fluoroalkyl, benzyl, C₁₋₆-acyl and hydroxy-C₁₋₆-alkyl;
Rₑ₁ represents a group -NR₄R₅ or a cyclic monoamine optionally comprising an oxygen atom, the cyclic monoamine being optionally substituted with one or more substituents chosen from a fluorine atom and the groups C₁₋₆-alkyl, C₁₋₆-alkyloxy and hydroxyl;
The two groups Rₑ₂ form, with the carbon atom that bears them, a pyrrolidine, piperidine or morpholine group optionally substituted with one or more groups R_{f}, which may be identical to or different than each other;
R_{f} represents a group C₁₋₆-alkyl, C₃₋₇-cycloalkyl, C₃₋₇-cycloalkyl-C₁₋₆-alkyl, C₁₋₆-alkyloxy-C₁₋₆-alkyl, hydroxy-C₁₋₆-alkyl, C₁₋₆-fluoroalkyl or benzyl;
R₄ and R₅ represent, independently of each other, a hydrogen atom or a group C₁₋₄ alkyl, C₃₋₇-cycloalkyl or C₃₋₇-cycloalkyl-C₁₋₆-alkyl;
- R₇ and R₈ represent, independently of each other, a hydrogen atom or a group C₁₋₆-alkyl;
in the form of base or of acid-addition salt.

2. Compound of general formula (I) according to Claim 1, **characterized in that**:
R₃ represents a group chosen from a hydrogen atom and a group C₁₋₃ alkyl, C₁₋₄ alkyloxy or -NR₄R₅; R₄ and R₅ represent a hydrogen atom or a group C₁₋₄-alkyl.

3. Compound of general formula (I) according to Claim 2, **characterized in that**:
R₃ represents a group chosen from a hydrogen atom and a methyl or methoxy group.

4. Compound of general formula (I) according to Claim 2, **characterized in that**:
R₃ represents a group chosen from -NH₂, methyl-amino and dimethylamino.

5. Compound of general formula (I) according to any one of Claims 1 to 4, **characterized in that**:
R₇ and R₈ represent, independently of each other, a hydrogen atom or a methyl group.

6. Compound of general formula (I) according to any one of Claims 1 to 5, **characterized in that**:
- A represents a group C₁₋₇-alkylene;
- B represents a group C₁₋₇-alkylene;
- L represents a carbon substituted with a group Rₑ₁ and a group R_{d};
- R_{d} represents a hydrogen atom;
- Rₑ₁ represents a group NR₄R₅, in which R₄ and R₅ represent, independently of each other, a group C₁₋₄-alkyl; or alternatively Rₑ₁ represents a cyclic monoamine optionally comprising an oxygen atom, the monoamine being optionally substituted with one or more groups chosen from a fluorine atom or the groups hydroxyl and C₁₋₆-alkyl.

7. Compound of general formula (I) according to any one of Claims 1 to 5, **characterized in that**:
- A represents a group C₁₋₇-alkylene optionally substituted with one or two groups R_{a;}
- B represents a group C₁₋₇-alkylene optionally substituted with a group R_{b};
- L represents a nitrogen atom optionally substituted with a group R_{c} or R_{d};
the carbon atoms of A and B being optionally substituted with one or more groups R_{f}, which may be identical to or different than each other;
When they are present, Rₐ, R_{b} and R_{c} are defined in combination as follows:
- either two groups Rₐ together form a group C₁₋₆-alkylene;
- or Rₐ and R_{b} together form a bond or a group C₁₋₆-alkylene;
- or Rₐ and R_{c} together form a bond or a group C₁₋₆-alkylene;
- or R_{b} and R_{c} together form a bond or a group C₁₋₆-alkylene;
- R_{d} represents a substituent chosen from a group C₁₋₆-alkyl, C₃₋₇-cycloalkyl, C₃₋₇-cycloalkyl-C₁₋₆-alkyl, hydroxy-C₁₋₆-alkyl, C₁₋₆-alkyloxy-C₁₋₆-alkyl, C₁₋₆-fluoroalkyl, benzyl or C₁₋₆-acyl;
- R_{f} represents a group C₁₋₆-alkyl.

8. Compound of general formula (I) according to any one of Claims 1 to 5, **characterized in that**:
- A represents a group C₁₋₇-alkylene;
- B represents a group C₁₋₇-alkylene;
- L represents a carbon atom substituted with two groups Rₑ₂;
the carbon atoms of A and B being optionally substituted with one or more groups R_{f}, which may be identical to or different than each other;
- two groups Rₑ₂ form, with the carbon atom that bears them, a pyrrolidine, piperidine, morpholine group;
- R_{f} represents a group C₁₋₆-alkyl.

9. Compound of general formula (I) according to any one of Claims 1 to 5, **characterized in that**:
- R₂ represents a phenyl group optionally substituted with one or more substituents chosen from fluorine and chlorine atoms and the methyl group;
- R₃ represents a hydrogen atom;
the cyclic amine formed by -N-A-L-B- represents a (±)-3-dimethylaminopyrrolidin-1-yl, 4-(pyrrolidin-1-yl)piperid-1-yl, 4-(morpholin-4-yl)piperid-1-yl, 4-(2.6-dimethylmorpholin-4-yl)piperid-1-yl, 4-dimethylaminopiperid-1-yl, 4-(3-hydroxypyrrolidin-1-yl)piperid-1-yl, 4-(-3-fluoropyrrolidin-1-yl)piperid-1-yl;
- R₇ and R₈ represent a hydrogen atom.

10. Compound of general formula (I) according to any one of Claims 1 to 5, **characterized in that**:
- R₂ represents a phenyl group optionally substituted with one or more substituents chosen from fluorine and chlorine atoms and methyl, methoxy and trifluoromethyl groups;
- R₃ represents a hydrogen atom or a methyl, methoxy,-NH₂, methylamino or dimethylamino group;
- the cyclic amine formed by -N-A-L-B- represents a piperazin-1-yl, 3-methylpiperazin-1-yl, 4-methylpiperazin-1-yl, 3,3-dimethylpiperazin1-yl, 3,4-dimethylpiperazin-1-yl, *cis*-*3,5*-dimethylpiperazin-1-yl, 4-(2-hydroxyethyl)piperazin-1-yl, 4-(2-methoxyethyl)piperazin-1-yl, 4-(2-fluoro-ethyl)piperazin-1-yl, 4-(2,2,2-trifluoroethyl)piperazin-1-yl, 4-cyclopropylpiperazin-1-yl, 4-isopropylpiperazin-1-yl, 4-(2-hydroxy-2-methylpropyl)piperazin-1-yl, 4-*n*-butylpiperazin-1-yl, 4-(3-hydroxy-3-methylbutyl)piperazin-1-yl, cyclohexylpiperazin-1-yl, 4-acetylpiperazin-1-yl, 4-isobutyrylpiperazin-1-yl, (±)-hexahydropyrrolo[1,2-a]pyrazin-2-yl, (*S*)-hexahydropyrrolo[1,2-*a*]pyrazin-2-yl, (1*S*,4*S*)-2,5-diazabicyclo[2.2.1]hept-2-yl, (1*S*, 4*S*)-5-benzyl-2,5-diazabicyclo[2.2.1]hept-2-yl, (1S,4S)-5-(2-hydroxyethyl)-2,5-diazabicyclo[2.2.1]hept-2-yl, 5-isopropyl-2,5-diaza[2.2.1]hept-2-yl, 4-methyl[1.4]diazepan-1-yl, (±)-octahydropyrrolo[1,2-*d*][1.4]diazepin-2-yl, 1,4-diazabicyclo[3.3.2]non-4-yl, (±)-3,6-diazabicyclo[3.2.0]hept-3-yl, hexahydropyrrolo[3,4-*b*]pyrrol-5(1*H*)-yl, hexahydropyrrolo[3,4-*c*]pyrrol-2(1*H*)-yl, 5-benzylhexahydropyrrolo[3,4-*c*]pyrrol-2(1*H*)-yl, 5-cyclopropylhexahydropyrrolo[3,4-*c*]pyrrol-2(1*H*)-yl, 5-isopropylhexahydropyrrolo[3,4-*c*]pyrrol-2(1*H*)-yl, octahydro-6*H*-pyrrolo[3,4-*b*]pyrid-6-yl or (±)-(*cis*)-decahydro[2.6]naphthyridin-2-yl, 3-ethylpiperazin-1-yl, 3,3-diethylpiperazin-1-yl, 3-fluoromethylpiperazin-1-yl, 4-(3-hydroxymethyl)piperazin-1-yl, 3-(1-hydroxy-1-methylethyl)piperazin-1-yl, 3-isopropylpiperazin-1-yl, (1R,5*R*)-3,6-diazabicyclo[3.2.0]hept-2-yl, 5-methyl-2,5-diazabicyclo[2.2.1]hept-2-yl, 1-((1*S*,4*S*)-(2,5-diazabicyclo[2.2.1]hept-2-yl)-2-methylpropan-2-ol, 3,6-diazabicyclo[3.1.1]hept-3-yl, 5-methylhexahydro-pyrrolo[3,4-*c*]pyrrol-2(1*H*)-yl, (±)-hexahydropyrrolo[3,4-*b*]pyrrol-5(1*H*)-yl, (+)-hexahydropyrrolo[3,4-*b*]pyrrol-5(1*H*)-yl, (-)-hexahydropyrrolo[3,4-*b*]pyrrol-5(1*H*)-yl, (4a*S*, 7a*S*)-octahydropyrrolo[3,4-*b*]pyrid-6-yl, 6,9-diazaspiro[4.5]dec-9-yl:
- R₇ and R₈ represent, independently of each other, a hydrogen atom or a methyl group.

11. Compound of general formula (I) according to any one of Claims 1 to 5, **characterized in that**:
- R₂ represents a phenyl group optionally substituted with one or more substituents chosen from fluorine and chlorine atoms;
- R₃ represents a hydrogen atom or a methyl, methoxy or
- NH₂ group;
- the cyclic amine formed by -N-A-L-B- represents a (±)-2,7-diazaspiro[4.4]non-2-yl, (±)-2,8-diazaspiro[4.5]dec-2-yl, (±)-2,7-diazaspiro[4.5]dec-2-yl, (±)-2,8-diazaspiro[4.5]dec-8-yl, (±)-2,7-diazaspiro[4.5]dec-7-yl, 3,9-diazaspiro[5.5]undec-3-yl, 2,9-diazaspiro[5.5]undec-9-yl, (±)-2,8-diazaspiro[5.5]undec-2-yl or 1-oxa-4,9-diazaspiro[5.5]undec-9-yl.
- R₇ and R₈ represent a hydrogen atom.

12. Compound of general formula (I) according to Claim 1, **characterized in that** it is chosen from the following compounds:
2-Phenyl-6-piperazin-1-yl-3-pyrid-4-ylimidazo[1,2-*b*]pyridazine;
2-(4-Fluorophenyl)-6-piperazin-1-yl-3-pyrid-4-ylimidazo[1,2-b]pyridazine;
2-(4-Fluorophenyl)-7,8-dimethyl-6-piperazin-1-yl-3-pyrid-4-ylimidazo[1,2-b]pyridazine;
4-[2-(4-Fluorophenyl)-6-piperazin-1-ylimidazo[1,2-*b*]pyridazin-3-yl]pyrid-2-ylamine;
2-(4-Fluorophenyl)-3-(2-methoxypyrid-4-yl)-6-piperazin-1-ylimidazo[1,2-*b*]pyridazine;
2-(4-Chlorophenyl)-6-piperazin-1-yl-3-pyrid-4-ylimidazo[1,2-*b*]pyridazine;
2-(4-Chlorophenyl)-7-methyl-6-piperazin-1-yl-3-pyrid-4-ylimidazo[1,2-*b*]pyridazine;
2-(4-Chlorophenyl)-8-methyl-6-piperazin-1-yl-3-pyrid-4-ylimidazo[1,2-*b*]pyridazine;
2-(3,5-Dimethylphenyl)-6-piperazin-1-yl-3-pyrid-4-ylimidazo[1,2-*b*]pyridazine;
2-(3,5-Difluorophenyl)-6-piperazin-1-yl-3-pyrid-4-ylimidazo[1,2-*b*]pyridazine;
2-(3,5-Difluorophenyl)-3-(2-methylpyrid-4-yl)-6-piperazin-1-ylimidazo[1,2-*b*]pyridazine;
2-(3,4-Difluorophenyl)-6-piperazin-1-yl-3-pyrid-4-ylimidazo[1,2-*b*]pyridazine;
2-(3,5-Dichlorophenyl)-6-piperazin-1-yl-3-pyrid-4-ylimidazo[1,2-*b*]pyridazine;
2-(3,5-Dichlorophenyl)-3-(2-methylpyrid-4-yl)-6-piperazin-1-ylimidazo[1,2-b]pyridazine;
(±)-6-(3-Methylpiperazin-1-yl)-2-phenyl-3-pyrid-4-ylimidazo[1,2-b]pyridazine;
6-((*R*)-3-Methylpiperazin-1-yl)-2-phenyl-3-pyrid-4-ylimidazo[1,2-b]pyridazine;
6-((*R*)-3-Methylpiperazin-1-yl)-3-(2-methylpyrid-4-yl)-2-phenylimidazo[1,2-*b*]pyridazine;
6-((*S*)-3-Methylpiperazin-1-yl)-2-phenyl-3-pyrid-4-ylimidazo[1,2-*b*]pyridazine;
2-(3-Fluorophenyl)-6-((*R*)-3-methylpiperazin-1-yl)-3-pyrid-4-ylimidazo[1,2-*b*]pyridazine;
2-(4-Fluorophenyl)-6-(3-methylpiperazin-1-yl)-3-pyrid-4-ylimidazo[1,2-*b*]pyridazine;
2-(4-Fluorophenyl)-6-((*R*)-3-methylpiperazin-1-yl)-3-pyrid-4-ylimidazo[1,2-*b*]pyridazine;
2-(4-Fluorophenyl)-6-((*S*)-3-methylpiperazin-1-yl)-3-pyrid-4-ylimidazo[1,2-*b*]pyridazine;
2-(4-Fluorophenyl)-3-(2-methoxypyrid-4-yl)-6-((*R*)-3-methylpiperazin-1-yl)imidazo[1,2-*b*]pyridazine;
2-(3,4-Difluorophenyl)-6-((*R*)-3-methylpiperazin-1-yl)-3-pyrid-4-ylimidazo[1,2-*b*]pyridazine;
6-(3,3-Dimethylpiperazin-1-yl)-2-phenyl-3-pyrid-4-ylimidazo[1,2-*b*]pyridazine;
6-(3,3-Dimethylpiperazin-1-yl)-2-(4-fluorophenyl)-3-pyrid-4-ylimidazo[1,2-*b*]pyridazine;
6-(3,3-Dimethylpiperazin-1-yl)-2-(4-fluorophenyl)-3-(2-methoxypyrid-4-yl)imidazo[1,2-*b*]pyridazine;
2-(3,5-Dimethylphenyl)-6-(3,3-dimethylpiperazin-1-yl)-3-pyrid-4-ylimidazo[1,2-*b*]pyridazine;
2-(3,5-Dimethylphenyl)-6-(3,3-dimethylpiperazin-1-yl)-3-(2-methylpyrid-4-yl)imidazo[1,2-*b*]pyridazine;
4-[2-(3,5-Dimethylphenyl)-6-(3,3-dimethylpiperazin-1-yl)imidazo[1,2-*b*]pyridazin-3-yl]pyrid-2-ylamine;
2-(3,5-Difluorophenyl)-6-(3,3-dimethylpiperazin-1-yl)-3-pyrid-4-ylimidazo[1,2-*b*]pyridazine;
2-(3,5-Difluorophenyl)-6-(3,3-dimethylpiperazin-1-yl)-3-(2-methylpyrid-4-yl)imidazo[1,2-*b*]pyridazine;
4-[2-(3,5-Difluorophenyl)-6-(3,3-dimethylpiperazin-1-yl)imidazo[1,2-*b*]pyridazin-3-yl]pyrid-2-ylamine;
2-(3,5-Dichlorophenyl)-6-(3,3-dimethylpiperazin-1-yl)-3-pyrid-4-ylimidazo[1,2-*b*]pyridazine;
2-(3,5-Dichlorophenyl)-6-(3,3-dimethylpiperazin-1-yl)-3-(2-methylpyrid-4-yl)imidazo[1,2-*b*]pyridazine;
(±)-6-(3,4-Dimethylpiperazin-1-yl)-2-(4-fluorophenyl)-3-pyrid-4-ylimidazo[1,2-*b*]pyridazine;
6-((*cis*)-3,5-Dimethylpiperazin-1-yl)-2-phenyl-3-pyrid-4-ylimidazo[1,2-*b*]pyridazine;
6-((*cis*)-3,5-Dimethylpiperazin-1-yl)-3-(2-methylpyrid-4-yl)-2-phenylimidazo[1,2-*b*]pyridazine;
6-((*cis*)-3,5-Dimethylpiperazin-1-yl)-2-(3-fluorophenyl)-3-pyrid-4-ylimidazo[1,2-*b*]pyridazine;
6-((*cis*)3,5-Dimethylpiperazin-1-yl)-2-(4-fluorophenyl)-3-pyrid-4-ylimidazo[1,2-*b*]pyridazine;
6-((*cis*)-3,5-Dimethylpiperazin-1-yl)-2-(4-fluorophenyl)-3-(2-methoxypyrid-4-yl)imidazo[1,2-*b*]pyridazine;
2-(3,5-Dimethylphenyl)-6-((*cis*)-3,5-dimethylpiperazin-1-yl)-3-pyrid-4-ylimidazo[1,2-*b*]pyridazine;
2-(3,4-Difluorophenyl)-6-((*cis*)-3,5-dimethylpiperazin-1-yl)-3-pyrid-4-ylimidazo[1,2-*b*]pyridazine;
2-(4-Chlorophenyl)-6-((*cis*)-3,5-dimethylpiperazin-1-yl)-7-methyl-3-pyrid-4-ylimidazo[1,2-*b*]pyridazine;
6-((*cis*)-3,5-Dimethylpiperazin-1-yl)-2-(4-fluorophenyl)-8-methyl-3-pyrid-4-ylimidazo[1,2-*b*]pyridazine;
2-(2-Chlorophenyl)-6-(4-methylpiperazin-1-yl)-3-pyrid-4-ylimidazo[1,2-*b*]pyridazine;
2-(3-Chlorophenyl)-6-(4-methylpiperazin-1-yl) -3-pyrid-4-ylimidazo[1,2-*b*]pyridazine;
2-(4-Chlorophenyl)-6-(4-methylpiperazin-1-yl)-3-pyrid-4-ylimidazo[1,2-*b*]pyridazine;
2-(3-Fluorophenyl)-6-(4-methylpiperazin-1-yl)-3-pyrid-4-ylimidazo[1,2-*b*]pyridazine;
2-(4-Fluorophenyl)-6-(4-methylpiperazin-1-yl)-3-pyrid-4-ylimidazo[1,2-*b*]pyridazine;
2-(3,4-Difluorophenyl)-6-(4-methylpiperazin-1-yl)-3-pyrid-4-ylimidazo[1,2-*b*]pyridazine;
2-(3-Methoxyphenyl)-6-(4-methylpiperazin-1-yl)-3-pyrid-4-ylimidazo[1,2-*b*]pyridazine;
2-(4-Methoxyphenyl)-6-(4-methylpiperazin-1-yl)-3-pyrid-4-ylimidazo[1,2-*b*]pyridazine;
6-(4-Methylpiperazin-1-yl)-3-pyrid-4-yl-2-p-tolylimidazo[1,2-*b*]pyridazine;
6-(4-Methylpiperazin-1-yl)-3-pyrid-4-yl-2-(4-trifluoromethylphenyl)imidazo[1,2-*b*]pyridazine;
2-(4-Fluorophenyl)-6-(4-methylpiperazin-1-yl)-3-(2-methylpyrid-4-yl)imidazo[1,2-*b*]pyridazine;
4-[2-(4-Fluorophenyl)-6-(4-methylpiperazin-1-yl)imidazo[1,2-*b*]pyridazin-3-yl]pyrid-2-ylamine;
{4-[2-(4-Fluorophenyl)-6-(4-methylpiperazin-1-yl)imidazo[1,2-*b*]pyridazin-3-yl]pyrid-2-yl}-dimethylamine;
2-(4-Fluorophenyl)-3-(2-methoxypyrid-4-yl)-6-(4-methylpiperazin-1-yl)imidazo[1,2-*b*]pyridazine;
6-(4-Ethylpiperazin-1-yl)-2-(4-fluorophenyl)-3-pyrid-4-ylimidazo[1,2-*b*]pyridazine;
2-(3,5-Dimethylphenyl)-6-(4-ethylpiperazin-1-yl)-3-pyrid-4-ylimidazo[1,2-*b*]pyridazine;
2-[4-(2-Phenyl-3-pyrid-4-ylimidazo[1,2-*b*]pyridazin-6-yl)piperazin-1-yl]ethanol;
2-{4-[2-(3-Fluorophenyl)-3-pyrid-4-ylimidazo[1,2-*b*]pyridazin-6-yl]piperazin-1-yl}ethanol;
2-{4-[2-(4-Fluorophenyl)-3-pyrid-4-ylimidazo[1,2-*b*]pyridazin-6-yl]piperazin-1-yl}ethanol;
2-{4-[2-(4-Fluorophenyl)-3-(2-methoxypyrid-4-yl)imidazo[1,2-*b*]pyridazin-6-yl]piperazin-1-yl}ethanol;
2-{4-[2-(3,4-Difluorophenyl)-3-pyrid-4-ylimidazo[1,2-*b*]pyridazin-6-yl]piperazin-1-yl}ethanol;
2-{4-[2-(4-Chlorophenyl)-3-pyrid-4-ylimidazo[1,2-*b*]pyridazin-6-yl]piperazin-1-yl}ethanol;
2-{4-[3-(2-Aminopyrid-4-yl)-2-(4-chlorophenyl)imidazo[1,2-*b*]pyridazin-6-yl]piperazin-1-yl}ethanol;
2-(4-Chlorophenyl)-6-[4-(2-methoxyethyl)piperazin-1-yl]-3-pyrid-4-ylimidazo[1,2-*b*]pyridazine;
6-[4-(2-Fluoroethyl)piperazin-1-yl]-2-(4-fluorophenyl)-3-pyrid-4-ylimidazo[1,2-*b*]pyridazine;
6-[4-(2-Fluoroethyl)piperazin-1-yl]-2-(4-fluorophenyl)-3-(2-methoxypyrid-4-yl)imidazo[1,2-*b*]pyridazine;
2-Phenyl-3-pyrid-4-yl-6-[4-(2,2,2-trifluoroethyl)piperazin-1-yl]imidazo[1,2-*b*]pyridazine;
2-(4-Fluorophenyl)-3-pyrid-4-yl-6-[4-(2,2,2-trifluoroethyl)piperazin-1-yl]imidazo[1,2-*b*]pyridazine;
6-(4-Cyclopropylpiperazin-1-yl)-2-(4-fluorophenyl)-3-pyrid-4-ylimidazo[1,2-*b*]pyridazine;
6-(4-Cyclopropylpiperazin-1-yl)-2-(4-fluorophenyl)-3-(2-methoxypyrid-4-yl)imidazo[1,2-*b*]pyridazine;
6-(4-Isopropylpiperazin-1-yl)-3-(2-methoxypyrid-4-yl)-2-phenylimidazo[1,2-*b*]pyridazine;
2-(3-Fluorophenyl)-6-(4-isopropylpiperazin-1-yl)-3-pyrid-4-ylimidazo[1,2-*b*]pyridazine;
2-(4-Fluorophenyl)-6-(4-isopropylpiperazin-1-yl)-3-pyrid-4-ylimidazo[1,2-*b*]pyridazine;
2-(4-Fluorophenyl)-6-(4-isopropylpiperazin-1-yl)-3-(2-methoxypyrid-4-yl)imidazo[1,2-*b*]pyridazine;
2-(3,4-Difluorophenyl)-6-(4-isopropylpiperazin-1-yl)-3-pyrid-4-ylimidazo[1,2-*b*]pyridazine;
4-[2-(4-Chlorophenyl)-6-(4-isopropylpiperazin-1-yl)imidazo[1,2-*b*]pyridazin-3-yl]pyrid-2-ylamine;
2-Methyl-1-[4-(2-phenyl-3-pyrid-4-ylimidazo[1,2-*b*]pyridazin-6-yl)piperazin-1-yl]propan-2-ol;
1-{4-[2-(3-Fluorophenyl)-3-pyrid-4-ylimidazo[1,2-*b*]pyridazin-6-yl]piperazin-1-yl}-2-methylpropan-2-ol;
1-{4-[2-(4-Fluorophenyl)-3-(2-methoxypyrid-4-yl)imidazo[1,2-*b*]pyridazin-6-yl]piperazin-1-yl}-2-methylpropan-2-ol;
6-(4-Butylpiperazin-1-yl)-2-(4-chlorophenyl)-3-pyrid-4-ylimidazo[1,2-*b*]pyridazine;
4-{4-[2-(4-Fluorophenyl)-3-(2-methoxypyrid-4-yl)imidazo[1,2-*b*]pyridazin-6-yl]piperazin-1-yl}-2-methylbutan-2-ol;
6-(4-Cyclohexylpiperazin-1-yl)-2-(4-fluorophenyl)-3-pyrid-4-ylimidazo[1,2-*b*]pyridazine;
1-{4-[2-(4-Fluorophenyl)-3-pyrid-4-ylimidazo[1,2-*b*]pyridazin-6-yl]piperazin-1-yl}ethanone;
1-{4-[2-(4-Fluorophenyl)-3-pyrid-4-ylimidazo[1,2-*b*]pyridazin-6-yl]piperazin-1-yl}-2-methylpropan-1-one;
(±)-2-(4-Fluorophenyl)-6-(hexahydropyrrolo[1,2-*a*]pyrazin-2-yl)-3-pyrid-4-ylimidazo[1,2-*b*]pyridazine;
2-(4-Fluorophenyl)-6-(*S*)-hexahydropyrrolo[1,2-*a*]pyrazin-2-yl-3-(2-methoxypyrid-4-yl)imidazo[1,2-b]pyridazine;
6-((1*S*,4*S*)-2,5-Diazabicyclo[2.2.1]hept-2-yl)-2-phenyl-3-pyrid-4-ylimidazo[1,2-*b*]pyridazine;
6-((1*S*,4*S*)-5-Benzyl-2,5-diazabicyclo[2.2.1]hept-2-yl)-2-(3-fluorophenyl)-3-pyrid-4-ylimidazo[1,2-*b*]pyridazine;
6-((1*S*,4*S*)-2,5-Diazabicyclo[2.2.1]hept-2-yl)-2-(4-fluorophenyl)-3-pyrid-4-ylimidazo[1,2-*b*]pyridazine;
6-((1*S*,4*S*)-5-Benzyl-2,5-diazabicyclo[2.2.1]hept-2-yl)-2-(4-fluorophenyl)-3-pyrid-4-ylimidazo[1,2-*b*]pyridazine;
6-((1*S*,4*S*)-2,5-Diazabicyclo[2.2.1]hept-2-yl)-2-(4-fluorophenyl)-3-(2-methoxypyrid-4-yl)imidazo[1,2-*b*]pyridazine;
2-{(1*S*,4*S*)-5-[2-(4-Fluorophenyl)-3-(2-methoxypyrid-4-yl)imidazo[1,2-*b*]pyridazin-6-yl]-2,5-diazabicyclo[2.2.1]hept-2-yl}ethanol;
2-(4-Fluorophenyl)-6-(5-isopropyl-2,5-diazabicyclo[2.2.1]hept-2-yl)-3-(2-methoxypyrid-4-yl)imidazo[1,2-*b*]pyridazine;
2-(4-Fluorophenyl)-6-(4-methyl[1,4]diazepan-1-yl)-3-pyrid-4-ylimidazo[1,2-b]pyridazine;
(±)-3-[2-(4-Fluorophenyl)-3-pyrid-4-ylimidazo[1,2-b]pyridazin-6-yl]octahydro(1*H*)pyrrolo[1,2-*d*] [1,4]diazepine;
(±)-4-[2-(4-Fluorophenyl)-3-pyrid-4-ylimidazo[1,2-*b*]pyridazin-6-yl]-1,4-diazabicyclo[3.2.2]nonane;
(±)-4-[2-(3,5-Dimethylphenyl)-3-pyrid-4-ylimidazo[1,2-*b*]pyridazin-6-yl]-1,4-diazabicyclo[3.2.2]nonane;
(±)-3,6-diazabicyclo[3.2.0]hept-3-yl-2-phenyl-3-pyrid-4-ylimidazo[1,2-*b*]pyridazine;
(±)-6-(Hexahydropyrrolo[3,4-*b*]pyrrol-5(1*H*)-yl)-2-phenyl-3-pyrid-4-ylimidazo[1,2-*b*]pyridazine;
(±)-6-(Hexahydropyrrolo[3,4-*b*]pyrrol-5(1*H*)-yl)-3-(2-methylpyrid-4-yl)-2-phenylimidazo[1,2-*b*]pyridazine;
6-Hexahydropyrrolo[3,4-*c*]pyrrol-2(1*H*)-yl-2-phenyl-3-pyrid-4-ylimidazo[1,2-*b*]pyridazine;
6-Hexahydropyrrolo[3,4-*c*]pyrrol-2(1*H*)-yl-3-(2-methylpyrid-4-yl)-2-phenylimidazo[1,2-*b*]pyridazine;
2-(3-Fluorophenyl)-6-hexahydropyrrolo[3,4-*c*]pyrrol-2(1*H*)-yl-3-(2-methylpyrid-4-yl)imidazo[1,2-*b*]pyridazine;
2-(4-Fluorophenyl)-6-hexahydropyrrolo[3,4-*c*]pyrrol-2(1*H*)-yl-3-pyrid-4-ylimidazo[1,2-*b*]pyridazine;
2-(4-Fluorophenyl)-6-hexahydropyrrolo[3,4-*c*]pyrrol-2(1*H*)-yl-3-(2-methylpyrid-4-yl)imidazo[1,2-*b*]pyridazine;
4-[2-(4-Chlorophenyl)-6-(hexahydropyrrolo[3,4-*c*]pyrrol-2(1*H*)-yl)imidazo[1,2-*b*]pyridazin-3-yl]pyrid-2-ylamine;
2-(4-Fluorophenyl)-6-(hexahydropyrrolo[3,4-*c*]pyrrol-2(1*H*)-yl)-3-(2-methoxypyrid-4-yl)imidazo[1,2-*b*]pyridazine;
4-[6-(5-Benzylhexahydropyrrolo[3,4-*c*]pyrrol-2(1H)-yl)-7,8-dimethyl-2-(4-fluorophenyl)imidazo[1,2-b]pyridazin-3-yl]pyrid-2-ylamine;
4-[2-(4-Fluorophenyl)-6-hexahydropyrrolo[3,4-*c*]pyrrol-2(1*H*)-yl-7,8-dimethylimidazo[1,2-*b*]pyridazin-3-yl]pyrid-2-ylamine;
6-(5-Cyclopropylhexahydropyrrolo[3,4-*c*]pyrrol-2(1*H*)-yl)-2-(4-fluorophenyl)-3-pyrid-4-ylimidazo[1,2-*b*]pyridazine;
2-(4-Fluorophenyl)-6-(5-isopropylhexahydropyrrolo[3,4-*c*]pyrrol-2(1*H*)-yl)-3-pyrid-4-ylimidazo[1,2-*b*]pyridazine;
2-(4-Fluorophenyl)-6-(5-isopropylhexahydropyrrolo[3,4-*c*]pyrrol-2-(1*H*)-yl)-3-(2-methylpyrid-4-yl)imidazo[1,2-*b*]pyridazine;
2-(4-Fluorophenyl)-6-(5-isopropylhexahydropyrrolo[3,4-*c*]pyrrol-2(1*H*)-yl)-3-(2-methoxypyrid-4-yl)imidazo[1,2-*b*]pyridazine
(±)-6-(Octahydro-6*H*-pyrrolo[3,4-*b*]pyrid-6-yl)-2-phenyl-3-pyrid-4-ylimidazo[1,2-*b*]pyridazine;
(±)-3-(2-Methylpyrid-4-yl)-6-(octahydro-6*H*-pyrrolo[3,4-*b*]pyrid-6-yl)-2-phenylimidazo[1,2-*b*]pyridazine;
(±)-(*cis*)-2-[2-(4-Fluorophenyl)-3-pyrid-4-ylimidazo[1,2-*b*]pyridazin-6-yl]decahydro[2,6]-naphthyridine;
(±)-6-(2,7-Diazaspiro[4.4]non-2-yl)-2-(3-fluorophenyl)-3-pyrid-4-ylimidazo[1,2-*b*]pyridazine;
(±)-6-(2,7-Diazaspiro[4.4]non-2-yl)-2-(4-fluorophenyl)-3-pyrid-4-ylimidazo[1,2-*b*]pyridazine;
(±)-6-(2,8-Diazaspiro[4.5]dec-2-yl)-2-(4-fluorophenyl)-3-pyrid-4-ylimidazo[1,2-*b*]pyridazine;
(±)-6-(2,7-Diazaspiro[4.5]dec-2-yl)-2-(4-fluorophenyl)-3-pyrid-4-ylimidazo[1,2-*b*]pyridazine;
(±)-6-(2,8-Diazaspiro[4.5]dec-8-yl)-2-(4-fluorophenyl)-3-pyrid-4-ylimidazo[1,2-*b*]pyridazine;
(±)-6-(2,7-Diazaspiro[4.5]dec-7-yl)-2-(4-fluorophenyl)-3-pyrid-4-ylimidazo[1,2-*b*]pyridazine;
3-[2-(4-Fluorophenyl)-3-pyrid-4-ylimidazo[1,2-*b*]pyridazin-6-yl]-3,9-diazaspiro[5.5]undecane;
9-(2-Phenyl-3-pyrid-4-ylimidazo[1,2-*b*]pyridazin-6-yl)-2,9-diazaspiro[5.5] undecane;
9-[3-(2-Methylpyrid-4-yl)-2-phenylimidazo[1,2-*b*]pyridazin-6-yl]-2,9-diazaspiro[5.5]undecane;
9-[2-(3-Fluorophenyl)-3-(2-methylpyrid-4-yl)imidazo[1,2-*b*]pyridazin-6-yl]-2,9-diazaspiro[5.5]-undecane;
9-[2-(4-Fluorophenyl)-3-pyrid-4-ylimidazo[1,2-*b*]pyridazin-6-yl]-2,9-diazaspiro[5.5]undecane;
9-[2-(4-Fluorophenyl)-3-(2-methylpyrid-4-yl)imidazo[1,2-*b*]pyridazin-6-yl]-2,9-diazaspiro[5.5]-undecane;
4-[2-(4-Chlorophenyl)-6-(2,9-diazaspiro[5.5]undec-9-yl)imidazo[1,2-*b*]pyridazin-3-yl]pyrid-2-ylamine;
9-[2-(4-Fluorophenyl)-3-(2-methoxypyrid-4-yl)imidazo[1,2-b]pyridazin-6-yl]-2,9-diazaspiro[5.5]-undecane;
2-[2-(4-Fluorophenyl)-3-(pyrid-4-yl)imidazo[1,2-*b*]pyridazin-6-yl]-2,9-diazaspiro[5.5] undecane
2-[2-(4-Fluorophenyl)-3-(pyrid-4-yl)imidazo[1,2-*b*]pyridazin-6-yl]-2,8-diazaspiro[5.5] undecane
9-[2-(phenyl)-3-(pyrid-4-yl)imidazo[1,2-*b*]pyridazin-6-yl]-1-oxa-4,9-diazaspiro[5.5] undecane;
9-[2-(4-Fluorophenyl)-3-(pyrid-4-yl) imidazo[1,2-*b*]pyridazin-6-yl]-1-oxa-4,9-diazaspiro[5.5]undecane;
9-[2-(4-Fluorophenyl)-3-(2-methoxypyrid-4-yl)imidazo[1,2-*b*]pyridazin-6-yl]-1-oxa-4,9-diazaspiro[5.5]undecane;
4-{[2-(4-Chlorophenyl)-3-(2-methoxypyrid-4-yl)imidazo[1,2-*b*]pyridazin-6-yl]-1-oxa-4,9-diazaspiro[5.5]undec-9-yl}-2-ylamine;
(±)-{1-[2-(4-Fluorophenyl)-3-pyrid-4-ylimidazo[1,2-*b*]pyridazin-6-yl]pyrrolidin-3-yl}dimethylamine;
2-(3,5-Dimethylphenyl)-3-pyrid-4-yl-6-(4-pyrrolidin-1-ylpiperid-1-yl)imidazo[1,2-*b*]pyridazine;
6-(4-Morpholin-4-ylpiperid-1-yl)-2-phenyl-3-pyrid-4-ylimidazo[1,2-b]pyridazine;
6-[4-(2,6-Dimethylmorpholin-4-yl)piperid-1-yl]-2-phenyl-3-pyrid-4-ylimidazo[1,2-*b*]pyridazine;
{1-[2-(4-Fluorophenyl)-3-pyrid-4-ylimidazo[1,2-*b*]pyridazin-6-yl]piperid-4-yl}dimethylamine;
2-Phenyl-3-pyrid-4-yl-6-(4-pyrrolidin-1-ylpiperid-1-yl)imidazo[1,2-b]pyridazine;
2-(4-Fluorophenyl)-3-pyrid-4-yl-6-(4-pyrrolidin-1-ylpiperid-1-yl)imidazo[1,2-*b*]pyridazine;
2-(4-Fluorophenyl)-3-(2-methoxypyrid-4-yl)-6-(4-pyrrolidin-1-ylpiperid-1-yl)imidazo[1,2-*b*]pyridazine;
(*R*)-1-{1-[2-(4-Fluorophenyl)-3-pyrid-4-ylimidazo[1,2-*b*]pyridazin-6-yl]piperid-4-yl}pyrrolidin-3-ol;
6-(4-Morpholin-4-ylpiperid-1-yl)-2-phenyl-3-pyrid-4-ylimidazo[1,2-b]pyridazine;
2-(3-Fluoro-5-methylphenyl)-6-((*R*)-3-methylpiperazin-1-yl)-3-pyrid-4-ylimidazo[1,2-b]pyridazine;
{4-[2-(4-Fluorophenyl)-6-((*R*)-3-methylpiperazin-1-yl)imidazo[1,2-*b*]pyridazin-3-yl]pyrid-2-yl}methylamine;
6-(3,3-Dimethylpiperazin-1-yl)-2-(4-fluorophenyl)-7-methyl-3-pyrid-4-ylimidazo[1,2-*b*]pyridazine;
6-(3,3-Dimethylpiperazin-1-yl)-2-(4-fluorophenyl)-8-methyl-3-pyrid-4-ylimidazo[1,2-*b*]pyridazine;
6-(3,3-Dimethylpiperazin-1-yl)-2-(3-fluoro-5-methylphenyl)-3-pyrid-4-ylimidazo[1,2-b]pyridazine;
{4-[6-(3,3-Dimethylpiperazin-1-yl)-2-(4-fluorophenyl)imidazo[1,2-*b*]pyridazin-3-yl]pyrid-2-yl}methylamine;
6-((*R*)-3-Ethylpiperazin-1-yl)-2-(4-fluorophenyl)-3-pyrid-4-ylimidazo[1,2-*b*]pyridazine;
6-(3,3-Diethylpiperazin-1-yl)-2-(4-fluorophenyl)-3-pyrid-4-ylimidazo[1,2-*b*]pyridazine;
(±)-6-(3-Fluoromethylpiperazin-1-yl)-2-(4-fluorophenyl)-3-pyrid-4-ylimidazo[1,2-*b*]pyridazine;
(±)-{4-[2-(4-Fluorophenyl)-3-pyrid-4-ylimidazo[1,2-*b*]pyridazin-6-yl]piperazin-2-yl}methanol;
2-{4-[2-(3-Fluoro-5-methylphenyl)-3-pyrid-4-ylimidazo[1,2-*b*]pyridazin-6-yl]piperazin-1-yl}ethanol;
2-{4-[2-(4-Fluorophenyl)-3-(2-methylaminopyrid-4-yl)imidazo[1,2-*b*]pyridazin-6-yl]piperazin-1-yl}ethanol;
1-{4-[2-(4-Fluorophenyl)-3-pyrid-4-ylimidazo[1,2-*b*]pyridazin-6-yl]piperazin-1-yl}-2-methylpropan-2-ol;
1-{4-[2-(4-Fluorophenyl)-8-methyl-3-pyrid-4-ylimidazo[1,2-*b*]pyridazin-6-yl]piperazin-1-yl}-2-methylpropan-2-ol;
1-{4-[2-(4-Fluorophenyl)-7-methyl-3-pyrid-4-ylimidazo[1,2-*b*]pyridazin-6-yl]piperazin-1-yl}-2-methylpropan-2-ol;
1-{4-[2-(4-Fluorophenyl)-3-(2-methylaminopyrid-4-yl)imidazo[1,2-*b*]pyridazin-6-yl]piperazin-1-yl}-2-methylpropan-2-ol;
2-{(*R*)-4-[2-(4-Fluorophenyl)-3-pyrid-4-ylimidazo[1,2-*b*]pyridazin-6-yl]piperazin-2-yl}propan-2-ol;
2-(4-Fluorophenyl)-6-((*R*)-3-isopropylpiperazin-1-yl)-3-pyrid-4-ylimidazo[1,2-*b*]pyridazine;
{4-[2-(4-Fluorophenyl)-6-((*R*)-3-isopropylpiperazin-1-yl)imidazo[1,2-*b*]pyridazin-3-yl]pyrid-2-yl}methylamine;
2-(3-Fluorophenyl)-6-(4-isopropylpiperazin-1-yl)-3-(2-methoxypyrid-4-yl)imidazo[1,2-*b*]pyridazine;
2-(4-Fluorophenyl)-6-(4-isopropylpiperazin-l-yl)-7-methyl-3-pyrid-4-ylimidazo[1,2-*b*]pyridazine;
2-(4-Fluorophenyl)-6-(4-isopropylpiperazin-1-yl)-8-methyl-3-pyrid-4-ylimidazo[1,2-*b*]pyridazine;
{4-[2-(4-Fluorophenyl)-6-(4-isopropylpiperazin-1-yl)imidazo[1,2-*b*]pyridazin-3-yl]pyrid-2-yl}methylamine;
2-(3,4-Difluorophenyl)-6-(4-isopropylpiperazin-1-yl)-3-(2-methoxypyrid-4-yl)imidazo[1,2-*b*]pyridazine;
2-(3-Fluoro-5-methylphenyl)-6-(4-isopropylpiperazin-1-yl)-3-pyrid-4-ylimidazo[1,2-*b*]pyridazine;
(±)-6-(3,6-Diazabicyclo[3.2.0]hept-3-yl)-2-phenyl-3-pyrid-4-ylimidazo[1,2-*b*]pyridazine;
6-(1*S*,4*S*)-2,5-Diazabicyclo[2.2.1]hept-2-yl-2-phenyl-3-pyrid-4-ylimidazo[1,2-*b*]pyridazine;
{4-[(1*S*,4*S*)-6-2,5-Diazabicyclo[2.2.1]hept-2-yl-2-(4-fluorophenyl)imidazo[1,2-*b*]pyridazin-3-yl]pyrid-2-yl}methylamine;
2-(4-Fluorophenyl)-6-((1*S*,4*S*)-5-methyl-2,5-diazabicyclo[2.2.1]hept-2-yl)-3-pyrid-4-ylimidazo[1,2-*b*]pyridazine;
2-(4-Fluorophenyl)-7-methyl-6-((1*S*,4*S*)-5-methyl-2,5-diazabicyclo[2.2.1]hept-2-yl)-3-pyrid-4-ylimidazo[1,2-*b*]pyridazine;
2-(4-Fluorophenyl)-8-methyl-6-((1*S*,4*S*)-5-methyl-2,5-diazabicyclo[2.2.1]hept-2-yl)-3-pyrid-4-ylimidazo[1,2-*b*]pyridazine;
2-(4-Fluorophenyl)-6-((1*S*,4*S*)-5-methyl-2,5-diazabicyclo[2.2.1]hept-2-yl)-3-(2-methylpyrid-4-yl)imidazo[1,2-*b*]pyridazine;
2-Methyl-1-[(1*S*,4*S*)-5-(2-phenyl-3-pyrid-4-ylimidazo[1,2-*b*]pyridazin-6-yl)-2,5-diazabicyclo[2.2.1]hept-2-yl]propan-2-ol;
6-(3,6-Diazabicyclo[3.1.1]hept-3-yl)-2-(4-fluorophenyl)-3-pyrid-4-ylimidazo[1,2-*b*]pyridazine;
2-(4-Fluorophenyl)-6-(5-methylhexahydropyrrolo[3,4-*c*]pyrrol-2(1H)-yl)-3-pyrid-4-ylimidazo[1,2-*b*]pyridazine;
2-(4-Fluorophenyl)-7-methyl-6-(5-methylhexahydropyrrolo[3,4-*c*]pyrrol-2(1*H*)-yl)-3-pyrid-4-ylimidazo[1,2-*b*]pyridazine;
2-(4-Fluorophenyl)-8-methyl-6-(5-methylhexahydropyrrolo[3,4-c]pyrrol-2(1*H*)-yl)-3-pyrid-4-ylimidazo[1,2-*b*]pyridazine;
2-(4-Fluorophenyl)-8-methyl-6-(-5-methylhexahydropyrrolo[3,4-*c*]pyrrol-2(1*H*)-yl)-3-(2-methylpyrid-4-yl)imidazo[1,2-*b*]pyridazine;
{4-[2-(4-Fluorophenyl)-6-(5-methylhexahydropyrrolo[3,4-c]pyrrol-2(1*H*)-yl)imidazo[1,2-*b*]pyridazin-3-yl]pyrid-2-yl}methylamine;
2-(4-Fluorophenyl)-3-(2-methoxypyrid-4-yl)-6-(5-methylhexahydropyrrolo[3,4-*c*]pyrrol-2(1*H*)-yl)imidazo[1,2-*b*]pyridazine;
2-(4-Fluorophenyl)-6-(5-methylhexahydropyrrolo[3,4-*c*]pyrrol-2(1H)-yl)-3-(2-methylpyrid-4-yl)imidazo[1,2-*b*]pyridazine;
{4-[2-(4-Fluorophenyl)-8-methyl-6-((3a*R*,6a*S*)-5-methylhexahydropyrrolo[3,4-*c*]pyrrol-2(1*H*)-yl)imidazo[1,2-*b*]pyridazin-3-yl]pyrid-2-yl}methylamine;
{4-[2-(4-Fluorophenyl)-7-methyl-6-(5-methylhexahydropyrrolo[3,4-*c*]pyrrol-2(1*H*)-yl)-imidazo[1,2-*b*]pyridazin-3-yl]pyrid-2-yl}methylamine;
(±)-2-(4-Fluorophenyl)-6-(hexahydropyrrolo[3,4-*b*]pyrrol-5(1H)-yl)-3-pyrid-4-ylimidazo[1,2-*b*]pyridazine;
(+)-2-(4-Fluorophenyl)-6-hexahydropyrrolo[3,4-*b*]pyrrol-5(1*H*)-yl-3-pyrid-4-ylimidazo[1,2-*b*]pyridazine;
(-)-2-(4-Fluorophenyl)-6-hexahydropyrrolo[3,4-*b*]pyrrol-5(1*H*)-yl-3-pyrid-4-ylimidazo[1,2-*b*]pyridazine;
(±)-{4-[2-(4-Fluorophenyl)-6-(hexahydropyrrolo[3,4-*b*]pyrrol-5(1*H*)-yl)imidazo[1,2-*b*]pyridazin-3-yl]pyrid-2-yl}methylamine;
6-(4a*S*,7a*S*)-octahydropyrrolo[3,4-*b*]pyrid-6-yl-2-phenyl-3-pyrid-4-ylimidazo[1,2-*b*]pyridazine;
3-(2-Methylpyrid-4-yl)-6-(4a*S*,7a*S*)-octahydropyrrolo[3,4-*b*]pyrid-6-yl-2-phenylimidazo[1,2-*b*]pyridazine;
3-(2-Methylpyrid-4-yl)-6-(4a*R*, 7a*R*)-octahydropyrrolo[3,4-*b*]pyrid-6-yl-2-phenylimidazo[1,2-*b*]pyridazine;
2-(4-Fluorophenyl)-8-methyl-6-(4a*S*,7a*S*)-octahydropyrrolo[3,4-*b*]pyrid-6-yl-3-pyrid-4-ylimidazo[1,2-*b*]pyridazine;
2-(4-Fluorophenyl)-7-methyl-6-(4a*S*,7a*S*)-octahydropyrrolo[3,4-*b*]pyrid-6-yl-3-pyrid-4-ylimidazo[1,2-*b*]pyridazine;
2-(4-Fluorophenyl)-7-methyl-6-(4a*R*,7a*R*)-octahydropyrrolo[3,4-*b*]pyrid-6-yl-3-pyrid-4-ylimidazo[1,2-*b*]pyridazine;
2-(4-Fluorophenyl)-6-(4a*S*,7a*S*)-octahydropyrrolo[3,4-*b*]pyrid-6-yl-3-pyrid-4-ylimidazo[1,2-*b*]pyridazine;
2-(4-Fluorophenyl)-3-(2-methylpyrid-4-yl)-6-(4a*S*,7a*S*)-octahydropyrrolo[3,4-*b*]pyrid-6-ylimidazo[1,2-*b*]pyridazine;
{4-[2-(4-Fluorophenyl)-6-(octahydropyrrolo[3,4-*b*]pyrid-6-yl)imidazo[1,2-*b*]pyridazin-3-yl]pyrid-2-yl}methylamine;
6-(4a*R*,7a*R*)-Octahydropyrrolo[3,4-*b*]pyrid-6-yl-2-phenyl-3-pyrid-4-ylimidazo[1,2-*b*]pyridazine;
2-(4-Fluorophenyl)-6-(4a*R*,7a*R*)-octahydropyrrolo[3,4-*b*]pyrid-6-yl-3-pyrid-4-ylimidazo[1,2-*b*]pyridazine;
2-(4-Fluorophenyl)-3-(2-methylpyrid-4-yl)-6-(4a*R*,7a*R*)-octahydropyrrolo[3,4-*b*]pyrid-6-ylimidazo[1,2-*b*]pyridazine;
2-(4-Fluorophenyl)-8-methyl-6-(4a*R*,7a*R*)-octahydropyrrolo[3,4-*b*]pyrid-6-yl-3-pyrid-4-ylimidazo[1,2-*b*]pyridazine;
6-(2,7-Diazaspiro[4.4]non-2-yl)-2-(4-fluorophenyl)-8-methyl-3-pyrid-4-ylimidazo[1,2-*b*]pyridazine;
6-(6,9-Diazaspiro[4.5]dec-9-yl)-2-(4-fluorophenyl)-3-pyrid-4-ylimidazo[1,2-b]pyridazine;
(±)-2-[2-(4-Fluorophenyl)-8-methyl-3-pyrid-4-ylimidazo[1,2-b]pyridazin-6-yl]-2,8-diazaspiro[5.5]undecane;
9-[2-(4-Fluorophenyl)-8-methyl-3-pyrid-4-ylimidazo[1,2-*b*]pyridazin-6-yl]-2,9-diazaspiro[5.5] undecane;
{4-[6-(2,9-Diazaspiro[5.5]undec-9-yl)-2-(4-fluorophenyl)imidazo[1,2-*b*]pyridazin-3-yl]pyrid-2-yl}methylamine;
9-[2-(3,4-Difluorophenyl)-3-pyrid-4-ylimidazo[1,2-*b*]pyridazin-6-yl]-2,9-diazaspiro[5.5] undecane;
9-[2-(3-Fluoro-5-methylphenyl)-3-pyrid-4-ylimidazo[1,2-*b*]pyridazin-6-yl]-2,9-diazaspiro[5.5] undecane;
2-(3-Fluorophenyl)-3-pyrid-4-yl-6-(4-pyrrolidin-1-ylpiperid-1-yl)imidazo[1,2-*b*]pyridazine;
{4-[2-(4-Fluorophenyl)-6-(4-pyrrolidin-1-ylpiperid-1-yl)imidazo[1,2-*b*]pyridazin-3-yl]pyrid-2-yl}methylamine;
2-(4-Fluorophenyl)-6-[4-((*R*)-3-fluoropyrrolidin-1-yl)piperid-1-yl]-3-pyrid-4-ylimidazo[1,2-*b*]pyridazine;
(*R*)-1-[1-(2-Phenyl-3-pyrid-4-ylimidazo[1,2-*b*]pyridazin-6-yl)piperid-4-yl]pyrrolidin-3-ol;
(*R*)-1-{1-[2-(3-Fluoro-5-methylphenyl)-3-pyrid-4-ylimidazo[1,2-*b*]pyridazin-6-yl]piperid-4-yl}-pyrrolidin-3-ol.

13. Process for preparing a compound of formula (I) according to Claim 1, **characterized in that** a compound of general formula (IIa) 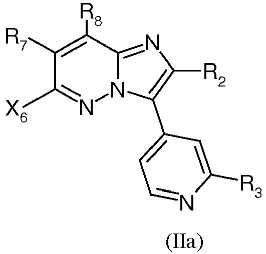 in which in which R₂, R₃, R₇ and R₈ are as defined according to Claim 1 and X₆ represents a leaving group, is reacted with an amine of general formula (IIIa) 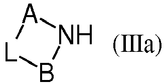 in which A, L and B are as defined according to Claim 1, in a polar solvent.

14. Process for preparing a compound of formula (I) according to Claim 1, **characterized in that** a compound of general formula (Vb) 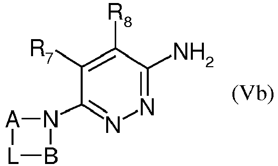 in which A, L, B, R₇ and R₈ are as defined according to Claim 1, is reacted with a compound of general formula (VIb) 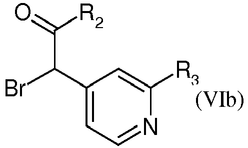 in which R₂ and R₃ are as defined according to Claim 1, in a polar solvent.

15. Process for preparing a compound of formula (I) according to Claim 1, **characterized in that** a compound of general formula (IId) 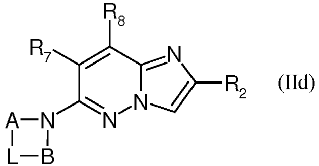 in which R₂, A, L, B, R₇ and R₈ are as defined according to Claim 1, is reacted with a compound of general formula (IVd) 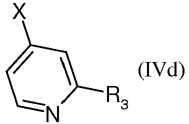 in which R₃ is as defined according to Claim 1 and X represents an iodine atom, in the presence of a catalyst and a mineral base, and in a polar aprotic solvent.

16. Process for preparing a compound of formula (I) according to Claim 1, **characterized in that** a compound of general formula (IIc) 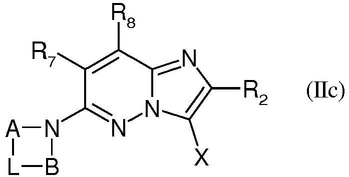 in which R₂, A, L, B, R₇ and R₈ are as defined according to Claim 1 and X represents an iodine or bromine atom, is reacted with a compound of general formula (IVc) 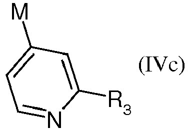 in which R₃ is as defined according to Claim 1 and M represents a trialkylstannyl group or a dihydroxyboryl or dialkyloxyboryl group.

17. Process for preparing a compound of formula (I) according to Claim 1, in which R₃ represents a hydrogen atom or a group C₁₋₃-alkyl, **characterized in that**:
a) a compound of general formula (IId) 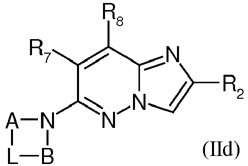 in which R₂, A, L, B, R₇ and R₈ are as defined according to Claim 1, is reacted with a mixture of compounds of formula (IVe) 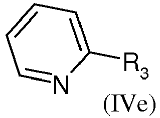 in which R₃ represents a hydrogen atom or a group C₁₋₃-alkyl, in the presence of an alkyl chloroformate, to obtain a compound of formula (IIe) 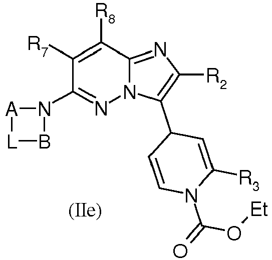 in which R₂, A, L, B, R₇ and R₈ are as defined according to Claim 1 and in which R₃ represents a hydrogen atom or a group C₁₋₃-alkyl;
b) the product of formula (IIe) obtained in step b) is reacted with an oxidizing agent to obtain a compound of general formula (I) according to Claim 1 and in which R₃ represents a hydrogen atom or a group C₁₋₃-alkyl.

18. Medicament, **characterized in that** it comprises a compound of formula (I) according to any one of Claims 1 to 12, in the form of a base or of an addition salt with a pharmaceutically acceptable acid.

19. Pharmaceutical composition, **characterized in that** it comprises a compound of formula (I) according to any one of Claims 1 to 12, in the form of a base or of an addition salt with a pharmaceutically acceptable acid, and also at least one pharmaceutically acceptable excipient.

20. Compound of general formula (I) according to any one of Claims 1 to 12, for use in treating or preventing sleep disorders or circadian rhythm disorders.

21. Compound of general formula (I) according to any one of Claims 1 to 12, for use in treating or preventing diseases associated with dependency on abuse substances.
